(19) Europäisches Patentamt
European Patent Office
Office européen des brevets

(11) Publication number: **0 073 562 B1**

(12) **EUROPEAN PATENT SPECIFICATION**

(45) Date of publication of patent specification: **09.11.88**

(21) Application number: **82303611.6**

(22) Date of filing: **09.07.82**

(51) Int. Cl.[4]: **C 07 D 249/14,**
C 07 D 233/88,
C 07 D 401/12,
C 07 D 405/12,
C 07 D 409/12, A 01 N 43/50,
A 01 N 43/64

(54) **Herbicidal triazole ureas.**

(30) Priority: **10.07.81 US 282174**
**28.05.82 US 382711**
**10.07.81 US 282173**
**01.06.82 US 382877**

(43) Date of publication of application:
**09.03.83 Bulletin 83/10**

(45) Publication of the grant of the patent:
**09.11.88 Bulletin 88/45**

(84) Designated Contracting States:
**CH DE FR GB IT LI NL SE**

(56) References cited:
**EP-A-0 015 683**
**EP-A-0 029 663**
**EP-A-0 030 142**
**US-A-4 127 405**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Selby, Thomas Paul**
**1 Fox Run Drive, R.D. 1, Quail Hollow**
**Hockessin, DE 19707 (US)**
Inventor: **Wolf, Anthony David**
**151 Kirkcaldy Drive**
**Elkton, MD 21921 (US)**
Inventor: **Fitzgerald, David Joseph**
**Box 91B, R.D. 2**
**West Grove, PA 19390 (US)**

(74) Representative: **Hildyard, Edward Martin et al**
**Frank B. Dehn & Co. Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 073 562 B1

**Description**

This invention relates to triazole ureas and in particular their use as agricultural chemicals and particularly as herbicides.

U.S. Patent 4,127,405 teaches compounds which are useful for controlling weeds in wheat having the formula:

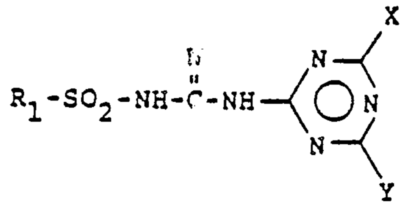

(I)

wherein

$R_1$ is

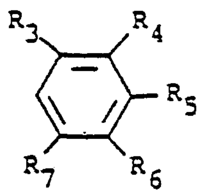

,

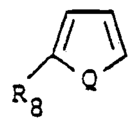

,

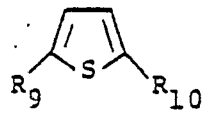

or

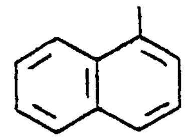

;

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atoms;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1—3 carbon atoms, trifluoromethyl, $CH_3S$— or $CH_3OCH_2$—; and

Y is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

French Patent No. 1,468,747 discloses the following *para*-substituted phenylsulfonamides, useful as antidiabetic agents:

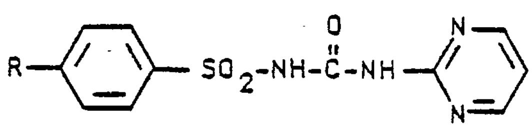

wherein

R = H, halogen, $CF_3$ or alkyl.

Logemann et al., Chem Ab., *53,* 18052g (1959), disclose a number of sulfonamides, including uracil derivatives and those having the formula:

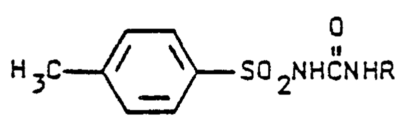

wherein

R is butyl, phenyl or

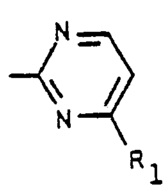

;

and

$R_1$ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon. Pharm. *19,* p. 121—5 (1962) [Chem. Ab., *59* 1633 e] describes the synthesis of N-[(2,6-dimethoxypyrimidin-4-yl)aminocarbonyl]-4-methylbenzenesulfonamide:

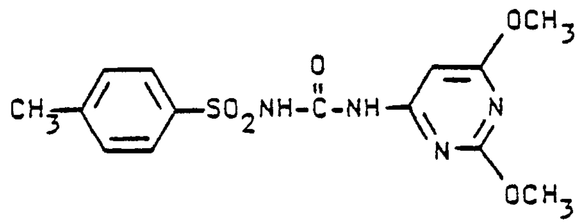

Based upon similarity to a known compound, the author predicted hypoglycemic activity for the foregoing compound.

In EP—A—30,142 and EP—A—15,683 we have disclosed arylsulfonylureas wherein the non-arylated urea nitrogen atom is substituted by a six-membered nitrogen-containing heterocycle, which optionally is fused to a five-membered ring.

Netherlands Patent 121,788, published September 15, 1966, teaches the preparation of compounds of Formula (i), and their use as general or selective herbicides,

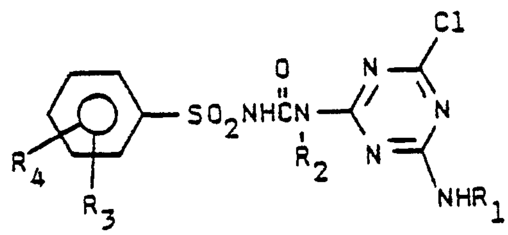

(i)

wherein

$R_1$ and $R_2$ may independently be alkyl of 1—4 carbon atoms; and

$R_3$ and $R_4$ may independently be hydrogen, chlorine or alkyl of 1—4 carbon atoms.

Compounds of Formula (ii), and their use as antidiabetic agents, are reported in *J. Drug. Res. 6,* 123 (1974),

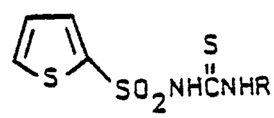

(ii)

wherein

R is pyridyl.

The presence of undesired vegetation causes substantial damage to useful crops, especially agricultural products that satisfy man's basic food needs, such as soybeans, barley, wheat, and the like. The current population explosion and concomitant world food shortage demand improvements in the efficiency of producing these crops. Prevention or minimizing the loss of a portion of valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. The need exists, however, for still more effective herbicides that destroy or retard weeds without causing significant damage to useful crops.

SUMMARY OF THE INVENTION

This invention relates to novel compounds of Formula I, suitable agricultural compositions containing them, and their method of use as pre-emergence and post-emergence herbicides.

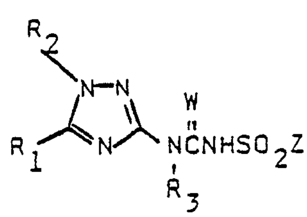

I

wherein

$R_1$ is H, $C_1$—$C_4$ alkyl, $C_3$—$C_4$ alkenyl, $C_3$—$C_4$ alkynyl, $SR_4$, $OR_5$, $CH_2OR_6$, $CH_2CH_2OR_6$, $N(CH_3)_2$, $CF_3$, $CF_2CF_3$, Cl, $NHCH_3$, $N(CH_3)_2$, $CH(OCH_3)_2$ or

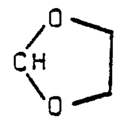 ;

$R_2$ is $C_1$—$C_4$ alkyl, $C_3$—$C_4$ alkenyl, $C_3$—$C_4$ alkynyl, $CH_2OCH_3$, $CH_2CH_2OCH_3$, $CH_2SCH_3$, $CH_2CH_2SCH_3$ or $C_1$—$C_4$ alkyl substituted with 1—3 F atoms;

$R_3$ is H or $CH_3$;

$R_4$ is $C_1$—$C_4$ alkyl, $C_3$—$C_4$ alkenyl, $C_3$—$C_4$ alkynyl, $CH_2CO_2R_6$ or $CH(CH_3)CO_2R_6$;

$R_5$ is $C_1$—$C_4$ alkyl, $C_3$—$C_4$ alkenyl, $C_3$—$C_4$ alkynyl, $CH_2CO_2R_6$, $CH(CH_3)CO_2R_6$ or $CH_2CF_3$;

$R_6$ is $C_1$—$C_4$ alkyl;

W is O or S;

Z is

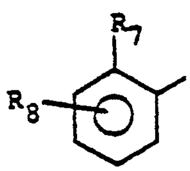 , 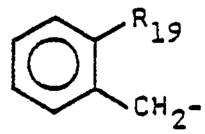 , 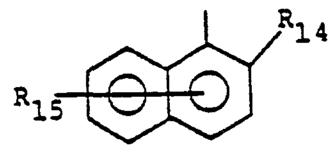

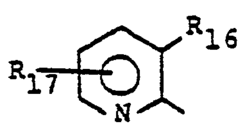 , 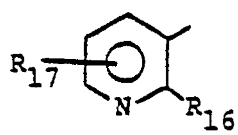 , 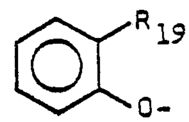 ,

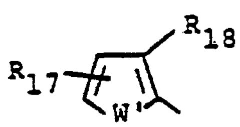 , 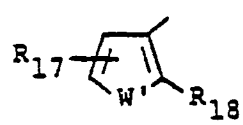 or 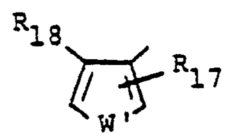

$R_7$ is H, $C_1$—$C_4$ alkyl, $C_1$—$C_4$ alkoxy, F, $C(O)NR_{21}R_{22}$, Cl, Br, $NO_2$, $CF_3$, $CO_2R_9$, $SO_2NR_{10}R_{11}$, $C(O)SR_{10}$, $SO_2N(OCH_3)CH_3$, $QSO_2R_{12}$, $S(O)_nR_{13}$, $CH_2CO_2R_{20}$, $CH(CH_3)CO_2R_{20}$, $CH_2S(O)_nR_{13}$, $CH(CH_3)S(O)_nR_{13}$, $C_3$—$C_4$ alkenyloxy, $C_3$—$C_4$ alkynyloxy, $C_1$—$C_2$ alkyl substituted with either $OCH_3$ or $OC_2H_5$, or $C_1$—$C_3$ alkoxy substituted with either a) 1—5 atoms of Cl, Br or F or b) $OCH_3$ or $OC_2H_5$;

$R_8$ is H, F, Cl, Br, $CF_3$, $NO_2$, $C_1$—$C_3$ alkyl or $C_1$—$C_3$ alkoxy;

$R_9$ is $C_1$—$C_6$ alkyl, $CH_2CH_2OCH_3$, $CH_2CH_2OCH_2CH_3$, $CH_2CH_2CH_2OCH_2CH_3$, $C_3$—$C_6$ alkenyl, $C_3$—$C_6$ alkynyl or $C_1$—$C_3$ alkyl substituted with 1—3 atoms of Cl or F;

$R_{10}$ and $R_{11}$ are independently $C_1$—$C_3$ alkyl;

$R_{12}$ is $C_1$—$C_4$ alkyl, $CH_2CH_2OCH_3$, $CH_2CH_2CH_2OCH_3$ or $C_1$—$C_4$ alkyl substituted with 1—3 atoms of F, Cl or Br;

$R_{13}$ is $C_1$—$C_4$ alkyl, allyl, $C_1$—$C_3$ alkyl substituted with 1—5 atoms of F, Cl or Br;

n is 0, 1 or 2;

Q is O or $NCH_3$;

$R_{14}$ is H, $CH_3$, $OCH_3$, F, Cl, Br, $NO_2$, $SO_2NR_{10}R_{11}$, $SO_2N(OCH_3)CH_3$, $OSO_2R_{12}$ or $S(O)_nR_{13}$;

$R_{15}$ is H, Cl, Br, $CH_3$, $OCH_3$ or $NO_2$;

$R_{16}$ is H, $C_1$—$C_4$ alkyl, $C_1$—$C_4$ alkoxy, F, Cl, Br, $CF_3$, $CO_2R_{20}$, $SO_2NR_{10}R_{11}$, $SO_2N(OCH_3)CH_3$ or $S(O)_nR_{13}$;

$R_{17}$ is H, F, Cl, Br, $CH_3$ or $OCH_3$;

W' is O or S;

$R_{18}$ is H, $C_1$—$C_4$ alkyl, $C_1$—$C_4$ alkoxy, F, Cl, Br, $NO_2$, $CO_2R_{20}$, $SO_2NR_{10}R_{11}$, $SO_2N(OCH_3)CH_3$ or $S(O)_nR_{13}$;

$R_{19}$ is Cl, $NO_2$, $CF_3$, $CO_2R_9$, $SO_2N(OCH_3)CH_3$, $SO_2NR_{10}R_{11}$, $QSO_2R_{12}$, $S(O)_nR_{13}$ or $C_1$—$C_3$ alkoxy substituted with 1—5 atoms of Cl or F;

$R_{20}$ is $C_1$—$C_4$ alkyl, $CH_2CH_2OCH_3$, $CH_2CH_2Cl$ or $CH_2CH{=}CH_2$;

$R_{21}$ is $C_1$—$C_3$ alkyl or $C_6H_5$;

$R_{22}$ is $C_1$—$C_3$ alkyl; and

$R_{21}$ and $R_{22}$ may be taken together to be

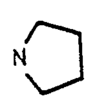 ;

provided that

(1) the total number of carbon atoms of $R_{10}$ and $R_{11}$ is less than or equal to 4;
(2) the total number of carbon atoms of $R_1$ and $R_2$ is less than or equal to 6;
(3) when W' is O, then $R_{18}$ is H, Cl, Br, $CH_3$ or $CO_2R_{20}$;
(4) when W' is O and $R_{18}$ is H, Cl, Br or $CH_3$, then Z is

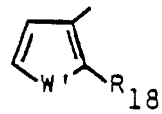 ;

(5) when W is S, then $R_3$ is H;
(6) when $R_7$ is H, then $R_8$ is H; and
(7) $R_{14}$ and $R_{15}$ may not both be $NO_2$.

Preferred for reasons of higher herbicidal activity or more favorable ease of synthesis are:

(1) Compounds of Formula I wherein
W is O and $R_3$ is H.

(2) Compounds of Preferred 1 where
Z is

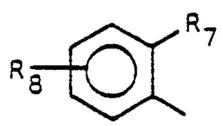 , 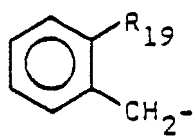 , 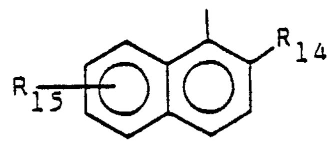 ,

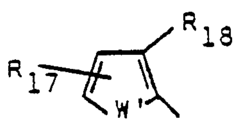 , 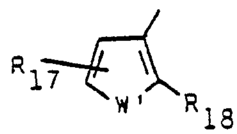 , 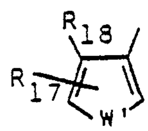 or 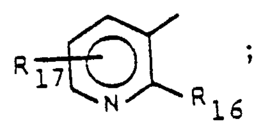 ;

W' is S;
$R_{19}$ is $CO_2CH_3$, $SO_2N(CH_3)_2$ or $SO_2CH_3$; and
$R_{15}$ and $R_{17}$ are H.

(3) Compounds of Preferred 2 where
Z is

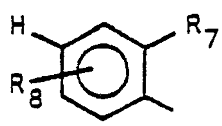 , 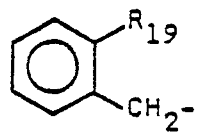 , 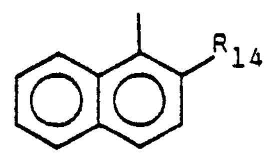 ,

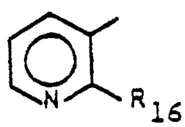 , 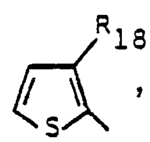 , 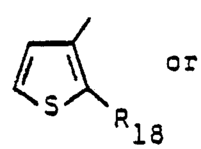 or 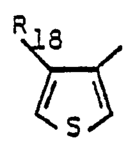 .

(4) Compounds of Preferred 3 where
$R_1$ is $C_1$—$C_2$ alkyl, $SR_4$, $OR_5$, $CH_2OCH_3$, $N(CH_3)_2$ or Cl;
$R_2$ is $C_1$—$C_2$ alkyl, $CH_2CF_3$, $CH_2CH{=}CH_2$ or $CH_2C{\equiv}CH$; and
$R_4$ and $R_5$ are independently $CH_3$ or $C_2H_5$.

(5) Compounds of Preferred 4 where Z is

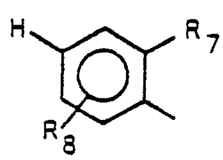 ;

$R_7$ is other than H; and
$R_8$ is H, F, Cl, Br, $CF_3$, $CH_3$ or $OCH_3$.

(6) Compounds of Preferred 5 where
$R_7$ is Cl, $NO_2$, $CO_2R_9$, $SO_2NR_{10}R_{11}$ or $OSO_2R_{12}$;

$R_8$ is H;

$R_9$ is $C_1$—$C_3$ alkyl, $CH_2CH{=}CH_2$, $CH_2CH_2OCH_3$ or $CH_2CH_2Cl$;

$R_{10}$ and $R_{11}$ are independently $C_1$—$C_2$ alkyl; and

$R_{12}$ and $R_{13}$ are independently $C_1$—$C_3$ alkyl.

(7) Compounds of Preferred 6 where $R_9$, $R_{10}$, $R_{11}$, $R_{12}$ and $R_{13}$ are $CH_3$.

Specifically preferred for reasons of their highest herbicidal activity and/or most favorable ease of synthesis are:

2-[[(5-methylthio-1-methyl-1H-1,2,4-triazol-3-yl)aminocarbonyl]aminosulfonyl]benzoic acid, methyl ester;

2-[[(5-ethylthio-1-methyl-1H-1,2,4-triazol-3-yl)aminocarbonyl]aminosulfonyl]benzoic acid, methyl ester;

2-[[(5-ethyl-1-methyl-1H-1,2,4-triazol-3-yl)aminocarbonyl]aminosulfonyl]benzoic acid, methyl ester;

2-[[(5-methoxy-1-methyl-1H-1,2,4-triazol-3-yl)aminocarbonyl]aminosulfonyl]benzoic acid, methyl ester;

N-[(5-methoxy-1-methyl-1H-1,2,4-triazol-3-yl)aminocarbonyl]-2-chlorobenbenzenesulfonamide; and

N-[(5-methoxy-1-methyl-1H-1,2,4-triazol-3-yl)aminocarbonyl]-N',N'-dimethyl-1,2-benzenedisulfonamide.

Synthesis

Many of the compounds of Formula I can be prepared by reacting an appropriate 3-amino-1,2,4-triazole of Formula II with an appropriately substituted sulfonylisocyanate or isothiocyanate of Formula III, as shown in Equation 1.

Equation 1

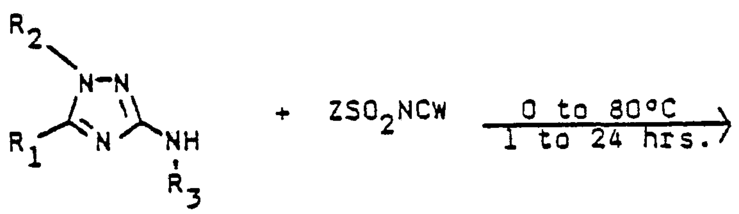


I

II III

wherein

Z, $R_1$, $R_2$, $R_3$ and W are as previously defined. The reaction of Equation 1 is best carried out in inert organic solvents such as methylene chloride, tetrahydrofuran, or acetonitrile at ambient pressure and temperature. The mode of addition is not critical; however, it is often convenient to add the sulfonylisocyanate or isothiocyanate to a stirred suspension or solution of the aminotriazole. Since such isocyanates and isothiocyanates usually are liquids, their additions can be easily controlled.

The reaction is generally exothermic. In some cases, the desired product is insoluble in the warm reaction medium and crystallizes from it in pure form. Products soluble in the reaction medium are isolated by evaporation of the solvent, trituration of the solid residue with solvents such as 1-chlorobutane or ethyl ether, and filtration.

An alternate method for synthesizing compounds of Formula I in which W = O is illustrated in Equation 2. An appropriate sulfonylisocyanate of Formula III is allowed to react with an appropriate 3-amino-1H-1,2,4-triazole of Formula IV to give an intermediate urea of Formula V which is then alkylated with a reagent of Formula $R_2X$ to yield compounds of Formula I:

Equation 2

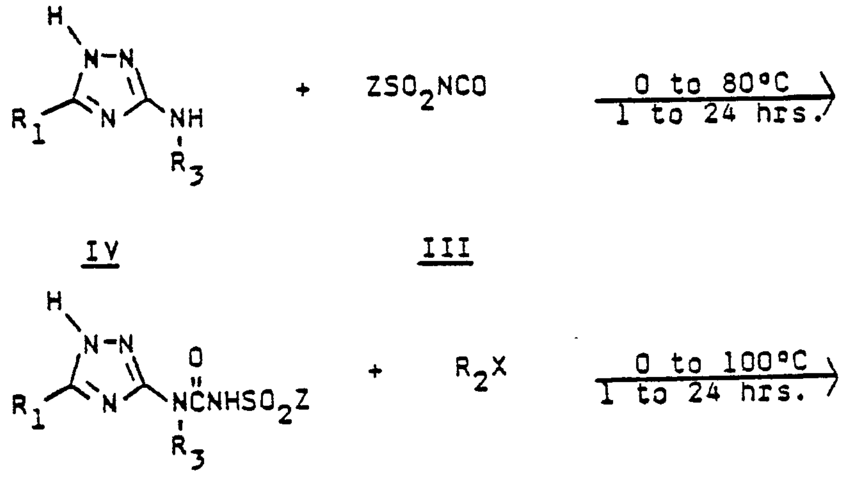


IV III

V I

wherein

X is a good leaving group such as bromine, iodine, chlorine, alkylsulfonate, $R_2OSO_3^{\ominus}$, or *p*-toluenesulfonate, and Z, $R_1$, $R_2$ and $R_3$ are as previously defined.

The conditions for reacting the 3-amino-1H-triazole of Formula IV and sulfonylisocyanate or sulfonylisothiocyanate in Equation 2 are similar to that described for the condensation involving triazole II in Equation 1, however it is desirable to have an equimolar amount of sulfonylisocyanate react with triazole IV.

The alkylation reaction illustrated in Equation 2 between the sulfonylurea intermediate V and $R_2X$ (wherein X is a good leaving group such as bromine, iodine, chlorine, alkylsulfonate, $R_2OSO_3^{\ominus}$, or *p*-toluenesulfonate) is preferably carried out in the presence of an alkaline material and preferably in the presence of a solvent. Suitable alkaline materials, include, for example, the alkali and alkaline earth metal carbonates, bicarbonates, acetates, and hydroxides such as potassium carbonate, sodium carbonate, potassium carbonate, potassium acetate, sodium and potassium hydroxide. Other organic bases such as pyridine and triethylamine may also be used. Suitable solvents include inert aprotic solvents such as acetone, methyl ethyl ketone, acetonitrile, methylene chloride, dimethylformamide, dimethylacetamide, and dimethylsulfoxide. The alkylation reaction conditions of Equation 2 vary according to the nature of the reactants, the base and solvent present. Usually, the reaction is facilitated by the application of heat (between 45° and 128°) however, lower reaction temperatures may be desirable in some instances as will be readily apparent to one skilled in the art.

The intermediate aryl sulfonylisocyanates of Formula III (W = O) can be prepared by reacting the corresponding aryl sulfonamides with phosgene in the presence of *n*-butyl isocyanate at reflux in a solvent such as chlorobenzene or xylenes, according to the procedure of H. Ulrich and A. A. Y. Sayigh, *Newer Methods of Preparative Organic Chemistry*, Vol. VI, p. 223—241, Academic Press, New York and London, W. Forest Ed., or by the methods taught in U.S. Patent 4,127,405 (1978), U.S. 4,238,671 (1980) and EP—A—23,141.

The intermediate pyridyl sulfonylisocyanates of Formula III (W = O) can be prepared by reacting an N-(alkylaminocarbonyl)pyridinesulfonamide with phosgene as described in EP—A—13,480, to which the reader is referred for further information. The N-(alkylaminocarbonyl)pyridinesulfonamide can be prepared, as described in USSN 966,258, by the reaction of a pyridinesulfonamide, an alkyl isocyanate and an anhydrous base in an anhydrous solvent.

Similarly, the preparation of the furan sulfonyl isocyanates is described in U.S. Patent 4,127,405 (1978); the thiophene and naphthalene sulfonylisocyanates can be prepared as shown in Equations 3 and 4 respectively, wherein $R_{14}$, $R_{15}$, $R_{17}$ and $R_{18}$ are as previously defined.

Equation 3

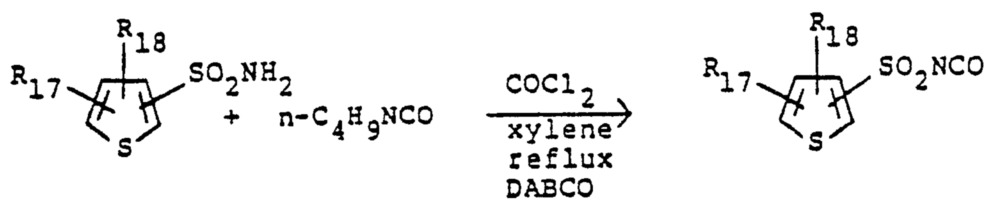


VI VII

A mixture of the appropriate sulfonamide, e.g., an 2-alkoxycarbonyl-3-thiophene sulfonamide VI such as the methyl ester, which is known in the art, an alkyl isocyanate such as butyl isocyanate and a catalytic amount of 1,4-diaza[2.2.2]bicyclooctane (DABCO) in xylene or other inert solvent of sufficiently high boiling point (e.g. >135°C) is heated to approximately 130—150°C. Phosgene is added to the mixture until an excess of phosgene is present as indicated by a drop in the boiling point. After the mixture is cooled and filtered to remove a small amount of insoluble by-products, the solvent and alkyl isocyanate are distilled off *in-vacuo* leaving a residue which is the crude sulfonyl isocyanate VII.

Equation 4

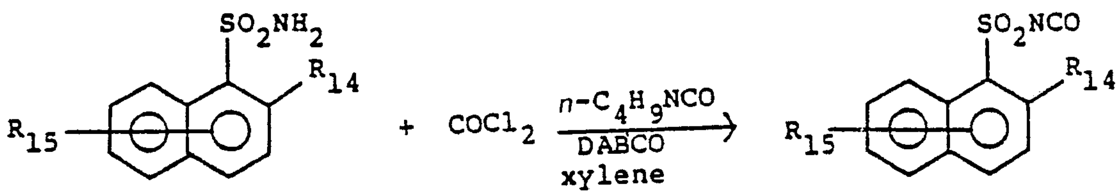


VIII IX

The preparations of naphthalene sulfonylisocyanates IX can be obtained from the corresponding sulfonamides VIII. Reaction conditions for the phosgenation would be the same as that for the thiophene sulfonamides in Equation 3.

The phenyloxysulfonylisocyanates of Formula XI are readily prepared from the corresponding phenols of Formula X as taught in U.S. 4,191,553 and as shown in Equation 5. Reaction of X with chlorosulfonylisocyanate in a high boiling solvent, for example, dichlorobenzene, toluene or xylene, will produce the sulfonylisocyanate of Formula *XI*.

Equation 5

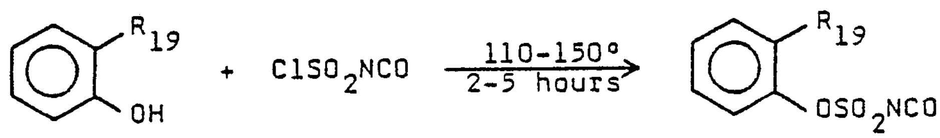


X XI

Arylsulfonylisothiocyanates of Formula III (W = S) can be prepared by treatment of sulfonamides with carbon disulfide and potassium hydroxide followed by reaction of the dipotassium salt with phosgene according to the teaching of K. Hartke, *Arch. Pharm. 229,* 174 (1966).

Pyridine sulfonylisothiocyanates can be prepared according to the procedure taught by K. Dickere and E. Kuhle in U.S. Patent 3,346,590. A suitable pyridinesulfonyliminodithiocarbonate is reacted with phosgene in the presence of a solvent such as toluene or xylene.

The thiophene and furan sulfonylisothiocyanate intermediates of Formula XIII prepared according to Equations 6 and 7 are useful for the preparation of compounds of Formula I where W = S.

Equation 6

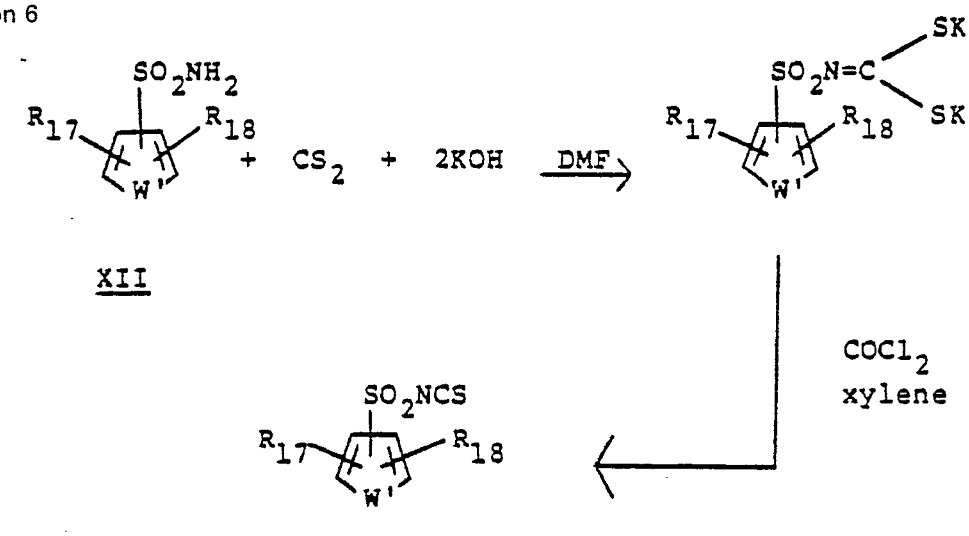


XII

XIII

The substituted sulfonamide is dissolved in dimethylformamide (DMF) with an equivalent amount of carbon disulfide and two equivalents of potassium hydroxide are added portionwise at room temperature. The mixture is stirred for 1—8 hours and diluted with ethyl acetate, ethyl ether or similar aprotic solvent to cause the dipotassium salt of the dithiocarbamic acid to precipitate. The salt is isolated, dried and suspended in an inert solvent such as xylene, benzene, carbon tetrachloride or methylene chloride. Phosgene is added to the stirred suspension at or below room temperature and the mixture stirred for 1—3 hours. In place of phosgene, a chloroformic ester (e.g. methyl chloroformate), phosphorous pentachloride, sulfuryl chloride or thionyl chloride can be used.

The sulfonylisothiocyanate which is formed is usually soluble in the solvent and is isolated by filtering off the insoluble potassium chloride and concentrating the filtrate. These isothiocyanates tend to be unstable and dimerize readily, (Equation 7), however, the dimers can be used in the same manner as the parent isothiocyanates for the purposes of this invention.

Equation 7

XIII 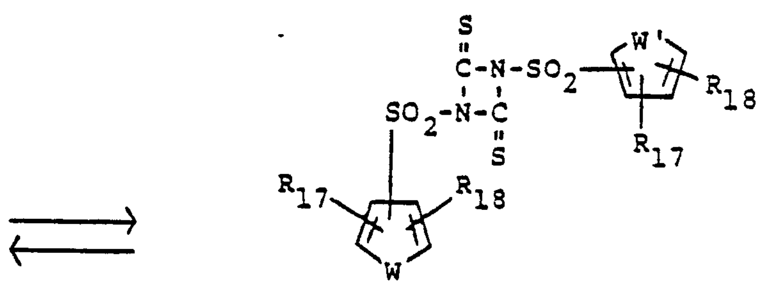

The naphthalene sulfonylisothiocyanate intermediate XIV may also be prepared from the sulfonamide VIII according to Equation 8 and following similar reaction conditions as that in Equation 6. These isothiocyanates may also be unstable and dimerize, however, these dimers can be used in the same way as the parent isothiocyanates for the purpose of the invention.

Equation 8

VIII + $CS_2$ + KOH 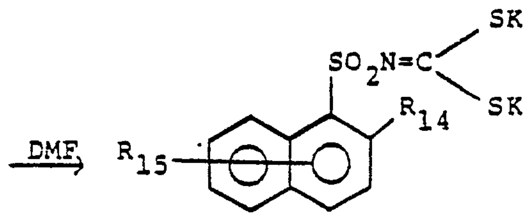

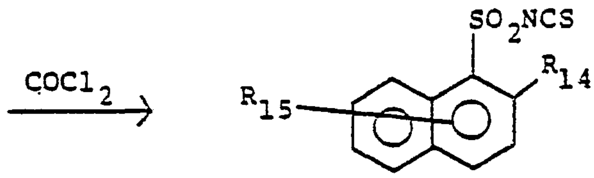


XIV

The preparation of sulfonamides from ammonium hydroxide and sulfonyl chloride is widely reported in the literature, e.g., Crossley et al., *J. Am. Chem. Soc. 60,* 2223 (1938). Certain sulfonyl chlorides are best prepared by chlorosulfonation of a substituted aromatic in carbon tetrachloride according to the teaching of H. T. Clarke et al., *Org. Synth.,* coll. Vol. 1, 2nd Ed., 1941, p. 85. Other benzenesulfonyl chlorides are best made by diazotization of the appropriate aniline with sodium nitrite in HCl, followed by reaction of the diazonium salt with sulfur dioxide and cuprous chloride in acetic acid according to the teaching of H. L. Yale and F. Sowinski, *J. Org. Chem. 25,* 1824 (1960). The preparation of pyridyl sulfonyl chlorides is described in *Chem. Abs. 88,* 190603 m (1978).

Preparations of the aryl sulfonamides are given in U.S. Patents 4,127,405 (1978) wherein $R_7$ = H, $C_1$—$C_4$ alkoxy, F, C, Br, $NO_2$, $CF_3$; EP—A—7687 wherein $R_7 = CO_2R_9$, $CO_2NR_{21}R_{22}$, $C(O)SR_{10}$; EP—A—23,141 ($R_7 = SO_2NR_{10}R_{11}$, $SO_2N(OCH_3)CH_3$); EP—A—23,422 ($R_7 = OCF_3$, $OCHF_2$, $OCF_2CF_2H$, $S(O)_nR_{13}$ where $R_{13}$ is fluorinated alkyl); EP—A—44,209 ($R_7 = CH_2CO_2R_{20}$, $CH(CH_3)CO_2R_{20}$, $CH_2S(O)_nR_{13}$, $CH(CH_3)S(O)_nR_{13}$); EP—A—44,212 ($R_7 = QSO_2R_{12}$ where Q = O or $NCH_3$); EP—A—44,807 ($R_7$ = $C_3$—$C_4$ alkenyloxy, $C_1$—$C_3$ alkoxy substituted with $OCH_3$ or $OC_2H_5$, $S(O)_nR_{13}$ where $R_{13}$ = allyl); and EP—A—44,808 ($R_7$ = $C_1$—$C_3$ alkoxy substituted with 1—5 atoms of Cl, Br or F). The arylsulfonamides wherein $R_7$ = $C_3$—$C_4$ alkynyloxy may be prepared by methods taught in EP—A—44,807. The synthesis of pyridylsulfonamides is described in G. Machek, *Monatsch* 2, 84 (1939); L. Thunus and C. L. Lapiere, *Ann. Farn. 33,* 663 (1975), and EP—A—13,480.

Equation 9 describes the procedure for making aryl and naphthalene intermediates of Formula III when $R_7$ and $R_{14}$ are $S(O)_nR_{13}$. The thioether of Formula XVb may be prepared from the appropriate 2-aminothiophenol or aminothionaphthalene and an alkyl halide as described in the literature, e.g., R. N. Prasad et al., *Can. J. Chem. 44,* 1247 (1966). The formation of the sulfonamide XVd is accomplished in the following manner.

Equation 9

9a

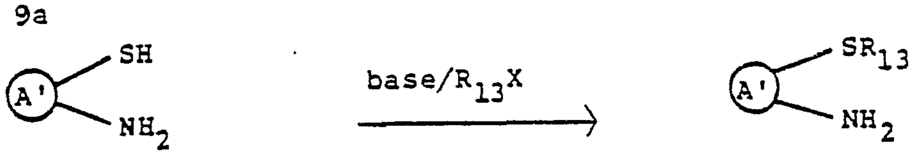


XVa XVb

9b

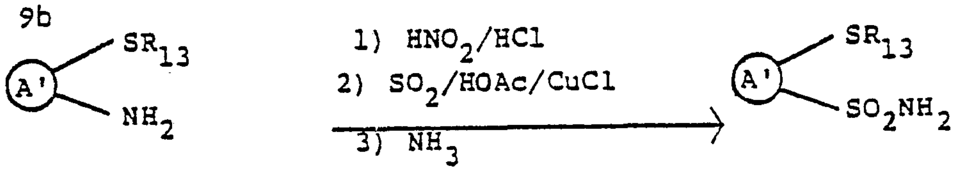


XVb XVc

9c

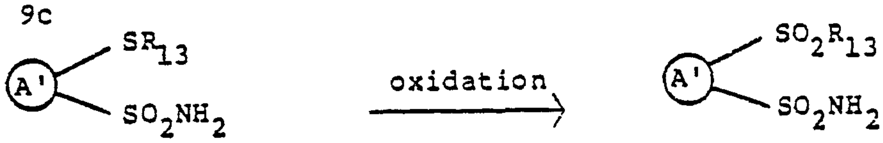


XVc XVd

wherein 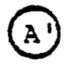 is 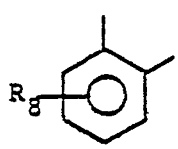 or 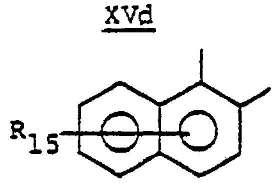

A solution of the thioether of Formula XVb in a mixture of concentrated hydrochloric acid and glacial acetic acid is treated with a solution of sodium nitrite in water at −5° to 0°. After stirring for 10—15 minutes at 0° to insure complete diazotization, this solution is added to a mixture of an excess of sulfur dioxide and a catalytic amount of cuprous chloride in glacial acetic acid at 0—5°. The temperature is kept at 0—5° for ¼ to 1 hour and is then raised to 20—25° and held at that temperature for 2—4 hours. This solution is then poured into a large excess of ice water. The sulfonyl chloride products can be isolated by filtration or by extraction into solvent such as ethyl ether or methylene chloride followed by evaporation of the solvent.

The amination described in step (9b) is conveniently carried out by treating a solution of the sulfonyl chloride with an excess of anhydrous ammonia in a solvent such as ethyl ether or methylene chloride at 0—25°. If the product sulfonamide is insoluble, it may be isolated by filtration followed by washing out the salts with water. If the product sulfonamide is soluble in the reaction solution, it may be isolated by filtering off the precipitated ammonium chloride and evaporating the solvent.

Sulfonamides of Formula XVc wherein A′ = aryl may also be prepared from the appropriate chlorobenzenesulfonamides (synthesis taught in U.S. 4,127,405) as shown in Equation 10. Heating an equimolar mixture of the appropriate solfonamide and mercaptan in the presence of two equivalents of base will yield XVc following acidic work-up. As in Equation 9, the sulfonamides XVd can be prepared by oxidation of XVc.

Equation 10

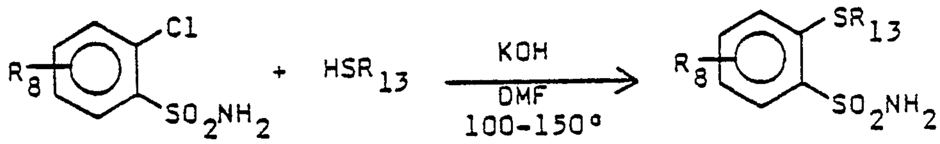


XVc

Synthesis of thiophene sulfonamides of Formula XVIe and XVIf may be accomplished by the method outlined in Equation 11. The sulfonyl chlorides of Formula XVIa are described by H. D. Hartough in "The Chemistry of Heterocyclic Compounds," v. 3, Interscience Publishers, Inc., N.Y. 1952. These may be converted to the corresponding *N-t*-butyl sulfonamides XVIb by admixture with at least twice the equivalent amount of *t*-butylamine in an inert solvent, such as ether, filtration of the amine hydrochloride, and evaporation of the solvent. The lithiation of thiophenes and of aromatic *N-t*-butylsulfonamides with *n*-butyllithium, *t*-butyllithium, lithium diisopropylamide and lithium 2,2,6,6-tetramethyl piperidide is reviewed by H. W. Gschwend and H. R. Rodriguez in *Org. React., 26,* 1 (1979), and is generally carried out by cooling to −78° a solution of twice the equimolar amount of base kept under an inert atmosphere, in an ethereal solvent such as diethyl ether or THF, and adding a solution of the compound of Formula XVIb. The compounds of Formula XVIc may be prepared as shown in Equation (11b) by adding an equimolar quantity of the appropriate disulfide $(R_{13}S)_2$, allowing the mixture to warm to room temperature, washing the mixture with acidic brine, and evaporation of the solvent.

Equation 11

(11a)

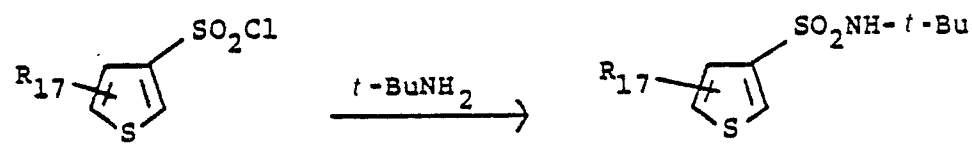


XVIa XVIb

(11b)

XVIb

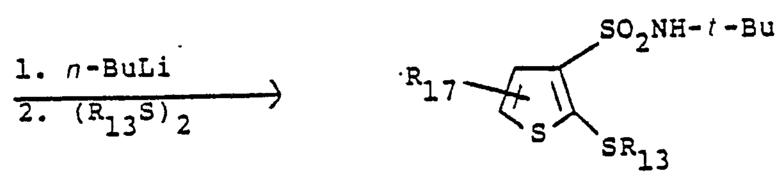


XVIc

(11c)

XVIb

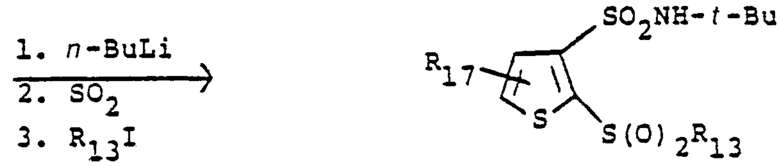


XVId

(11d)

XVIc

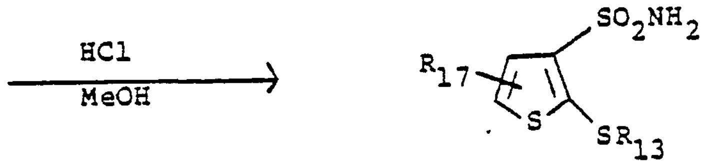


XVIe

(11e)

XVId

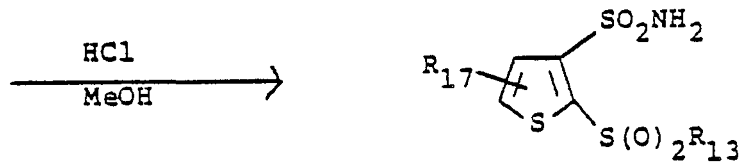


XVIf

Alternatively, as shown in Equation (11c), in order to prepare the compounds of Formula XVId, the lithiation mixture may be treated with an equimolar quantity of sulfur dioxide, allowing the mixture to warm to room temperature, filtration of the solid precipitate, dissolution of this salt in ethanol and adding an equimolar amount of the appropriate alkyl iodide. This alkylation step may be carried out at temperatures of 25 to 78°. The cooled reaction mixture may be diluted with dilute aqueous hydrochloric acid to precipitate the product XVId. The *t*-butyl sulfonamides of Formula XVIc and XVId may be converted to the compounds of Formulae XVIe and XVIf, respectively, by heating in methanol containing at least an equimolar quantity of hydrochloric acid, followed by concentration of the reaction mixture and precipitation of the product with ether.

An alternate preparation of the thiophene sulfonamides of Formula XVIf is described in Equation 12.

Equation 12

(12a)

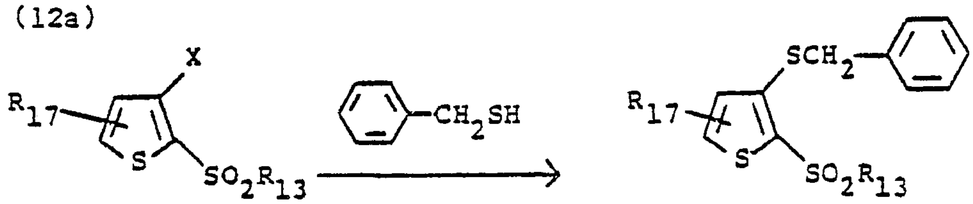

XVIg XVIh

(12b)

XVIh

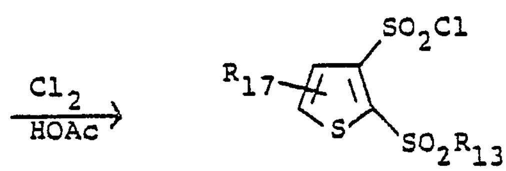


XVIi

(12c)

XVIi

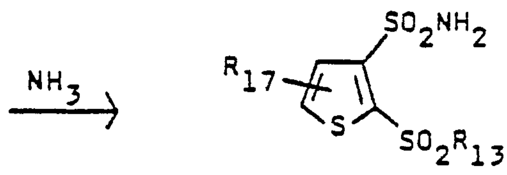


XVIf

wherein

X = Cl or Br and $R_{13}$ and $R_{17}$ are as previously defined.

The reaction of Equation (12a) is accomplished by mixing equimolar quantities of the appropriate halide XVIg with an equimolar quantity of benzyl mercaptan in a polar solvent, such as dimethylformamide, containing an equimolar amount of a strong base, such as sodium methoxide or sodium hydride, heating at a temperature between 50 and 120°, and isolating the product by precipitation with ice-water and washing with hexane. The sulfides of Formula XVIh are converted to the sulfonyl chlorides XVIi as shown in Equation (12b) by contacting with at least three equivalents of chlorine in acetic acid according to the procedure of R. F. Langler, *Can. J. Chem., 54,* 498 (1976). The sulfonyl chlorides can be precipitated by the addition of ice-water to the chlorination mixture. Ammonolysis of thiophene sulfonyl chlorides gives XVIf.

Compounds of Formulae XVIIb (Equation 13) wherein $R_{13}$ and $R_{17}$ are as previously defined may be prepared by adding twice the equimolar amount of chlorosulfonic acid, diluted in an inert solvent, such as dichloromethane, to the appropriate 3-thienyl sulfide XVIIa at temperatures between −30° and 25°, washing the mixture with ice-water and evaporating the solvent. These may be converted to the appropriate compounds of Formula XVIIc by treatment with ammonia.

The sulfides of Formula XVIIc may be oxidized to compounds of Formula XVIId.

Equation 13

(13a)

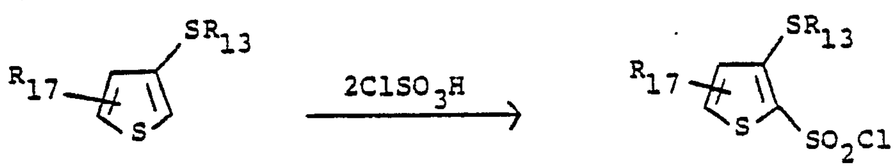


XVIIa XVIIb

(13b)

XVIIb

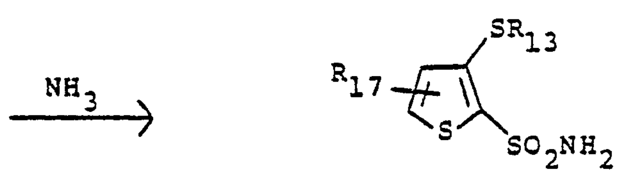


XVIIc

(13c)

XVIIc

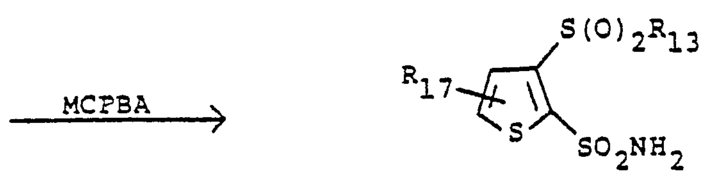


XVIId

Also, the methods described in Equation 11 may be applied in making compounds of Formula XVIId.

According to the method outlined in Equation 14, compounds of Formulae XVIIIf and XVIIIh can be prepared, wherein $R'_{17}$ equals H, chlorine, or bromine, X = chlorine, bromine and $R_{13}$ is as previously defined. Starting with sulfonyl chlorides of Formula XVIIIa, the dihalo-compounds may be partially

dehalogenated by contacting with two equivalents of 5% sodium amalgam in an alcoholic or aqueous alcoholic solution at 25 to 78°, followed by acidification of the products of Formulae XVIIIe and XVIIIg wherein $R'_{17}$ = Cl or Br. The 2-halo and 5-halo isomers may be separated by column chromatography. The totally dehalogenated compounds where $R'_{17}$ = H, may be prepared by using three or more equivalents of the sodium amalgam in the reaction. These compounds may be converted to XVIIIf and XVIIIh as previously described.

Equation 14

(14a)

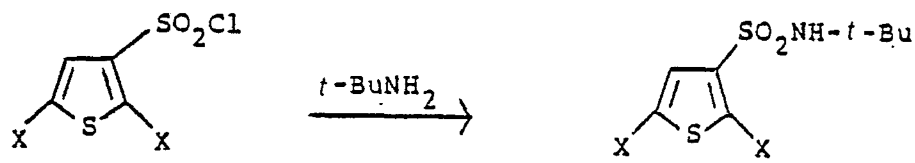


XVIIIa XVIIIb

(14b)

XVIIIb

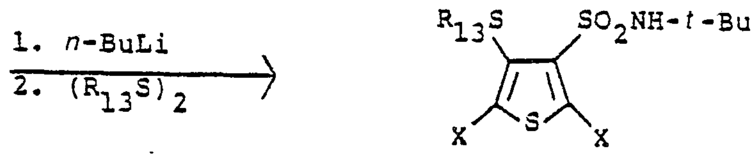


XVIIIc

(14c)

XVIIIb

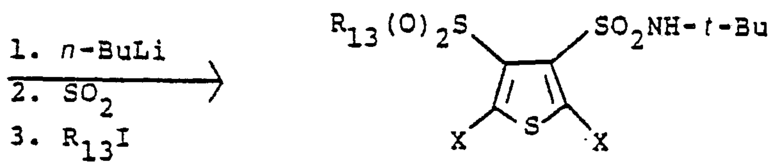


XVIIId

(14d)

XVIIIc

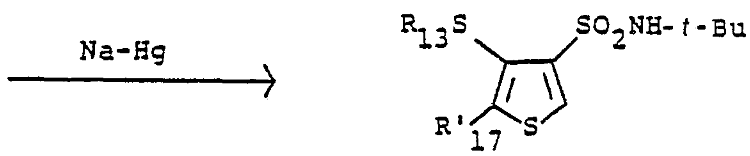


XVIIIe

(14e)

XVIIIe

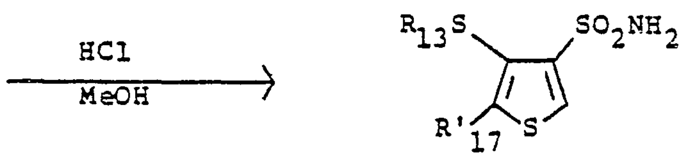


XVIIIf

(14f)

XVIIId

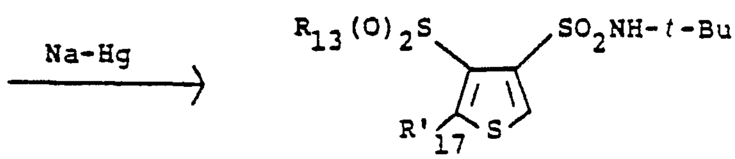


XVIIIg

(14g)

XVIIIg

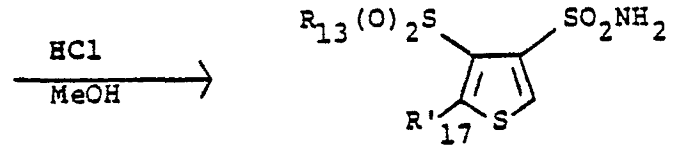


XVIIIh

Precursors to the required thiophene sulfonyl chlorides and sulfonamides are prepared by a variety of synthetic routes depending on the chemical properties of the substituent and its position on the thiophene ring.

Direct sulfonation or chlorosulfonation to sulfonic acid or sulfonyl chloride derivatives can be carried out according to the references cited in "Thiophene and its Derivatives," H. D. Hartough, Interscience, New York, 1952. The structure of sulfonation products of 3-alkyl thiophenes has been reported as uncertain. Nuclear magnetic resonance studies indicate the chlorosulfonation occurs predominantly at the 2- rather than the 5-position.

Sulfonic acids are readily converted to sulfonyl chlorides, using methods well known in the art, by chlorinating agents such as phosphorus pentachloride, phosphorus oxychloride or thionyl chloride. A mixture of sulfuryl chloride in dimethylformamide can also be used to prepare thiophenesulfonyl chlorides of active thiophene intermediates according to the method of E. Testa et al., *Helv. Chim. Acta.*, *47*, 766 (1963).

Other intermediates can be prepared via lithiation reactions. A review of this chemistry appears in Organic Reactions, Vol. 26, Gschwind, H. W. and Rodriguez, H. R., John Wiley and Sons, Inc., New York, 1979. Examples of the application of this chemistry to the preparation of intermediates used here is shown in the following equations.

Equation 15 shows the preparation of sulfamyl thiophene sulfonamides via lithiated intermediates.

Equation 15

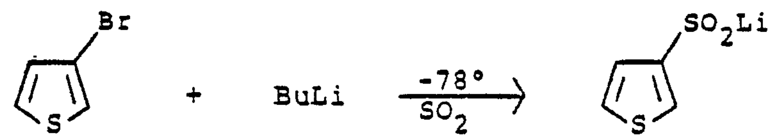


IXXa

IXXa +

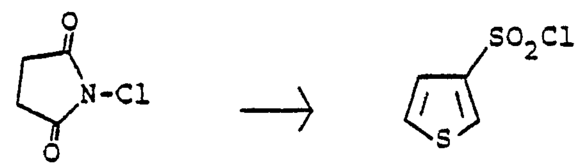

IXXb

IXXb +

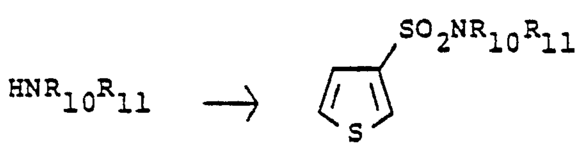


IXXc

IXXc +

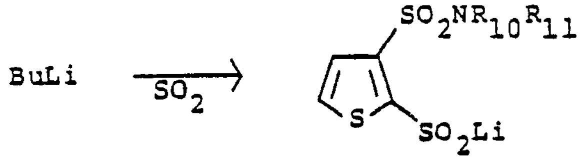


IXXd

As shown in Equation 15, 3-bromothiophene is converted to 3-lithiothiophene at −78° in an inert solvent such as tetrahydrofuran and the mixture is then contacted with sulfur dioxide. The resultant lithio

sulfinate is stirred at room temperature in acetic acid or aqueous 2-propanol with N-chlorosuccinimide to yield the 3-thiophenesulfonyl chloride IXXb. This product is then contacted with an amine, $HNR_{10}R_{11}$ wherein $R_{10}$ and $R_{11}$ are as previously defined. The 3-thiophenesulfonamide IXXc, thus formed is reacted with butyl lithium at −40 to 0°C followed by sulfur dioxide to form the lithio 3-sulfamyl-2-thiophenesulfinate IXXd which is converted to the sulfonyl chloride as described above. Conversion of this sulfonyl chloride to the sulfonamide and sulfonylisocyanate is carried out as previously described.

The synthesis of other intermediates via lithiation is shown in Equation 16, wherein $R_{18}$ is optionally Cl, Br, $C_1$—$C_4$ alkyl, $C_3$ alkenyl or $OCH_3$.

Equation 16

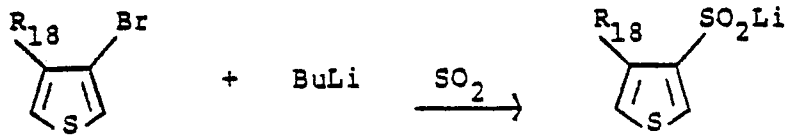


XXa

XXb

XXb +

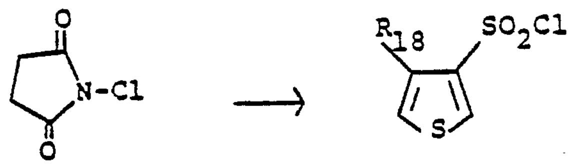

XXc

The reactions described in Equation 16 are carried out in the same manner as those described in Equation 15 as would be expected by one skilled in the art. Displacement of activated halogen atoms from the thiophene nucleus by benzyl mercaptan and chlorination of the resulting product is also a useful route to intermediates for compounds of this invention as illustration by Equation 17.

Equation 17

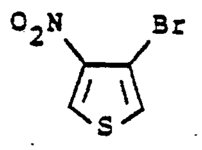

+

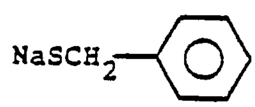

XXIa

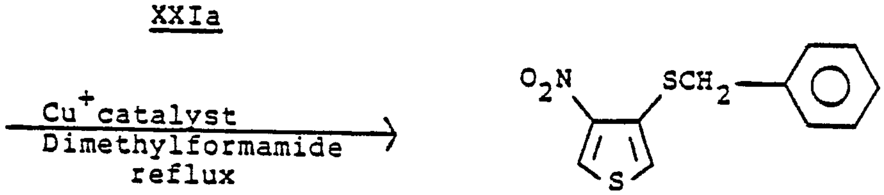


XXIb

XXIb + $Cl_2$

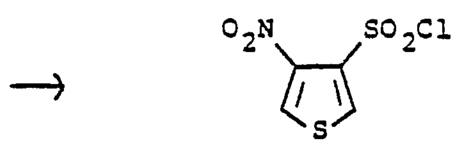

XXIc

The reaction of benzyl mercaptan with a halothiophene, wherein the halo atom is susceptible to replacement by a nucleophile, is best carried out in an inert polar, high boiling solvent such as dimethylformamide or N-methylpyrrolidone at reflux in the presence of a copper catalyst over six to forty-eight hours. The thioether intermediate XXIb is converted to the sulfonyl chloride XXIc by passing chlorine gas into aqueous hydrochloric acid or acetic acid solution or suspension of XXIb.

Disulfides such as structure XXII, reported by Henriksen and Autruys, *Acta. Chem. Scands., 24,* 2629 (1970), are also useful intermediates for conversion to sulfonyl chlorides as shown in Equation 18.

Equation 18

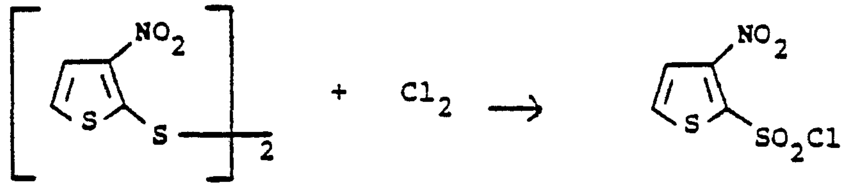


XXII XXIII

The chlorination shown in Equation 18 is carried out in the same manner as described for the chlorination of structure XXIb.

Alternatively, the diazotization reaction of thiophene amines to sulfonyl chlorides such as structure XXIV shown in Equation 19 are carried out according to the general procedure of H. L. Yale and F. Sowinski, *J. Org. Chem., 25,* 1824 (1960).

Equation 19

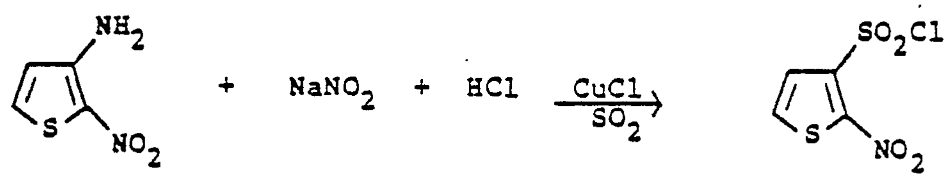


XXIV XXIVa

The *o*-alkoxymethyl nitrobenzenes XXVb are in turn prepared via "Williamson Synthesis", according to Equations 20a or 20b.

Equation 20a

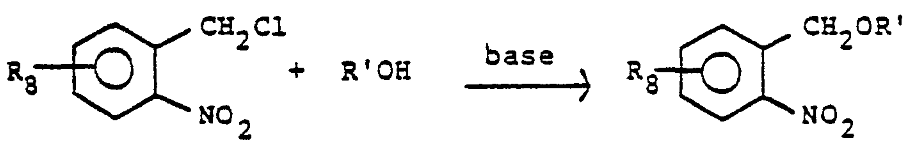


XXVa XXVb

Equation 20b

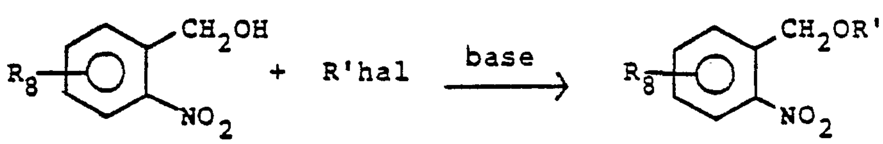


XXVc XXVb

$R' = C_1\text{-}C_2$ alkyl.

"Williamson Synthesis" has been widely used for the preparation of ethers as reviewed by W. Theilheimer, *Syn. Methods of Org. Chem.,* Vol. VII, p. 112.

Alternatively, *o*-alkoxymethyl methylbenzenesulfonyl chlorides, XXVf, can be obtained from an appropriately substituted α-hydroxy-*o*-toluenesulfonic acid-α-sultone, XXVd, via ring-opening reaction with an alkoxide anion as depicted in Equations 20c and 20d.

Equation 20c

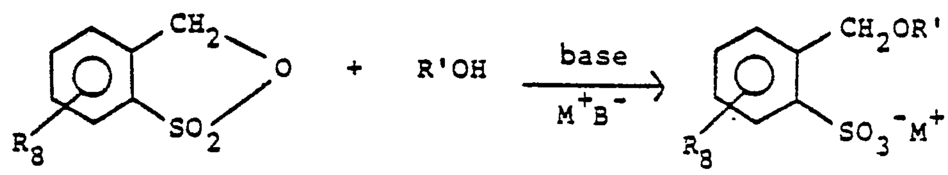


XXVd XXVe

Equation 20d

XXVe + $PCl_5$

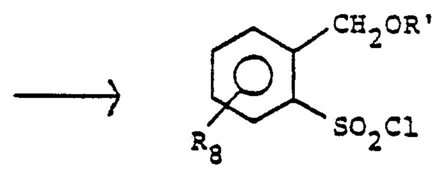

XXVf

Reaction 20c is closely related to the alkylation of acyloxides and acetamide with sultones as disclosed by J. H. Helberger et al., *Ann., 565* 22 (1949). Conversion of the sulfonic acid salt to the sulfonyl chloride is then carried out according to the teaching of *Org. Synthesis,* Coll. Vol. IV, 846, 693.

Benzenesulfonamides of Formula XXVIb can also be derived from compound XXVIa as illustrated in Equation 21.

Equation 21

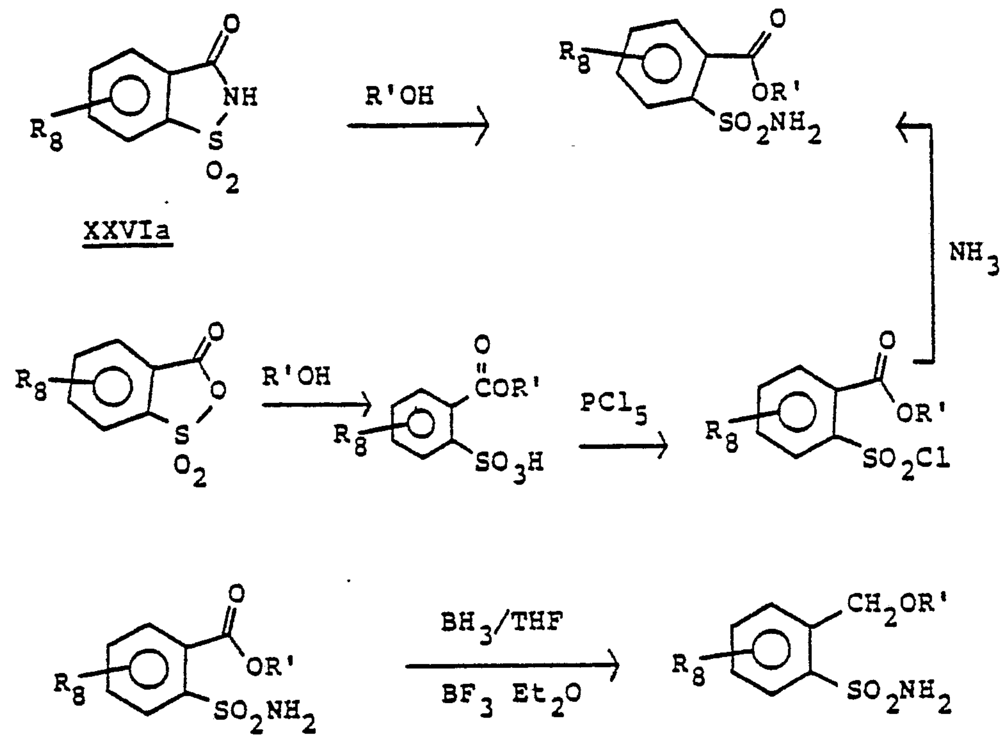


XXVIb

The *o*-alkoxyethylbenzenesulfonamides of Formula XXVIIb can be prepared from the appropriate benzenesulfonyl chlorides of Formula XXVIIa via lithiation chemistry as shown in Equation 22. Reactions of this type are well known in the literature and have been reviewed in Organic Reactions, Vol. 26, Gschwend, H. W. and Rodriguez, H. R., John Wiley and Sons, Inc., New York, 1979.

Equation 22

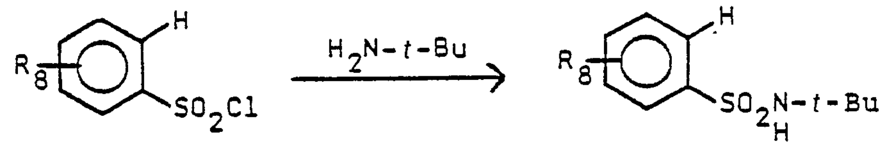


XXVIIa

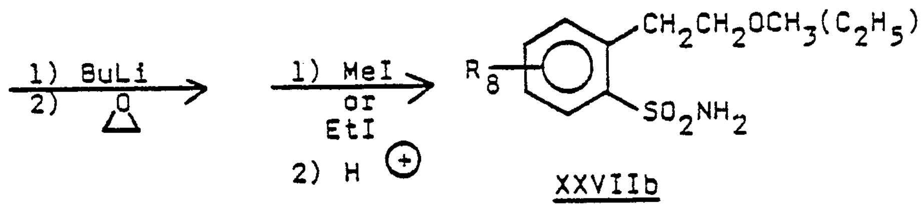


XXVIIb

Preparation of *o*-sulfamylbenzoic acid esters, from saccharin or sulfobenzoic acid anhydride is well known in the art, e.g., B. Loev and M. Kormendy, *J. Org. Chem. 27*, 1703 (1962). The esters can be readily reduced to the esters with diborane in a suitable organic solvent, e.g., tetrahydrofuran, in the presence of fifteen fold excess of boron trifluoride etherate under reflux for 18 hours, as described by R. P. Graber and M. B. Meyers, *J. Org. Chem., 26*, 4773 (1961).

Most generally, the naphthalene sulfonamides may be prepared from the sulfonyl chlorides XXVIIIb (Equation 23) as described in "Preparative Organic Chemistry", ed. G. Hilgetag and A. Martini, J. Wiley and Sons, New York (1972). The sulfonyl chlorides may be prepared by chlorination of the sulfonic acids XXVIIIa by methods described by Hilgetag and Martini, op. cit. The preparation of these acids is described in the art. These compounds may be further transferred by methods known in the art to yield other disclosed sulfonic acids.

Equation 23

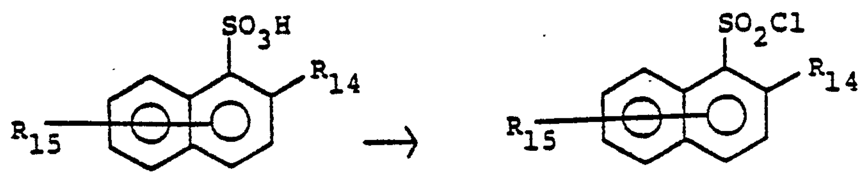

XXVIIIa XXVIIIb

The desired sulfamyl naphthalene sulfonamides can be prepared by methods analogous to those taught in European Patent 23,141.

The benzylsulfonamides of Formula XXIXd are most easily prepared as shown in Equation 24. The appropriately substituted toluene derivatives of Formula XXIXa are brominated and then reacted with thiourea to give the thiouronium salts of Formula XXIXb. Preparation of the sulfonyl chlorides of Formula XXIXc is readily accomplished by oxidation/chlorination of XXIXb [for the preparation and oxidative chlorination of thiouronium salts see: T. B. Johnson and J. M. Sprague, *J. Am. Chem. Soc., 58*, 1348 (1936); *ibid., 59*, 1837, 2439 (1937); *ibid., 61*, 176 (1939)]. Amination of XXIXc wil give the sulfonamides of Formula XXIXd.

Equation 24

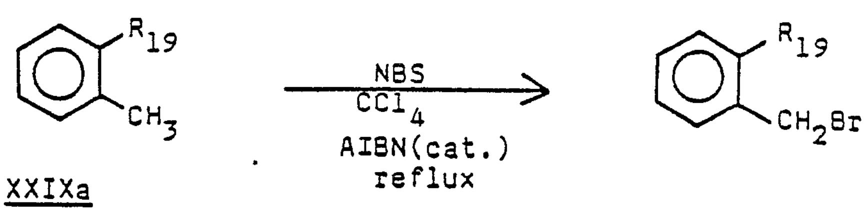


XXIXa

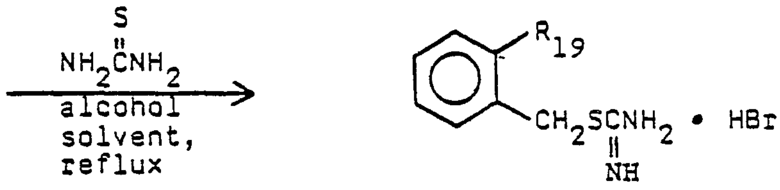


XXIXb

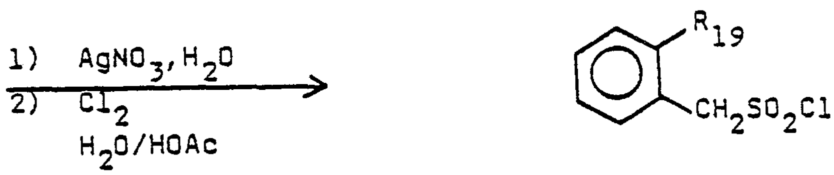


XXIXc

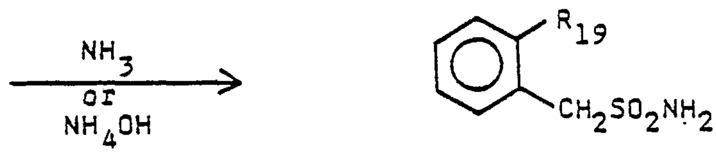


XXIXd

Alternatively, the benzyl chlorides of Formula XXIXe can be converted to the benzylsulfonamides of Formula XXIXd as shown in Equation 25.

Equation 25

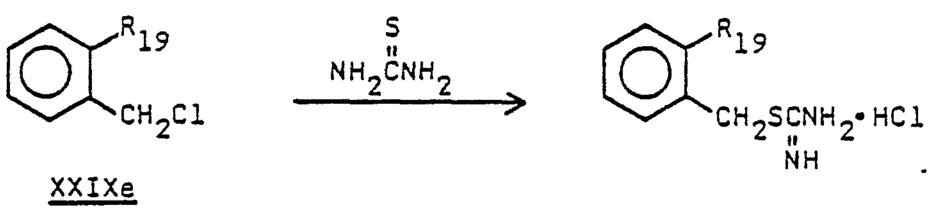


XXIXe

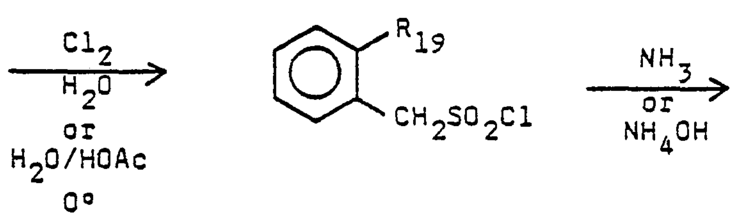


XXIXd

Phosgenation of XXIXd to the corresponding benzylsulfonylisocyanates of Formula XXIXf proceeds readily as shown in Equation 26. (See Equation 3 for complete details.)

Equation 26

XXIXd + $n$-$C_4H_9NCO$

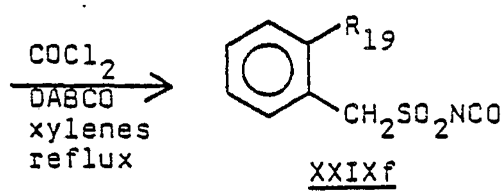


XXIXf

Preparations of 3-amino-1,2,4-triazoles are known in the art and 1,2,4-triazoles are reviewed in *The Chemistry of Heterocyclic Compounds* "Triazoles 1,2,4" (John Wiley & Sons, New York, 1981). Commonly used starting materials containing nitrogen are N-aminoguanidine, hydrazine, alkylhydrazines, cyanamide, ethyl cyanoacetimidate, dimethyl cyanodithioimidocarbonate, dimethyl cyanoimidocarbonate, ethoxy-

methylenecyanamide, and acylhydrazines. Some literature syntheses are illustrated below. Using these techniques or suitable modifications that would be apparent to one skilled in the art, the 3-amino-1,2,4-triazole intermediates can be readily prepared.

Heating equimolar amounts of ethyl propionimidate hydrochloride and N-aminoguanidine nitrate in pyridine gives 3-amino-ethyltriazole; *German Patent* 1,073,499 (1960); *Berichte, 96,* 1064 (1963).

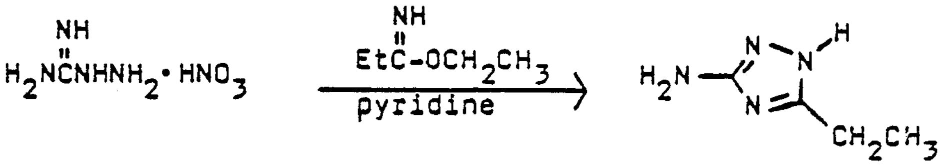


Condensation of hydrazine with ethyl N-cyanoacetimidate yields 3-amino-5-methyltriazole; *Journal of Organic Chemistry, 28,* 1816 (1963).

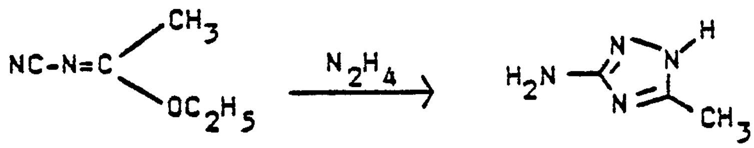


Trifluoromethyl 3-aminotriazole can be obtained by thermal dehydration of the hydrazide of trifluoroacetic acid. *Zh. Obshch. Khim., 39,* 2525 (1969); *Chemical Abstracts, 72:* 78954v (1970).

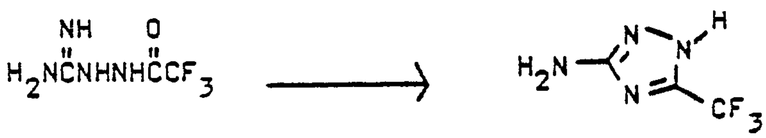

U.S. Patent 2,835,581 (1958) teaches the preparation of 3-amino-5-(hydroxymethyl)triazole from N-aminoguanidine and glycolic acid and *British Patent* 736,568 (1955) describes the synthesis of 3-amino-5-mercaptotriazole.

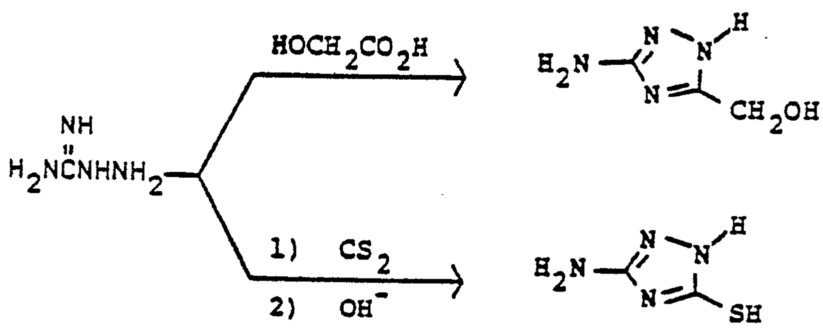


Condensing hydrazine with dimethyl cyanodithioimidocarbonate in acetonitrile gives 3-amino-5-methylthio-1,2,4-triazole while reaction of hydrazine with dimethyl N-cyanoimidocarbonate produces 3-amino-5-methoxy-1,2,4-triazole; *Journal of Organic Chemistry, 39,* 1522 (1974).

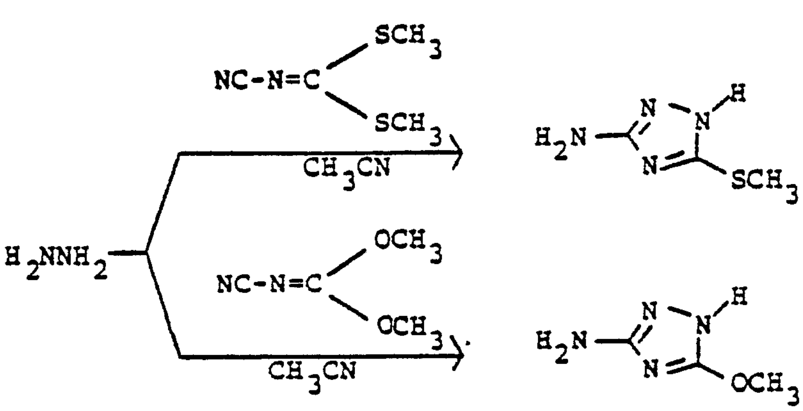

Reaction of substituted hydrazines with N-cyanothioimidocarbonates (prepared according to the procedure given in D. M. Wieland, Ph.D. Thesis, 1971, pp. 123—124) yields disubstituted aminotriazoles as shown below.

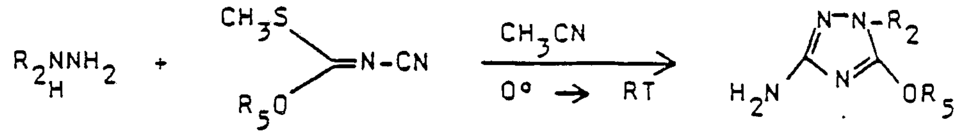


The following examples teach the preparation of compounds of this invention in more detail. Unless otherwise indicated, all parts are by weight and temperatures in °C.

Example I

2-[[(5-Methylthio-1H-1,2,4-triazol-3-yl)aminocarbonyl]aminosulfonyl]benzoic acid, methyl ester

To a mixture of 5.0 grams (0.0385 mole) 3-amino-5-methylthio-1H-1,2,4-triazole (Aldrich Chemicals) and 75 ml of dry methylene chloride stirring in a 200 ml RB single neck flask, 10.5 grams of 2-(methoxycarbonyl)benzenesulfonylisocyanate was added at ambient temperature; a clear solution gradually formed. After stirring overnight at room temperature, the white solid which precipitated was filtered and washed with methylene chloride, yield 11.7 grams, m.p. 147—153°.

NMR (tfa-D): δ 2.85 (s, 3H, $SCH_3$), 4.10 (s, 3H, $CO_2CH_3$), 7.60—8.55 (m, ArH). IR (Nujol): 3.0—3.20 (NH), 5.75 (C=O), 6.20 (C=N) microns.

Example II

2-[[(1-Methyl-5-methylthio-1H-1,2,4-triazol-3-yl)aminocarbonyl]aminosulfonyl]benzoic acid, methyl ester

Method A: Methyl iodide (0.6 ml) was added by syringe at ambient temperature to a stirred mixture of 0.8 grams (0.0022 mole) of methyl 2-[(5-methylthio-1H-1,2,4-triazol-3-yl)aminocarbonyl]aminosulfonylbenzoate and 0.6 grams of potassium carbonate in 30 ml of acetone in a 100 ml RB single neck flask. The mixture was stirred at room temperature overnight. The reaction mixture was then poured into 250 ml of ice water and acidified to pH 3—4 with glacial acetic acid. After extracting the aqueous mixture with 1:1 mixture of ethyl acetate and ethyl ether, the organic layer was washed with brine dried ($MgSO_4$), filtered, and the solvent evaporated to dryness to yield a white solid residue which was suspended in acetonitrile, filtered, and recrystallized from the acetonitrile; yield 0.3 g, m.p. 198—200°.

NMR (tfa-D): δ 2.95 (s, 3H, $SCH_3$), 4.0 (s, 3H, $CH_3$), 4.15 (s, 3H, $CO_2CH_3$), 7.80—8.60 (m, ArH). IR (Nujol): 5.75—5.85 (C=O), 6.30 (C=N), 13.10, 13.55 microns.

Method B: To 1.0 gram (0.0069 mole) of 3-amino-1-methyl-5-methylthio-1H-1,2,4-triazole stirring in 15 ml of dry methylene chloride, 3.0 grams of 2-(methoxycarbonyl)benzenesulfonylisocyanate was added at ambient temperature and a cloudy solution immediately formed followed by precipitation of a white solid. The white suspension was stirred at room temperature overnight. Chlorobutane (4 ml) was added to the mixture and the white solid filtered, washed with methylene chloride followed by a chlorobutane wash, yield 2.0 g, m.p. 198—200° (recrystallized from acetonitrile). The spectral characteristics and melting points were identical to the solid obtained in Method A. A mixed melting point was not depressed.

Example III

3-Amino-1-methyl-5-methylthio-1H-1,2,4-triazole

At 0°, 6.5 grams of methyl hydrazine was added dropwise to a stirred suspension of 20.0 grams (0.137 mole) dimethyl cyanodithioimidocarbonate in 35 ml of acetonitrile. A yellow solution immediately formed after the addition followed by precipitation of a white solid. The ice bath was removed, and upon warming to room temperature a yellow solution formed which was stirred at room temperature overnight. After stirring overnight, a solid precipitated which was filtered, washed with cold acetonitrile, followed by 1-chlorobutane, yield 8.5 grams, m.p. 104—109°. The literature [*Journal of Organic Chemistry, 39,* 1522 (1974)] reported a similar melting point of 103—106° for the title compound, however, a different isomeric structure was assigned to the compound (5-amino-1-methyl-3-methylthio-1H-1,2,4-triazole). The assignment of 3-amino-1-methyl-5-methylthio-1H-1,2,4-triazole as the correct structure was verified by X-ray crystal structure analysis.

Example IV

2-[[(1,5-Dimethyl-1H-1,2,4-triazol-3-yl)aminocarbonyl]aminosulfonyl]benzoic acid, methyl ester

In 20 ml of acetonitrile at room temperature 0.63 grams of 3-amino-1,5-dimethyl-1H-1,2,4-triazole [*Journal of Organic Chemistry, 39,* 1522 (1974)] and 1.49 grams of 2-(methoxycarbonyl)benzenesulfonylisocyanate were stirred for 12 hours. The white solid which precipitated was filtered and washed with ether to yield 1.3 grams of the title product melting at 175—178°.

Example V

2-[[(1-Methyl-5-methylthio-1H-1,2,4-triazol-3-yl)aminocarbonyl]aminosulfonyl]benzoic acid, 2-propyl ester

A mixture of 0.7 grams (0.00486 mole) of 3-amino-1-methyl-5-methylthio-1H-1,2,4-triazole and 4.0 grams of 2-(isopropoxycarbonyl)benzenesulfonylisocyanate was stirred in 12 ml of dry methylene chloride at ambient temperature overnight. After addition of 4 ml of 1-chlorobutane, a white solid precipitated which was filtered, washed with 1-chlorobutane and dried, yield 1.3 grams, m.p. 192—194°.

NMR (tfa-D): δ 1.55 (d, 6H, $2CH_3$), 2.90 (s, 3H, $SCH_3$), 3.95 (s, 3H, N—$CH_3$), 4.90—5.40 (m, 1H, $CO_2CH$), 7.60—8.50 (m's, ArH). IR (Nujol): 2.95 (NH), 5.75 (C=O), 6.30 (C=N), 13.10, 13.50 microns.

Example VI

2-[(1-Methyl-5-methylthio-1H-1,2,4-triazol-3-yl)aminocarbonyl]-2-nitrobenzenesulfonamide

After the addition of 3.0 grams 2-nitrobenzenesulfonylisocyanate to 0.7 grams (0.00486 mole) 3-amino-1-methyl-5-methylthio-1H-1,2,4-triazole stirring in 15 ml of dry methylene chloride, a solid precipitated and the suspension was stirred at room temperature overnight. The solid was filtered and washed with 1-chlorobutane to yield 1.6 grams of product, m.p. 210—213°.

NMR (tfa-D): δ 2.90 (s, 3H, $SCH_3$), 4.00 (s, 3H, N—$CH_3$), 7.85—8.60 (m, ArH). IR (Nujol): 3.0—3.20 (NH), 5.80 (C=O), 6.25 (C=N), 12.70, 13.45, 13.60 microns.

Example VII

2-[[(1-Ethyl-5-methylthio-1H-1,2,4-triazol-3-yl)aminocarbonyl]aminosulfonyl]benzoic acid, methyl ester

To a stirred mixture of 1.0 gram (0.0027 mole) of methyl 2-[(5-methylthio-1H-1,2,4-triazol-3-yl)aminocarbonyl]aminosulfonylbenzoate and 0.7 grams of potassium carbonate in 30 ml of acetone at room temperature, 0.8 ml of ethyl iodide was added by way of syringe and the resulting white suspension stirred 3 days. The mixture was poured into 250 ml of ice water and acidified to pH 3—4 with glacial acetic acid. The aqueous acidic mixture was extracted with 1:1 mixture of ethyl ether/ethyl acetate and the extract washed with brine, dried ($MgSO_4$), and evaporated to yield a white solid residue which was suspended in acetonitrile and filtered, yield 0.3 grams, m.p. 188—191°.

NMR (tfa-D): δ 1.45 (t, 3H, $CH_3$), 2.80 (s, 3H, $SCH_3$), 4.05 (s, 3H, $CO_2CH_3$), 4.25 (q, 2H, $CH_2$), 7.60—8.45 (m, ArH).

Example VIII

3-Amino-5-methoxy-1-methyl-1H-1,2,4-triazole

Methylhydrazine (98%, 6.30 g, 0.136 mol) was added dropwise to a solution of dimethyl-N-cyanothioimidocarbonate (17.3 g, 0.133 mol, prepared according to procedure given in D. M. Wieland, Ph. D. Thesis, 1971, pp. 123—124.) in acetonitrile (35 ml) at -5-0° with stirring under nitrogen. The reaction mixture was allowed to warm to room temperature and stirred for 2 days. Isolation of the product by filtration and washing with acetonitrile gave 12.7 g of a white powder, m.p. 177—180°.

NMR (DSO/$CDCl_3$): δ 3.35 (s, N—$CH_3$), 3.95 (s, O—$CH_3$); and 5.0 (broad, $NH_2$).

Example IX

2-[[(5-Methoxy-1-methyl-1H-1,2,4-triazol-3-yl)aminocarbonyl]aminosulfonyl]benzoic acid, methyl ester

To a solution of 2-(methoxycarbonyl)benzenesulfonylisocyanate (80%, 1.41 g, 4.7 mmol) in methylene chloride (10 ml) at room temperature under nitrogen was added 3-amino-5-methoxy-1-methyl-1H-1,2,4-triazole (0.50 g, 3.9 mmol). DABCO (catalytic amount) was added, and the reaction mixture was stirred for 3 days. Isolation of the product by filtration and washing with methylene chloride gave 1.05 g of a white powder, m.p. 177—180°.

NMR ($CDCl_3$/DMSO): δ 3.5 (s, N—$CH_3$); 3.9 (s, $CO_2CH_3$); 4.1 (s, O—$CH_3$); 7.5—7.8 (m, Ar-H); 8.1—8.4 (m, Ar-H); 9.7 (broad, NH); and 11.0 (broad, NH).

IR (KBr) 3150 (NH), 1740 (c=o), 1700 (c=o), 1600, 1530, 1475, 1420, 1360 ($SO_2$), 1340, 1295, 1260, 1180 ($SO_2$), 1115 and 1050 $cm^{-1}$.

By application of one or more of the procedures of Examples I—IX and/or the methods described above and using the appropriate reactants, the compunds of Tables 1 to 18 can be prepared.

Table 1

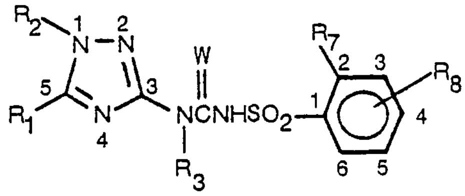

| $R_1$ | $R_2$ | $R_3$ | W | $R_7$ | $R_8$ | m.p.(°C) |
|---|---|---|---|---|---|---|
| H | $n-C_3H_7$ | H | O | $CO_2CH_3$ | H | |
| H | $C_2H_5$ | H | O | $CO_2C_2H_5$ | H | |
| H | $CH(CH_3)_2$ | H | O | $SO_2CH_3$ | H | |
| $CH_3$ | $C_2H_5$ | H | O | $CO_2CH_3$ | H | |
| $CH_3$ | $C_2H_5$ | H | S | Cl | H | |
| $CH_3$ | $C_2H_5$ | $CH_3$ | O | $CO_2CH_3$ | H | |
| $CH_3$ | $C_2H_5$ | $CH_3$ | O | $SO_2CH_3$ | H | |
| $CH_3$ | $C_2H_5$ | H | O | $CF_3$ | H | |
| $CH_3$ | $C_2H_5$ | H | O | Br | H | |
| $CH_3$ | $C_2H_5$ | H | O | Cl | H | |
| $CH_3$ | $C_2H_5$ | H | S | $CH_3$ | H | |
| $CH_3$ | $C_2H_5$ | H | O | $C_2H_5$ | H | |
| $CH_3$ | $C_2H_5$ | H | O | $NO_2$ | H | |
| $CH_3$ | $C_2H_5$ | H | O | $SO_2(CH_2)_2CH_3$ | H | |
| $CH_3$ | $C_2H_5$ | H | O | $SO_2N(CH_3)_2$ | H | |
| $CH_3$ | $C_2H_5$ | $CH_3$ | O | $NO_2$ | H | |
| $CH_3$ | $C_2H_5$ | H | O | $OCH_3$ | H | |
| $OCH_3$ | $C_2H_5$ | H | O | $CO_2CH(CH_3)_2$ | H | |
| $CH_3$ | $C_2H_5$ | H | O | $CO_2CH_2CH=CH_2$ | H | |
| $OCH_3$ | $C_2H_5$ | H | O | $OCF_2CF_2H$ | H | |
| $CH_3$ | $C_2H_5$ | H | O | $CH_2OCH_3$ | H | |
| $OCH_3$ | $C_2H_5$ | H | O | $OSO_2CHCl_2$ | H | |
| $CH_3$ | $C_2H_5$ | H | O | $CF_3$ | H | |
| $CH_3$ | $C_2H_5$ | H | O | $SC_2H_5$ | H | |
| $OCH_3$ | $C_2H_5$ | H | O | $OSO_2C_2H_5$ | H | |

Table 1 (continued)

| $R_1$ | $R_2$ | $R_3$ | W | $R_7$ | $R_8$ | m.p.(°C) |
|---|---|---|---|---|---|---|
| $OCH_3$ | $C_2H_5$ | H | O | $OSO_2CH_2CF_3$ | H | |
| $CH_3$ | $C_2H_5$ | H | O | $CO_2CH_2CH_2Cl$ | H | |
| $OCH_3$ | $C_2H_5$ | H | O | $OSO_2CH_2CH_2OCH_3$ | H | |
| $OC_2H_5$ | $CH_3$ | H | O | $SO_2N(OCH_3)CH_3$ | H | |
| $C_2H_5$ | $CH_3$ | H | O | $CO_2CH_3$ | H | 165-166° |
| $C_2H_5$ | $CH_3$ | H | O | Br | H | |
| $C_2H_5$ | $CH_3$ | H | O | $NO_2$ | H | |
| $C_2H_5$ | $CH_3$ | $CH_3$ | O | $CO_2CH_3$ | H | |
| $C_2H_5$ | $CH_3$ | $CH_3$ | O | $SO_2CH_3$ | H | |
| $OC_2H_5$ | $CH_3$ | $CH_3$ | O | $CO_2CH_2CH_2OCH_3$ | H | |
| $OC_2H_5$ | $CH_3$ | $CH_3$ | O | $OSO_2CH_2(CH_2)_2OCH_3$ | H | |
| $C_2H_5$ | $CH_3$ | H | S | $CH_3$ | H | |
| $C_2H_5$ | $CH_3$ | H | S | Cl | H | |
| $C_2H_5$ | $CH_3$ | $CH_3$ | O | $CH_3$ | H | |
| $C_2H_5$ | $CH_3$ | H | O | $CO_2CH(CH_3)_2$ | H | |
| $C_2H_5$ | $CH_3$ | H | O | Cl | H | |
| $C_2H_5$ | $CH_3$ | H | O | $CO_2CH_2CH{=}CH_2$ | H | |
| $OC_2H_5$ | $CH_3$ | H | O | $OSO_2CH_2CF_2H$ | H | |
| $OC_2H_5$ | $CH_3$ | H | O | $OSO_2CCl_3$ | H | |
| $OC_2H_5$ | $CH_3$ | H | O | $OSO_2CHCl_2$ | H | |
| $C_2H_5$ | $CH_3$ | H | O | $OSO_2CH_3$ | H | |
| $C_2H_5$ | $CH_3$ | H | O | $CH_2OC_2H_5$ | H | |
| $C_2H_5$ | $CH_3$ | H | O | $SO_2C_2H_5$ | H | |
| $OC_2H_5$ | $CH_3$ | H | O | $OSO_2CF_3$ | H | |
| $C_2H_5$ | $CH_3$ | H | O | $OCF_3$ | H | |
| $OC_2H_5$ | $CH_3$ | H | O | $OCH_3$ | H | |
| $C_2H_5$ | $CH_3$ | H | O | $NO_2$ | 5-$CF_3$ | |
| $C_2H_5$ | $CH_3$ | H | O | $NO_2$ | 3-Cl | |
| $C_2H_5$ | $CH_3$ | H | O | Cl | 6-$NO_2$ | |
| $C_2H_5$ | $CH_3$ | H | O | $CF_3$ | 5-$NO_2$ | |
| $CH_3$ | $C_2H_5$ | H | O | Cl | 5-Cl | |
| $OCH_3$ | $C_2H_5$ | H | O | $NO_2$ | 5-Cl | |

Table 1 (continued)

| $R_1$ | $R_2$ | $R_3$ | W | $R_7$ | $R_8$ | m.p.(°C) |
|---|---|---|---|---|---|---|
| $CH_3$ | $C_2H_5$ | H | O | $SO_2N(CH_3)_2$ | 3-Cl | |
| $OC_2H_5$ | $CH_3$ | H | O | $CO_2CH_3$ | 5-$C_2H_5$ | |
| $C_2H_5$ | $CH_3$ | H | O | Cl | 3-Br | |
| $OC_2H_5$ | $CH_3$ | H | O | Cl | 5-Br | |
| $C_2H_5$ | $CH_3$ | H | O | $NO_2$ | 6-F | |
| $OC_2H_5$ | $CH_3$ | H | O | Cl | 5-F | |
| $C_2H_5$ | $CH_3$ | H | O | Cl | 4-F | |
| $OC_2H_5$ | $CH_3$ | H | O | Br | 3-F | |
| $C_2H_5$ | $CH_3$ | H | O | $NO_2$ | 5-$NO_2$ | |
| $C_2H_5$ | $CH_3$ | H | O | $CO_2CH_3$ | 3-$CF_3$ | |
| $OC_2H_5$ | $CH_3$ | H | O | $CH_3$ | 3-$NO_2$ | |
| $C_2H_5$ | $C_2H_5$ | H | O | $CO_2CH_3$ | H | |
| $C_2H_5$ | $CH_3$ | H | O | $CO_2C_2H_5$ | H | |
| $C_2H_5$ | $C_2H_5$ | H | S | Cl | H | |
| $C_2H_5$ | $C_2H_5$ | H | O | $NO_2$ | H | |
| $C_2H_5$ | $C_2H_5$ | H | S | $CH_3$ | H | |
| $C_2H_5$ | $C_2H_5$ | H | O | $CO_2CH_2CH{=}CH_2$ | H | |
| $C_2H_5$ | $C_2H_5$ | H | O | $CO_2CH(CH_3)_2$ | H | |
| $C_2H_5$ | $C_2H_5$ | H | O | $SO_2CH_3$ | H | |
| $C_2H_5$ | $C_2H_5$ | H | O | $SO_2(CH_2)_2CH_3$ | H | |
| $OC_2H_5$ | $C_2H_5$ | H | O | $SO_2CHF_2$ | H | |
| $C_2H_5$ | $C_2H_5$ | H | O | $SO_2N(CH_3)_2$ | H | |
| $C_2H_5$ | $C_2H_5$ | H | O | $SO_2N(OCH_3)CH_3$ | H | |
| $OC_2H_5$ | $C_2H_5$ | H | O | $OSO_2CH_3$ | H | |
| $C_2H_5$ | $C_2H_5$ | H | O | $(CH_2)_2CH_3$ | H | |
| $C_2H_5$ | $C_2H_5$ | H | O | $OC_2H_5$ | H | |
| $C_2H_5$ | $C_2H_5$ | H | O | $SCH_3$ | H | |
| $CH(CH_3)_2$ | $CH_3$ | H | O | $NO_2$ | H | |
| $n$-$C_3H_7$ | $CH_3$ | H | O | Cl | H | |
| $CH_2CH(CH_3)_2$ | $CH_3$ | H | O | $CO_2CH_3$ | H | |
| $CH_2CH{=}CH_2$ | $CH_3$ | H | O | $CF_3$ | H | |
| $CH_2C(CH_3){=}CH_2$ | $CH_3$ | H | O | $CO_2CH_3$ | H | |

Table 1 (continued)

| $R_1$ | $R_2$ | $R_3$ | W | $R_7$ | $R_8$ | m.p.(°C) |
|---|---|---|---|---|---|---|
| $CH_2C{\equiv}CH$ | $CH_3$ | H | O | $CO_2CH_3$ | H | |
| $CH_2C{\equiv}CCH_3$ | $CH_3$ | H | O | $CO_2CH_3$ | H | |
| $OCH_3$ | $CH_2CH{=}CH_2$ | H | O | $CO_2C_2H_5$ | H | |
| $CH_3$ | $CH_2C(CH_3){=}CH_2$ | H | O | $SO_2CH_3$ | H | |
| $CH_3$ | $CH_2CH{=}CHCH_3$ | H | O | $CO_2CH_3$ | H | |
| $CH_3$ | $CH_2C{\equiv}CCH_3$ | H | O | $CO_2CH_3$ | H | |
| $OCH_3$ | $CH_2C{\equiv}CH$ | H | O | $NO_2$ | H | |
| $SCH_3$ | $CH_3$ | $CH_3$ | O | $CO_2CH_3$ | H | |
| $SCH_3$ | $CH_3$ | H | O | $SO_2CH_3$ | H | 208-213° |
| $SCH_3$ | $CH_3$ | $CH_3$ | O | $NO_2$ | H | |
| $SCH_3$ | $CH_3$ | $CH_3$ | O | $SO_2N(CH_3)_2$ | H | |
| $SCH_3$ | $CH_3$ | $CH_3$ | O | $SO_2N(CH_3)C_2H_5$ | H | |
| $SCH_3$ | $CH_3$ | H | S | $CH_3$ | H | |
| $SCH_3$ | $CH_3$ | H | S | $NO_2$ | H | |
| $SCH_3$ | $CH_3$ | H | O | Cl | H | 230-237° |
| $SCH_3$ | $CH_3$ | H | O | $OSO_2CH_3$ | H | |
| $SCH_3$ | $CH_3$ | H | O | $CO_2CH_2CH_2CH_3$ | H | |
| $SCH_3$ | $CH_3$ | H | O | $CO_2CH_2CH{=}CH_2$ | H | 160-163° |
| $SCH_3$ | $CH_3$ | H | O | $CO_2CH_2CH_2Cl$ | H | |
| $SCH_3$ | $CH_3$ | H | O | $CO_2CH_2CH_2OCH_3$ | H | |
| $SCH_3$ | $CH_3$ | H | O | $CO_2CH(CH_3)C_2H_5$ | H | |
| $SCH_3$ | $CH_3$ | H | O | $CO_2(CH_2)_3CH_3$ | H | |
| $SCH_3$ | $CH_3$ | H | O | $OSO_2(CH_2)_3CH_3$ | H | |
| $SCH_3$ | $CH_3$ | H | O | $OSO_2(CH_2)_3OCH_3$ | H | |
| $SCH_3$ | $CH_3$ | H | O | $CH_2OCH_3$ | H | |
| $SCH_3$ | $CH_3$ | H | O | $OSO_2CH_2CF_2H$ | H | |
| $SCH_3$ | $CH_3$ | H | O | H | H | |
| $SCH_3$ | $CH_3$ | H | O | F | H | |
| $SCH_3$ | $CH_3$ | H | O | H | 4-F | |
| $SCH_3$ | $CH_3$ | H | O | H | 5-F | |
| $SCH_3$ | $CH_3$ | H | O | $OSO_2CH_2CCl_3$ | H | |
| $SCH_3$ | $CH_3$ | H | O | $OSO_2CH_2CHCl_2$ | H | |
| $SCH_3$ | $CH_3$ | $CH_3$ | O | $SO_2CH_3$ | H | |

Table 1 (continued)

| $R_1$ | $R_2$ | $R_3$ | W | $R_7$ | $R_8$ | m.p.(°C) |
|---|---|---|---|---|---|---|
| $SCH_3$ | $CH_3$ | H | O | $OSO_2CF_3$ | H | |
| $SCH_3$ | $CH_3$ | H | O | $OSO_2CHCl_2$ | H | |
| $SCH_3$ | $CH_3$ | H | O | $SO_2(CH_2)_2CH_3$ | H | |
| $SCH_3$ | $CH_3$ | H | O | $SO_2N(CH_3)_2$ | H | |
| $SCH_3$ | $CH_3$ | H | O | $SO_2N(C_2H_5)_2$ | H | |
| $SCH_3$ | $CH_3$ | H | O | $SO_2N(CH_3)[CH(CH_3)_2]$ | H | |
| $SCH_3$ | $CH_3$ | H | O | $SO_2CH(CH_3)_2$ | H | |
| $SCH_3$ | $CH_3$ | H | O | $CH_2OC_2H_5$ | H | |
| $SCH_3$ | $CH_3$ | H | O | $C_2H_5$ | H | |
| $SCH_3$ | $CH_3$ | H | O | $CF_3$ | H | |
| $SCH_3$ | $CH_3$ | H | O | Cl | 5-Br | |
| $SCH_3$ | $CH_3$ | H | O | Cl | 6-Cl | |
| $SCH_3$ | $CH_3$ | H | O | $NO_2$ | 5-Cl | |
| $SCH_3$ | $CH_3$ | H | O | $CO_2CH_3$ | 3-Br | |
| $SCH_3$ | $CH_3$ | H | O | $SO_2CH_3$ | 4-$OCH_3$ | |
| $SCH_3$ | $CH_3$ | H | O | $CO_2CH_3$ | 4-$OCH_3$ | |
| $SCH_3$ | $CH_3$ | H | O | Cl | 5-$NO_2$ | |
| $SCH_3$ | $CH_3$ | H | O | $SO_2N(CH_3)_2$ | 5-$NO_2$ | |
| $SCH_3$ | $CH_3$ | H | O | Cl | 5-$CH(CH_3)_2$ | |
| $SCH_3$ | $CH_3$ | H | O | $NO_2$ | 5-$C_2H_5$ | |
| $SCH_3$ | $CH_3$ | H | O | $NO_2$ | 5-$CF_3$ | |
| $SCH_3$ | $CH_3$ | H | O | $NO_2$ | 3-$CF_3$ | |
| $SCH_3$ | $CH_3$ | H | O | $SCF_3$ | H | |
| $SCH_3$ | $CH_3$ | H | O | $OCF_3$ | H | |
| $SCH_3$ | $CH_3$ | H | O | $NO_2$ | 6-F | |
| $SCH_3$ | $CH_3$ | H | O | Cl | 5-$OC_2H_5$ | |
| $SCH_3$ | $CH_3$ | H | O | Br | 5-$NO_2$ | |
| $SCH_3$ | $CH_3$ | H | O | $NO_2$ | 3-F | |
| $SCH_3$ | $CH_3$ | H | O | Cl | 5-F | |
| $SCH_3$ | $CH_3$ | H | O | $NO_2$ | 6-F | |
| $SCH_3$ | $CH_3$ | H | O | Cl | 4-F | |
| $SCH_3$ | $CH_3$ | H | O | $OSO_2CHF_2$ | H | |

Table 1 (continued)

| $R_1$ | $R_2$ | $R_3$ | W | $R_7$ | $R_8$ | m.p.(°C) |
|---|---|---|---|---|---|---|
| $SCH_3$ | $CH_3$ | H | O | $OSO_2(CH_2)_3Br$ | H | |
| $SC_2H_5$ | $CH_3$ | H | O | $CO_2CH_3$ | H | 205-208° |
| $SC_2H_5$ | $CH_3$ | H | O | $SO_2CH_3$ | H | |
| $SC_2H_5$ | $CH_3$ | $CH_3$ | O | $SO_2CH_3$ | H | |
| $SC_2H_5$ | $CH_3$ | H | O | $NO_2$ | H | 186-190° |
| $SC_2H_5$ | $CH_3$ | H | S | Cl | H | |
| $SC_2H_5$ | $CH_3$ | H | S | $CH_3$ | H | |
| $SC_2H_5$ | $CH_3$ | H | O | $CF_3$ | H | |
| $SC_2H_5$ | $CH_3$ | H | O | $CO_2CH(CH_3)_2$ | H | 152-156° |
| $SC_2H_5$ | $CH_3$ | H | O | $CO_2CH_2CH{=}CH_2$ | H | 172-174° |
| $SC_2H_5$ | $CH_3$ | $CH_3$ | O | $NO_2$ | H | |
| $SC_2H_5$ | $CH_3$ | H | O | $CH_2OCH_3$ | H | |
| $SC_2H_5$ | $CH_3$ | H | O | $SO_2C_2H_5$ | H | |
| $SC_2H_5$ | $CH_3$ | H | O | $SO_2N(CH_3)C_2H_5$ | H | |
| $SC_2H_5$ | $CH_3$ | H | O | Br | H | |
| $SC_2H_5$ | $CH_3$ | H | O | $OCF_3$ | H | |
| $SCH_3$ | $C_2H_5$ | H | O | $CO_2CH_3$ | H | |
| $SCH_3$ | $C_2H_5$ | H | O | Cl | H | |
| $SCH_3$ | $C_2H_5$ | H | O | $NO_2$ | H | |
| $SCH_3$ | $C_2H_5$ | H | S | Br | H | |
| $SCH_3$ | $C_2H_5$ | H | S | $CH_3$ | H | |
| $SCH_3$ | $C_2H_5$ | H | O | $CF_3$ | H | |
| $SCH_3$ | $C_2H_5$ | H | O | $OCH_3$ | H | |
| $SCH_3$ | $C_2H_5$ | H | O | $CO_2(CH_2)_2CH_3$ | H | |
| $SCH_3$ | $C_2H_5$ | H | O | $OSO_2(CH_2)_2CH_3$ | H | |
| $SCH_3$ | $C_2H_5$ | H | O | $SO_2N(CH_3)C_2H_5$ | H | |
| $SCH_3$ | $C_2H_5$ | H | O | $SO_2N(OCH_3)CH_3$ | H | |
| $SCH_3$ | $C_2H_5$ | H | O | $SO_2(CH_2)_2CH_3$ | H | |
| $SCH_3$ | $C_2H_5$ | H | O | $OCHF_2$ | H | |
| $SCH_3$ | $C_2H_5$ | H | O | $CH(CH_3)_2$ | H | |

Table 1 (continued)

| $R_1$ | $R_2$ | $R_3$ | W | $R_7$ | $R_8$ | m.p. (°C) |
|---|---|---|---|---|---|---|
| $SCH_3$ | $C_2H_5$ | H | O | $SCF_3$ | H | |
| $SCH_3$ | $C_2H_5$ | H | O | $OSO_2(CH_2)_2OCH_3$ | H | |
| $SCH_3$ | $C_2H_5$ | H | O | $SC_2H_5$ | H | |
| $S(CH_2)_2CH_3$ | $CH_3$ | H | O | Cl | H | |
| $S(CH_2)_2CH_3$ | $CH_3$ | H | O | $CO_2CH_3$ | H | |
| $SCH(CH_3)_2$ | $CH_3$ | H | O | $CO_2C_2H_5$ | H | |
| $S(CH_2)_3CH_3$ | $CH_3$ | H | O | $SO_2CH_3$ | H | |
| $SCH(CH_3)C_2H_5$ | $CH_3$ | H | O | $CO_2C_2H_5$ | H | |
| $SCH_2CH{=}CH_2$ | $CH_3$ | H | O | $NO_2$ | H | |
| $SCH_2C(CH_3){=}CH_2$ | $CH_3$ | H | O | $CO_2CH_3$ | H | |
| $SCH_2CH{=}CHCH_3$ | $CH_3$ | H | O | $SO_2CH_3$ | H | |
| $SCH_2CO_2CH_3$ | $CH_3$ | H | O | Cl | H | |
| $SCH_2CO_2(CH_2)_3CH_3$ | $CH_3$ | H | O | Br | H | |
| $SCH(CH_3)CO_2CH_3$ | $CH_3$ | H | O | $NO_2$ | H | |
| $SCH_2C{\equiv}CH$ | $CH_3$ | H | O | $CO_2CH_3$ | H | |
| $SCH_2C{\equiv}CCH_3$ | $CH_3$ | H | O | $CO_2CH_3$ | H | |
| $CH_3$ | $CH_2OCH_3$ | H | O | $CO_2C_2H_5$ | H | |
| $CH_3$ | $CH_2OCH_3$ | H | O | $SO_2C_2H_5$ | H | |
| $CH_3$ | $CH_2SCH_3$ | H | O | $CH_2OCH_3$ | H | |
| $CH_3$ | $CH_2CH_2OCH_3$ | H | O | Cl | H | |
| $CH_3$ | $CH_2CH_2SCH_3$ | H | O | $SO_2CH_3$ | H | |
| $CH_2OC_2H_5$ | $CH_3$ | H | O | $CH_3$ | H | |
| $CH_2OC_2H_5$ | $CH_3$ | H | O | $CO_2CH(CH_3)_2$ | H | |
| $CH_2OC_2H_5$ | $CH_3$ | $CH_3$ | O | $CO_2CH_3$ | H | |
| $CH_2OCH(CH_3)_2$ | $CH_3$ | H | O | Br | H | |
| $CH_2O(CH_2)_3CH_3$ | $CH_3$ | H | O | $SO_2C_2H_5$ | H | |
| $CH_2CH_2OCH_3$ | $CH_3$ | H | O | $NO_2$ | H | |
| $CH_2CH_2OC_2H_5$ | $CH_3$ | H | O | $CO_2CH_3$ | H | |
| $CH_2CH_2O(CH_2)_2CH_3$ | $CH_3$ | H | O | $CO_2CH_3$ | H | |
| $OCH_3$ | $CH_3$ | H | S | Cl | H | |
| $OCH_3$ | $C_2H_5$ | H | S | $NO_2$ | H | |

Table 1 (continued)

| $R_1$ | $R_2$ | $R_3$ | W | $R_7$ | $R_8$ | m.p.(°C) |
|---|---|---|---|---|---|---|
| $OCH_3$ | $C_2H_5$ | H | S | $CH_3$ | H | |
| $OCH_3$ | $CH_3$ | H | S | $CF_3$ | H | |
| $OCH_3$ | $CH_3$ | H | S | Br | H | |
| $OCH_3$ | $CH_3$ | $CH_3$ | O | $CO_2CH_3$ | H | |
| $OCH_3$ | $CH_3$ | $CH_3$ | O | $SO_2CH_3$ | H | |
| $OCH_3$ | $CH_3$ | $CH_3$ | O | $SCH_3$ | H | |
| $OCH_3$ | $CH_3$ | H | O | $OSO_2(CH_2)_3OCH_3$ | H | |
| $OCH_3$ | $C_2H_5$ | H | O | $OSO_2CH_2CHCl_2$ | H | |
| $OCH_3$ | $C_2H_5$ | H | O | $OSO_2CH_2CF_2H$ | H | |
| $OCH_3$ | $C_2H_5$ | H | O | $OCF_3$ | H | |
| $OCH_3$ | $C_2H_5$ | H | O | $SCHF_2$ | H | |
| $OCH_3$ | $C_2H_5$ | H | O | $OSO_2CH(CH_3)_2$ | H | |
| $OC_2H_5$ | $CH_3$ | H | O | H | H | |
| $OC_2H_5$ | $CH_3$ | H | O | F | H | |
| $OC_2H_5$ | $CH_3$ | H | O | $CO_2CH_3$ | 3-Br | |
| $OC_2H_5$ | $CH_3$ | H | O | $SO_2CH_3$ | H | 190-208° |
| $OC_2H_5$ | $CH_3$ | H | O | $S(CH_2)_3CH_3$ | H | |
| $OC_2H_5$ | $CH_3$ | H | O | $OSO_2(CH_2)_3CH_3$ | H | |
| $OC_2H_5$ | $CH_3$ | H | O | $OCF_3$ | H | |
| $OC_2H_5$ | $CH_3$ | H | O | $SCF_3$ | H | |
| $OC_2H_5$ | $CH_3$ | H | O | $OCHF_2$ | H | |
| $OC_2H_5$ | $CH_3$ | H | O | $SCHF_2$ | H | |
| $OC_2H_5$ | $CH_3$ | H | O | $OCF_2CF_2H$ | H | |
| $OC_2H_5$ | $CH_3$ | H | O | $SCF_2CF_2H$ | H | |
| $OC_2H_5$ | $CH_3$ | H | O | $CH_2OC_2H_5$ | H | |
| $OC_2H_5$ | $CH_3$ | H | O | $SCH_3$ | H | |
| $OC_2H_5$ | $CH_3$ | H | O | $CO_2CH_2CH_2Cl$ | H | |
| $OC_2H_5$ | $CH_3$ | H | O | $CO_2CH_2CH{=}CH_2$ | H | |
| $OC_2H_5$ | $CH_3$ | H | O | $CO_2CH(CH_3)_2$ | H | |
| $OC_2H_5$ | $CH_3$ | H | O | $OSO_2CHFCF_2H$ | H | |
| $OC_2H_5$ | $CH_3$ | H | O | $OSO_2CHFCCl_3$ | H | |
| $OC_2H_5$ | $CH_3$ | H | O | $OSO_2CHFCH_2F$ | H | |

Table 1 (continued)

| $R_1$ | $R_2$ | $R_3$ | W | $R_7$ | $R_8$ | m.p.(°C) |
|---|---|---|---|---|---|---|
| $OC_2H_5$ | $CH_3$ | H | O | $OSO_2CH_2CH_2Cl$ | H | |
| $OC_2H_5$ | $CH_3$ | H | O | $SO_2N(CH_3)_2$ | H | 169-175° |
| $OC_2H_5$ | $CH_3$ | H | O | $SO_2N(CH_3)[(CH_2)_2CH_3]$ | H | |
| $OC_2H_5$ | $CH_3$ | H | O | $CO_2CH_2CH(CH_3)_2$ | H | |
| $OC_2H_5$ | $CH_3$ | H | O | $OCH_2CH(CH_3)_2$ | H | |
| $OC_2H_5$ | $CH_3$ | H | O | $CO_2CH_3$ | H | 187-189° |
| $OC_2H_5$ | $CH_3$ | $CH_3$ | O | $SO_2CH_3$ | H | |
| $OC_2H_5$ | $CH_3$ | $CH_3$ | O | $CO_2CH_3$ | H | |
| $OC_2H_5$ | $CH_3$ | $CH_3$ | O | $OSO_2CH(CH_3)_2$ | H | |
| $OC_2H_5$ | $CH_3$ | $CH_3$ | O | $CF_3$ | H | |
| $OC_2H_5$ | $CH_3$ | $CH_3$ | O | $OCF_2CF_2H$ | H | |
| $OC_2H_5$ | $C_2H_5$ | $CH_3$ | O | Cl | H | |
| $OC_2H_5$ | $C_2H_5$ | H | O | $SCHF_2$ | H | |
| $OC_2H_5$ | $C_2H_5$ | H | O | $SO_2(CH_2)_2CH_3$ | H | |
| $OC_2H_5$ | $C_2H_5$ | H | O | $CO_2C_2H_5$ | H | |
| $OC_2H_5$ | $CH_3$ | H | O | $OSO_2(CH_2)_3Cl$ | H | |
| $O(CH)_2CH_3$ | $CH_3$ | H | O | Cl | H | |
| $O(CH_2)_2CH_3$ | $CH_3$ | H | O | $C_2H_5$ | H | |
| $OCH(CH_3)_2$ | $CH_3$ | H | O | $CO_2CH(CH_3)_2$ | H | |
| $O(CH_2)_3CH_3$ | $CH_3$ | H | O | $CO_2CH_3$ | H | |
| $OCH_2CH{=}CH_2$ | $CH_3$ | H | O | $SO_2CH_3$ | H | |
| $OCH_2C(CH_3){=}CH_2$ | $CH_3$ | H | O | $CO_2CH_3$ | H | |
| $OCH_2C{\equiv}CCH_3$ | $CH_3$ | H | O | $CO_2CH_3$ | H | |
| $OCH_2C{\equiv}CH$ | $CH_3$ | H | O | $CO_2C_2H_5$ | H | |
| $CF_3$ | $CH_3$ | H | O | $CO_2CH_3$ | H | |
| $CF_3$ | $CH_3$ | H | O | Cl | H | |
| $CF_3$ | $C_2H_5$ | H | O | $NO_2$ | H | |
| $CF_3$ | $C_2H_5$ | H | O | $CH_3$ | H | |
| $CF_2CF_3$ | $CH_3$ | H | O | $CO_2CH(CH_3)_2$ | H | |
| $CF_2CF_3$ | $CH_3$ | H | O | $CO_2CH_3$ | H | |
| $N(CH_3)_2$ | $CH_3$ | H | O | $CO_2CH_3$ | H | |
| $CF_3$ | $C_2H_5$ | H | O | $CO_2CH_3$ | H | |

Table 1 (continued)

| $R_1$ | $R_2$ | $R_3$ | W | $R_7$ | $R_8$ | m.p.(°C) |
|---|---|---|---|---|---|---|
| $CF_3$ | $C_2H_5$ | H | O | $CF_3$ | H | |
| $CF_3$ | $C_2H_5$ | H | O | $CH_2OCH_3$ | H | |
| $SCH_3$ | $CH_2CH{=}CH_2$ | H | O | $CO_2CH_3$ | H | 146-149° |
| $SC_2H_5$ | $CH_3$ | H | O | $CH_3$ | 5-$CH_3$ | 193-195° |
| $SC_2H_5$ | $C_2H_5$ | H | O | $CO_2CH_3$ | H | 150-152° |
| $CH(CH_3)_2$ | $CH_3$ | H | O | $CO_2CH_3$ | H | 190-197° |
| $OCH_3$ | $CH_3$ | H | H | H | H | |
| $OCH_3$ | $CH_3$ | H | H | $CH_3$ | H | 210-213° |
| $OCH_3$ | $CH_3$ | H | H | $C_2H_5$ | H | |
| $OCH_3$ | $CH_3$ | H | H | $CH_2CH_2CH_3$ | H | |
| $OCH_3$ | $CH_3$ | H | H | $CH(CH_3)_2$ | H | |
| $OCH_3$ | $CH_3$ | H | H | $(CH_2)_3CH_3$ | H | |
| $OCH_3$ | $CH_3$ | H | H | $OCH_3$ | H | |
| $OCH_3$ | $CH_3$ | H | H | $OCH_2CH_3$ | H | |
| $OCH_3$ | $CH_3$ | H | H | $OCH_2CH_2CH_3$ | H | 154-157° |
| $OCH_3$ | $CH_3$ | H | H | $OCH(CH_3)C_2H_5$ | H | |
| $OCH_3$ | $CH_3$ | H | H | F | H | |
| $OCH_3$ | $CH_3$ | H | H | Cl | H | 200-205° |
| $OCH_3$ | $CH_3$ | H | H | Br | H | |
| $OCH_3$ | $CH_3$ | H | H | $NO_2$ | H | 185-189° |
| $OCH_3$ | $CH_3$ | H | H | $CF_3$ | H | |
| $OCH_3$ | $CH_3$ | H | H | $CO_2C_2H_5$ | H | 144-148° |
| $OCH_3$ | $CH_3$ | H | H | $CO_2(CH_2)_2CH_3$ | H | 117-120° |
| $OCH_3$ | $CH_3$ | H | H | $CO_2CH(CH_3)_2$ | H | 165-169° |
| $OCH_3$ | $CH_3$ | H | H | $CO_2CH(CH_3)C_2H_5$ | H | |
| $OCH_3$ | $CH_3$ | H | H | $CO_2(CH_2)_5CH_3$ | H | |
| $OCH_3$ | $CH_3$ | H | H | $CO_2CH_2CH{=}CH_2$ | H | 139-144° |
| $OCH_3$ | $CH_3$ | H | H | $CO_2CH_2CH{=}CHCH_3$ | H | |
| $OCH_3$ | $CH_3$ | H | H | $CO_2CH_2C{\equiv}CH$ | H | |
| $OCH_3$ | $CH_3$ | H | O | $CO_2CH_2CH_2OCH_3$ | H | |
| $OCH_3$ | $CH_3$ | H | O | $CO_2CH_2CH_2Cl$ | H | |
| $OCH_3$ | $CH_3$ | H | O | $CO_2CH_2CH_2OC_2H_5$ | H | |

Table 1 (continued)

| $R_1$ | $R_2$ | $R_3$ | W | $R_7$ | $R_8$ | m.p.(°C) |
|---|---|---|---|---|---|---|
| $OCH_3$ | $CH_3$ | H | O | $CO_2(CH_2)_3OC_2H_5$ | H | |
| $OCH_3$ | $CH_3$ | H | O | $CO_2CH_2C{\equiv}CC_2H_5$ | H | |
| $OCH_3$ | $CH_3$ | H | O | $CO_2CF_2CFH_2$ | H | |
| $OCH_3$ | $CH_3$ | H | O | $CO_2CH_2CH_2\text{-}F$ | H | |
| $OCH_3$ | $CH_3$ | H | O | $CO_2CH_2CHFCF_2H$ | H | |
| $OCH_3$ | $CH_3$ | H | O | $SO_2N(CH_3)_2$ | H | 183-186° |
| $OCH_3$ | $CH_3$ | H | O | $SO_2N(C_2H_5)_2$ | H | |
| $OCH_3$ | $CH_3$ | H | O | $SO_2N(CH_3)C_2H_5$ | H | |
| $OCH_3$ | $CH_3$ | H | O | $SO_2N(CH_3)CH_2CH_2CH_3$ | H | |
| $OCH_3$ | $CH_3$ | H | O | $SO_2N(OCH_3)CH_3$ | H | |
| $OCH_3$ | $CH_3$ | H | O | $OSO_2CH_3$ | H | 174-176° |
| $OCH_3$ | $CH_3$ | H | O | $OSO_2C_2H_5$ | H | |
| $OCH_3$ | $CH_3$ | H | O | $OSO_2CH_2CH_2CH_3$ | H | |
| $OCH_3$ | $CH_3$ | H | O | $OSO_2CH_2CH_2OCH_3$ | H | |
| $OCH_3$ | $CH_3$ | H | O | $OSO_2CF_3$ | H | |
| $OCH_3$ | $CH_3$ | H | O | $OSO_2CH_2CH_2Cl$ | H | |
| $OCH_3$ | $CH_3$ | H | O | $SOCH_3$ | H | |
| $OCH_3$ | $CH_3$ | H | O | $SCH_3$ | H | |
| $OCH_3$ | $CH_3$ | H | O | $SO_2CH_3$ | H | 183-190° |
| $OCH_3$ | $CH_3$ | H | O | $SC_2H_5$ | H | |
| $OCH_3$ | $CH_3$ | H | O | $SO_2C_2H_5$ | H | |
| $OCH_3$ | $CH_3$ | H | O | $SO_2CH_2CH_2CH_3$ | H | 174-177° |
| $OCH_3$ | $CH_3$ | H | O | $SO_2CH(CH_3)_2$ | H | |
| $OCH_3$ | $CH_3$ | H | O | $SO_2(CH_2)_3CH_3$ | H | |
| $OCH_3$ | $CH_3$ | H | O | $SO_2CH_2CH{=}CH_2$ | H | |
| $OCH_3$ | $CH_3$ | H | O | $SO_2CF_3$ | H | |
| $OCH_3$ | $CH_3$ | H | O | $SO_2CH_2CH_2Cl$ | H | |
| $OCH_3$ | $CH_3$ | H | O | $SO_2CF_2CF_3$ | H | |
| $OCH_3$ | $CH_3$ | H | O | $SO_2CCl_2CF_3$ | H | |
| $OCH_3$ | $CH_3$ | H | O | $N(CH_3)SO_2CH_3$ | H | |
| $OCH_3$ | $CH_3$ | H | O | $N(CH_3)SO_2C_2H_5$ | H | |
| $OCH_3$ | $CH_3$ | H | O | $N(CH_3)SO_2(CH_2)_3CH_3$ | H | |

Table 1 (continued)

| $R_1$ | $R_2$ | $R_3$ | W | $R_7$ | $R_8$ | m.p.(°C) |
|---|---|---|---|---|---|---|
| $OCH_3$ | $CH_3$ | H | O | $N(CH_3)SO_2CH_2CH_2OCH_3$ | H | |
| $OCH_3$ | $CH_3$ | H | O | $N(CH_3)SO_2CF_3$ | H | |
| $OCH_3$ | $CH_3$ | H | O | $N(CH_3)SO_2CH_2CH_2Cl$ | H | |
| $OCH_3$ | $CH_3$ | H | O | $CH_2CO_2CH_3$ | H | |
| $OCH_3$ | $CH_3$ | H | O | $CH_2CO_2C_2H_5$ | H | |
| $OCH_3$ | $CH_3$ | H | O | $CH_2CO_2CH(CH_3)_2$ | H | |
| $OCH_3$ | $CH_3$ | H | O | $CH_2CO_2CH_2CH_2OCH_3$ | H | |
| $OCH_3$ | $CH_3$ | H | O | $CH_2CO_2CH_2CH_2Cl$ | H | |
| $OCH_3$ | $CH_3$ | H | O | $CH_2CO_2CH_2CH{=}CH_2$ | H | |
| $OCH_3$ | $CH_3$ | H | O | $CH(CH_3)CO_2CH_3$ | H | |
| $OCH_3$ | $CH_3$ | H | O | $CH_2SCH_3$ | H | |
| $OCH_3$ | $CH_3$ | H | O | $CH_2SOC_2H_5$ | H | |
| $OCH_3$ | $CH_3$ | H | O | $CH_2SO_2CH_3$ | H | |
| $OCH_3$ | $CH_3$ | H | O | $CH_2SO_2CH_2CH{=}CH_2$ | H | |
| $OCH_3$ | $CH_3$ | H | O | $CH_2SO_2CF_3$ | H | |
| $OCH_3$ | $CH_3$ | H | O | $CH_2SO_2CF_2CF_3$ | H | |
| $OCH_3$ | $CH_3$ | H | O | $CH_2SO_2CH_2CH_2CH_3$ | H | |
| $OCH_3$ | $CH_3$ | H | O | $CH(CH_3)SO_2CH_3$ | H | |
| $OCH_3$ | $CH_3$ | H | O | $OCH_2CH{=}CH_2$ | H | |
| $OCH_3$ | $CH_3$ | H | O | $OCH_2CH{=}CHCH_3$ | H | |
| $OCH_3$ | $CH_3$ | H | O | $OCH_2C{\equiv}CH$ | H | |
| $OCH_3$ | $CH_3$ | H | O | $CH_2CH_2OCH_3$ | H | |
| $OCH_3$ | $CH_3$ | H | O | $CH_2CH_2OC_2H_5$ | H | |
| $OCH_3$ | $CH_3$ | H | O | $OCF_3$ | H | |
| $OCH_3$ | $CH_3$ | H | O | $OCF_2CF_3$ | H | |
| $OCH_3$ | $CH_3$ | H | O | $OCH_2CH_2Cl$ | H | |
| $OCH_3$ | $CH_3$ | H | O | $OCH_2CF_2CF_3$ | H | |
| $OCH_3$ | $CH_3$ | H | O | $OCH_2OCH_3$ | H | |
| $OCH_3$ | $CH_3$ | H | O | $OCH_2CH_2OCH_3$ | H | |
| $OCH_3$ | $CH_3$ | H | O | $OCH_2OC_2H_5$ | H | |
| $OCH_3$ | $CH_3$ | H | O | $OCH_2CH_2OC_2H_5$ | H | |
| $OCH_3$ | $CH_3$ | H | O | $CH_2OCH_3$ | H | |

Table 1 (continued)

| $R_1$ | $R_2$ | $R_3$ | W | $R_7$ | $R_8$ | m.p.(°C) |
|---|---|---|---|---|---|---|
| $OCH_3$ | $CH_3$ | H | O | $CH_2OC_2H_5$ | H | |
| $OCH_3$ | $CH_2CF_3$ | H | O | $CO_2CH_3$ | H | 154-157° |
| $OCH_3$ | $CH_2CF_3$ | H | O | $SO_2N(CH_3)_2$ | H | 150-157° |
| $OCH_3$ | $CH_2CF_3$ | H | O | Cl | H | 90-100° |
| $OCH_3$ | $CH_2CF_3$ | H | O | $SO_2CH_3$ | H | 195-225° |
| $OCH_3$ | $CH_2CF_3$ | H | O | $NO_2$ | H | 158-160° |
| $OC_2H_5$ | $CH_3$ | H | O | $CH_3$ | H | |
| $OC_2H_5$ | $CH_3$ | H | O | $CH_2CH_3$ | H | |
| $OC_2H_5$ | $CH_3$ | H | O | $CH_2(CH_3)_2$ | H | |
| $OC_2H_5$ | $CH_3$ | H | O | $OCH_2CH_2CH_3$ | H | |
| $OC_2H_5$ | $CH_3$ | H | O | Cl | H | 180-183° |
| $OC_2H_5$ | $CH_3$ | H | O | Br | H | |
| $OC_2H_5$ | $CH_3$ | H | O | $NO_2$ | H | |
| $OC_2H_5$ | $CH_3$ | H | O | $CF_3$ | H | |
| $OC_2H_5$ | $CH_3$ | H | O | $CO_2(CH_2)_5CH_3$ | H | |
| $OC_2H_5$ | $CH_3$ | H | O | $CO_2CH_2CH_2OCH_3$ | H | |
| $OC_2H_5$ | $CH_3$ | H | O | $CO_2CH_2C{\equiv}CH$ | H | |
| $OC_2H_5$ | $CH_3$ | H | O | $N(CH_3)SO_2CH_3$ | H | |
| $OC_2H_5$ | $CH_3$ | H | O | $N(CH_3)SO_2C_2H_5$ | H | |
| $OC_2H_5$ | $CH_3$ | H | O | $OSO_2CH_3$ | H | |
| $OC_2H_5$ | $CH_3$ | H | O | $SO_2CH_2CH_2CH_3$ | H | |
| $OC_2H_5$ | $CH_3$ | H | O | $SO_2CH_2CH{=}CH_2$ | H | |
| $OC_2H_5$ | $CH_3$ | H | O | $SO_2CF_3$ | H | |
| $OC_2H_5$ | $CH_3$ | H | O | $CH_2CO_2CH_3$ | H | |
| $OC_2H_5$ | $CH_3$ | H | O | $CH(CH_3)CO_2CH_3$ | H | |
| $OC_2H_5$ | $CH_3$ | H | O | $CH_2CO_2CH_2CH_2Cl$ | H | |
| $OC_2H_5$ | $CH_3$ | H | O | $CH_2SO_2CH_3$ | H | |
| $OC_2H_5$ | $CH_3$ | H | O | $CH_2SCH_3$ | H | |
| $OC_2H_5$ | $CH_3$ | H | O | $CH(CH_3)SO_2CH_3$ | H | |
| $OC_2H_5$ | $CH_3$ | H | O | $OCH_2CH{=}CH_2$ | H | |
| $OC_2H_5$ | $CH_3$ | H | O | $OCH_2C{=}CCH_3$ | H | |
| $OC_2H_5$ | $CH_3$ | H | O | $CH_2OCH_3$ | H | |

Table 1 (continued)

| $R_1$ | $R_2$ | $R_3$ | W | $R_7$ | $R_8$ | m.p.(°C) |
|---|---|---|---|---|---|---|
| $OC_2H_5$ | $CH_3$ | H | O | $CH_2CH_2OC_2H_5$ | H | |
| $OC_2H_5$ | $CH_3$ | H | O | $OCF_2CF_2CH_3$ | H | |
| $OC_2H_5$ | $CH_3$ | H | O | $OCH_2OCH_3$ | H | |
| $OC_2H_5$ | $CH_3$ | H | O | $OCH_2CH_2OCH_3$ | H | |
| $OC_2H_5$ | $CH_3$ | H | O | $OCH_2CH_2OCH_2CH_3$ | H | |
| $OC_2H_5$ | $CH_3$ | H | O | $O(CH_2)_3OCH_3$ | H | |
| Cl | $CH_3$ | H | O | $CH_3$ | H | |
| Cl | $CH_3$ | H | O | $CH(CH_3)_2$ | H | |
| Cl | $CH_3$ | H | O | $OCH_3$ | H | |
| Cl | $CH_3$ | H | O | $OCH_2CH_2CH_3$ | H | |
| Cl | $CH_3$ | H | O | Cl | H | |
| Cl | $CH_3$ | H | O | $NO_2$ | H | |
| Cl | $CH_3$ | H | O | $CF_3$ | H | |
| Cl | $CH_3$ | H | O | $CO_2CH_3$ | H | |
| Cl | $CH_3$ | H | O | $CO_2C_2H_5$ | H | |
| Cl | $CH_3$ | H | O | $CO_2CH_2CH=CH_2$ | H | |
| Cl | $CH_3$ | H | O | $SO_2N(CH_3)_2$ | H | |
| Cl | $CH_3$ | H | O | $OSO_2CH_3$ | H | |
| Cl | $CH_3$ | H | O | $N(CH_3)SO_2CH_3$ | H | |
| Cl | $CH_3$ | H | O | $SO_2CH_3$ | H | |
| Cl | $CH_3$ | H | O | $SO_2CH_2CH_2CH_3$ | H | |
| Cl | $CH_3$ | H | O | $CH_2CO_2CH_3$ | H | |
| Cl | $CH_3$ | H | O | $CH_2SCH_3$ | H | |
| Cl | $CH_3$ | H | O | $CH_2SO_2CH_3$ | H | |
| Cl | $CH_3$ | H | O | $OCH_2CH=CH_2$ | H | |
| Cl | $CH_3$ | H | O | $OCH_2C{\equiv}CCH_3$ | H | |
| Cl | $CH_3$ | H | O | $CH_2OCH_3$ | H | |
| Cl | $CH_3$ | H | O | $CH_2CH_2OCH_3$ | H | |
| Cl | $CH_3$ | H | O | $CH_2CH_2Cl$ | H | |
| $NHCH_3$ | $CH_3$ | H | O | $CH_3$ | H | |
| $NHCH_3$ | $CH_3$ | H | O | $CH_2CH_3$ | H | |
| $NHCH_3$ | $CH_3$ | H | O | $OCH_3$ | H | |

Table 1 (continued)

| $R_1$ | $R_2$ | $R_3$ | W | $R_7$ | $R_8$ | m.p.(°C) |
|---|---|---|---|---|---|---|
| $NHCH_3$ | $CH_3$ | H | O | Br | H | |
| $NHCH_3$ | $CH_3$ | H | O | Cl | H | |
| $NHCH_3$ | $CH_3$ | H | O | $NO_2$ | H | |
| $NHCH_3$ | $CH_3$ | H | O | $CF_3$ | H | |
| $NHCH_3$ | $CH_3$ | H | O | $CO_2CH_3$ | H | |
| $NHCH_3$ | $CH_3$ | H | O | $CO_2CH(CH_3)_2$ | H | |
| $NHCH_3$ | $CH_3$ | H | O | $CO_2CH_2CH_2Cl$ | H | |
| $NHCH_3$ | $CH_3$ | H | O | $SO_2N(CH_3)_2$ | H | |
| $NHCH_3$ | $CH_3$ | H | O | $SO_2N(OCH_3)CH_3$ | H | |
| $NHCH_3$ | $CH_3$ | H | O | $OSO_2CF_3$ | H | |
| $NHCH_3$ | $CH_3$ | H | O | $SO_2CH_3$ | H | |
| $NHCH_3$ | $CH_3$ | H | O | $SO_2CH_2CH_2CH_3$ | H | |
| $NHCH_3$ | $CH_3$ | H | O | $SO_2CH_2CH{=}CH_2$ | H | |
| $NHCH_3$ | $CH_3$ | H | O | $CH_2CO_2CH_3$ | H | |
| $NHCH_3$ | $CH_3$ | H | O | $CH_2CO_2CH_2CH_2Cl$ | H | |
| $NHCH_3$ | $CH_3$ | H | O | $CH_2SOCH_3$ | H | |
| $NHCH_3$ | $CH_3$ | H | O | $CH_2SO_2CH_3$ | H | |
| $NHCH_3$ | $CH_3$ | H | O | $OCH_2CH{=}CH_2$ | H | |
| $NHCH_3$ | $CH_3$ | H | O | $OCH_2C{\equiv}CH$ | H | |
| $NHCH_3$ | $CH_3$ | H | O | $CH_2CH_2OC_2H_5$ | H | |
| $NHCH_3$ | $CH_3$ | H | O | $OCH_2CF_2CF_3$ | H | |
| $NHCH_3$ | $CH_3$ | H | O | $OCH_2OCH_3$ | H | |
| $NHCH_3$ | $CH_3$ | H | O | $OCH_2CH_2OCH_3$ | H | |
| $NHCH_3$ | $CH_3$ | H | O | $OCH_2CH_2OC_2H_5$ | H | |
| $CH(OCH_3)_2$ | $CH_3$ | H | O | $CH_3$ | H | |
| $CH(OCH_3)_2$ | $CH_3$ | H | O | $CH(CH_3)_2$ | H | |
| $CH(OCH_3)_2$ | $CH_3$ | H | O | $OCH_3$ | H | |
| $CH(OCH_3)_2$ | $CH_3$ | H | O | $OCH_2CH_2CH_3$ | H | |
| $CH(OCH_3)_2$ | $CH_3$ | H | O | F | H | |
| $CH(OCH_3)_2$ | $CH_3$ | H | O | Cl | H | |
| $CH(OCH_3)_2$ | $CH_3$ | H | O | Br | H | |
| $CH(OCH_3)_2$ | $CH_3$ | H | O | $NO_2$ | H | |

Table 1 (continued)

| $R_1$ | $R_2$ | $R_3$ | W | $R_7$ | $R_8$ | m.p.(°C) |
|---|---|---|---|---|---|---|
| $CH(OCH_3)_2$ | $CH_3$ | H | O | $CF_3$ | H | |
| $CH(OCH_3)_2$ | $CH_3$ | H | O | $CO_2CH_3$ | H | |
| $CH(OCH_3)_2$ | $CH_3$ | H | O | $CO_2C_2H_5$ | H | |
| $CH(OCH_3)_2$ | $CH_3$ | H | O | $CO_2CH_2CH{=}CH_2$ | H | |
| $CH(OCH_3)_2$ | $CH_3$ | H | O | $SO_2N(CH_3)_2$ | H | |
| $CH(OCH_3)_2$ | $CH_3$ | H | O | $SO_2N(CH_3)C_2H_5$ | H | |
| $CH(OCH_3)_2$ | $CH_3$ | H | O | $OSO_2CF_3$ | H | |
| $CH(OCH_3)_2$ | $CH_3$ | H | O | $SO_2CH_3$ | H | |
| $CH(OCH_3)_2$ | $CH_3$ | H | O | $SO_2CH_2CH_2CH_3$ | H | |
| $CH(OCH_3)_2$ | $CH_3$ | H | O | $SO_2CH_2CH{=}CH_2$ | H | |
| $CH(OCH_3)_2$ | $CH_3$ | H | O | $CH_2CO_2CH_3$ | H | |
| $CH(OCH_3)_2$ | $CH_3$ | H | O | $CH_2CO_2CH_2CH_2OCH_3$ | H | |
| $CH(OCH_3)_2$ | $CH_3$ | H | O | $CH_2SO_2CH_3$ | H | |
| $CH(OCH_3)_2$ | $CH_3$ | H | O | $OCH_2CH{=}CH_2$ | H | |
| $CH(OCH_3)_2$ | $CH_3$ | H | O | $CH_2CH_2OCH_3$ | H | |
| $CH(OCH_3)_2$ | $CH_3$ | H | O | $CH_2OCH_3$ | H | |
| $CH(OCH_3)_2$ | $CH_3$ | H | O | $OCH_2CH_2OCH_3$ | H | |
| $CH(OCH_3)_2$ | $CH_3$ | H | O | $OCF_2CF_3$ | H | |
| $CH(OCH_3)_2$ | $CH_3$ | H | O | $OCH_2CF_2CF_3$ | H | |
| 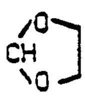 | $CH_3$ | H | O | $CH_3$ | H | |
| 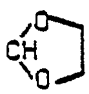 | $CH_3$ | H | O | $CH(CH_3)_2$ | H | |
| 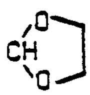 | $CH_3$ | H | O | $OCH_2CH_3$ | H | |
| 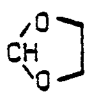 | $CH_3$ | H | O | Cl | H | |
| 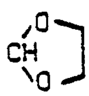 | $CH_3$ | H | O | $NO_2$ | H | |

Table 1 (continued)

| $R_1$ | $R_2$ | $R_3$ | W | $R_7$ | $R_8$ | m.p.(°C) |
|---|---|---|---|---|---|---|
| 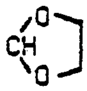 | $CH_3$ | H | O | $CF_3$ | H | |
| 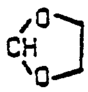 | $CH_3$ | H | O | $CO_2CH_3$ | H | |
| 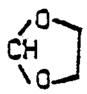 | $CH_3$ | H | O | $CO_2CH_2CH_2Cl$ | H | |
| 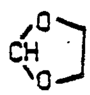 | $CH_3$ | H | O | $CO_2CH(CH_3)$ | H | |
| 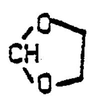 | $CH_3$ | H | O | $SO_2N(CH_3)_2$ | H | |
| 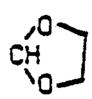 | $CH_3$ | H | O | $SO_2N(OCH_3)CH_3$ | H | |
| 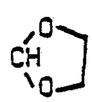 | $CH_3$ | H | O | $N(CH_3)SO_2CH_3$ | H | |
| 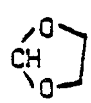 | $CH_3$ | H | O | $OSO_2CH_3$ | H | |
| 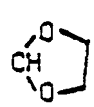 | $CH_3$ | H | O | $SO_2CH_3$ | H | |
| 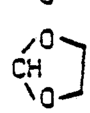 | $CH_3$ | H | O | $SO_2CH_2CH_2CH_3$ | H | |
| 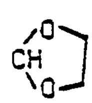 | $CH_3$ | H | O | $CH_2CO_2CH_3$ | H | |
| 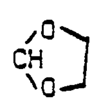 | $CH_3$ | H | O | $CH(CH_3)CO_2CH_3$ | H | |
| 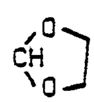 | $CH_3$ | H | O | $CH_2SO_2CH_3$ | H | |
| 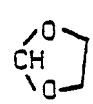 | $CH_3$ | H | O | $CH_2SC_2H_5$ | H | |

Table 1 (continued)

| $R_1$ | $R_2$ | $R_3$ | W | $R_7$ | $R_8$ | m.p.(°C) |
|---|---|---|---|---|---|---|
| 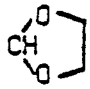 | $CH_3$ | H | O | $OCH_2CH{=}CH_2$ | H | |
| 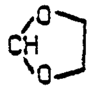 | $CH_3$ | H | O | $CH_2OCH_3$ | H | |
| 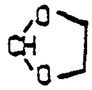 | $CH_3$ | H | O | $CH_2CH_2OCH_3$ | H | |
| 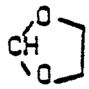 | $CH_3$ | H | O | $OCH_2CH_2OCH_3$ | H | |
| 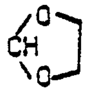 | $CH_3$ | H | O | $OCF_2CF_3$ | H | |
| 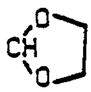 | $CH_3$ | H | O | $OCH_2CF_2CF_3$ | H | |
| 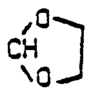 | $CH_3$ | H | O | $OCH_2CH_2CH_2OCH_3$ | H | |
| 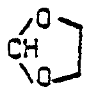 | $CH_3$ | H | O | $OCCl_2CF_3$ | H | |
| $OCH_3$ | $CH_3$ | H | O | $CO_2N(CH_3)_2$ | H | |
| $OCH_3$ | $CH_3$ | H | O | $CO_2N(C_2H_5)_2$ | H | |
| $OCH_3$ | $CH_3$ | H | O | $CO_2N(CH_3)C_2H_5$ | H | |
| $OCH_3$ | $CH_3$ | H | O | $CO_2N(CH_3)CH_2CH_2CH_3$ | H | |
| $OCH_3$ | $CH_3$ | H | O | $CO_2(CH_3)C_6H_5$ | H | |
| $OCH_3$ | $CH_3$ | H | O | $CO_2N(CH_2)_4$ | H | |
| $OCH_3$ | $CH_3$ | H | O | $C(O)SCH_3$ | H | |
| $OCH_3$ | $CH_3$ | H | O | $C(O)SC_2H_5$ | H | |
| $OCH_3$ | $CH_3$ | H | O | $C(O)SCH_2CH_2CH_3$ | H | |
| $OCH_3$ | $CH_3$ | H | O | $C(O)SCH(CH_3)_2$ | H | |
| $OC_2H_5$ | $CH_3$ | H | O | $CO_2N(CH_3)_2$ | H | |
| $OC_2H_5$ | $CH_3$ | H | O | $CO_2N(C_2H_5)_2$ | H | |
| $OC_2H_5$ | $CH_3$ | H | O | $CO_2N(CH_3)C_2H_5$ | H | |

Table 1 (continued)

| $R_1$ | $R_2$ | $R_3$ | W | $R_7$ | $R_8$ | m.p.(°C) |
|---|---|---|---|---|---|---|
| $OC_2H_5$ | $CH_3$ | H | O | $CO_2N(CH_3)CH_2CH_2CH_3$ | H | |
| $OC_2H_5$ | $CH_3$ | H | O | $CO_2(CH_3)C_6H_5$ | H | |
| $OC_2H_5$ | $CH_3$ | H | O | $CO_2N(CH_2)_4$ | H | |
| $OC_2H_5$ | $CH_3$ | H | O | $C(O)SCH_3$ | H | |
| $OC_2H_5$ | $CH_3$ | H | O | $C(O)SC_2H_5$ | H | |
| $OC_2H_5$ | $CH_3$ | H | O | $C(O)SCH_2CH_2CH_3$ | H | |
| $OC_2H_5$ | $CH_3$ | H | O | $C(O)SCH(CH_3)_2$ | H | |
| $SCH_3$ | $CH_3$ | H | O | $CO_2N(CH_3)_2$ | H | |
| $SCH_3$ | $CH_3$ | H | O | $CO_2N(C_2H_5)_2$ | H | |
| $SCH_3$ | $CH_3$ | H | O | $CO_2N(CH_3)C_2H_5$ | H | |
| $SCH_3$ | $CH_3$ | H | O | $CO_2N(CH_3)CH_2CH_2CH_3$ | H | |
| $SCH_3$ | $CH_3$ | H | O | $CO_2(CH_3)C_6H_5$ | H | |
| $SCH_3$ | $CH_3$ | H | O | $CO_2N(CH_2)_4$ | H | |
| $SCH_3$ | $CH_3$ | H | O | $C(O)SCH_3$ | H | |
| $SCH_3$ | $CH_3$ | H | O | $C(O)SC_2H_5$ | H | |
| $SCH_3$ | $CH_3$ | H | O | $C(O)SCH_2CH_2CH_3$ | H | |
| $SCH_3$ | $CH_3$ | H | O | $C(O)SCH(CH_3)_2$ | H | |

Table 2

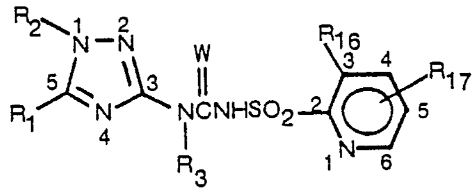

| $R_1$ | $R_2$ | $R_3$ | W | $R_{16}$ | $R_{17}$ | m.p.(°C) |
|---|---|---|---|---|---|---|
| H | $C_2H_5$ | H | O | $CO_2CH_3$ | H | |
| H | $n$-$C_3H_7$ | H | O | Cl | H | |
| $CH_3$ | $CH_2CH(CH_3)_2$ | H | O | $CO_2CH_3$ | H | |
| $CH_3$ | $C_2H_5$ | H | O | Cl | H | |
| $CH_3$ | $C_2H_5$ | H | O | H | H | |
| $CH_3$ | $C_2H_5$ | H | S | Cl | H | |
| $CH_3$ | $C_2H_5$ | H | O | $CO_2CH(CH_3)C_2H_5$ | H | |
| $CH_3$ | $C_2H_5$ | $CH_3$ | O | $CO_2CH_3$ | H | |
| $C_2H_5$ | $CH_3$ | H | O | $CO_2(CH_2)_3CH_3$ | H | |
| $C_2H_5$ | $CH_3$ | $CH_3$ | O | Cl | H | |
| $C_2H_5$ | $CH_3$ | H | S | Cl | H | |
| $C_2H_5$ | $CH_3$ | H | O | $CO_2CH_2CH_2OCH_3$ | H | |
| $C_2H_5$ | $C_2H_5$ | H | O | $CO_2CH_2CH(CH_3)_2$ | H | |
| $n$-$C_4H_9$ | $CH_3$ | H | O | $CO_2CH_3$ | H | |
| $CH(CH_3)_2$ | $CH_3$ | H | O | $NO_2$ | H | |
| $n$-$C_3H_7$ | $CH_3$ | H | O | $CF_3$ | H | |
| $CH_3$ | $n$-$C_3H_7$ | H | O | $CO_2CH_3$ | H | |
| $CH_3$ | $CH_2CH{=}CH_2$ | H | O | Cl | H | |
| $CH_3$ | $CH_2C(CH_3){=}CH_2$ | H | O | $OCH_3$ | H | |
| $CH_2CH{=}CH_2$ | $CH_3$ | H | O | $C_2H_5$ | H | |
| $CH_2C(CH_3){=}CH_2$ | $CH_3$ | H | O | $SO_2CH_3$ | H | |
| $CH_3$ | $CH_2C{\equiv}CCH_3$ | H | O | $SO_2CH_3$ | H | |
| $CH_2C{\equiv}C{-}CH_3$ | $CH_3$ | H | O | $SO_2C_2H_5$ | H | |
| $C_2H_5$ | $CH_3$ | H | O | $SO_2N(CH_3)_2$ | H | |
| $C_2H_5$ | $CH_3$ | H | O | $SO_2N(CH_3)CCH_3$ | H | |

Table 2 (continued)

| $R_1$ | $R_2$ | $R_3$ | W | $R_{16}$ | $R_{17}$ | m.p.(°C) |
|---|---|---|---|---|---|---|
| $CH_3$ | $C_2H_5$ | H | O | $SCH_3$ | H | |
| $C_2H_5$ | $CH_3$ | H | O | $S(CH_2)_3CH_3$ | H | |
| $C_2H_5$ | $CH_3$ | H | O | F | H | |
| $C_2H_5$ | $CH_3$ | H | S | $CH_3$ | H | |
| $C_2H_5$ | $CH_3$ | $CH_3$ | O | $SO_2CH_3$ | H | |
| $C_2H_5$ | $C_2H_5$ | H | O | $n$-$C_4H_9$ | H | |
| $CH_3$ | $CH_2C{\equiv}CH$ | H | O | Cl | H | |
| $SCH_3$ | $CH_3$ | H | O | $CH_3$ | H | |
| $SCH_3$ | $CH_3$ | H | O | H | H | |
| $SCH_3$ | $CH_3$ | H | O | Cl | H | |
| $SCH_3$ | $CH_3$ | H | O | $NO_2$ | H | |
| $SCH_3$ | $CH_3$ | H | S | Cl | H | |
| $SCH_3$ | $CH_3$ | $CH_3$ | O | Cl | H | |
| $SCH_3$ | $CH_3$ | H | O | Cl | 5-F | |
| $SCH_3$ | $CH_3$ | H | O | $SO_2CH_3$ | 4-Br | |
| $SCH_3$ | $C_2H_5$ | H | O | $SO_2CH_3$ | 6-Br | |
| $SCH_3$ | $CH_3$ | H | O | $SO_2N(CH_3)_2$ | 6-Cl | |
| $CH_3$ | $C_2H_5$ | H | O | $NO_2$ | 5-F | |
| $C_2H_5$ | $CH_3$ | H | O | $CH_3$ | 4-Cl | |
| $SCH_3$ | $CH_3$ | $CH_3$ | O | Cl | 6-Cl | |
| $SCH_3$ | $C_2H_5$ | H | S | $OCH_3$ | H | |
| $SCH_3$ | $CH_3$ | H | O | $CO_2CH_2CH{=}CH_2$ | H | |
| $SCH_3$ | $CH_3$ | H | O | $CO_2CH(CH_3)_2$ | H | |
| $SCH_3$ | $C_2H_5$ | H | O | $CO_2CH_2CH_2Cl$ | H | |
| $SCH_3$ | $CH_3$ | H | O | $CO_2CH_2CH(CH_3)_2$ | H | |
| $SCH_3$ | $C_2H_5$ | H | O | $n$-$C_4H_9$ | H | |
| $SCH_3$ | $CH_3$ | H | O | $SCH_3$ | H | |
| $SCH_3$ | $CH_3$ | H | O | $S(CH_2)_2CH_3$ | H | |
| $SCH_3$ | $CH_3$ | H | O | F | H | |
| $SC_2H_5$ | $CH_3$ | H | S | $CH_3$ | H | |
| $SC_2H_5$ | $CH_3$ | H | O | $SO_2CH_3$ | H | |

Table 2 (continued)

| $R_1$ | $R_2$ | $R_3$ | W | $R_{16}$ | $R_{17}$ | m.p.(°C) |
|---|---|---|---|---|---|---|
| $S(CH_2)_2CH_3$ | $CH_3$ | H | O | $SO_2CH_3$ | H | |
| $SCH(CH_3)C_2H_5$ | $CH_3$ | H | O | Cl | H | |
| $SCH(CH_3)_2$ | $CH_3$ | H | O | $SO_2C_2H_5$ | H | |
| $SCH_2CH{=}CH_2$ | $CH_3$ | H | O | $CO_2C_2H_5$ | H | |
| $SCH_2C(CH_3){=}CH_2$ | $CH_3$ | H | O | $SO_2CH_3$ | H | |
| $SCH_2C{\equiv}C{-}CH_3$ | $CH_3$ | H | O | $CO_2CH_3$ | H | |
| $SCH_3C{\equiv}CH$ | $CH_3$ | H | O | Cl | H | |
| $SCH_2CO_2CH_3$ | $CH_3$ | H | O | $CF_3$ | H | |
| $SCH_2CO_2CH(CH_3)_2$ | $CH_3$ | H | O | $NO_2$ | H | |
| $SCH(CH_3)CO_2CH_3$ | $CH_3$ | H | O | $CO_2CH_3$ | H | |
| $OCH_3$ | $C_2H_5$ | H | S | Cl | H | |
| $OCH_3$ | $C_2H_5$ | $CH_3$ | O | Cl | H | |
| $OCH_3$ | $C_2H_5$ | H | O | H | H | |
| $OCH_3$ | $C_2H_5$ | H | O | F | H | |
| $OCH_3$ | $C_2H_5$ | H | O | H | 4-F | |
| $OCH_3$ | $C_2H_5$ | H | O | H | 5-F | |
| $OCH_3$ | $C_2H_5$ | H | O | Cl | H | |
| $OCH_3$ | $C_2H_5$ | H | O | $SO_2CH_3$ | H | |
| $OCH_3$ | $C_2H_5$ | H | O | $CF_3$ | H | |
| $OCH_3$ | $C_2H_5$ | H | O | $NO_2$ | H | |
| $OC_2H_5$ | $CH_3$ | H | S | $CH_3$ | H | |
| $OC_2H_5$ | $CH_3$ | H | O | $OC_2H_5$ | H | |
| $OC_2H_5$ | $CH_3$ | $CH_3$ | O | Cl | H | |
| $OC_2H_5$ | $CH_3$ | $CH_3$ | O | $SO_2CH_3$ | H | |
| $OC_2H_5$ | $CH_3$ | H | S | $SO_2CH_3$ | H | |
| $OC_2H_5$ | $CH_3$ | H | O | $SO_2N(CH_3)[(CH_2)_2CH_3]$ | H | |
| $OCH_3$ | $C_2H_5$ | H | O | $SO_2N(CH_3)_2$ | H | |
| $OCH_3$ | $C_2H_5$ | H | O | $SO_2N(CH_3)OCH_3$ | H | |
| $OCH_3$ | $C_2H_5$ | H | O | $SO_2C_2H_5$ | H | |
| $OC_2H_5$ | $CH_3$ | H | O | $SO_2(CH_2)_3CH_3$ | H | |

Table 2 (continued)

| $R_1$ | $R_2$ | $R_3$ | W | $R_{16}$ | $R_{17}$ | m.p.(°C) |
|---|---|---|---|---|---|---|
| $OC_2H_5$ | $CH_3$ | H | O | Cl | 6-$CH_3$ | |
| $OCH_3$ | $C_2H_5$ | H | O | $NO_2$ | 5-Cl | |
| $OCH_3$ | $C_2H_5$ | H | O | Cl | 5-$CH_3$ | |
| $OCH_3$ | $C_2H_5$ | H | O | Br | 4-Cl | |
| $OCH_3$ | $C_2H_5$ | H | O | $OCH_3$ | 5-F | |
| $OCH_3$ | $C_2H_5$ | H | O | $SO_2CH_3$ | 4-Br | |
| $OCH_3$ | $C_2H_5$ | H | O | $SO_2CH_3$ | 6-Cl | |
| $OC_2H_5$ | $CH_3$ | H | O | $SO_2CH_3$ | 5-$OCH_3$ | |
| $OCH_3$ | $C_2H_5$ | H | O | $SO_2CH_3$ | 4-$OCH_3$ | |
| $OCH_3$ | $C_2H_5$ | H | O | $SCH_3$ | H | |
| $OC_2H_5$ | $CH_3$ | H | O | $S(CH_2)_3CH_3$ | H | |
| $OC_2H_5$ | $CH_3$ | H | O | $SO_2N(CH_3)C_2H_5$ | H | |
| $OC_2H_5$ | $CH_3$ | H | O | $CF_3$ | H | |
| $OCH_3$ | $C_2H_5$ | H | O | Br | H | |
| $OCH_3$ | $C_2H_5$ | H | O | $NO_2$ | H | |
| $OCH_3$ | $C_2H_5$ | H | O | $n$-$C_3H_7$ | H | |
| $OC_2H_5$ | $CH_3$ | H | O | $OCH_2(CH_2)_2CH_3$ | H | |
| $OC_2H_5$ | $CH_3$ | H | O | $OCH_2CH(CH_3)_2$ | H | |
| $OC_2H_5$ | $CH_3$ | H | O | $OC_2H_5$ | H | |
| $OCH_3$ | $CH_3$ | H | O | $CO_2CH_2CH_2OCH_3$ | H | |
| $OCH_3$ | $C_2H_5$ | H | O | $CO_2(CH_2)_3OCH_3$ | H | |
| $OCH_3$ | $C_2H_5$ | H | O | $CO_2(CH_2)_2OCH_3$ | H | |
| $OC_2H_5$ | $C_2H_5$ | H | O | $CO_2CH_2CH_2Cl$ | H | |
| $OCH_3$ | $C_2H_5$ | $CH_3$ | O | $CO_2C_2H_5$ | H | |
| $OCH_3$ | $C_2H_5$ | $CH_3$ | O | $CO_2CH_3$ | H | |
| $OC_2H_5$ | $C_2H_5$ | $CH_3$ | O | $SO_2N(C_2H_5)_2$ | H | |
| $OCH_2CH=CH_2$ | $CH_3$ | H | O | $SO_2CH_3$ | H | |
| $OCH_2C(CH_3)=CH_2$ | $CH_3$ | H | O | Cl | H | |
| $OCH(CH_3)C_2H_5$ | $CH_3$ | H | O | $SO_2CH_3$ | H | |
| $O(CH_2)_2CH_3$ | $CH_3$ | H | O | $SO_2CH_3$ | H | |
| $OCH(CH_3)_2$ | $CH_3$ | H | O | $CO_2C_2H_5$ | H | |

Table 2 (continued)

| $R_1$ | $R_2$ | $R_3$ | W | $R_{16}$ | $R_{17}$ | m.p.(°C) |
|---|---|---|---|---|---|---|
| $O(CH_2)_2CH_3$ | $CH_3$ | H | O | $CO_2CH(CH_3)_2$ | H | |
| $OCH_2CH{=}CHCH_3$ | $CH_3$ | H | O | Cl | H | |
| $OCH_2C{\equiv}CCH_3$ | $CH_3$ | H | O | $SO_2CH_3$ | H | |
| $CH_2OCH_3$ | $CH_3$ | H | O | $NO_2$ | H | |
| $CH_2OCH_3$ | $CH_3$ | H | O | $SO_2N(CH_3)[CH(CH_3)_2]$ | H | |
| $CH_2OCH_3$ | $CH_3$ | H | O | $SO_2N(CH_3)_2$ | H | |
| $CH_2OCH_3$ | $CH_3$ | H | O | $SO_2CH_3$ | H | |
| $CH_2OCH_3$ | $CH_3$ | H | O | Cl | H | |
| $CH_2OCH_3$ | $CH_3$ | H | O | F | H | |
| $CH_2OCH_3$ | $CH_3$ | H | O | H | H | |
| $CH_2OCH_3$ | $CH_3$ | H | S | Cl | H | |
| $CH_2OCH_3$ | $CH_3$ | $CH_3$ | O | $CH_3$ | H | |
| $CH_2OCH_3$ | $CH_3$ | $CH_3$ | O | Cl | H | |
| $CH_2OCH_3$ | $CH_3$ | H | O | $SC_2H_5$ | H | |
| $CH_2OCH_3$ | $CH_3$ | H | O | $OCH_3$ | H | |
| $CH_2OCH_3$ | $CH_3$ | H | O | $CO_2CH_2CH_2Cl$ | H | |
| $CH_2OC_2H_5$ | $CH_3$ | $CH_3$ | O | $CO_2CH(CH_3)_2$ | H | |
| $CH_2OC_2H_5$ | $CH_3$ | H | O | $CO_2CH_2CH{=}CH_2$ | H | |
| $CH_2O(CH_2)_3CH_3$ | $CH_3$ | H | O | $CO_2CH_3$ | H | |
| $CH_2CH(CH_3)_2$ | $CH_3$ | H | O | Cl | H | |
| $CH_2CH_2OCH_3$ | $CH_3$ | H | O | $CO_2C_2H_5$ | H | |
| $CH_2CH_2OCH(CH_3)_2$ | $CH_3$ | H | O | $CO_2CH(CH_3)_2$ | H | |
| $CH_2CH_2O(CH_2)_2CH_3$ | $CH_3$ | H | O | $SO_2CH_3$ | H | |
| $N(CH_3)_2$ | $CH_3$ | H | O | Cl | H | |
| $CF_2CF_3$ | $CH_3$ | H | O | $SO_2CH_3$ | H | |
| $CF_3$ | $CH_3$ | H | O | Cl | H | |
| $CF_2CF_3$ | $CH_3$ | H | O | $CO_2CH_3$ | H | |
| $CF_2CF_3$ | $CH_3$ | H | O | $NO_2$ | H | |
| $CF_2CF_3$ | $CH_3$ | H | O | $SO_2CH(CH_3)_2$ | H | |
| $CH_3$ | $CH_2OCH_3$ | H | S | Cl | H | |
| $CH_3$ | $CH_2OCH_3$ | H | O | $SO_2C_2H_5$ | H | |

Table 2 (continued)

| $R_1$ | $R_2$ | $R_3$ | W | $R_{16}$ | $R_{17}$ | m.p.(°C) |
|---|---|---|---|---|---|---|
| $CH_3$ | $CH_2OCH_3$ | H | O | $CO_2CH_3$ | H | |
| $CH_3$ | $CH_2CH_2OCH_3$ | H | O | Cl | H | |
| $CH_3$ | $CH_2SCH_3$ | H | O | $CO_2CH_3$ | H | |
| $CH_3$ | $CH_2CH_2SCH_3$ | H | O | $SO_2C_2H_5$ | H | |
| $SCH_3$ | $CH_3$ | $CH_3$ | O | $SO_2N(CH_3)_2$ | H | |
| $SCH_3$ | $CH_3$ | H | O | $SO_2(CH_2)_3CH_3$ | H | |
| $SCH_3$ | $CH_3$ | H | O | $S(CH_2)_3CH_3$ | H | |
| $SCH_3$ | $CH_3$ | H | O | $SO_2CH_2CH(CH_3)_2$ | H | |
| $SCH_3$ | $CH_3$ | H | O | $SCH(CH_3)_2$ | H | |
| $OC_2H_5$ | $CH_3$ | H | O | $NO_2$ | 4-F | |
| $SCH_3$ | $CH_3$ | H | O | $NO_2$ | 4-$OCH_3$ | |
| $SCH_3$ | $CH_3$ | $CH_3$ | O | $CO_2CH_2CH_2OCH_3$ | H | |
| $SCH_3$ | $CH_3$ | H | O | $O-CH(CH_3)C_2H_5$ | H | |
| $SCH_3$ | $CH_3$ | $CH_3$ | O | $SO_2(CH_2)CH_3$ | H | |
| $OC_2H_5$ | $C_2H_5$ | H | O | F | H | |
| $CF_3$ | $C_2H_5$ | $CH_3$ | O | $SO_2CH_3$ | H | |
| $SCH_3$ | $CH_3$ | $CH_3$ | O | $SO_2N(OCH_3)CH_3$ | H | |
| $OCH_3$ | $CH_3$ | H | O | $CH_3$ | H | |
| $OCH_3$ | $CH_3$ | H | O | $CH(CH_3)_2$ | H | |
| $OCH_3$ | $CH_3$ | H | O | $OCH_3$ | H | |
| $OCH_3$ | $CH_3$ | H | O | $O(CH_2)_3CH_3$ | H | |
| $OCH_3$ | $CH_3$ | H | O | F | H | |
| $OCH_3$ | $CH_3$ | H | O | Cl | H | |
| $OCH_3$ | $CH_3$ | H | O | Br | H | |
| $OCH_3$ | $CH_3$ | H | O | $CF_3$ | H | |
| $OCH_3$ | $CH_3$ | H | O | $CO_2CH_3$ | H | |
| $OCH_3$ | $CH_3$ | H | O | $CO_2C_2H_5$ | H | |
| $OCH_3$ | $CH_3$ | H | O | $CO_2CH(CH_3)_2$ | H | |
| $OCH_3$ | $CH_3$ | H | O | $CO_2CH_2CH_2CH_2CH_3$ | H | |
| $OCH_3$ | $CH_3$ | H | O | $CO_2CH_2CH_2Cl$ | H | |
| $OCH_3$ | $CH_3$ | H | O | $CO_2CH_2CH=CH_2$ | H | |
| $OCH_3$ | $CH_3$ | H | O | $SO_2N(CH_3)_2$ | H | |

Table 2 (continued)

| $R_1$ | $R_2$ | $R_3$ | W | $R_{16}$ | $R_{17}$ | m.p.(°C) |
|---|---|---|---|---|---|---|
| $OCH_3$ | $CH_3$ | H | O | $SO_2N(CH_3)C_2H_5$ | H | |
| $OCH_3$ | $CH_3$ | H | O | $SO_2N(C_2H_5)_2$ | H | |
| $OCH_3$ | $CH_3$ | H | O | $SO_2N(OCH_3)CH_3$ | H | |
| $OCH_3$ | $CH_3$ | H | O | $SO_2CH_3$ | H | |
| $OCH_3$ | $CH_3$ | H | O | $SOCH_3$ | H | |
| $OCH_3$ | $CH_3$ | H | O | $SCH_3$ | H | |
| $OCH_3$ | $CH_3$ | H | O | $SC_2H_5$ | H | |
| $OCH_3$ | $CH_3$ | H | O | $SO_2C_2H_5$ | H | |
| $OCH_3$ | $CH_3$ | H | O | $SO_2CH_2CH_2CH_3$ | H | |
| $OCH_3$ | $CH_3$ | H | O | $SO_2CH(CH_3)_2$ | H | |
| $OCH_3$ | $CH_3$ | H | O | $SO_2CH_2CH=CH_2$ | H | |
| $OCH_3$ | $CH_3$ | H | O | $SCH_2CH=CH_2$ | H | |
| $OCH_3$ | $CH_3$ | H | O | $SO_2CF_3$ | H | |
| $OCH_3$ | $CH_3$ | H | O | $SO_2CF_2CF_3$ | H | |
| $OC_2H_5$ | $CH_3$ | H | O | Cl | H | |
| $OC_2H_5$ | $CH_3$ | H | O | Br | H | |
| $OC_2H_5$ | $CH_3$ | H | O | $SO_2CH_3$ | H | |
| $OC_2H_5$ | $CH_3$ | H | O | $CO_2CH_3$ | H | |
| $OC_2H_5$ | $CH_3$ | H | O | $CO_2C_2H_5$ | H | |
| $OC_2H_5$ | $CH_3$ | H | O | $CO_2CH_2CH=CH_2$ | H | |
| $OC_2H_5$ | $CH_3$ | H | O | $SO_2N(CH_3)_2$ | H | |
| $OC_2H_5$ | $CH_3$ | H | O | $CH_3$ | H | |
| $OC_2H_5$ | $CH_3$ | H | O | $CH(CH_3)_2$ | H | |
| $OC_2H_5$ | $CH_3$ | H | O | $CO_2CH(CH_3)_2$ | H | |
| $OCH_3$ | $CH_2CF_3$ | H | O | Cl | H | |
| $OCH_3$ | $CH_2CF_3$ | H | O | $SO_2CH_3$ | H | |
| $OCH_3$ | $CH_2CF_3$ | H | O | $SO_2N(CH_3)_2$ | H | |
| $OCH_3$ | $CH_2CF_3$ | H | O | $CO_2CH_3$ | H | |
| $OCH_3$ | $CH_2CF_3$ | H | O | Br | H | |
| Cl | $CH_3$ | H | O | Cl | H | |
| Cl | $CH_3$ | H | O | Br | H | |
| Cl | $CH_3$ | H | O | $CH_3$ | H | |

Table 2 (continued)

| $R_1$ | $R_2$ | $R_3$ | W | $R_{16}$ | $R_{17}$ | m.p.(°C) |
|---|---|---|---|---|---|---|
| Cl | $CH_3$ | H | O | $OCH_3$ | | H |
| Cl | $CH_3$ | H | O | $CF_3$ | | H |
| Cl | $CH_3$ | H | O | $CO_2CH_3$ | | H |
| Cl | $CH_3$ | H | O | $CO_2CH_2CH{=}CH_2$ | | H |
| Cl | $CH_3$ | H | O | $SO_2N(CH_3)_2$ | | H |
| Cl | $CH_3$ | H | O | $SO_2CH_3$ | | H |
| $NHCH_3$ | $CH_3$ | H | O | Cl | | H |
| $NHCH_3$ | $CH_3$ | H | O | $CO_2CH_3$ | | H |
| $NHCH_3$ | $CH_3$ | H | O | $SO_2CH_3$ | | H |
| $CH(OCH_3)_2$ | $CH_3$ | H | O | Cl | | H |
| $CH(OCH_3)_2$ | $CH_3$ | H | O | $CO_2CH_3$ | | H |
| $CH(OCH_3)_2$ | $CH_3$ | H | O | $SO_2N(CH_3)_2$ | | H |
| 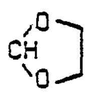 | $CH_3$ | H | O | Cl | | H |
| 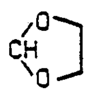 | $CH_3$ | H | O | $CO_2CH_3$ | | H |
| 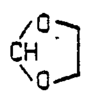 | $CH_3$ | H | O | $CO_2CH_2CH{=}CH_2$ | | H |
| 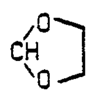 | $CH_3$ | H | O | $SO_2CH_2CH{=}CH_2$ | | H |

Table 3

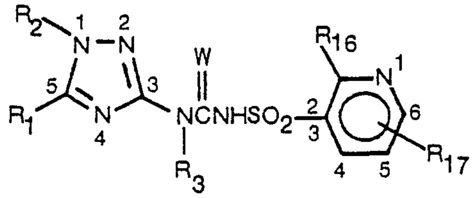

| $R_1$ | $R_2$ | $R_3$ | W | $R_{16}$ | $R_{17}$ | m.p.(°C) |
|---|---|---|---|---|---|---|
| H | $C_2H_5$ | H | O | $CO_2CH_3$ | H | |
| H | $n$-$C_3H_7$ | H | O | Cl | H | |
| $CH_3$ | $CH_2CH(CH_3)_2$ | H | O | $CO_2CH_3$ | H | |
| $CH_3$ | $C_2H_5$ | H | O | Cl | H | |
| $CH_3$ | $C_2H_5$ | H | O | H | H | |
| $CH_3$ | $C_2H_5$ | H | S | Cl | H | |
| $CH_3$ | $C_2H_5$ | H | O | $CO_2CH(CH_3)C_2H_5$ | H | |
| $CH_3$ | $C_2H_5$ | $CH_3$ | O | $CO_2CH_3$ | H | |
| $C_2H_5$ | $CH_3$ | H | O | $CO_2(CH_2)_3CH_3$ | H | |
| $C_2H_5$ | $CH_3$ | $CH_3$ | O | Cl | H | |
| $C_2H_5$ | $CH_3$ | H | S | Cl | H | |
| $C_2H_5$ | $CH_3$ | H | O | $CO_2CH_2CH_2OCH_3$ | H | |
| $C_2H_5$ | $C_2H_5$ | H | O | $CO_2CH_2CH(CH_3)_2$ | H | |
| $n$-$C_4H_9$ | $CH_3$ | H | O | $CO_2CH_3$ | H | |
| $CH(CH_3)_2$ | $CH_3$ | H | O | $NO_2$ | H | |
| $n$-$C_3H_7$ | $CH_3$ | H | O | $CF_3$ | H | |
| $CH_3$ | $n$-$C_3H_7$ | H | O | $CO_2CH_3$ | H | |
| $CH_3$ | $CH_2CH=CH_2$ | H | O | Cl | H | |
| $CH_3$ | $CH_2C(CH_3)=CH_2$ | H | O | $OCH_3$ | H | |
| $CH_2CH=CH_2$ | $CH_3$ | H | O | $C_2H_5$ | H | |
| $CH_2C(CH_3)=CH_2$ | $CH_3$ | H | O | $SO_2CH_3$ | H | |
| $CH_3$ | $CH_2C\equiv CCH_3$ | H | O | $SO_2CH_3$ | H | |
| $CH_2C\equiv C$-$CH_3$ | $CH_3$ | H | O | $SO_2C_2H_5$ | H | |
| $C_2H_5$ | $CH_3$ | H | O | $SO_2N(CH_3)_2$ | H | |
| $C_2H_5$ | $CH_3$ | H | O | $SO_2N(CH_3)OCH_3$ | H | |

Table 3 (continued)

| $R_1$ | $R_2$ | $R_3$ | W | $R_{16}$ | $R_{17}$ | m.p.(°C) |
|---|---|---|---|---|---|---|
| $CH_3$ | $C_2H_5$ | H | O | $SCH_3$ | H | |
| $C_2H_5$ | $CH_3$ | H | O | $S(CH_2)_3CH_3$ | H | |
| $C_2H_5$ | $CH_3$ | H | O | F | H | |
| $C_2H_5$ | $CH_3$ | H | S | $CH_3$ | H | |
| $C_2H_5$ | $CH_3$ | $CH_3$ | O | $SO_2CH_3$ | H | |
| $C_2H_5$ | $C_2H_5$ | H | O | $n$-$C_4H_9$ | H | |
| $CH_3$ | $CH_2C{\equiv}CH$ | H | O | Cl | H | |
| $SCH_3$ | $CH_3$ | H | O | $CH_3$ | H | |
| $SCH_3$ | $CH_3$ | H | O | H | H | |
| $SCH_3$ | $CH_3$ | H | O | Cl | H | |
| $SCH_3$ | $CH_3$ | H | O | $NO_2$ | H | |
| $SCH_3$ | $CH_3$ | H | S | Cl | H | |
| $SCH_3$ | $CH_3$ | $CH_3$ | O | Cl | H | |
| $SCH_3$ | $CH_3$ | H | O | Cl | 5-F | |
| $SCH_3$ | $CH_3$ | H | O | $SO_2CH_3$ | 4-Br | |
| $SCH_3$ | $C_2H_5$ | H | O | $SO_2CH_3$ | 6-Br | |
| $SCH_3$ | $CH_3$ | H | O | $SO_2N(CH_3)_2$ | 6-Cl | |
| $CH_3$ | $C_2H_5$ | H | O | $NO_2$ | 5-F | |
| $C_2H_5$ | $CH_3$ | H | O | $CH_3$ | 4-Cl | |
| $SCH_3$ | $CH_3$ | $CH_3$ | O | Cl | 6-Cl | |
| $SCH_3$ | $C_2H_5$ | H | S | $OCH_3$ | H | |
| $SCH_3$ | $CH_3$ | H | O | $CO_2CH_2CH{=}CH_2$ | H | |
| $SCH_3$ | $CH_3$ | H | O | $CO_2CH(CH_3)_2$ | H | |
| $SCH_3$ | $C_2H_5$ | H | O | $CO_2CH_2CH_2Cl$ | H | |
| $SCH_3$ | $CH_3$ | H | O | $CO_2CH_2CH(CH_3)_2$ | H | |
| $SCH_3$ | $C_2H_5$ | H | O | $n$-$C_4H_9$ | H | |
| $SCH_3$ | $CH_3$ | H | O | $SCH_3$ | H | |
| $SCH_3$ | $CH_3$ | H | O | $S(CH_2)_2CH_3$ | H | |
| $SCH_3$ | $CH_3$ | H | O | F | H | |
| $SC_2H_5$ | $CH_3$ | H | S | $CH_3$ | H | |
| $SC_2H_3$ | $CH_3$ | H | O | $SO_2CH_3$ | H | |

Table 3 (continued)

| $R_1$ | $R_2$ | $R_3$ | W | $R_{16}$ | $R_{17}$ | m.p.(°C) |
|---|---|---|---|---|---|---|
| $S(CH_2)_2CH_3$ | $CH_3$ | H | O | $SO_2CH_3$ | H | |
| $SCH(CH_3)C_2H_5$ | $CH_3$ | H | O | Cl | H | |
| $SCH(CH_3)_2$ | $CH_3$ | H | O | $SO_2C_2H_5$ | H | |
| $SCH_2CH{=}CH_2$ | $CH_3$ | H | O | $CO_2C_2H_5$ | H | |
| $SCH_2C(CH_3){=}CH_2$ | $CH_3$ | H | O | $SO_2CH_3$ | H | |
| $SCH_2C{\equiv}C{-}CH_3$ | $CH_3$ | H | O | $CO_2CH_3$ | H | |
| $SCH_2C{\equiv}CH$ | $CH_3$ | H | O | Cl | H | |
| $SCH_2CO_2CH_3$ | $CH_3$ | H | O | $CF_3$ | H | |
| $SCH_2CO_2CH(CH_3)_2$ | $CH_3$ | H | O | $NO_2$ | H | |
| $SCH(CH_3)CO_2CH_3$ | $CH_3$ | H | O | $CO_2CH_3$ | H | |
| $OCH_3$ | $C_2H_5$ | H | S | Cl | H | |
| $OCH_3$ | $C_2H_5$ | $CH_3$ | O | Cl | H | |
| $OCH_3$ | $C_2H_5$ | H | O | H | H | |
| $OCH_3$ | $C_2H_5$ | H | O | F | H | |
| $OCH_3$ | $C_2H_5$ | H | O | H | 4-F | |
| $OCH_3$ | $C_2H_5$ | H | O | H | 5-F | |
| $OCH_3$ | $C_2H_5$ | H | O | Cl | H | |
| $OCH_3$ | $C_2H_5$ | H | O | $SO_2CH_3$ | H | |
| $OCH_3$ | $C_2H_5$ | H | O | $CF_3$ | H | |
| $OCH_3$ | $C_2H_5$ | H | O | $NO_2$ | H | |
| $OC_2H_5$ | $CH_3$ | H | S | $CH_3$ | H | |
| $OC_2H_5$ | $CH_3$ | H | O | $OC_2H_5$ | H | |
| $OC_2H_5$ | $CH_3$ | $CH_3$ | O | Cl | H | |
| $OC_2H_5$ | $CH_3$ | $CH_3$ | O | $SO_2CH_3$ | H | |
| $OC_2H_5$ | $CH_3$ | H | S | $SO_2CH_3$ | H | |
| $OC_2H_5$ | $CH_3$ | H | O | $SO_2N(CH_3)[(CH_2)_2CH_3]$ | H | |
| $OCH_3$ | $C_2H_5$ | H | O | $SO_2N(CH_3)_2$ | H | |
| $OCH_3$ | $C_2H_5$ | H | O | $SO_2N(CH_3)OCH_3$ | H | |
| $OCH_3$ | $C_2H_5$ | H | O | $SO_2C_2H_5$ | H | |
| $OC_2H_5$ | $CH_3$ | H | O | $SO_2(CH_2)_3CH_3$ | H | |

Table 3 (continued)

| $R_1$ | $R_2$ | $R_3$ | W | $R_{16}$ | $R_{17}$ | m.p.(°C) |
|---|---|---|---|---|---|---|
| $OC_2H_5$ | $CH_3$ | H | O | Cl | 6-$CH_3$ | |
| $OCH_3$ | $C_2H_5$ | H | O | $NO_2$ | 5-Cl | |
| $OCH_3$ | $C_2H_5$ | H | O | Cl | 5-$CH_3$ | |
| $OCH_3$ | $C_2H_5$ | H | O | Br | 4-Cl | |
| $OCH_3$ | $C_2H_5$ | H | O | $OCH_3$ | 5-F | |
| $OCH_3$ | $C_2H_5$ | H | O | $SO_2CH_3$ | 4-Br | |
| $OCH_3$ | $C_2H_5$ | H | O | $SO_2CH_3$ | 6-Cl | |
| $OC_2H_5$ | $CH_3$ | H | O | $SO_2CH_3$ | 5-$OCH_3$ | |
| $OCH_3$ | $C_2H_5$ | H | O | $SO_2CH_3$ | 4-$OCH_3$ | |
| $OCH_3$ | $C_2H_5$ | H | O | $SCH_3$ | H | |
| $OC_2H_5$ | $CH_3$ | H | O | $S(CH_2)_3CH_3$ | H | |
| $OC_2H_5$ | $CH_3$ | H | O | $SO_2N(CH_3)C_2H_5$ | H | |
| $OC_2H_5$ | $CH_3$ | H | O | $CF_3$ | H | |
| $OCH_3$ | $C_2H_5$ | H | O | Br | H | |
| $OCH_3$ | $C_2H_5$ | H | O | $NO_2$ | H | |
| $OCH_3$ | $C_2H_5$ | H | O | $n$-$C_3H_7$ | H | |
| $OC_2H_5$ | $CH_3$ | H | O | $OCH_2(CH_2)_2CH_3$ | H | |
| $OC_2H_5$ | $CH_3$ | H | O | $OCH_2CH(CH_3)_2$ | H | |
| $OC_2H_5$ | $CH_3$ | H | O | $OC_2H_5$ | H | |
| $OCH_3$ | $CH_3$ | H | O | $CO_2CH_2CH_2OCH_3$ | H | |
| $OCH_3$ | $C_2H_5$ | H | O | $CO_2(CH_2)_3OCH_3$ | H | |
| $OCH_3$ | $C_2H_5$ | H | O | $CO_2(CH_2)_2OCH_3$ | H | |
| $OC_2H_5$ | $C_2H_5$ | H | O | $CO_2CH_2CH_2Cl$ | H | |
| $OCH_3$ | $C_2H_5$ | $CH_3$ | O | $CO_2C_2H_5$ | H | |
| $OCH_3$ | $C_2H_5$ | $CH_3$ | O | $CO_2CH_3$ | H | |
| $OC_2H_5$ | $C_2H_5$ | $CH_3$ | O | $SO_2N(C_2H_5)_2$ | H | |
| $OCH_2CH{=}CH_2$ | $CH_3$ | H | O | $SO_2CH_3$ | H | |
| $OCH_2C(CH_3){=}CH_2$ | $CH_3$ | H | O | Cl | H | |
| $OCH(CH_3)C_2H_5$ | $CH_3$ | H | O | $SO_2CH_3$ | H | |
| $O(CH_2)_2CH_3$ | $CH_3$ | H | O | $SO_2CH_3$ | H | |
| $OCH(CH_3)_2$ | $CH_3$ | H | O | $CO_2C_2H_5$ | H | |

Table 3 (continued)

| $R_1$ | $R_2$ | $R_3$ | W | $R_{16}$ | $R_{17}$ | m.p.(°C) |
|---|---|---|---|---|---|---|
| $O(CH_2)_2CH_3$ | $CH_3$ | H | O | $CO_2CH(CH_3)_2$ | H | |
| $OCH_2CH=CHCH_3$ | $CH_3$ | H | O | Cl | H | |
| $OCH_2C\equiv CCH_3$ | $CH_3$ | H | O | $SO_2CH_3$ | H | |
| $CH_2OCH_3$ | $CH_3$ | H | O | $NO_2$ | H | |
| $CH_2OCH_3$ | $CH_3$ | H | O | $SO_2N(CH_3)[CH(CH_3)_2]$ | H | |
| $CH_2OCH_3$ | $CH_3$ | H | O | $SO_2N(CH_3)_2$ | H | |
| $CH_2OCH_3$ | $CH_3$ | H | O | $SO_2CH_3$ | H | |
| $CH_2OCH_3$ | $CH_3$ | H | O | Cl | H | |
| $CH_2OCH_3$ | $CH_3$ | H | O | F | H | |
| $CH_2OCH_3$ | $CH_3$ | H | O | H | H | |
| $CH_2OCH_3$ | $CH_3$ | H | S | Cl | H | |
| $CH_2OCH_3$ | $CH_3$ | $CH_3$ | O | $CH_3$ | H | |
| $CH_2OCH_3$ | $CH_3$ | $CH_3$ | O | Cl | H | |
| $CH_2OCH_3$ | $CH_3$ | H | O | $SC_2H_5$ | H | |
| $CH_2OCH_3$ | $CH_3$ | H | O | $OCH_3$ | H | |
| $CH_2OCH_3$ | $CH_3$ | H | O | $CO_2CH_2CH_2Cl$ | H | |
| $CH_2OC_2H_5$ | $CH_3$ | $CH_3$ | O | $CO_2CH(CH_3)_2$ | H | |
| $CH_2OC_2H_5$ | $CH_3$ | H | O | $CO_2CH_2CH=CH_2$ | H | |
| $CH_2O(CH_2)_3CH_3$ | $CH_3$ | H | O | $CO_2CH_3$ | H | |
| $CH_2CH(CH_3)_2$ | $CH_3$ | H | O | Cl | H | |
| $CH_2CH_2OCH_3$ | $CH_3$ | H | O | $CO_2C_2H_5$ | H | |
| $CH_2CH_2OCH(CH_3)_2$ | $CH_3$ | H | O | $CO_2CH(CH_3)_2$ | H | |
| $CH_2CH_2O(CH_2)_2CH_3$ | $CH_3$ | H | O | $SO_2CH_3$ | H | |
| $N(CH_3)_2$ | $CH_3$ | H | O | Cl | H | |
| $CF_2CF_3$ | $CH_3$ | H | O | $SO_2CH_3$ | H | |
| $CF_3$ | $CH_3$ | H | O | Cl | H | |
| $CF_2CF_3$ | $CH_3$ | H | O | $CO_2CH_3$ | H | |
| $CF_2CF_3$ | $CH_3$ | H | O | $NO_2$ | H | |
| $CF_2CF_3$ | $CH_3$ | H | O | $SO_2CH(CH_3)_2$ | H | |
| $CH_3$ | $CH_2OCH_3$ | H | S | Cl | H | |
| $CH_3$ | $CH_2OCH_3$ | H | O | $SO_2C_2H_5$ | H | |

Table 3 (continued)

| $R_1$ | $R_2$ | $R_3$ | W | $R_{16}$ | $R_{17}$ | m.p.(°C) |
|---|---|---|---|---|---|---|
| $CH_3$ | $CH_2OCH_3$ | H | O | $CO_2CH_3$ | H | |
| $CH_3$ | $CH_2CH_2OCH_3$ | H | O | Cl | H | |
| $CH_3$ | $CH_2SCH_3$ | H | O | $CO_2CH_3$ | H | |
| $CH_3$ | $CH_2CH_2SCH_3$ | H | O | $SO_2C_2H_5$ | H | |
| $SCH_3$ | $CH_3$ | $CH_3$ | O | $SO_2N(CH_3)_2$ | H | |
| $SCH_3$ | $CH_3$ | H | O | $SO_2(CH_2)_3CH_3$ | H | |
| $SCH_3$ | $CH_3$ | H | O | $S(CH_2)_3CH_3$ | H | |
| $SCH_3$ | $CH_3$ | H | O | $SO_2CH_2CH(CH_3)_2$ | H | |
| $SCH_3$ | $CH_3$ | H | O | $SCH(CH_3)_2$ | H | |
| $OC_2H_5$ | $CH_3$ | H | O | $NO_2$ | 4-F | |
| $SCH_3$ | $CH_3$ | H | O | $NO_2$ | 4-$OCH_3$ | |
| $SCH_3$ | $CH_3$ | $CH_3$ | O | $CO_2CH_2CH_2OCH_3$ | H | |
| $SCH_3$ | $CH_3$ | H | O | $O-CH(CH_3)C_2H_5$ | H | |
| $SCH_3$ | $CH_3$ | $CH_3$ | O | $SO_2(CH_2)CH_3$ | H | |
| $OC_2H_5$ | $C_2H_5$ | H | O | F | H | |
| $CF_3$ | $C_2H_5$ | $CH_3$ | O | $SO_2CH_3$ | H | |
| $SCH_3$ | $CH_3$ | $CH_3$ | O | $SO_2N(OCH_3)CH_3$ | H | |
| $OCH_3$ | $CH_3$ | H | O | $CH_3$ | H | |
| $OCH_3$ | $CH_3$ | H | O | $CH(CH_3)_2$ | H | |
| $OCH_3$ | $CH_3$ | H | O | $OCH_3$ | H | |
| $OCH_3$ | $CH_3$ | H | O | $O(CH_2)_3CH_3$ | H | |
| $OCH_3$ | $CH_3$ | H | O | F | H | |
| $OCH_3$ | $CH_3$ | H | O | Cl | H | 165-168° |
| $OCH_3$ | $CH_3$ | H | O | Br | H | |
| $OCH_3$ | $CH_3$ | H | O | $CF_3$ | H | |
| $OCH_3$ | $CH_3$ | H | O | $CO_2CH_3$ | H | |
| $OCH_3$ | $CH_3$ | H | O | $CO_2C_2H_5$ | H | |
| $OCH_3$ | $CH_3$ | H | O | $CO_2CH(CH_3)_2$ | H | |
| $OCH_3$ | $CH_3$ | H | O | $CO_2CH_2CH_2CH_2CH_3$ | H | |
| $OCH_3$ | $CH_3$ | H | O | $CO_2CH_2CH_2Cl$ | H | |
| $OCH_3$ | $CH_3$ | H | O | $CO_2CH_2CH=CH_2$ | H | |
| $OCH_3$ | $CH_3$ | H | O | $SO_2N(CH_3)_2$ | H | |

Table 3 (continued)

| $R_1$ | $R_2$ | $R_3$ | W | $R_{16}$ | $R_{17}$ | m.p.(°C) |
|---|---|---|---|---|---|---|
| $OCH_3$ | $CH_3$ | H | O | $SO_2N(CH_3)C_2H_5$ | H | |
| $OCH_3$ | $CH_3$ | H | O | $SO_2N(C_2H_5)_2$ | H | |
| $OCH_3$ | $CH_3$ | H | O | $SO_2N(OCH_3)CH_3$ | H | |
| $OCH_3$ | $CH_3$ | H | O | $SO_2CH_3$ | H | |
| $OCH_3$ | $CH_3$ | H | O | $SOCH_3$ | H | |
| $OCH_3$ | $CH_3$ | H | O | $SCH_3$ | H | |
| $OCH_3$ | $CH_3$ | H | O | $SC_2H_5$ | H | |
| $OCH_3$ | $CH_3$ | H | O | $SO_2C_2H_5$ | H | |
| $OCH_3$ | $CH_3$ | H | O | $SO_2CH_2CH_2CH_3$ | H | |
| $OCH_3$ | $CH_3$ | H | O | $SO_2CH(CH_3)_2$ | H | |
| $OCH_3$ | $CH_3$ | H | O | $SO_2CH_2CH=CH_2$ | H | |
| $OCH_3$ | $CH_3$ | H | O | $SCH_2CH=CH_2$ | H | |
| $OCH_3$ | $CH_3$ | H | O | $SO_2CF_3$ | H | |
| $OCH_3$ | $CH_3$ | H | O | $SO_2CF_2CF_3$ | H | |
| $OC_2H_5$ | $CH_3$ | H | O | Cl | H | |
| $OC_2H_5$ | $CH_3$ | H | O | Br | H | |
| $OC_2H_5$ | $CH_3$ | H | O | $SO_2CH_3$ | H | |
| $OC_2H_5$ | $CH_3$ | H | O | $CO_2CH_3$ | H | |
| $OC_2H_5$ | $CH_3$ | H | O | $CO_2C_2H_5$ | H | |
| $OC_2H_5$ | $CH_3$ | H | O | $CO_2CH_2CH=CH_2$ | H | |
| $OC_2H_5$ | $CH_3$ | H | O | $SO_2N(CH_3)_2$ | H | |
| $OC_2H_5$ | $CH_3$ | H | O | $CH_3$ | H | |
| $OC_2H_5$ | $CH_3$ | H | O | $CH(CH_3)_2$ | H | |
| $OC_2H_5$ | $CH_3$ | H | O | $CO_2CH(CH_3)_2$ | H | |
| $OCH_3$ | $CH_2CF_3$ | H | O | Cl | H | |
| $OCH_3$ | $CH_2CF_3$ | H | O | $SO_2CH_3$ | H | |
| $OCH_3$ | $CH_2CF_3$ | H | O | $SO_2N(CH_3)_2$ | H | |
| $OCH_3$ | $CH_2CF_3$ | H | O | $CO_2CH_3$ | H | |
| $OCH_3$ | $CH_2CF_3$ | H | O | Br | H | |
| Cl | $CH_3$ | H | O | Cl | H | |
| Cl | $CH_3$ | H | O | Br | H | |
| Cl | $CH_3$ | H | O | $CH_3$ | H | |

Table 3 (continued)

| $R_1$ | $R_2$ | $R_3$ | W | $R_{16}$ | $R_{17}$ | m.p.(°C) |
|---|---|---|---|---|---|---|
| Cl | $CH_3$ | H | O | $OCH_3$ | | H |
| Cl | $CH_3$ | H | O | $CF_3$ | | H |
| Cl | $CH_3$ | H | O | $CO_2CH_3$ | | H |
| Cl | $CH_3$ | H | O | $CO_2CH_2CH{=}CH_2$ | | H |
| Cl | $CH_3$ | H | O | $SO_2N(CH_3)_2$ | | H |
| Cl | $CH_3$ | H | O | $SO_2CH_3$ | | H |
| $NHCH_3$ | $CH_3$ | H | O | Cl | | H |
| $NHCH_3$ | $CH_3$ | H | O | $CO_2CH_3$ | | H |
| $NHCH_3$ | $CH_3$ | H | O | $SO_2CH_3$ | | H |
| $CH(OCH_3)_2$ | $CH_3$ | H | O | Cl | | H |
| $CH(OCH_3)_2$ | $CH_3$ | H | O | $CO_2CH_3$ | | H |
| $CH(OCH_3)_2$ | $CH_3$ | H | O | $SO_2N(CH_3)_2$ | | H |
| 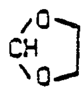 | $CH_3$ | H | O | Cl | | H |
| 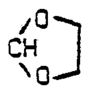 | $CH_3$ | H | O | $CO_2CH_3$ | | H |
| 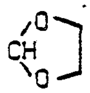 | $CH_3$ | H | O | $CO_2CH_2CH{=}CH_2$ | | H |
| 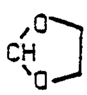 | $CH_3$ | H | O | $SO_2CH_2CH{=}CH_2$ | | H |

Table 4

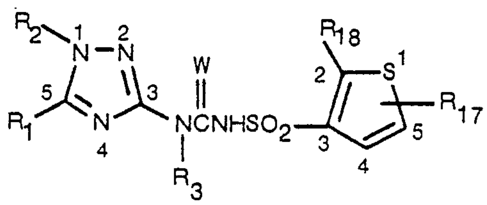

| $R_1$ | $R_2$ | $R_3$ | W | $R_{17}$ | $R_{18}$ | m.p.(°C) |
|---|---|---|---|---|---|---|
| H | $C_2H_5$ | H | O | H | $CO_2CH_3$ | |
| H | $n$-$C_3H_7$ | H | O | H | $CO_2C_2H_5$ | |
| $CH_3$ | $C_2H_5$ | H | O | H | Br | |
| $CH_3$ | $C_2H_5$ | H | O | H | $CO_2CH(CH_3)_2$ | |
| $CH_3$ | $CH_2CH(CH_3)_2$ | H | O | H | $CO_2CH_3$ | |
| $CH_3$ | $C_2H_5$ | H | O | H | $NO_2$ | |
| $CH_3$ | $C_2H_5$ | H | O | H | H | |
| $CH_3$ | $C_2H_5$ | H | S | H | Cl | |
| $CH_3$ | $C_2H_5$ | H | O | H | Cl | |
| $CH_3$ | $C_2H_5$ | $CH_3$ | O | H | $CO_2CH_2CH{=}CH_2$ | |
| $CH_3$ | $C_2H_5$ | H | O | H | $CO_2CH_2CH_2Cl$ | |
| $CH_3$ | $C_2H_5$ | $CH_3$ | O | H | $CO_2CH_3$ | |
| $CH_3$ | $C_2H_5$ | $CH_3$ | O | H | $SO_2CH_3$ | |
| $C_2H_5$ | $CH_3$ | $CH_3$ | O | H | $SO_2CH_3$ | |
| $C_2H_5$ | $CH_3$ | $CH_3$ | O | H | $OCH_3$ | |
| $CH_3$ | $C_2H_5$ | $CH_3$ | O | H | $SO_2N(CH_3)_2$ | |
| $CH_3$ | $C_2H_5$ | H | S | H | $NO_2$ | |
| $CH_3$ | $C_2H_5$ | H | O | H | $SO_2N(CH_3)OCH_3$ | |
| $C_2H_5$ | $CH_3$ | H | S | H | $SO_2CH_3$ | |
| $C_2H_5$ | $CH_3$ | H | O | 5-F | $SO_2CH_3$ | |
| $C_2H_5$ | $CH_3$ | H | O | 4-F | Br | |
| $CH_3$ | $C_2H_5$ | H | O | 5-Cl | Br | |
| $CH_3$ | $C_2H_5$ | H | O | 5-$CH_3$ | Cl | |
| $C_2H_5$ | $C_2H_5$ | H | O | H | Br | |
| $C_2H_5$ | $C_2H_5$ | H | O | H | $CO_2CH_3$ | |

Table 4 (continued)

| $R_1$ | $R_2$ | $R_3$ | W | $R_{17}$ | $R_{18}$ | m.p.(°C) |
|---|---|---|---|---|---|---|
| $C_2H_5$ | $C_2H_5$ | H | O | H | $SO_2CH_3$ | |
| $C_2H_5$ | $C_2H_5$ | H | O | H | $SO_2N(CH_3)C_2H_5$ | |
| $CH_3$ | $C_2H_5$ | H | O | H | $SO_2(CH_2)_2CH_3$ | |
| $C_2H_5$ | $CH_3$ | H | O | H | $SCH_3$ | |
| $C_2H_5$ | $C_2H_5$ | $CH_3$ | O | H | $SO_2CH_3$ | |
| $C_2H_5$ | $C_2H_5$ | $CH_3$ | O | H | $CO_2CH_3$ | |
| $C_2H_5$ | $C_2H_5$ | H | O | H | H | |
| $CH_3$ | $C_2H_5$ | H | O | 5-F | H | |
| $CH_3$ | $C_2H_5$ | H | O | 5-Cl | H | |
| $C_2H_5$ | $C_2H_5$ | H | O | H | $SO_2N(CH_3)_2$ | |
| $C_2H_5$ | $C_2H_5$ | H | S | H | Br | |
| $CH_3$ | $C_2H_5$ | H | O | H | F | |
| $C_2H_5$ | $C_2H_5$ | H | O | H | F | |
| $n$-$C_3H_7$ | $CH_3$ | H | O | H | $CO_2CH_3$ | |
| $CH(CH_3)_2$ | $CH_3$ | H | O | H | $SO_2CH_3$ | |
| $CH_2CH(CH_3)_2$ | $CH_3$ | H | O | H | $CO_2C_2H_5$ | |
| $CH_3$ | $n$-$C_3H_7$ | H | O | H | Br | |
| $CH_3$ | $CH_2CH=CH_2$ | H | O | H | $CO_2CH(CH_3)_2$ | |
| $CH_3$ | $CH_2C(CH_3)=CH_2$ | H | O | H | $CO_2CH_3$ | |
| $CH_2CH=CH_2$ | $CH_3$ | H | O | H | $C_2H_5$ | |
| $CH_2C(CH_3)=CH_2$ | $CH_3$ | H | O | H | $SO_2CH_3$ | |
| $CH_3$ | $CH_2C\equiv CCH_3$ | H | O | H | $CO_2CH_3$ | |
| $CH_2C\equiv CCH_3$ | $CH_3$ | H | O | H | $SO_2C_2H_5$ | |
| $SCH_3$ | $CH_3$ | H | S | H | Br | |
| $SCH_3$ | $CH_3$ | $CH_3$ | O | H | Br | |
| $SCH_3$ | $CH_3$ | H | O | 4-Cl | Br | |
| $SCH_3$ | $CH_3$ | H | O | 5-$OCH_3$ | H | |
| $SCH_3$ | $CH_3$ | H | O | 5-Cl | Cl | |
| $SCH_3$ | $CH_3$ | H | O | H | $NO_2$ | |
| $SCH_3$ | $CH_3$ | H | O | H | $SO_2N(CH_3)C_2H_5$ | |
| $SCH_3$ | $CH_3$ | H | O | H | $SO_2N(CH_3)[CH(CH_3)_2]$ | |

Table 4 (continued)

| $R_1$ | $R_2$ | $R_3$ | W | $R_{17}$ | $R_{18}$ | m.p.(°C) |
|---|---|---|---|---|---|---|
| $SCH_3$ | $CH_3$ | H | O | H | F | |
| $SCH_3$ | $CH_3$ | H | O | 5-F | H | |
| $SCH_3$ | $CH_3$ | H | O | 4-Br | H | |
| $SCH_3$ | $CH_3$ | H | O | 5-Br | H | |
| $SCH_3$ | $CH_3$ | $CH_3$ | O | H | $SO_2CH_3$ | |
| $SCH_3$ | $CH_3$ | H | O | H | $CO_2CH_3$ | 200-202° |
| $SCH_3$ | $CH_3$ | $CH_3$ | O | H | $SO_2N(CH_3)_2$ | |
| $SCH_3$ | $CH_3$ | H | O | H | $OC_2H_5$ | |
| $SCH_3$ | $CH_3$ | H | S | H | H | |
| $SCH_3$ | $CH_3$ | H | O | H | $CO_2(CH_2)_3CH_3$ | |
| $SCH_3$ | $CH_3$ | H | O | H | $CO_2CH_2CH_2OCH_3$ | |
| $SCH_3$ | $CH_3$ | H | O | 5-Cl | H | |
| $SCH_3$ | $CH_3$ | H | O | H | $OCH(CH_3)C_2H_5$ | |
| $SCH_3$ | $CH_3$ | H | O | 5-$CH_3$ | $CH_3$ | |
| $SCH_3$ | $CH_3$ | H | O | H | $SCH_3$ | |
| $SCH_3$ | $CH_3$ | H | O | H | $S(CH_2)_3CH_3$ | |
| $SCH_3$ | $C_2H_5$ | H | O | H | $SO_2C_2H_5$ | |
| $SCH_3$ | $CH_3$ | H | O | H | $CO_2CH(CH_3)_2$ | |
| $SCH_3$ | $CH_3$ | H | O | H | $CO_2CH_2CH=CH_2$ | |
| $SCH_3$ | $C_2H_5$ | H | O | H | $CO_2CH_3$ | |
| $SCH_3$ | $C_2H_5$ | H | O | H | Br | |
| $SCH_3$ | $C_2H_5$ | H | O | H | $SO_2CH_3$ | |
| $SCH_3$ | $C_2H_5$ | H | S | H | Cl | |
| $SCH_3$ | $C_2H_5$ | H | O | H | $NO_2$ | |
| $SC_2H_5$ | $CH_3$ | H | O | H | $CO_2CH(CH_3)_2$ | |
| $SC_2H_5$ | $CH_3$ | H | O | H | $CH_3$ | |
| $SC_2H_5$ | $CH_3$ | H | O | H | $CO_2CH_3$ | |
| $SC_2H_5$ | $CH_3$ | H | O | H | $SO_2CH_3$ | |
| $SC_2H_5$ | $CH_3$ | H | O | H | Br | |
| $SC_2H_5$ | $CH_3$ | $CH_3$ | O | H | Cl | |
| $SC_2H_5$ | $CH_3$ | H | S | H | Br | |

Table 4 (continued)

| $R_1$ | $R_2$ | $R_3$ | W | $R_{17}$ | $R_{18}$ | m.p.(°C) |
|---|---|---|---|---|---|---|
| $SCH(CH_3)_2$ | $CH_3$ | H | O | H | $CO_2CH_3$ | |
| $S(CH_2)_3CH_3$ | $CH_3$ | H | O | H | $SO_2CH_3$ | |
| $SCH_2CH{=}CH_2$ | $CH_3$ | H | O | H | $CO_2CH_3$ | |
| $S(CH_2)_2CH_3$ | $CH_3$ | H | O | H | $CO_2CH_3$ | |
| $SCH_2C(CH_3){=}CH_2$ | $CH_3$ | H | O | H | $CO_2C_2H_5$ | |
| $SCH_2C{\equiv}CCH_3$ | $CH_3$ | H | O | H | $CO_2CH_3$ | |
| $SCH_2CO_2CH_3$ | $CH_3$ | H | O | H | $NO_2$ | |
| $SCH_2CO_2(CH_2)_3CH_3$ | $CH_3$ | H | O | H | $CO_2CH_3$ | |
| $SCH(CH_3)CO_2CH_3$ | $CH_3$ | H | O | H | $CO_2CH_3$ | |
| $OCH_3$ | $CH_3$ | H | O | H | $CO_2CH_3$ | 183-186° |
| $OCH_3$ | $CH_3$ | H | O | H | $SO_2N(CH_3)C_2H_5$ | |
| $OCH_3$ | $CH_3$ | H | O | H | Br | |
| $OCH_3$ | $CH_3$ | H | O | H | Cl | |
| $OCH_3$ | $CH_3$ | H | O | H | $NO_2$ | |
| $OCH_3$ | $CH_3$ | H | S | H | $SO_2CH_3$ | |
| $OCH_3$ | $CH_3$ | $CH_3$ | O | H | $SO_2CH_3$ | |
| $OCH_3$ | $CH_3$ | H | O | H | $SO_2CH_3$ | |
| $OCH_3$ | $CH_3$ | H | O | H | $SO_2(CH_2)_2CH_3$ | |
| $OCH_3$ | $CH_3$ | H | O | H | $OCH_3$ | |
| $OC_2H_5$ | $CH_3$ | H | O | H | $O(CH_2)_3CH_3$ | |
| $OC_2H_5$ | $CH_3$ | H | O | H | $CO_2CH_2CH{=}CH_2$ | |
| $OCH_3$ | $CH_3$ | H | O | H | $CO_2CH_2CH_2Cl$ | |
| $OCH_3$ | $C_2H_5$ | H | O | H | $CO_2CH(CH_3)_2$ | |
| $OCH_3$ | $C_2H_5$ | H | O | H | $CO_2(CH_2)_2CH_3$ | |
| $OC_2H_5$ | $CH_3$ | H | O | H | $CO_2C_2H_5$ | |
| $OCH_3$ | $C_2H_5$ | $CH_3$ | O | H | $CO_2CH_3$ | |
| $OCH_3$ | $C_2H_5$ | H | S | H | Cl | |
| $OC_2H_5$ | $CH_3$ | H | S | H | Br | |
| $OCH_3$ | $C_2H_5$ | H | O | H | $SO_2CH_3$ | |
| $OCH_3$ | $C_2H_5$ | H | O | H | $SO_2(CH_2)_3CH_3$ | |

Table 4 (continued)

| $R_1$ | $R_2$ | $R_3$ | W | $R_{17}$ | $R_{18}$ | m.p.(°C) |
|---|---|---|---|---|---|---|
| $OCH_3$ | $C_2H_5$ | H | O | H | $SO_2CH_2CH(CH_3)_2$ | |
| $OCH_3$ | $C_2H_5$ | $CH_3$ | O | H | $SO_2CH_3$ | |
| $OCH_3$ | $C_2H_5$ | $CH_3$ | O | 5-Cl | $SO_2CH_3$ | |
| $OCH_3$ | $C_2H_5$ | $CH_3$ | O | 5-Br | $SO_2CH_3$ | |
| $OCH_3$ | $C_2H_5$ | H | O | H | $OC_2H_5$ | |
| $OCH_3$ | $C_2H_5$ | H | O | H | F | |
| $OCH_3$ | $C_2H_5$ | H | O | 5-F | F | |
| $OCH_3$ | $C_2H_5$ | H | O | H | $SC_2H_5$ | |
| $OC_2H_5$ | $CH_3$ | H | O | H | $SO_2N(OCH_3)CH_3$ | |
| $OC_2H_5$ | $CH_3$ | H | O | H | $SO_2N(CH_3)[(CH_2)_2CH_3]$ | |
| $OC_2H_5$ | $CH_3$ | H | S | H | H | |
| $OC_2H_5$ | $CH_3$ | H | S | H | $NO_2$ | |
| $OC_2H_5$ | $CH_3$ | H | O | H | $CO_2CH(CH_3)_2$ | |
| $OC_2H_5$ | $CH_3$ | H | O | H | $CO_2CH_2CH_2Cl$ | |
| $OC_2H_5$ | $CH_3$ | H | O | H | $CH_3$ | |
| $OC_2H_5$ | $CH_3$ | H | O | H | $CH(CH_3)_2$ | |
| $OC_2H_5$ | $CH_3$ | H | O | H | $CH_2CH(CH_3)_2$ | |
| $OC_2H_5$ | $CH_3$ | $CH_3$ | O | H | $CH_3$ | |
| $OC_2H_5$ | $CH_3$ | $CH_3$ | O | H | $SO_2CH_3$ | |
| $OC_2H_5$ | $CH_3$ | $CH_3$ | O | H | Cl | |
| $OC_2H_5$ | $CH_3$ | H | O | H | $C_2H_5$ | |
| $OC_2H_5$ | $CH_3$ | H | O | H | $NO_2$ | |
| $OC_2H_5$ | $CH_3$ | H | O | 4-Br | Br | |
| $OC_2H_5$ | $CH_3$ | H | O | 4-Cl | Cl | |
| $OC_2H_5$ | $C_2H_5$ | H | O | H | Cl | |
| $OC_2H_5$ | $C_2H_5$ | H | O | H | $CO_2CH_3$ | |
| $OC_2H_5$ | $C_2H_5$ | H | O | H | $SO_2CH_3$ | |
| $OC_2H_5$ | $C_2H_5$ | H | O | H | $SO_2N(CH_3)_2$ | |
| $OC_2H_5$ | $C_2H_5$ | H | O | H | $NO_2$ | |
| $OC_2H_5$ | $C_2H_5$ | H | O | H | Br | |
| $OC_2H_5$ | $C_2H_5$ | H | O | H | $CO_2CH(CH_3)_2$ | |

Table 4 (continued)

| $R_1$ | $R_2$ | $R_3$ | W | $R_{17}$ | $R_{18}$ | m.p.(°C) |
|---|---|---|---|---|---|---|
| $OCH_3$ | $C_2H_5$ | H | O | H | $SO_2C_2H_5$ | |
| $OCH_3$ | $C_2H_5$ | H | O | 4-F | F | |
| $OC_2H_5$ | $CH_3$ | H | O | H | $OCH(CH_3)_2$ | |
| $OCH_2CH{=}CH_2$ | $CH_3$ | H | O | H | $SO_2CH_3$ | |
| $OCH_2C(CH_3){=}CH_2$ | $CH_3$ | H | O | H | $CO_2CH_3$ | |
| $OCH(CH_3)C_2H_5$ | $CH_3$ | H | O | H | $CO_2CH_3$ | |
| $OCH(CH_3)_2$ | $CH_3$ | H | O | H | $CO_2C_2H_5$ | |
| $O(CH_2)_2CH_3$ | $CH_3$ | H | O | H | Cl | |
| $OCH_2C{\equiv}CCH_3$ | $CH_3$ | H | O | H | $NO_2$ | |
| $CH_2OCH_3$ | $CH_3$ | H | O | H | Br | |
| $CH_2OCH_3$ | $CH_3$ | H | O | H | Cl | |
| $CH_2OCH_3$ | $CH_3$ | $CH_3$ | O | H | $SO_2CH_3$ | |
| $CH_2OCH_3$ | $CH_3$ | $CH_3$ | O | H | $CO_2CH_3$ | |
| $CH_2OCH_3$ | $CH_3$ | H | O | H | $CO_2CH_3$ | |
| $CH_2OCH_3$ | $CH_3$ | H | O | H | $NO_2$ | |
| $CH_2OCH_3$ | $CH_3$ | H | S | H | Cl | |
| $CH_2OCH_3$ | $CH_3$ | H | O | H | H | |
| $CH_2OCH_3$ | $CH_3$ | H | O | H | $SO_2N(CH_3)_2$ | |
| $CH_2OCH_3$ | $CH_3$ | $CH_3$ | O | H | $SO_2N(CH_3)C_2H_5$ | |
| $CH_2OCH_3$ | $C_2H_5$ | H | O | H | $SO_2C_2H_5$ | |
| $CH_2OCH_3$ | $C_2H_5$ | H | O | H | $CO_2CH_3$ | |
| $CH_2OC_2H_5$ | $CH_3$ | H | O | H | Br | |
| $CH_2OCH(CH_3)_2$ | $CH_3$ | H | O | H | $CO_2CH_3$ | |
| $CH_2O(CH_2)_3CH_3$ | $CH_3$ | H | O | H | $CO_2CH_3$ | |
| $CH_2CH_2OCH_3$ | $CH_3$ | H | O | H | Cl | |
| $CH_2CH_2OCH(CH_3)_2$ | $CH_3$ | H | O | H | $CO_2CH_3$ | |
| $N(CH_3)_2$ | $CH_3$ | H | O | H | $CO_2CH_3$ | |
| $CF_3$ | $CH_3$ | H | O | H | $SO_2CH_3$ | |
| $CF_3$ | $CH_3$ | H | O | H | $CO_2CH_3$ | |
| $CF_3$ | $C_2H_5$ | H | O | H | $CO_2CH(CH_3)_2$ | |
| $CF_3$ | $C_2H_5$ | H | O | H | $SO_2CH_3$ | |

Table 4 (continued)

| $R_1$ | $R_2$ | $R_3$ | W | $R_{17}$ | $R_{18}$ | m.p.(°C) |
|---|---|---|---|---|---|---|
| $CF_3$ | $C_2H_5$ | H | O | H | Br | |
| $CF_2CF_3$ | $CH_3$ | H | O | H | $CO_2CH_3$ | |
| $CF_2CF_3$ | $CH_3$ | H | O | H | Cl | |
| $CH_3$ | $CH_2OCH_3$ | H | O | H | $CO_2CH_3$ | |
| $CH_3$ | $CH_2SCH_3$ | H | O | H | Cl | |
| $CH_3$ | $CH_2CH_2OCH_3$ | H | O | H | $CO_2CH_3$ | |
| $CH_3$ | $CH_2CH_2SCH_3$ | H | O | H | Br | |
| $CH_2OCH_3$ | $C_2H_5$ | H | O | H | $CO_2CH_3$ | |
| $CH_2OCH_3$ | $CH_3$ | $CH_3$ | O | H | $CO_2CH_3$ | |
| $OC_2H_5$ | $CH_3$ | $CH_3$ | O | H | $SO_2(CH_2)_2CH_3$ | |
| $OC_2H_5$ | $CH_3$ | H | O | H | $SO_2CH(CH_3)_2$ | |
| $OC_2H_5$ | $CH_3$ | H | O | H | $SO_2N(C_2H_5)_2$ | |
| $OCH_3$ | $CH_2CF_3$ | H | O | H | $CO_2CH_3$ | 152-155° |
| $OCH_3$ | $CH_3$ | H | O | H | $CH_3$ | |
| $OCH_3$ | $CH_3$ | H | O | H | $C_2H_5$ | |
| $OCH_3$ | $CH_3$ | H | O | H | $CH(CH_3)_2$ | |
| $OCH_3$ | $CH_3$ | H | O | H | $OCH_2CH_2CH_3$ | |
| $OCH_3$ | $CH_3$ | H | O | H | F | |
| $OCH_3$ | $CH_3$ | H | O | H | $CO_2C_2H_5$ | |
| $OCH_3$ | $CH_3$ | H | O | H | $CO_2CH_2CH_2OCH_3$ | |
| $OCH_3$ | $CH_3$ | H | O | H | $CO_2CH_2CH=CH_2$ | |
| $OCH_3$ | $CH_3$ | H | O | H | $SO_2N(CH_3)_2$ | |
| $OCH_3$ | $CH_3$ | H | O | H | $SO_2N(OCH_3)CH_3$ | |
| $OCH_3$ | $CH_3$ | H | O | H | $SO_2CH_2CH=CH_2$ | |
| $OC_2H_5$ | $CH_3$ | H | O | H | F | |
| $OC_2H_5$ | $CH_3$ | H | O | H | Cl | |
| $OC_2H_5$ | $CH_3$ | H | O | H | Br | |
| $OC_2H_5$ | $CH_3$ | H | O | H | $CO_2CH_3$ | 194-197° |
| $OC_2H_5$ | $CH_3$ | H | O | H | $SO_2N(CH_3)_2$ | |
| $OC_2H_5$ | $CH_3$ | H | O | H | $SO_2CH_3$ | |
| $OC_2H_5$ | $CH_3$ | H | O | H | $SO_2CH_2CH=CH_2$ | |

Table 4 (continued)

| $R_1$ | $R_2$ | $R_3$ | W | $R_{17}$ | $R_{18}$ | m.p.(°C) |
|---|---|---|---|---|---|---|
| $OCH_3$ | $CH_2CF_3$ | H | O | H | $CH_3$ | |
| $OCH_3$ | $CH_2CF_3$ | H | O | H | $OCH_3$ | |
| $OCH_3$ | $CH_2CF_3$ | H | O | H | Cl | |
| $OCH_3$ | $CH_2CF_3$ | H | O | H | $NO_2$ | |
| $OCH_3$ | $CH_2CF_3$ | H | O | H | $CO_2CH_2CH_3$ | |
| $OCH_3$ | $CH_2CF_3$ | H | O | H | $SO_2N(CH_3)_2$ | |
| $OCH_3$ | $CH_2CF_3$ | H | O | H | $SO_2CH_3$ | |
| Cl | $CH_3$ | H | O | H | $CH_3$ | |
| Cl | $CH_3$ | H | O | H | $OCH_3$ | |
| Cl | $CH_3$ | H | O | H | Cl | |
| Cl | $CH_3$ | H | O | H | $NO_2$ | |
| Cl | $CH_3$ | H | O | H | $CO_2CH_3$ | |
| Cl | $CH_3$ | H | O | H | $SO_2N(CH_3)_2$ | |
| Cl | $CH_3$ | H | O | H | $SO_2CH_3$ | |
| $NHCH_3$ | $CH_3$ | H | O | H | $CH_3$ | |
| $NHCH_3$ | $CH_3$ | H | O | H | $OCH_3$ | |
| $NHCH_3$ | $CH_3$ | H | O | H | Cl | |
| $NHCH_3$ | $CH_3$ | H | O | H | $NO_2$ | |
| $NHCH_3$ | $CH_3$ | H | O | H | $CO_2CH_3$ | |
| $NHCH_3$ | $CH_3$ | H | O | H | $SO_2N(CH_3)_2$ | |
| $NHCH_3$ | $CH_3$ | H | O | H | $SO_2CH_3$ | |
| $CH(OCH_3)_2$ | $CH_3$ | H | O | H | $CH_3$ | |
| $CH(OCH_3)_2$ | $CH_3$ | H | O | H | $OCH_3$ | |
| $CH(OCH_3)_2$ | $CH_3$ | H | O | H | Cl | |
| $CH(OCH_3)_2$ | $CH_3$ | H | O | H | $NO_2$ | |
| $CH(OCH_3)_2$ | $CH_3$ | H | O | H | $CO_2CH_3$ | |
| $CH(OCH_3)_2$ | $CH_3$ | H | O | H | $SO_2N(CH_3)_2$ | |
| $CH(OCH_3)_2$ | $CH_3$ | H | O | H | $SO_2CH_3$ | |
| 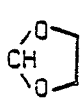 | $CH_3$ | H | O | H | $CH_3$ | |

Table 4 (continued)

| $R_1$ | $R_2$ | $R_3$ | W | $R_{17}$ | $R_{18}$ | m.p.(°C) |
|---|---|---|---|---|---|---|
| 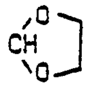 | $CH_3$ | H | O | H | $OCH_3$ | |
| 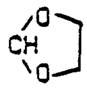 | $CH_3$ | H | O | H | Cl | |
| 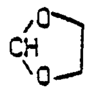 | $CH_3$ | H | O | H | $NO_2$ | |
| 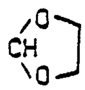 | $CH_3$ | H | O | H | $CO_2CH_3$ | |
| 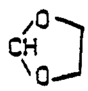 | $CH_3$ | H | O | H | $SO_2N(CH_3)_2$ | |
| 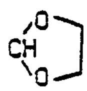 | $CH_3$ | H | O | H | $SO_2CH_3$ | |

Table 5

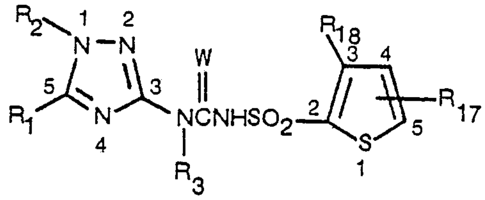

| $R_1$ | $R_2$ | $R_3$ | W | $R_{17}$ | $R_{18}$ | m.p.(°C) |
|---|---|---|---|---|---|---|
| H | $C_2H_5$ | H | O | H | $CO_2CH_3$ | |
| H | $n$-$C_3H_7$ | H | O | H | $CO_2C_2H_5$ | |
| $CH_3$ | $C_2H_5$ | H | O | H | Br | |
| $CH_3$ | $C_2H_5$ | H | O | H | $CO_2CH(CH_3)_2$ | |
| $CH_3$ | $CH_2CH(CH_3)_2$ | H | O | H | $CO_2CH_3$ | |
| $CH_3$ | $C_2H_5$ | H | O | H | $NO_2$ | |
| $CH_3$ | $C_2H_5$ | H | O | H | H | |
| $CH_3$ | $C_2H_5$ | H | S | H | Cl | |
| $CH_3$ | $C_2H_5$ | H | O | H | Cl | |
| $CH_3$ | $C_2H_5$ | $CH_3$ | O | H | $CO_2CH_2CH{=}CH_2$ | |
| $CH_3$ | $C_2H_5$ | H | O | H | $CO_2CH_2CH_2Cl$ | |
| $CH_3$ | $C_2H_5$ | $CH_3$ | O | H | $CO_2CH_3$ | |
| $CH_3$ | $C_2H_5$ | $CH_3$ | O | H | $SO_2CH_3$ | |
| $C_2H_5$ | $CH_3$ | $CH_3$ | O | H | $SO_2CH_3$ | |
| $C_2H_5$ | $CH_3$ | $CH_3$ | O | H | $OCH_3$ | |
| $CH_3$ | $C_2H_5$ | $CH_3$ | O | H | $SO_2N(CH_3)_2$ | |
| $CH_3$ | $C_2H_5$ | H | S | H | $NO_2$ | |
| $CH_3$ | $C_2H_5$ | H | O | H | $SO_2N(CH_3)OCH_3$ | |
| $C_2H_5$ | $CH_3$ | H | S | H | $SO_2CH_3$ | |
| $C_2H_5$ | $CH_3$ | H | O | 5-F | $SO_2CH_3$ | |
| $C_2H_5$ | $CH_3$ | H | O | 4-F | Br | |
| $CH_3$ | $C_2H_5$ | H | O | 5-Cl | Br | |
| $CH_3$ | $C_2H_5$ | H | O | 5-$CH_3$ | Cl | |
| $C_2H_5$ | $C_2H_5$ | H | O | H | Br | |
| $C_2H_5$ | $C_2H_5$ | H | O | H | $CO_2CH_3$ | |

Table 5 (continued)

| $R_1$ | $R_2$ | $R_3$ | W | $R_{17}$ | $R_{18}$ | m.p.(°C) |
|---|---|---|---|---|---|---|
| $C_2H_5$ | $C_2H_5$ | H | O | H | $SO_2CH_3$ | |
| $C_2H_5$ | $C_2H_5$ | H | O | H | $SO_2N(CH_3)C_2H_5$ | |
| $CH_3$ | $C_2H_5$ | H | O | H | $SO_2(CH_2)_2CH_3$ | |
| $C_2H_5$ | $CH_3$ | H | O | H | $SCH_3$ | |
| $C_2H_5$ | $C_2H_5$ | $CH_3$ | O | H | $SO_2CH_3$ | |
| $C_2H_5$ | $C_2H_5$ | $CH_3$ | O | H | $CO_2CH_3$ | |
| $C_2H_5$ | $C_2H_5$ | H | O | H | H | |
| $CH_3$ | $C_2H_5$ | H | O | 5-F | H | |
| $CH_3$ | $C_2H_5$ | H | O | 5-Cl | H | |
| $C_2H_5$ | $C_2H_5$ | H | O | H | $SO_2N(CH_3)_2$ | |
| $C_2H_5$ | $C_2H_5$ | H | S | H | Br | |
| $CH_3$ | $C_2H_5$ | H | O | H | F | |
| $C_2H_5$ | $C_2H_5$ | H | O | H | F | |
| $n$-$C_3H_7$ | $CH_3$ | H | O | H | $CO_2CH_3$ | |
| $CH(CH_3)_2$ | $CH_3$ | H | O | H | $SO_2CH_3$ | |
| $CH_2CH(CH_3)_2$ | $CH_3$ | H | O | H | $CO_2C_2H_5$ | |
| $CH_3$ | $n$-$C_3H_7$ | H | O | H | Br | |
| $CH_3$ | $CH_2CH{=}CH_2$ | H | O | H | $CO_2CH(CH_3)_2$ | |
| $CH_3$ | $CH_2C(CH_3){=}CH_2$ | H | O | H | $CO_2CH_3$ | |
| $CH_2CH{=}CH_2$ | $CH_3$ | H | O | H | $C_2H_5$ | |
| $CH_2C(CH_3){=}CH_2$ | $CH_3$ | H | O | H | $SO_2CH_3$ | |
| $CH_3$ | $CH_2C{\equiv}CCH_3$ | H | O | H | $CO_2CH_3$ | |
| $CH_2C{\equiv}CCH_3$ | $CH_3$ | H | O | H | $SO_2C_2H_5$ | |
| $SCH_3$ | $CH_3$ | H | S | H | Br | |
| $SCH_3$ | $CH_3$ | $CH_3$ | O | H | Br | |
| $SCH_3$ | $CH_3$ | H | O | 4-Cl | Br | |
| $SCH_3$ | $CH_3$ | H | O | 5-$OCH_3$ | H | |
| $SCH_3$ | $CH_3$ | H | O | 5-Cl | Cl | |
| $SCH_3$ | $CH_3$ | H | O | H | $NO_2$ | |
| $SCH_3$ | $CH_3$ | H | O | H | $SO_2N(CH_3)C_2H_5$ | |
| $SCH_3$ | $CH_3$ | H | O | H | $SO_2N(CH_3)[CH(CH_3)_2]$ | |

Table 5 (continued)

| $R_1$ | $R_2$ | $R_3$ | W | $R_{17}$ | $R_{18}$ | m.p.(°C) |
|---|---|---|---|---|---|---|
| $SCH_3$ | $CH_3$ | H | O | H | F | |
| $SCH_3$ | $CH_3$ | H | O | 5-F | H | |
| $SCH_3$ | $CH_3$ | H | O | 4-Br | H | |
| $SCH_3$ | $CH_3$ | H | O | 5-Br | H | |
| $SCH_3$ | $CH_3$ | $CH_3$ | O | H | $SO_2CH_3$ | |
| $SCH_3$ | $CH_3$ | $CH_3$ | O | H | $CO_2CH_3$ | |
| $SCH_3$ | $CH_3$ | $CH_3$ | O | H | $SO_2N(CH_3)_2$ | |
| $SCH_3$ | $CH_3$ | H | O | H | $OC_2H_5$ | |
| $SCH_3$ | $CH_3$ | H | S | H | H | |
| $SCH_3$ | $CH_3$ | H | O | H | $CO_2(CH_2)_3CH_3$ | |
| $SCH_3$ | $CH_3$ | H | O | H | $CO_2CH_2CH_2OCH_3$ | |
| $SCH_3$ | $CH_3$ | H | O | 5-Cl | H | |
| $SCH_3$ | $CH_3$ | H | O | H | $OCH(CH_3)C_2H_5$ | |
| $SCH_3$ | $CH_3$ | H | O | 5-$CH_3$ | $CH_3$ | |
| $SCH_3$ | $CH_3$ | H | O | H | $SCH_3$ | |
| $SCH_3$ | $CH_3$ | H | O | H | $S(CH_2)_3CH_3$ | |
| $SCH_3$ | $C_2H_5$ | H | O | H | $SO_2C_2H_5$ | |
| $SCH_3$ | $CH_3$ | H | O | H | $CO_2CH(CH_3)_2$ | |
| $SCH_3$ | $CH_3$ | H | O | H | $CO_2CH_2CH{=}CH_2$ | |
| $SCH_3$ | $C_2H_5$ | H | O | H | $CO_2CH_3$ | |
| $SCH_3$ | $C_2H_5$ | H | O | H | Br | |
| $SCH_3$ | $C_2H_5$ | H | O | H | $SO_2CH_3$ | |
| $SCH_3$ | $C_2H_5$ | H | S | H | Cl | |
| $SCH_3$ | $C_2H_5$ | H | O | H | $NO_2$ | |
| $SC_2H_5$ | $CH_3$ | H | O | H | $CO_2CH(CH_3)_2$ | |
| $SC_2H_5$ | $CH_3$ | H | O | H | $CH_3$ | |
| $SC_2H_5$ | $CH_3$ | H | O | H | $CO_2CH_3$ | |
| $SC_2H_5$ | $CH_3$ | H | O | H | $SO_2CH_3$ | |
| $SC_2H_5$ | $CH_3$ | H | O | H | Br | |
| $SC_2H_5$ | $CH_3$ | $CH_3$ | O | H | Cl | |
| $SC_2H_5$ | $CH_3$ | H | S | H | Br | |

Table 5 (continued)

| $R_1$ | $R_2$ | $R_3$ | W | $R_{17}$ | $R_{18}$ | m.p.(°C) |
|---|---|---|---|---|---|---|
| $SCH(CH_3)_2$ | $CH_3$ | H | O | H | $CO_2CH_3$ | |
| $S(CH_2)_3CH_3$ | $CH_3$ | H | O | H | $SO_2CH_3$ | |
| $SCH_2CH{=}CH_2$ | $CH_3$ | H | O | H | $CO_2CH_3$ | |
| $S(CH_2)_2CH_3$ | $CH_3$ | H | O | H | $CO_2CH_3$ | |
| $SCH_2C(CH_3){=}CH_2$ | $CH_3$ | H | O | H | $CO_2C_2H_5$ | |
| $SCH_2C{\equiv}CCH_3$ | $CH_3$ | H | O | H | $CO_2CH_3$ | |
| $SCH_2CO_2CH_3$ | $CH_3$ | H | O | H | $NO_2$ | |
| $SCH_2CO_2(CH_2)_3CH_3$ | $CH_3$ | H | O | H | $CO_2CH_3$ | |
| $SCH(CH_3)CO_2CH_3$ | $CH_3$ | H | O | H | $CO_2CH_3$ | |
| $OCH_3$ | $CH_3$ | H | O | H | $CO_2CH_3$ | |
| $OCH_3$ | $CH_3$ | H | O | H | $SO_2N(CH_3)C_2H_5$ | |
| $OCH_3$ | $CH_3$ | H | O | H | Br | |
| $OCH_3$ | $CH_3$ | H | O | H | Cl | |
| $OCH_3$ | $CH_3$ | H | O | H | $NO_2$ | |
| $OCH_3$ | $CH_3$ | H | S | H | $SO_2CH_3$ | |
| $OCH_3$ | $CH_3$ | $CH_3$ | O | H | $SO_2CH_3$ | |
| $OCH_3$ | $CH_3$ | H | O | H | $SO_2CH_3$ | |
| $OCH_3$ | $CH_3$ | H | O | H | $SO_2(CH_2)_2CH_3$ | |
| $OCH_3$ | $CH_3$ | H | O | H | $OCH_3$ | |
| $OC_2H_5$ | $CH_3$ | H | O | H | $O(CH_2)_3CH_3$ | |
| $OC_2H_5$ | $CH_3$ | H | O | H | $CO_2CH_2CH{=}CH_2$ | |
| $OCH_3$ | $CH_3$ | H | O | H | $CO_2CH_2CH_2Cl$ | |
| $OCH_3$ | $C_2H_5$ | H | O | H | $CO_2CH(CH_3)_2$ | |
| $OCH_3$ | $C_2H_5$ | H | O | H | $CO_2(CH_2)_2CH_3$ | |
| $OC_2H_5$ | $CH_3$ | H | O | H | $CO_2C_2H_5$ | |
| $OCH_3$ | $C_2H_5$ | $CH_3$ | O | H | $CO_2CH_3$ | |
| $OCH_3$ | $C_2H_5$ | H | S | H | Cl | |
| $OC_2H_5$ | $CH_3$ | H | S | H | Br | |
| $OCH_3$ | $C_2H_5$ | H | O | H | $SO_2CH_3$ | |
| $OCH_3$ | $C_2H_5$ | H | O | H | $SO_2(CH_2)_3CH_3$ | |

Table 5 (continued)

| $R_1$ | $R_2$ | $R_3$ | W | $R_{17}$ | $R_{18}$ | m.p.(°C) |
|---|---|---|---|---|---|---|
| $OCH_3$ | $C_2H_5$ | H | O | H | $SO_2CH_2CH(CH_3)_2$ | |
| $OCH_3$ | $C_2H_5$ | $CH_3$ | O | H | $SO_2CH_3$ | |
| $OCH_3$ | $C_2H_5$ | $CH_3$ | O | 5-Cl | $SO_2CH_3$ | |
| $OCH_3$ | $C_2H_5$ | $CH_3$ | O | 5-Br | $SO_2CH_3$ | |
| $OCH_3$ | $C_2H_5$ | H | O | H | $OC_2H_5$ | |
| $OCH_3$ | $C_2H_5$ | H | O | H | F | |
| $OCH_3$ | $C_2H_5$ | H | O | 5-F | F | |
| $OCH_3$ | $C_2H_5$ | H | O | H | $SC_2H_5$ | |
| $OC_2H_5$ | $CH_3$ | H | O | H | $SO_2N(OCH_3)CH_3$ | |
| $OC_2H_5$ | $CH_3$ | H | O | H | $SO_2N(CH_3)[(CH_2)_2CH_3]$ | |
| $OC_2H_5$ | $CH_3$ | H | S | H | H | |
| $OC_2H_5$ | $CH_3$ | H | S | H | $NO_2$ | |
| $OC_2H_5$ | $CH_3$ | H | O | H | $CO_2CH(CH_3)_2$ | |
| $OC_2H_5$ | $CH_3$ | H | O | H | $CO_2CH_2CH{=}CH_2$ | |
| $OC_2H_5$ | $CH_3$ | H | O | H | $CO_2CH_2CH_2Cl$ | |
| $OC_2H_5$ | $CH_3$ | H | O | H | $CH_3$ | |
| $OC_2H_5$ | $CH_3$ | H | O | H | $CH(CH_3)_2$ | |
| $OC_2H_5$ | $CH_3$ | H | O | H | $CH_2CH(CH_3)_2$ | |
| $OC_2H_5$ | $CH_3$ | $CH_3$ | O | H | $CH_3$ | |
| $OC_2H_5$ | $CH_3$ | $CH_3$ | O | H | $SO_2CH_3$ | |
| $OC_2H_5$ | $CH_3$ | $CH_3$ | O | H | Cl | |
| $OC_2H_5$ | $CH_3$ | H | O | H | $C_2H_5$ | |
| $OC_2H_5$ | $CH_3$ | H | O | H | $NO_2$ | |
| $OC_2H_5$ | $CH_3$ | H | O | 4-Br | Br | |
| $OC_2H_5$ | $CH_3$ | H | O | 4-Cl | Cl | |
| $OC_2H_5$ | $C_2H_5$ | H | O | H | Cl | |
| $OC_2H_5$ | $C_2H_5$ | H | O | H | $CO_2CH_3$ | |
| $OC_2H_5$ | $C_2H_5$ | H | O | H | $SO_2CH_3$ | |
| $OC_2H_5$ | $C_2H_5$ | H | O | H | $SO_2N(CH_3)_2$ | |

Table 5 (continued)

| $R_1$ | $R_2$ | $R_3$ | W | $R_{17}$ | $R_{18}$ | m.p.(°C) |
|---|---|---|---|---|---|---|
| $OC_2H_5$ | $C_2H_5$ | H | O | H | $NO_2$ | |
| $OC_2H_5$ | $C_2H_5$ | H | O | H | Br | |
| $OC_2H_5$ | $C_2H_5$ | H | O | H | $CO_2CH(CH_3)_2$ | |
| $OCH_3$ | $C_2H_5$ | H | O | H | $SO_2C_2H_5$ | |
| $OCH_3$ | $C_2H_5$ | H | O | 4-F | F | |
| $OC_2H_5$ | $CH_3$ | H | O | H | $OCH(CH_3)_2$ | |
| $OCH_2CH{=}CH_2$ | $CH_3$ | H | O | H | $SO_2CH_3$ | |
| $OCH_2C(CH_3){=}CH_2$ | $CH_3$ | H | O | H | $CO_2CH_3$ | |
| $OCH(CH_3)C_2H_5$ | $CH_3$ | H | O | H | $CO_2CH_3$ | |
| $OCH(CH_3)_2$ | $CH_3$ | H | O | H | $CO_2C_2H_5$ | |
| $O(CH_2)_2CH_3$ | $CH_3$ | H | O | H | Cl | |
| $OCH_2C{\equiv}CCH_3$ | $CH_3$ | H | O | H | $NO_2$ | |
| $CH_2OCH_3$ | $CH_3$ | H | O | H | Br | |
| $CH_2OCH_3$ | $CH_3$ | H | O | H | Cl | |
| $CH_2OCH_3$ | $CH_3$ | $CH_3$ | O | H | $SO_2CH_3$ | |
| $CH_2OCH_3$ | $CH_3$ | $CH_3$ | O | H | $CO_2CH_3$ | |
| $CH_2OCH_3$ | $CH_3$ | H | O | H | $CO_2CH_3$ | |
| $CH_2OCH_3$ | $CH_3$ | H | O | H | $NO_2$ | |
| $CH_2OCH_3$ | $CH_3$ | H | S | H | Cl | |
| $CH_2OCH_3$ | $CH_3$ | H | O | H | H | |
| $CH_2OCH_3$ | $CH_3$ | H | O | H | $SO_2N(CH_3)_2$ | |
| $CH_2OCH_3$ | $CH_3$ | $CH_3$ | O | H | $SO_2N(CH_3)C_2H_5$ | |
| $CH_2OCH_3$ | $C_2H_5$ | H | O | H | $SO_2C_2H_5$ | |
| $CH_2OCH_3$ | $C_2H_5$ | H | O | H | $CO_2CH_3$ | |
| $CH_2OC_2H_5$ | $CH_3$ | H | O | H | Br | |
| $CH_2OCH(CH_3)_2$ | $CH_3$ | H | O | H | $CO_2CH_3$ | |
| $CH_2O(CH_2)_3CH_3$ | $CH_3$ | H | O | H | $CO_2CH_3$ | |
| $CH_2CH_2OCH_3$ | $CH_3$ | H | O | H | Cl | |
| $CH_2CH_2OCH(CH_3)_2$ | $CH_3$ | H | O | H | $CO_2CH_3$ | |
| $N(CH_3)_2$ | $CH_3$ | H | O | H | $CO_2CH_3$ | |
| $CF_3$ | $CH_3$ | H | O | H | $SO_2CH_3$ | |

Table 5 (continued)

| $R_1$ | $R_2$ | $R_3$ | W | $R_{17}$ | $R_{18}$ | m.p.(°C) |
|---|---|---|---|---|---|---|
| $CF_3$ | $CH_3$ | H | O | H | $CO_2CH_3$ | |
| $CF_3$ | $C_2H_5$ | H | O | H | $CO_2CH(CH_3)_2$ | |
| $CF_3$ | $C_2H_5$ | H | O | H | $SO_2CH_3$ | |
| $CF_3$ | $C_2H_5$ | H | O | H | Br | |
| $CF_2CF_3$ | $CH_3$ | H | O | H | $CO_2CH_3$ | |
| $CF_2CF_3$ | $CH_3$ | H | O | H | Cl | |
| $CH_3$ | $CH_2OCH_3$ | H | O | H | $CO_2CH_3$ | |
| $CH_3$ | $CH_2SCH_3$ | H | O | H | Cl | |
| $CH_3$ | $CH_2CH_2OCH_3$ | H | O | H | $CO_2CH_3$ | |
| $CH_3$ | $CH_2CH_2SCH_3$ | H | O | H | Br | |
| $CH_2OCH_3$ | $C_2H_5$ | H | O | H | $CO_2CH_3$ | |
| $CH_2OCH_3$ | $CH_3$ | $CH_3$ | O | H | $CO_2CH_3$ | |
| $OC_2H_5$ | $CH_3$ | $CH_3$ | O | H | $SO_2(CH_2)_2CH_3$ | |
| $OC_2H_5$ | $CH_3$ | H | O | H | $SO_2CH(CH_3)_2$ | |
| $OC_2H_5$ | $CH_3$ | H | O | H | $SO_2N(C_2H_5)_2$ | |
| $OCH_3$ | $CH_3$ | H | O | H | $CH_3$ | |
| $OCH_3$ | $CH_3$ | H | O | H | $C_2H_5$ | |
| $OCH_3$ | $CH_3$ | H | O | H | $CH(CH_3)_2$ | |
| $OCH_3$ | $CH_3$ | H | O | H | $OCH_2CH_2CH_3$ | |
| $OCH_3$ | $CH_3$ | H | O | H | F | |
| $OCH_3$ | $CH_3$ | H | O | H | $CO_2C_2H_5$ | |
| $OCH_3$ | $CH_3$ | H | O | H | $CO_2CH_2CH_2OCH_3$ | |
| $OCH_3$ | $CH_3$ | H | O | H | $CO_2CH_2CH=CH_2$ | |
| $OCH_3$ | $CH_3$ | H | O | H | $SO_2N(CH_3)_2$ | |
| $OCH_3$ | $CH_3$ | H | O | H | $SO_2N(OCH_3)CH_3$ | |
| $OCH_3$ | $CH_3$ | H | O | H | $SO_2CH_2CH=CH_2$ | |
| $OC_2H_5$ | $CH_3$ | H | O | H | F | |
| $OC_2H_5$ | $CH_3$ | H | O | H | Cl | |
| $OC_2H_5$ | $CH_3$ | H | O | H | Br | |
| $OC_2H_5$ | $CH_3$ | H | O | H | $CO_2CH_3$ | |
| $OC_2H_5$ | $CH_3$ | H | O | H | $SO_2N(CH_3)_2$ | |

Table 5 (continued)

| $R_1$ | $R_2$ | $R_3$ | W | $R_{17}$ | $R_{18}$ | m.p.(°C) |
|---|---|---|---|---|---|---|
| $OC_2H_5$ | $CH_3$ | H | O | H | $SO_2CH_3$ | |
| $OC_2H_5$ | $CH_3$ | H | O | H | $SO_2CH_2CH{=}CH_2$ | |
| $OCH_3$ | $CH_2CF_3$ | H | O | H | $CH_3$ | |
| $OCH_3$ | $CH_2CF_3$ | H | O | H | $OCH_3$ | |
| $OCH_3$ | $CH_2CF_3$ | H | O | H | Cl | |
| $OCH_3$ | $CH_2CF_3$ | H | O | H | $NO_2$ | |
| $OCH_3$ | $CH_2CF_3$ | H | O | H | $CO_2CH_2CH_3$ | |
| $OCH_3$ | $CH_2CF_3$ | H | O | H | $SO_2N(CH_3)_2$ | |
| $OCH_3$ | $CH_2CF_3$ | H | O | H | $SO_2CH_3$ | |
| Cl | $CH_3$ | H | O | H | $CH_3$ | |
| Cl | $CH_3$ | H | O | H | $OCH_3$ | |
| Cl | $CH_3$ | H | O | H | Cl | |
| Cl | $CH_3$ | H | O | H | $NO_2$ | |
| Cl | $CH_3$ | H | O | H | $CO_2CH_3$ | |
| Cl | $CH_3$ | H | O | H | $SO_2N(CH_3)_2$ | |
| Cl | $CH_3$ | H | O | H | $SO_2CH_3$ | |
| $NHCH_3$ | $CH_3$ | H | O | H | $CH_3$ | |
| $NHCH_3$ | $CH_3$ | H | O | H | $OCH_3$ | |
| $NHCH_3$ | $CH_3$ | H | O | H | Cl | |
| $NHCH_3$ | $CH_3$ | H | O | H | $NO_2$ | |
| $NHCH_3$ | $CH_3$ | H | O | H | $CO_2CH_3$ | |
| $NHCH_3$ | $CH_3$ | H | O | H | $SO_2N(CH_3)_2$ | |
| $NHCH_3$ | $CH_3$ | H | O | H | $SO_2CH_3$ | |
| $CH(OCH_3)_2$ | $CH_3$ | H | O | H | $CH_3$ | |
| $CH(OCH_3)_2$ | $CH_3$ | H | O | H | $OCH_3$ | |
| $CH(OCH_3)_2$ | $CH_3$ | H | O | H | Cl | |
| $CH(OCH_3)_2$ | $CH_3$ | H | O | H | $NO_2$ | |
| $CH(OCH_3)_2$ | $CH_3$ | H | O | H | $CO_2CH_3$ | |
| $CH(OCH_3)_2$ | $CH_3$ | H | O | H | $SO_2N(CH_3)_2$ | |
| $CH(OCH_3)_2$ | $CH_3$ | H | O | H | $SO_2CH_3$ | |
| 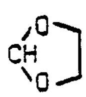 | $CH_3$ | H | O | H | $CH_3$ | |

Table 5 (continued)

| $R_1$ | $R_2$ | $R_3$ | W | $R_{17}$ | $R_{18}$ | m.p.(°C) |
|---|---|---|---|---|---|---|
| 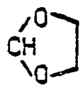 | $CH_3$ | H | O | H | $OCH_3$ | |
| 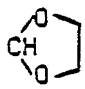 | $CH_3$ | H | O | H | Cl | |
| 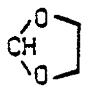 | $CH_3$ | H | O | H | $NO_2$ | |
| 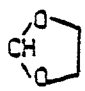 | $CH_3$ | H | O | H | $CO_2CH_3$ | |
| 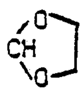 | $CH_3$ | H | O | H | $SO_2N(CH_3)_2$ | |
| 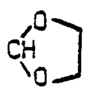 | $CH_3$ | H | O | H | $SO_2CH_3$ | |

Table 6

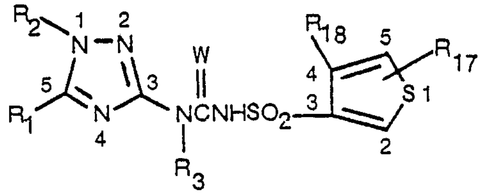

| $R_1$ | $R_2$ | $R_3$ | W | $R_{17}$ | $R_{18}$ | m.p.(°C) |
|---|---|---|---|---|---|---|
| H | $C_2H_5$ | H | O | H | $CO_2CH_3$ | |
| H | $n$-$C_3H_7$ | H | O | H | $CO_2C_2H_5$ | |
| $CH_3$ | $C_2H_5$ | H | O | H | Br | |
| $CH_3$ | $C_2H_5$ | H | O | H | $CO_2CH(CH_3)_2$ | |
| $CH_3$ | $CH_2CH(CH_3)_2$ | H | O | H | $CO_2CH_3$ | |
| $CH_3$ | $C_2H_5$ | H | O | H | $NO_2$ | |
| $CH_3$ | $C_2H_5$ | H | O | H | H | |
| $CH_3$ | $C_2H_5$ | H | S | H | Cl | |
| $CH_3$ | $C_2H_5$ | H | O | H | Cl | |
| $CH_3$ | $C_2H_5$ | $CH_3$ | O | H | $CO_2CH_2CH{=}CH_2$ | |
| $CH_3$ | $C_2H_5$ | H | O | H | $CO_2CH_2CH_2Cl$ | |
| $CH_3$ | $C_2H_5$ | $CH_3$ | O | H | $CO_2CH_3$ | |
| $CH_3$ | $C_2H_5$ | $CH_3$ | O | H | $SO_2CH_3$ | |
| $C_2H_5$ | $CH_3$ | $CH_3$ | O | H | $SO_2CH_3$ | |
| $C_2H_5$ | $CH_3$ | $CH_3$ | O | H | $OCH_3$ | |
| $CH_3$ | $C_2H_5$ | $CH_3$ | O | H | $SO_2N(CH_3)_2$ | |
| $CH_3$ | $C_2H_5$ | H | S | H | $NO_2$ | |
| $CH_3$ | $C_2H_5$ | H | O | H | $SO_2N(CH_3)OCH_3$ | |
| $C_2H_5$ | $CH_3$ | H | S | H | $SO_2CH_3$ | |
| $C_2H_5$ | $CH_3$ | H | O | 5-F | $SO_2CH_3$ | |
| $C_2H_5$ | $CH_3$ | H | O | 2-F | Br | |
| $CH_3$ | $C_2H_5$ | H | O | 5-Cl | Br | |
| $CH_3$ | $C_2H_5$ | H | O | 5-$CH_3$ | Cl | |
| $C_2H_5$ | $C_2H_5$ | H | O | H | Br | |
| $C_2H_5$ | $C_2H_5$ | H | O | H | $CO_2CH_3$ | |

Table 6 (continued)

| $R_1$ | $R_2$ | $R_3$ | W | $R_{17}$ | $R_{18}$ | m.p.(°C) |
|---|---|---|---|---|---|---|
| $C_2H_5$ | $C_2H_5$ | H | O | H | $SO_2CH_3$ | |
| $C_2H_5$ | $C_2H_5$ | H | O | H | $SO_2N(CH_3)C_2H_5$ | |
| $CH_3$ | $C_2H_5$ | H | O | H | $SO_2(CH_2)_2CH_3$ | |
| $C_2H_5$ | $CH_3$ | H | O | H | $SCH_3$ | |
| $C_2H_5$ | $C_2H_5$ | $CH_3$ | O | H | $SO_2CH_3$ | |
| $C_2H_5$ | $C_2H_5$ | $CH_3$ | O | H | $CO_2CH_3$ | |
| $C_2H_5$ | $C_2H_5$ | H | O | H | H | |
| $CH_3$ | $C_2H_5$ | H | O | 5-F | H | |
| $CH_3$ | $C_2H_5$ | H | O | 5-Cl | H | |
| $C_2H_5$ | $C_2H_5$ | H | O | H | $SO_2N(CH_3)_2$ | |
| $C_2H_5$ | $C_2H_5$ | H | S | H | Br | |
| $CH_3$ | $C_2H_5$ | H | O | H | F | |
| $C_2H_5$ | $C_2H_5$ | H | O | H | F | |
| $n$-$C_3H_7$ | $CH_3$ | H | O | H | $CO_2CH_3$ | |
| $CH(CH_3)_2$ | $CH_3$ | H | O | H | $SO_2CH_3$ | |
| $CH_2CH(CH_3)_2$ | $CH_3$ | H | O | H | $CO_2C_2H_5$ | |
| $CH_3$ | $n$-$C_3H_7$ | H | O | H | Br | |
| $CH_3$ | $CH_2CH{=}CH_2$ | H | O | H | $CO_2CH(CH_3)_2$ | |
| $CH_3$ | $CH_2C(CH_3){=}CH_2$ | H | O | H | $CO_2CH_3$ | |
| $CH_2CH{=}CH_2$ | $CH_3$ | H | O | H | $C_2H_5$ | |
| $CH_2C(CH_3){=}CH_2$ | $CH_3$ | H | O | H | $SO_2CH_3$ | |
| $CH_3$ | $CH_2C{\equiv}CCH_3$ | H | O | H | $CO_2CH_3$ | |
| $CH_2C{\equiv}CCH_3$ | $CH_3$ | H | O | H | $SO_2C_2H_5$ | |
| $SCH_3$ | $CH_3$ | H | S | H | Br | |
| $SCH_3$ | $CH_3$ | $CH_3$ | O | H | Br | |
| $SCH_3$ | $CH_3$ | H | O | 2-Cl | Br | |
| $SCH_3$ | $CH_3$ | H | O | 5-$OCH_3$ | H | |
| $SCH_3$ | $CH_3$ | H | O | 5-Cl | Cl | |
| $SCH_3$ | $CH_3$ | H | O | H | $NO_2$ | |
| $SCH_3$ | $CH_3$ | H | O | H | $SO_2N(CH_3)C_2H_5$ | |
| $SCH_3$ | $CH_3$ | H | O | H | $SO_2N(CH_3)[CH(CH_3)_2]$ | |

Table 6 (continued)

| $R_1$ | $R_2$ | $R_3$ | W | $R_{17}$ | $R_{18}$ | m.p.(°C) |
|---|---|---|---|---|---|---|
| $SCH_3$ | $CH_3$ | H | O | H | F | |
| $SCH_3$ | $CH_3$ | H | O | 5-F | H | |
| $SCH_3$ | $CH_3$ | H | O | 2-Br | H | |
| $SCH_3$ | $CH_3$ | H | O | 5-Br | H | |
| $SCH_3$ | $CH_3$ | $CH_3$ | O | H | $SO_2CH_3$ | |
| $SCH_3$ | $CH_3$ | $CH_3$ | O | H | $CO_2CH_3$ | |
| $SCH_3$ | $CH_3$ | $CH_3$ | O | H | $SO_2N(CH_3)_2$ | |
| $SCH_3$ | $CH_3$ | H | O | H | $OC_2H_5$ | |
| $SCH_3$ | $CH_3$ | H | S | H | H | |
| $SCH_3$ | $CH_3$ | H | O | H | $CO_2(CH_2)_3CH_3$ | |
| $SCH_3$ | $CH_3$ | H | O | H | $CO_2CH_2CH_2OCH_3$ | |
| $SCH_3$ | $CH_3$ | H | O | 5-Cl | H | |
| $SCH_3$ | $CH_3$ | H | O | H | $OCH(CH_3)C_2H_5$ | |
| $SCH_3$ | $CH_3$ | H | O | 5-$CH_3$ | $CH_3$ | |
| $SCH_3$ | $CH_3$ | H | O | H | $SCH_3$ | |
| $SCH_3$ | $CH_3$ | H | O | H | $S(CH_2)_3CH_3$ | |
| $SCH_3$ | $C_2H_5$ | H | O | H | $SO_2C_2H_5$ | |
| $SCH_3$ | $CH_3$ | H | O | H | $CO_2CH(CH_3)_2$ | |
| $SCH_3$ | $CH_3$ | H | O | H | $CO_2CH_2CH=CH_2$ | |
| $SCH_3$ | $C_2H_5$ | H | O | H | $CO_2CH_3$ | |
| $SCH_3$ | $C_2H_5$ | H | O | H | Br | |
| $SCH_3$ | $C_2H_5$ | H | O | H | $SO_2CH_3$ | |
| $SCH_3$ | $C_2H_5$ | H | S | H | Cl | |
| $SCH_3$ | $C_2H_5$ | H | O | H | $NO_2$ | |
| $SC_2H_5$ | $CH_3$ | H | O | H | $CO_2CH(CH_3)_2$ | |
| $SC_2H_5$ | $CH_3$ | H | O | H | $CH_3$ | |
| $SC_2H_5$ | $CH_3$ | H | O | H | $CO_2CH_3$ | |
| $SC_2H_5$ | $CH_3$ | H | O | H | $SO_2CH_3$ | |
| $SC_2H_5$ | $CH_3$ | H | O | H | Br | |
| $SC_2H_5$ | $CH_3$ | $CH_3$ | O | H | Cl | |
| $SC_2H_5$ | $CH_3$ | H | S | H | Br | |

Table 6 (continued)

| $R_1$ | $R_2$ | $R_3$ | W | $R_{17}$ | $R_{18}$ | m.p.(°C) |
|---|---|---|---|---|---|---|
| $SCH(CH_3)_2$ | $CH_3$ | H | O | H | $CO_2CH_3$ | |
| $S(CH_2)_3CH_3$ | $CH_3$ | H | O | H | $SO_2CH_3$ | |
| $SCH_2CH=CH_2$ | $CH_3$ | H | O | H | $CO_2CH_3$ | |
| $S(CH_2)_2CH_3$ | $CH_3$ | H | O | H | $CO_2CH_3$ | |
| $SCH_2C(CH_3)=CH_2$ | $CH_3$ | H | O | H | $CO_2C_2H_5$ | |
| $SCH_2C{\equiv}CCH_3$ | $CH_3$ | H | O | H | $CO_2CH_3$ | |
| $SCH_2CO_2CH_3$ | $CH_3$ | H | O | H | $NO_2$ | |
| $SCH_2CO_2(CH_2)_3CH_3$ | $CH_3$ | H | O | H | $CO_2CH_3$ | |
| $SCH(CH_3)CO_2CH_3$ | $CH_3$ | H | O | H | $CO_2CH_3$ | |
| $OCH_3$ | $CH_3$ | H | O | H | $CO_2CH_3$ | |
| $OCH_3$ | $CH_3$ | H | O | H | $SO_2N(CH_3)C_2H_5$ | |
| $OCH_3$ | $CH_3$ | H | O | H | Br | |
| $OCH_3$ | $CH_3$ | H | O | H | Cl | |
| $OCH_3$ | $CH_3$ | H | O | H | $NO_2$ | |
| $OCH_3$ | $CH_3$ | H | S | H | $SO_2CH_3$ | |
| $OCH_3$ | $CH_3$ | $CH_3$ | O | H | $SO_2CH_3$ | |
| $OCH_3$ | $CH_3$ | H | O | H | $SO_2CH_3$ | |
| $OCH_3$ | $CH_3$ | H | O | H | $SO_2(CH_2)_2CH_3$ | |
| $OCH_3$ | $CH_3$ | H | O | H | $OCH_3$ | |
| $OC_2H_5$ | $CH_3$ | H | O | H | $O(CH_2)_3CH_3$ | |
| $OC_2H_5$ | $CH_3$ | H | O | H | $CO_2CH_2CH=CH_2$ | |
| $OCH_3$ | $CH_3$ | H | O | H | $CO_2CH_2CH_2Cl$ | |
| $OCH_3$ | $C_2H_5$ | H | O | H | $CO_2CH(CH_3)_2$ | |
| $OCH_3$ | $C_2H_5$ | H | O | H | $CO_2(CH_2)_2CH_3$ | |
| $OC_2H_5$ | $CH_3$ | H | O | H | $CO_2C_2H_5$ | |
| $OCH_3$ | $C_2H_5$ | $CH_3$ | O | H | $CO_2CH_3$ | |
| $OCH_3$ | $C_2H_5$ | H | S | H | Cl | |
| $OC_2H_5$ | $CH_3$ | H | S | H | Br | |
| $OCH_3$ | $C_2H_5$ | H | O | H | $SO_2CH_3$ | |
| $OCH_3$ | $C_2H_5$ | H | O | H | $SO_2(CH_2)_3CH_3$ | |

Table 6 (continued)

| $R_1$ | $R_2$ | $R_3$ | W | $R_{17}$ | $R_{18}$ | m.p.(°C) |
|---|---|---|---|---|---|---|
| $OCH_3$ | $C_2H_5$ | H | O | H | $SO_2CH_2CH(CH_3)_2$ | |
| $OCH_3$ | $C_2H_5$ | $CH_3$ | O | H | $SO_2CH_3$ | |
| $OCH_3$ | $C_2H_5$ | $CH_3$ | O | 5-Cl | $SO_2CH_3$ | |
| $OCH_3$ | $C_2H_5$ | $CH_3$ | O | 5-Br | $SO_2CH_3$ | |
| $OCH_3$ | $C_2H_5$ | H | O | H | $OC_2H_5$ | |
| $OCH_3$ | $C_2H_5$ | H | O | H | F | |
| $OCH_3$ | $C_2H_5$ | H | O | 5-F | F | |
| $OCH_3$ | $C_2H_5$ | H | O | H | $SC_2H_5$ | |
| $OC_2H_5$ | $CH_3$ | H | O | H | $SO_2N(OCH_3)CH_3$ | |
| $OC_2H_5$ | $CH_3$ | H | O | H | $SO_2N(CH_3)[(CH_2)_2CH_3]$ | |
| $OC_2H_5$ | $CH_3$ | H | S | H | H | |
| $OC_2H_5$ | $CH_3$ | H | S | H | $NO_2$ | |
| $OC_2H_5$ | $CH_3$ | H | O | H | $CO_2CH(CH_3)_2$ | |
| $OC_2H_5$ | $CH_3$ | H | O | H | $CO_2CH_2CH{=}CH_2$ | |
| $OC_2H_5$ | $CH_3$ | H | O | H | $CO_2CH_2CH_2Cl$ | |
| $OC_2H_5$ | $CH_3$ | H | O | H | $CH_3$ | |
| $OC_2H_5$ | $CH_3$ | H | O | H | $CH(CH_3)_2$ | |
| $OC_2H_5$ | $CH_3$ | H | O | H | $CH_2CH(CH_3)_2$ | |
| $OC_2H_5$ | $CH_3$ | $CH_3$ | O | H | $CH_3$ | |
| $OC_2H_5$ | $CH_3$ | $CH_3$ | O | H | $SO_2CH_3$ | |
| $OC_2H_5$ | $CH_3$ | $CH_3$ | O | H | Cl | |
| $OC_2H_5$ | $CH_3$ | H | O | H | $C_2H_5$ | |
| $OC_2H_5$ | $CH_3$ | H | O | H | $NO_2$ | |
| $OC_2H_5$ | $CH_3$ | H | O | 2-Br | Br | |
| $OC_2H_5$ | $CH_3$ | H | O | 2-Cl | Cl | |
| $OC_2H_5$ | $C_2H_5$ | H | O | H | Cl | |
| $OC_2H_5$ | $C_2H_5$ | H | O | H | $CO_2CH_3$ | |
| $OC_2H_5$ | $C_2H_5$ | H | O | H | $SO_2CH_3$ | |
| $OC_2H_5$ | $C_2H_5$ | H | O | H | $SO_2N(CH_3)_2$ | |

Table 6 (continued)

| $R_1$ | $R_2$ | $R_3$ | W | $R_{17}$ | $R_{18}$ | m.p.(°C) |
|---|---|---|---|---|---|---|
| $OC_2H_5$ | $C_2H_5$ | H | O | H | $NO_2$ | |
| $OC_2H_5$ | $C_2H_5$ | H | O | H | Br | |
| $OC_2H_5$ | $C_2H_5$ | H | O | H | $CO_2CH(CH_3)_2$ | |
| $OCH_3$ | $C_2H_5$ | H | O | H | $SO_2C_2H_5$ | |
| $OCH_3$ | $C_2H_5$ | H | O | 2-F | F | |
| $OC_2H_5$ | $CH_3$ | H | O | H | $OCH(CH_3)_2$ | |
| $OCH_2CH=CH_2$ | $CH_3$ | H | O | H | $SO_2CH_3$ | |
| $OCH_2C(CH_3)=CH_2$ | $CH_3$ | H | O | H | $CO_2CH_3$ | |
| $OCH(CH_3)C_2H_5$ | $CH_3$ | H | O | H | $CO_2CH_3$ | |
| $OCH(CH_3)_2$ | $CH_3$ | H | O | H | $CO_2C_2H_5$ | |
| $O(CH_2)_2CH_3$ | $CH_3$ | H | O | H | Cl | |
| $OCH_2C\equiv CCH_3$ | $CH_3$ | H | O | H | $NO_2$ | |
| $CH_2OCH_3$ | $CH_3$ | H | O | H | Br | |
| $CH_2OCH_3$ | $CH_3$ | H | O | H | Cl | |
| $CH_2OCH_3$ | $CH_3$ | $CH_3$ | O | H | $SO_2CH_3$ | |
| $CH_2OCH_3$ | $CH_3$ | $CH_3$ | O | H | $CO_2CH_3$ | |
| $CH_2OCH_3$ | $CH_3$ | H | O | H | $CO_2CH_3$ | |
| $CH_2OCH_3$ | $CH_3$ | H | O | H | $NO_2$ | |
| $CH_2OCH_3$ | $CH_3$ | H | S | H | Cl | |
| $CH_2OCH_3$ | $CH_3$ | H | O | H | H | |
| $CH_2OCH_3$ | $CH_3$ | H | O | H | $SO_2N(CH_3)_2$ | |
| $CH_2OCH_3$ | $CH_3$ | $CH_3$ | O | H | $SO_2N(CH_3)C_2H_5$ | |
| $CH_2OCH_3$ | $C_2H_5$ | H | O | H | $SO_2C_2H_5$ | |
| $CH_2OCH_3$ | $C_2H_5$ | H | O | H | $CO_2CH_3$ | |
| $CH_2OC_2H_5$ | $CH_3$ | H | O | H | Br | |
| $CH_2OCH(CH_3)_2$ | $CH_3$ | H | O | H | $CO_2CH_3$ | |
| $CH_2O(CH_2)_3CH_3$ | $CH_3$ | H | O | H | $CO_2CH_3$ | |
| $CH_2CH_2OCH_3$ | $CH_3$ | H | O | H | Cl | |
| $CH_2CH_2OCH(CH_3)_2$ | $CH_3$ | H | O | H | $CO_2CH_3$ | |
| $N(CH_3)_2$ | $CH_3$ | H | O | H | $CO_2CH_3$ | |
| $CF_3$ | $CH_3$ | H | O | H | $SO_2CH_3$ | |

Table 6 (continued)

| $R_1$ | $R_2$ | $R_3$ | W | $R_{17}$ | $R_{18}$ | m.p.(°C) |
|---|---|---|---|---|---|---|
| $CF_3$ | $CH_3$ | H | O | H | $CO_2CH_3$ | |
| $CF_3$ | $C_2H_5$ | H | O | H | $CO_2CH(CH_3)_2$ | |
| $CF_3$ | $C_2H_5$ | H | O | H | $SO_2CH_3$ | |
| $CF_3$ | $C_2H_5$ | H | O | H | Br | |
| $CF_2CF_3$ | $CH_3$ | H | O | H | $CO_2CH_3$ | |
| $CF_2CF_3$ | $CH_3$ | H | O | H | Cl | |
| $CH_3$ | $CH_2OCH_3$ | H | O | H | $CO_2CH_3$ | |
| $CH_3$ | $CH_2SCH_3$ | H | O | H | Cl | |
| $CH_3$ | $CH_2CH_2OCH_3$ | H | O | H | $CO_2CH_3$ | |
| $CH_3$ | $CH_2CH_2SCH_3$ | H | O | H | Br | |
| $CH_2OCH_3$ | $C_2H_5$ | H | O | H | $CO_2CH_3$ | |
| $CH_2OCH_3$ | $CH_3$ | $CH_3$ | O | H | $CO_2CH_3$ | |
| $OC_2H_5$ | $CH_3$ | $CH_3$ | O | H | $SO_2(CH_2)_2CH_3$ | |
| $OC_2H_5$ | $CH_3$ | H | O | H | $SO_2CH(CH_3)_2$ | |
| $OC_2H_5$ | $CH_3$ | H | O | H | $SO_2N(C_2H_5)_2$ | |
| $OCH_3$ | $CH_3$ | H | O | H | $CH_3$ | |
| $OCH_3$ | $CH_3$ | H | O | H | $C_2H_5$ | |
| $OCH_3$ | $CH_3$ | H | O | H | $CH(CH_3)_2$ | |
| $OCH_3$ | $CH_3$ | H | O | H | $OCH_2CH_2CH_3$ | |
| $OCH_3$ | $CH_3$ | H | O | H | F | |
| $OCH_3$ | $CH_3$ | H | O | H | $CO_2C_2H_5$ | |
| $OCH_3$ | $CH_3$ | H | O | H | $CO_2CH_2CH_2OCH_3$ | |
| $OCH_3$ | $CH_3$ | H | O | H | $CO_2CH_2CH=CH_2$ | |
| $OCH_3$ | $CH_3$ | H | O | H | $SO_2N(CH_3)_2$ | |
| $OCH_3$ | $CH_3$ | H | O | H | $SO_2N(OCH_3)CH_3$ | |
| $OCH_3$ | $CH_3$ | H | O | H | $SO_2CH_2CH=CH_2$ | |
| $OC_2H_5$ | $CH_3$ | H | O | H | F | |
| $OC_2H_5$ | $CH_3$ | H | O | H | Cl | |
| $OC_2H_5$ | $CH_3$ | H | O | H | Br | |
| $OC_2H_5$ | $CH_3$ | H | O | H | $CO_2CH_3$ | |
| $OC_2H_5$ | $CH_3$ | H | O | H | $SO_2N(CH_3)_2$ | |

Table 6 (continued)

| $R_1$ | $R_2$ | $R_3$ | W | $R_{17}$ | $R_{18}$ | m.p.(°C) |
|---|---|---|---|---|---|---|
| $OC_2H_5$ | $CH_3$ | H | O | H | $SO_2CH_3$ | |
| $OC_2H_5$ | $CH_3$ | H | O | H | $SO_2CH_2CH=CH_2$ | |
| $OCH_3$ | $CH_2CF_3$ | H | O | H | $CH_3$ | |
| $OCH_3$ | $CH_2CF_3$ | H | O | H | $OCH_3$ | |
| $OCH_3$ | $CH_2CF_3$ | H | O | H | Cl | |
| $OCH_3$ | $CH_2CF_3$ | H | O | H | $NO_2$ | |
| $OCH_3$ | $CH_2CF_3$ | H | O | H | $CO_2CH_2CH_3$ | |
| $OCH_3$ | $CH_2CF_3$ | H | O | H | $SO_2N(CH_3)_2$ | |
| $OCH_3$ | $CH_2CF_3$ | H | O | H | $SO_2CH_3$ | |
| Cl | $CH_3$ | H | O | H | $CH_3$ | |
| Cl | $CH_3$ | H | O | H | $OCH_3$ | |
| Cl | $CH_3$ | H | O | H | Cl | |
| Cl | $CH_3$ | H | O | H | $NO_2$ | |
| Cl | $CH_3$ | H | O | H | $CO_2CH_3$ | |
| Cl | $CH_3$ | H | O | H | $SO_2N(CH_3)_2$ | |
| Cl | $CH_3$ | H | O | H | $SO_2CH_3$ | |
| $NHCH_3$ | $CH_3$ | H | O | H | $CH_3$ | |
| $NHCH_3$ | $CH_3$ | H | O | H | $OCH_3$ | |
| $NHCH_3$ | $CH_3$ | H | O | H | Cl | |
| $NHCH_3$ | $CH_3$ | H | O | H | $NO_2$ | |
| $NHCH_3$ | $CH_3$ | H | O | H | $CO_2CH_3$ | |
| $NHCH_3$ | $CH_3$ | H | O | H | $SO_2N(CH_3)_2$ | |
| $NHCH_3$ | $CH_3$ | H | O | H | $SO_2CH_3$ | |
| $CH(OCH_3)_2$ | $CH_3$ | H | O | H | $CH_3$ | |
| $CH(OCH_3)_2$ | $CH_3$ | H | O | H | $OCH_3$ | |
| $CH(OCH_3)_2$ | $CH_3$ | H | O | H | Cl | |
| $CH(OCH_3)_2$ | $CH_3$ | H | O | H | $NO_2$ | |
| $CH(OCH_3)_2$ | $CH_3$ | H | O | H | $CO_2CH_3$ | |
| $CH(OCH_3)_2$ | $CH_3$ | H | O | H | $SO_2N(CH_3)_2$ | |
| $CH(OCH_3)_2$ | $CH_3$ | H | O | H | $SO_2CH_3$ | |
| 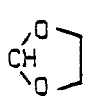 | $CH_3$ | H | O | H | $CH_3$ | |

Table 6 (continued)

| $R_1$ | $R_2$ | $R_3$ | W | $R_{17}$ | $R_{18}$ | m.p.(°C) |
|---|---|---|---|---|---|---|
| 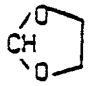 | $CH_3$ | H | O | H | $OCH_3$ | |
| 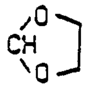 | $CH_3$ | H | O | H | Cl | |
| 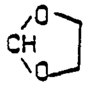 | $CH_3$ | H | O | H | $NO_2$ | |
| 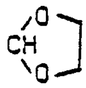 | $CH_3$ | H | O | H | $CO_2CH_3$ | |
| 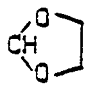 | $CH_3$ | H | O | H | $SO_2N(CH_3)_2$ | |
| 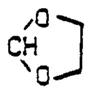 | $CH_3$ | H | O | H | $SO_2CH_3$ | |

Table 7

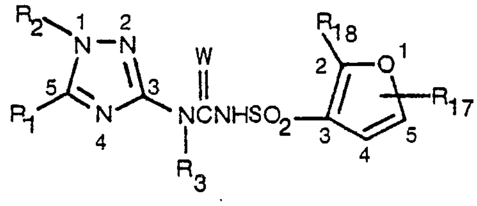

| $R_1$ | $R_2$ | $R_3$ | W | $R_{17}$ | $R_{18}$ | m.p.(°C) |
|---|---|---|---|---|---|---|
| H | $n$-$C_3H_7$ | H | O | H | $CO_2CH_3$ | |
| $CH_3$ | $CH(CH_3)_2$ | H | O | H | $CO_2CH(CH_3)_2$ | |
| $CH_3$ | $CH_3$ | H | O | H | $CO_2CH_2CH=CH_2$ | |
| $n$-$C_4H_9$ | $CH_3$ | H | O | H | Cl | |
| $CH_2CH=CHCH_3$ | $CH_3$ | H | O | H | Br | |
| $CH_2C\equiv CCH_3$ | $CH_3$ | H | O | H | $CH_3$ | |
| $SCH_3$ | $CH_3$ | H | O | H | $CO_2CH(CH_3)C_2H_5$ | |
| $SCH_2CH=CH_2$ | $CH_3$ | H | O | H | Cl | |
| $S(CH_2)_3CH_3$ | $CH_3$ | H | O | H | Br | |
| $SCH(CH_3)_2$ | $CH_3$ | H | O | H | $CO_2C_2H_5$ | |
| $SCH_3$ | $CH_3$ | $CH_3$ | O | H | $CH_3$ | |
| $SCH_2C\equiv CCH_3$ | $CH_3$ | H | O | H | $CO_2CH_3$ | |
| $SCH_2CO_2CH(CH_3)_2$ | $CH_3$ | H | O | H | Cl | |
| $SCH(CH_3)CO_2C_2H_5$ | $CH_3$ | H | O | H | Br | |
| $N(CH_3)_2$ | $CH_3$ | H | O | H | $CO_2CH_2CH_2CH_3$ | |
| $CF_3$ | $C_2H_5$ | $CH_3$ | O | H | H | |
| $CF_2CF_3$ | $CH_3$ | H | S | H | $CO_2CH_2CH_2Cl$ | |
| $OC_2H_5$ | $CH_3$ | H | S | H | $CH_3$ | |
| $OCH_2CH(CH_3)_2$ | $CH_3$ | H | O | H | $CO_2CH_3$ | |
| $CH_2OCH_3$ | $CH_3$ | $CH_3$ | O | H | $CO_2CH_2CH_2OCH_3$ | |
| $CH_2OCH(CH_3)_2$ | $CH_3$ | H | O | H | $CO_2CH(CH_3)_2$ | |
| $CH_2OCH_3$ | $CH_3$ | H | S | H | Cl | |
| $CH_3$ | $CH_2CH_2OCH_3$ | H | O | H | Cl | |
| $CH_3$ | $CH_2SCH_3$ | H | O | H | Br | |
| $CH_3$ | $CH_2OCH_3$ | H | O | H | $CO_2CH_2CH(CH_3)_2$ | |

Table 7 (continued)

| $R_1$ | $R_2$ | $R_3$ | W | $R_{17}$ | $R_{18}$ | m.p.(°C) |
|---|---|---|---|---|---|---|
| $CH_3$ | $CH_2CH_2SCH_3$ | H | O | H | $CO_2CH_3$ | |
| $SCH_3$ | $n$-$C_3H_7$ | H | S | H | $CH_3$ | |
| $CH_3$ | $CH_2CH(CH_3)_2$ | H | O | H | Cl | |
| $OC_2H_5$ | $CH_3$ | H | O | H | $CO_2CH_3$ | |
| $OCH_3$ | $CH_3$ | $CH_3$ | O | H | Cl | |
| $OCH_3$ | $C_2H_5$ | H | O | H | $CO_2CH_3$ | |
| $C_2H_5$ | $CH_3$ | H | S | H | Br | |
| $SCH_3$ | $CH_3$ | H | O | H | $CO_2CH_3$ | |
| $CH_2OCH_2CH_2CH_3$ | $CH_3$ | H | O | H | $CH_3$ | |
| $SCH_3$ | $CH_2C(CH_3)=CH_2$ | H | O | H | Cl | |
| $OC_2H_5$ | $CH_3$ | $CH_3$ | S | H | $CH_3$ | |
| $OC_2H_5$ | $CH_3$ | H | O | H | $CO_2CH(CH_3)_2$ | |
| $CH_2OCH_3$ | $CH_3$ | H | O | H | $CO_2CH_2CH=CH_2$ | |
| $SC_2H_5$ | $CH_3$ | H | O | H | $CO_2C_2H_5$ | |
| $CF_2CF_3$ | $CH_3$ | H | O | H | $CO_2CH_3$ | |
| $CF_3$ | $C_2H_5$ | H | O | H | $CO_2CH_3$ | |
| $CH_3$ | $CH_2CH=CH_2$ | H | O | H | $CO_2CH_3$ | |
| $CH_3$ | $CH_2C\equiv CCH_3$ | H | O | H | $CH_3$ | |
| $SCH_3$ | $CH_3$ | $CH_3$ | O | H | $CO_2CH(CH_3)_2$ | |
| $SCH_3$ | $CH_3$ | H | O | H | H | |
| $SCH_3$ | $CH_3$ | $CH_3$ | O | H | H | |
| $SCH(CH_3)CO_2CH_3$ | $C_2H_5$ | H | O | H | $SO_2CH_3$ | |
| $SCH_3$ | $C_2H_5$ | H | O | H | $CO_2CH_3$ | |
| $CH_2CH_2OCH(CH_3)_2$ | $CH_3$ | H | O | H | Cl | |
| $SCH_3$ | $CH_3$ | H | O | H | H | |
| $OCH_2CH=CH_2$ | $CH_3$ | H | O | H | $CO_2C_2H_5$ | |
| $OCH_3$ | $C_2H_5$ | H | O | H | Br | |
| $CH_2CH_2OCH_3$ | $CH_3$ | H | O | H | $CO_2CH_3$ | |
| $SCH_2CO_2C_2H_5$ | $CH_3$ | H | O | H | $SO_2CH_3$ | |
| $OCH_2C\equiv CH$ | $CH_3$ | H | O | H | $CH_3$ | |
| $OC_2H_5$ | $CH_3$ | $CH_3$ | O | H | $CO_2CH_3$ | |

Table 7 (continued)

| $R_1$ | $R_2$ | $R_3$ | W | $R_{17}$ | $R_{18}$ | m.p.(°C) |
|---|---|---|---|---|---|---|
| $OC_2H_5$ | $CH_3$ | H | O | H | $CO_2CH_2CH{=}CH_2$ | |
| $OC_2H_5$ | $CH_3$ | H | O | H | $CO_2CH_2CH_2OCH_3$ | |
| $OC_2H_5$ | $CH_3$ | H | O | H | $CO_2CH(CH_3)C_2H_5$ | |
| $OC_2H_5$ | $CH_3$ | H | O | H | Br | |
| $OC_2H_5$ | $CH_3$ | H | O | H | Cl | |
| $OC_2H_5$ | $CH_3$ | H | O | H | H | |
| $OC_2H_5$ | $CH_3$ | H | O | H | $CO_2(CH_2)_3CH_3$ | |
| $SCH_3$ | $CH_3$ | H | O | H | $CO_2CH_2CH{=}CH_2$ | |
| $SCH_3$ | $CH_3$ | H | O | H | $CO_2C_2H_5$ | |
| $SCH_3$ | $CH_3$ | H | O | H | $CH_3$ | |
| $SCH_3$ | $CH_3$ | H | O | H | $CO_2(CH_2)_2CH_3$ | |
| $SCH_3$ | $CH_3$ | H | O | H | Br | |
| $SCH_3$ | $CH_3$ | H | O | H | Cl | |
| $SCH_3$ | $CH_3$ | $CH_3$ | O | H | Cl | |
| $SCH_3$ | $CH_3$ | $CH_3$ | O | H | Br | |
| $SCH_3$ | $CH_3$ | $CH_3$ | O | H | $CO_2CH_3$ | |
| $SCH_3$ | $C_2H_5$ | H | O | H | $CH_3$ | |
| $SCH_3$ | $C_2H_5$ | H | O | H | $CO_2C_2H_5$ | |
| $SCH_3$ | $C_2H_5$ | H | O | H | Br | |
| $OCH_3$ | $C_2H_5$ | H | O | H | $CO_2(CH_2)_3CH_3$ | |
| $OCH_3$ | $C_2H_5$ | H | O | 4-Br | H | |
| $OCH_3$ | $C_2H_5$ | H | O | 4-Br | $CO_2CH_3$ | |
| $OCH_3$ | $C_2H_5$ | H | O | 5-Cl | $CO_2CH_3$ | |
| $OCH_3$ | $C_2H_5$ | H | O | 4-Cl | $CO_2CH_3$ | |
| $OCH_3$ | $C_2H_5$ | H | O | 4-$CH_3$ | $CO_2CH_3$ | |
| $OCH_3$ | $C_2H_5$ | H | O | H | $CO_2CH_2CH{=}CH_2$ | |
| $OCH_3$ | $C_2H_5$ | H | O | H | $CO_2CH(CH_3)_2$ | |
| $OCH_3$ | $CH_3$ | H | O | H | H | |
| $OCH_3$ | $CH_3$ | H | O | H | Cl | |
| $OCH_3$ | $CH_3$ | H | O | H | Br | |
| $OCH_3$ | $CH_3$ | H | O | H | $CH_3$ | |

Table 7 (continued)

| $R_1$ | $R_2$ | $R_3$ | W | $R_{17}$ | $R_{18}$ | m.p.(°C) |
|---|---|---|---|---|---|---|
| $OCH_3$ | $CH_3$ | H | O | H | $CO_2CH_3$ | |
| $OCH_3$ | $CH_3$ | H | O | H | $CO_2C_2H_5$ | |
| $OCH_3$ | $CH_3$ | H | O | H | $CO_2CH(CH_3)_2$ | |
| $OCH_3$ | $CH_3$ | H | O | H | $CO_2CH_2CH_2CH_3$ | |
| $OCH_3$ | $CH_3$ | H | O | H | $CO_2(CH_2)_3CH_3$ | |
| $OCH_3$ | $CH_3$ | H | O | H | $CO_2CH_2CH_2OCH_3$ | |
| $OCH_3$ | $CH_3$ | H | O | H | $CO_2CH_2CH_2Cl$ | |
| $OCH_3$ | $CH_3$ | H | O | H | $CO_2CH_2CH=CH_2$ | |
| $OCH_3$ | $CH_2CF_3$ | H | O | H | H | |
| $OCH_3$ | $CH_2CF_3$ | H | O | H | Cl | |
| $OCH_3$ | $CH_2CF_3$ | H | O | H | $CH_3$ | |
| $OCH_3$ | $CH_2CF_3$ | H | O | H | $CO_2CH_3$ | |
| $OCH_3$ | $CH_2CF_3$ | H | O | H | $CO_2CH_2CH=CH_2$ | |
| $OCH_3$ | $CH_2CF_3$ | H | O | H | $CO_2CH_2CH_2Cl$ | |
| Cl | $CH_3$ | H | O | H | Cl | |
| Cl | $CH_3$ | H | O | H | $CH_3$ | |
| Cl | $CH_3$ | H | O | H | $CO_2CH_3$ | |
| Cl | $CH_3$ | H | O | H | $CO_2CH_2CH=CH_2$ | |
| $NHCH_3$ | $CH_3$ | H | O | H | Br | |
| $NHCH_3$ | $CH_3$ | H | O | H | $CO_2C_2H_5$ | |
| $NHCH_3$ | $CH_3$ | H | O | H | $CO_2CH_2CH_2Cl$ | |
| $CH(OCH_3)_2$ | $CH_3$ | H | O | H | $CO_2CH_3$ | |
| 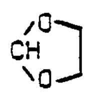 | $CH_3$ | H | O | H | $CO_2CH_3$ | |

Table 8

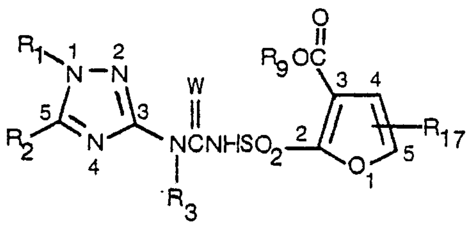

| $R_1$ | $R_2$ | $R_3$ | W | $R_9$ | $R_{17}$ | m.p.(°C) |
|---|---|---|---|---|---|---|
| H | $n$-$C_3H_7$ | H | O | $CH_3$ | H | |
| H | $C_2H_5$ | H | O | $C_2H_5$ | H | |
| $CH_3$ | $CH(CH_3)_2$ | H | O | $CH_3$ | H | |
| $CH_3$ | $CH_3$ | H | S | $CH(CH_3)_2$ | H | |
| $n$-$C_4H_9$ | $CH_3$ | H | O | $CH_3$ | H | |
| $CH_2CH{=}CH_2$ | $CH_3$ | H | O | $CH_3$ | H | |
| $CH_2C(CH_3){=}CH_2$ | $CH_3$ | H | O | $CH_3$ | H | |
| $CH_2C{\equiv}CCH_3$ | $CH_3$ | H | O | $CH_3$ | H | |
| $SCH_2CH{=}CH_2$ | $CH_3$ | H | O | $C_2H_5$ | H | |
| $SCH_2CH(CH_3)_2$ | $CH_3$ | H | O | $CH_3$ | H | |
| $SCH_2C{\equiv}CH$ | $CH_3$ | H | O | $CH_3$ | H | |
| $SC_2H_5$ | $CH_3$ | H | O | $CH(CH_3)C_2H_5$ | H | |
| $SCH_3$ | $CH_3$ | $CH_3$ | O | $CH_2CH_2Cl$ | H | |
| $SCH_2CO_2C_2H_5$ | $CH_3$ | H | O | $C_2H_5$ | H | |
| $N(CH_3)_2$ | $CH_3$ | H | O | $CH_3$ | H | |
| $CF_3$ | $C_2H_5$ | H | O | $CH_2CH_2OCH_3$ | H | |
| $CF_2CF_3$ | $CH_3$ | H | O | $n$-$C_3H_7$ | H | |
| $OC_2H_5$ | $CH_3$ | $CH_3$ | O | $CH(CH_3)C_2H_5$ | H | |
| $OCH_2CH(CH_3)_2$ | $CH_3$ | H | O | $CH_3$ | H | |
| $OCH(CH_3)_2$ | $CH_3$ | H | O | $C_2H_5$ | H | |
| $CH_2OCH_3$ | $CH_3$ | H | S | $CH_3$ | H | |
| $CH_3$ | $CH_2CH_2OCH_3$ | H | O | $CH_3$ | H | |
| $CH_3$ | $CH_2SCH_3$ | H | O | $CH_3$ | H | |
| $CH_3$ | $CH_2OCH_3$ | H | O | $CH(CH_3)_2$ | H | |

Table 8 (continued)

| $R_1$ | $R_2$ | $R_3$ | W | $R_9$ | $R_{17}$ | m.p.(°C) |
|---|---|---|---|---|---|---|
| $CH_3$ | $CH_2CH_2SCH_3$ | H | O | $CH_3$ | H | |
| $SCH_3$ | $n$-$C_3H_7$ | H | O | $CH_3$ | H | |
| $CH_3$ | $CH_2CH(CH_3)_2$ | H | O | $CH_3$ | H | |
| $OC_2H_5$ | $CH_3$ | H | O | $CH_3$ | H | |
| $OCH_3$ | $CH_3$ | $CH_3$ | O | $CH_3$ | H | |
| $OCH_3$ | $C_2H_5$ | H | O | $CH_3$ | 5-Cl | |
| $OCH_3$ | $C_2H_5$ | H | O | $CH_3$ | 5-Br | |
| $CH_3$ | $CH_2CH=CH_2$ | H | O | $CH_3$ | H | |
| $CH_3$ | $CH_2CH=CHCH_3$ | H | O | $CH_3$ | H | |
| $CH_3$ | $CH_2C≡CCH_3$ | H | O | $CH_3$ | H | |
| $OC_2H_5$ | $CH_3$ | H | O | $CH_3$ | 4-Cl | |
| $OCH_3$ | $C_2H_5$ | H | O | $CH_2CH_2Cl$ | H | |
| $C_2H_5$ | $CH_3$ | H | S | $CH_2CH_2OCH_3$ | H | |
| $SCH_3$ | $CH_3$ | H | O | $CH(CH_3)C_2H_5$ | H | |
| $CF_3$ | $C_2H_5$ | H | O | $CH_2CH=CH_2$ | H | |
| $CH_2CH_2OC_2H_5$ | $CH_3$ | H | O | $CH(CH_3)_2$ | H | |
| $CH_2O$-$CH_2CH(CH_3)_2$ | $CH_3$ | H | O | $C_2H_5$ | 4-$CH_3$ | |
| $OCH_3$ | $C_2H_5$ | $CH_3$ | O | $n$-$C_4H_9$ | H | |
| $SCH_3$ | $CH_3$ | $CH_3$ | O | $n$-$C_3H_7$ | H | |
| $CH_2OCH_3$ | $CH_3$ | $CH_3$ | O | $CH_3$ | H | |
| $CH_2OCH_3$ | $CH_3$ | H | O | $C_2H_5$ | H | |
| $CF_2CF_3$ | $C_2H_5$ | H | O | $CH_3$ | H | |
| $OCH_3$ | $C_2H_5$ | H | S | $CH(CH_3)C_2H_5$ | H | |
| $OCH_3$ | $C_2H_5$ | H | S | $CH_3$ | 5-$OCH_3$ | |
| $SCH_3$ | $CH_3$ | H | S | $CH_3$ | H | |
| S-$n$-$C_3H_7$ | $CH_3$ | H | O | $CH_3$ | H | |
| $OC_2H_5$ | $C_2H_5$ | H | O | $CH_3$ | H | |
| $OCH_2C≡CH$ | $C_2H_5$ | H | O | $CH_3$ | H | |
| $CH_3$ | $C_2H_5$ | H | O | $CH(CH_3)_2$ | H | |
| $SCH_3$ | $CH_3$ | H | O | $n$-$C_3H_7$ | H | |
| $OCH_2CH=CH_2$ | $CH_3$ | H | O | $CH_3$ | H | |

Table 8 (continued)

| $R_1$ | $R_2$ | $R_3$ | W | $R_9$ | $R_{17}$ | m.p.(°C) |
|---|---|---|---|---|---|---|
| $SCH_3$ | $CH_3$ | H | O | $CH_3$ | H | |
| $SCH_3$ | $CH_3$ | H | O | $C_2H_5$ | H | |
| $SCH_3$ | $C_2H_5$ | H | O | $CH_3$ | H | |
| $SCH_3$ | $C_2H_5$ | H | O | $C_2H_5$ | H | |
| $SCH_3$ | $CH_3$ | H | O | $CH_2CH=CH_2$ | H | |
| $SCH_3$ | $CH_3$ | H | O | $CH_2(CH_3)_2$ | H | |
| $SCH_3$ | $CH_3$ | $CH_3$ | S | $CH_3$ | H | |
| $SCH_3$ | $CH_3$ | H | S | $C_2H_5$ | H | |
| $OC_2H_5$ | $CH_3$ | H | O | $CH_3$ | 5-F | |
| $OC_2H_5$ | $CH_3$ | H | O | $C_2H_5$ | 5-Cl | |
| $OC_2H_5$ | $CH_3$ | H | O | $CH_3$ | 5-Br | |
| $OC_2H_5$ | $CH_3$ | H | O | $CH(CH_3)_2$ | H | |
| $OC_2H_5$ | $CH_3$ | H | O | $CH_3$ | 4-Br | |
| $OC_2H_5$ | $CH_3$ | H | O | $CH_3$ | 5-$CH_3$ | |
| $OC_2H_5$ | $CH_3$ | H | S | $CH_3$ | H | |
| $OC_2H_5$ | $CH_3$ | $CH_3$ | O | $CH_3$ | H | |
| $OC_2H_5$ | $CH_3$ | $CH_3$ | O | $CH(CH_3)_2$ | H | |
| $OC_2H_5$ | $CH_3$ | $CH_3$ | O | $CH_2CH=CH_2$ | H | |
| $OC_2H_5$ | $CH_3$ | H | O | $n$-$C_4H_9$ | H | |
| $OC_2H_5$ | $CH_3$ | H | O | $CH_2CH(CH_3)_2$ | H | |
| $OC_2H_5$ | $CH_3$ | H | O | $n$-$C_3H_7$ | H | |
| $OCH_3$ | $C_2H_5$ | H | O | $CH_3$ | H | |
| $OCH_3$ | $C_2H_5$ | H | S | $CH_3$ | H | |
| $OCH_3$ | $C_2H_5$ | H | O | $CH_3$ | 4-F | |
| $OCH_3$ | $C_2H_5$ | H | O | $CH_3$ | 5-F | |
| $OCH_3$ | $C_2H_5$ | $CH_3$ | O | $CH_3$ | H | |
| $OCH_3$ | $C_2H_5$ | $CH_3$ | O | $CH_2CH=CH_2$ | H | |
| $OCH_3$ | $CH_3$ | H | O | $CH_3$ | H | |
| $OCH_3$ | $CH_3$ | H | O | $CH(CH_3)_2$ | H | |
| $OCH_3$ | $CH_3$ | H | O | $C_2H_5$ | H | |
| $OCH_3$ | $CH_3$ | H | O | $CH_2CH_2CH_3$ | H | |

Table 8 (continued)

| $R_1$ | $R_2$ | $R_3$ | W | $R_9$ | $R_{17}$ | m.p.(°C) |
|---|---|---|---|---|---|---|
| $OCH_3$ | $CH_3$ | H | O | $CH_2CH=CH_2$ | H | |
| $OCH_3$ | $CH_3$ | H | O | $CH_2CH_2OCH_3$ | H | |
| $OCH_3$ | $CH_3$ | H | O | $CH_2CH_2Cl$ | H | |
| $OC_2H_5$ | $CH_3$ | H | O | $CH_3$ | H | |
| $OCH_3$ | $CH_2CF_3$ | H | O | $CH_3$ | H | |
| $OCH_3$ | $CH_2CF_3$ | H | O | $C_2H_5$ | H | |
| $OCH_3$ | $CH_2CF_3$ | H | O | $CH(CH_3)_2$ | H | |
| $OCH_3$ | $CH_2CF_3$ | H | O | $CH_2CH=CH_2$ | H | |
| $OCH_3$ | $CH_2CF_3$ | H | O | $CH_2CH_2OCH_3$ | H | |
| $OCH_3$ | $CH_2CF_3$ | H | O | $CH_2CH_2Cl$ | H | |
| Cl | $CH_3$ | H | O | $CH_3$ | H | |
| Cl | $CH_3$ | H | O | $C_2H_5$ | H | |
| Cl | $CH_3$ | H | O | $CH_2CH=CH_2$ | H | |
| $NHCH_3$ | $CH_3$ | H | O | $CH_3$ | H | |
| $NHCH_3$ | $CH_3$ | H | O | $CH_2CH_2Cl$ | H | |
| $NHCH_3$ | $CH_3$ | H | O | $CH_2CH_2OCH_3$ | H | |
| $NHCH_3$ | $CH_3$ | H | O | $C_2H_5$ | H | |
| $CH(OCH_3)_2$ | $CH_3$ | H | O | $CH_3$ | H | |
| $CH(OCH_3)_2$ | $CH_3$ | H | O | $C_2H_5$ | H | |
| $CH(OCH_3)_2$ | CH | H | O | $CH(CH_3)_2$ | H | |
| $CH(OCH_3)_2$ | $CH_3$ | H | O | $CH_2CH=CH_2$ | H | |
| $CH(OCH_3)_2$ | $CH_3$ | H | O | $CH_2CH_2Cl$ | H | |
| 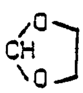 | $CH_3$ | H | O | $CH_3$ | H | |
| 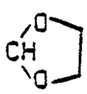 | $CH_3$ | H | O | $CH_2CH_2CH_3$ | H | |
| 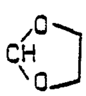 | $CH_3$ | H | O | $CH_2CH_2OCH_3$ | H | |
| 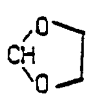 | $CH_3$ | H | O | $CH_2CH_2Cl$ | H | |

Table 9

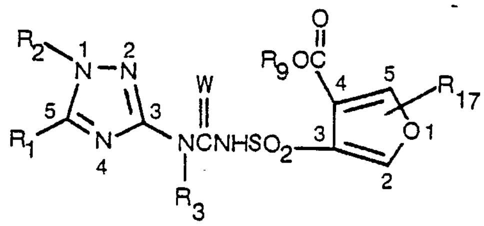

| $R_1$ | $R_2$ | $R_3$ | W | $R_9$ | $R_{17}$ | m.p.(°C) |
|---|---|---|---|---|---|---|
| H | $n$-$C_3H_7$ | H | O | $CH_3$ | H | |
| $CH_3$ | $C_2H_5$ | H | O | $C_2H_5$ | H | |
| $CH_3$ | $CH(CH_3)_2$ | H | O | $CH_3$ | H | |
| $CH_3$ | $CH_3$ | H | O | $CH_3$ | H | |
| $n$-$C_4H_9$ | $CH_3$ | H | O | $CH_3$ | H | |
| $CH_2CH=CH_2$ | $CH_3$ | H | O | $CH_3$ | H | |
| $CH_2CH(CH_3)=CH_2$ | $CH_3$ | H | O | $CH_3$ | H | |
| $CH_2C≡CCH_3$ | $CH_3$ | H | O | $CH_3$ | H | |
| $SCH_2CH=CH_2$ | $CH_3$ | H | O | $CH_3$ | H | |
| $CH(CH_3)C_2H_5$ | $CH_3$ | H | O | $CH_3$ | H | |
| $SCH_2(CH_3)_2$ | $CH_3$ | H | O | $CH_3$ | H | |
| $SCH_2C≡CH$ | $CH_3$ | H | O | $CH_3$ | H | |
| $SC_2H_5$ | $CH_3$ | H | O | $CH_3$ | H | |
| $SCH_3$ | $CH_3$ | $CH_3$ | O | $CH_3$ | H | |
| $SCH_2CO_2CH(CH_3)_2$ | $CH_3$ | H | O | $CH_3$ | H | |
| $N(CH_3)_2$ | $CH_3$ | H | O | $CH_3$ | H | |
| $SCH_3$ | $CH_3$ | $CH_3$ | O | $CH_3$ | H | |
| $SCH_3$ | $CH_3$ | H | S | $C_2H_5$ | H | |
| $SCH_3$ | $CH_3$ | H | O | $CH(CH_3)C_2H_5$ | H | |
| $SCH_3$ | $CH_3$ | H | O | $CH_2CH_2OCH_3$ | H | |
| $SCH_3$ | $C_2H_5$ | H | O | $CH(CH_3)_2$ | H | |
| $CF_3$ | $C_2H_5$ | H | O | $CH_2CH=CH_2$ | H | |
| $CF_2CF_3$ | $CH_3$ | H | S | $CH_3$ | H | |
| $OC_2H_5$ | $CH_3$ | H | O | $n$-$C_3H_7$ | H | |
| $OCH_2CH(CH_3)_2$ | $CH_3$ | H | O | $CH_3$ | H | |

Table 9 (continued)

| $R_1$ | $R_2$ | $R_3$ | W | $R_9$ | $R_{17}$ | m.p.(°C) |
|---|---|---|---|---|---|---|
| $OCH(CH_3)_2$ | $CH_3$ | H | S | $CH_3$ | H | |
| $CH_2OCH_3$ | $CH_3$ | H | O | $CH_2CH_2Cl$ | H | |
| $SCH_2CO_2CH_3$ | $CH_3$ | H | O | $CH_3$ | H | |
| $SCH(CH_3)CO_2C_2H_5$ | $CH_3$ | H | O | $CH_3$ | H | |
| $CH_3$ | $CH_2CH_2OCH_3$ | H | O | $CH_3$ | H | |
| $CH_3$ | $CH_2SCH_3$ | H | O | $CH(CH_3)_2$ | H | |
| $CF_3$ | $CH_2OCH_3$ | H | S | $C_2H_5$ | H | |
| $CH_2OCH(CH_3)_2$ | $CH_3$ | H | O | $CH_3$ | H | |
| $CH_2CH_2OC_2H_5$ | $CH_3$ | H | O | $CH_3$ | H | |
| $SCH_3$ | $n$-$C_3H_7$ | $CH_3$ | O | $CH_3$ | H | |
| $OC_2H_5$ | $CH_3$ | $CH_3$ | S | $CH_3$ | H | |
| $OCH_3$ | $CH_3$ | $CH_3$ | O | $CH_3$ | H | |
| $OCH_3$ | $CH_3$ | H | O | $CH_3$ | 5-Cl | |
| $OCH_3$ | $C_2H_5$ | H | O | $CH_3$ | 2-Br | |
| $OCH_3$ | $C_2H_5$ | H | S | $CH_3$ | H | |
| $C_2H_5$ | $CH_3$ | H | O | $CH_3$ | 5-$CH_3$ | |
| $CH_3$ | $CH_2CH=CH_2$ | H | O | $n$-$C_3H_7$ | H | |
| $CH_3$ | $CH_2CH(CH_3)_2$ | H | O | $CH(CH_3)_2$ | H | |
| $CH_3$ | $CH_2C{\equiv}C$-$CH_3$ | H | O | $CH_3$ | H | |
| $OC_2H_5$ | $CH_3$ | $CH_3$ | O | $C_2H_5$ | H | |
| $CF_3$ | $C_2H_5$ | H | S | $CH_3$ | H | |
| $OCH_2CH=CH_2$ | $CH_3$ | H | O | $CH_3$ | H | |
| $OCH_2C{\equiv}CH$ | $CH_3$ | H | O | $CH_3$ | H | |
| $SCH_3$ | $CH_3$ | H | O | $CH_2CH_2OCH_3$ | H | |
| $SCH_3$ | $CH_3$ | H | O | $CH_2CH_2Cl$ | H | |
| $CF_2CF_3$ | $CH_3$ | $CH_3$ | O | $CH_3$ | H | |
| $OCH_3$ | $C_2H_5$ | $CH_3$ | O | $CH_3$ | H | |
| $OCH_3$ | $C_2H_5$ | H | O | $CH_2CH=CH_2$ | H | |
| $CH_2OCH_3$ | $CH_3$ | H | O | $CH_3$ | H | |
| $SCH_3$ | $CH_3$ | H | O | $CH_3$ | H | |

Table 9 (continued)

| $R_1$ | $R_2$ | $R_3$ | W | $R_9$ | $R_{17}$ | m.p.(°C) |
|---|---|---|---|---|---|---|
| $SCH_3$ | $CH_3$ | H | O | $CH_3$ | H | |
| $SCH_3$ | $CH_3$ | H | O | $C_2H_5$ | H | |
| $SCH_3$ | $C_2H_5$ | H | O | $CH_3$ | H | |
| $SCH_3$ | $C_2H_5$ | H | O | $C_2H_5$ | H | |
| $SCH_3$ | $CH_3$ | H | O | $CH_2CH{=}CH_2$ | H | |
| $SCH_3$ | $CH_3$ | H | O | $CH_2(CH_3)_2$ | H | |
| $SCH_3$ | $CH_3$ | $CH_3$ | S | $CH_3$ | H | |
| $SCH_3$ | $CH_3$ | H | S | $C_2H_5$ | H | |
| $OC_2H_5$ | $CH_3$ | H | O | $CH_3$ | 5-F | |
| $OC_2H_5$ | $CH_3$ | H | O | $C_2H_5$ | 5-Cl | |
| $OC_2H_5$ | $CH_3$ | H | O | $CH_3$ | 5-Br | |
| $OC_2H_5$ | $CH_3$ | H | O | $CH(CH_3)_2$ | H | |
| $OC_2H_5$ | $CH_3$ | H | O | $CH_3$ | 2-Br | |
| $OC_2H_5$ | $CH_3$ | H | O | $CH_3$ | 5-$CH_3$ | |
| $OC_2H_5$ | $CH_3$ | H | S | $CH_3$ | H | |
| $OC_2H_5$ | $CH_3$ | $CH_3$ | O | $CH_3$ | H | |
| $OC_2H_5$ | $CH_3$ | $CH_3$ | O | $CH(CH_3)_2$ | H | |
| $OC_2H_5$ | $CH_3$ | $CH_3$ | O | $CH_2CH{=}CH_2$ | H | |
| $OC_2H_5$ | $CH_3$ | H | O | $n$-$C_4H_9$ | H | |
| $OC_2H_5$ | $CH_3$ | H | O | $CH_2CH(CH_3)_2$ | H | |
| $OC_2H_5$ | $CH_3$ | H | O | $n$-$C_3H_7$ | H | |
| $OCH_3$ | $C_2H_5$ | H | O | $CH_3$ | H | |
| $OCH_3$ | $C_2H_5$ | H | S | $CH_3$ | H | |
| $OCH_3$ | $C_2H_5$ | H | O | $CH_3$ | 2-F | |
| $OCH_3$ | $C_2H_5$ | H | O | $CH_3$ | 5-F | |
| $OCH_3$ | $C_2H_5$ | $CH_3$ | O | $CH_3$ | H | |
| $OCH_3$ | $C_2H_5$ | $CH_3$ | O | $CH_2CH{=}CH_2$ | H | |
| $OCH_3$ | $CH_3$ | H | O | $CH_3$ | H | |
| $OCH_3$ | $CH_3$ | H | O | $CH(CH_3)_2$ | H | |
| $OCH_3$ | $CH_3$ | H | O | $C_2H_5$ | H | |
| $OCH_3$ | $CH_3$ | H | O | $CH_2CH_2CH_3$ | H | |

Table 9 (continued)

| $R_1$ | $R_2$ | $R_3$ | W | $R_9$ | $R_{17}$ | m.p.(°C) |
|---|---|---|---|---|---|---|
| $OCH_3$ | $CH_3$ | H | O | $CH_2CH{=}CH_2$ | H | |
| $OCH_3$ | $CH_3$ | H | O | $CH_2CH_2OCH_3$ | H | |
| $OCH_3$ | $CH_3$ | H | O | $CH_2CH_2Cl$ | H | |
| $OCH_3$ | $CH_3$ | H | O | $CH_2CH_2CH_2CH_3$ | H | |
| $OC_2H_5$ | $CH_3$ | H | O | $CH_3$ | H | |
| $OCH_3$ | $CH_2CF_3$ | H | O | $CH_3$ | H | |
| $OCH_3$ | $CH_2CF_3$ | H | O | $C_2H_5$ | H | |
| $OCH_3$ | $CH_2CF_3$ | H | O | $CH(CH_3)_2$ | H | |
| $OCH_3$ | $CH_2CF_3$ | H | O | $CH_2CH{=}CH_2$ | H | |
| $OCH_3$ | $CH_2CF_3$ | H | O | $CH_2CH_2OCH_3$ | H | |
| $OCH_3$ | $CH_2CF_3$ | H | O | $CH_2CH_2Cl$ | H | |
| Cl | $CH_3$ | H | O | $CH_3$ | H | |
| Cl | $CH_3$ | H | O | $C_2H_5$ | H | |
| Cl | $CH_3$ | H | O | $CH_2CH{=}CH_2$ | H | |
| $NHCH_3$ | $CH_3$ | H | O | $CH_3$ | H | |
| $NHCH_3$ | $CH_3$ | H | O | $CH_2CH_2Cl$ | H | |
| $NHCH_3$ | $CH_3$ | H | O | $CH_2CH_2OCH_3$ | H | |
| $NHCH_3$ | $CH_3$ | H | O | $C_2H_5$ | H | |
| $CH(OCH_3)_2$ | $CH_3$ | H | O | $CH_3$ | H | |
| $CH(OCH_3)_2$ | $CH_3$ | H | O | $C_2H_5$ | H | |
| $CH(OCH_3)_2$ | CH | H | O | $CH(CH_3)_2$ | H | |
| $CH(OCH_3)_2$ | $CH_3$ | H | O | $CH_2CH{=}CH_2$ | H | |
| $CH(OCH_3)_2$ | $CH_3$ | H | O | $CH_2CH_2Cl$ | H | |
| 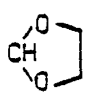 | $CH_3$ | H | O | $CH_3$ | H | |
| 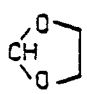 | $CH_3$ | H | O | $CH_2CH_2CH_3$ | H | |
| 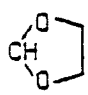 | $CH_3$ | H | O | $CH_2CH_2OCH_3$ | H | |
| 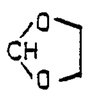 | $CH_3$ | H | O | $CH_2CH_2Cl$ | H | |

Table 10

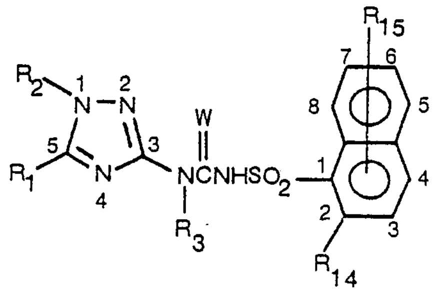

| $R_1$ | $R_2$ | $R_3$ | W | $R_{14}$ | $R_{15}$ | m.p.(°C) |
|---|---|---|---|---|---|---|
| H | $n$-$C_3H_7$ | H | O | Cl | H | |
| H | $C_2H_5$ | H | O | Br | H | |
| $CH_3$ | $C_2H_5$ | H | O | F | H | |
| $C_2H_5$ | $CH_3$ | H | O | H | H | |
| $CH_3$ | $CH(CH_3)_2$ | H | O | Cl | H | |
| $CH_3$ | $CH(CH_3)C_2H_5$ | H | O | Cl | H | |
| $CH_3$ | $n$-$C_3H_7$ | H | S | Cl | H | |
| $n$-$C_3H_7$ | $CH_3$ | H | O | $OCH_3$ | H | |
| $CH(CH_3)_2$ | $CH_3$ | H | O | $CH_3$ | H | |
| $CH(CH_3)C_2H_5$ | $CH_3$ | H | O | Br | H | |
| $n$-$C_4H_9$ | $CH_3$ | H | O | Cl | H | |
| $C_2H_5$ | $CH_3$ | H | O | $NO_2$ | 4-Cl | |
| $C_2H_5$ | $CH_3$ | H | O | $CH_3$ | 8-$NO_2$ | |
| $CH_3$ | $C_2H_5$ | H | O | Cl | 3-Cl | |
| $CH_3$ | $C_2H_5$ | H | O | $NO_2$ | 6-Br | |
| $CH_3$ | $C_2H_5$ | $CH_3$ | O | $NO_2$ | H | |
| $C_2H_5$ | $CH_3$ | H | S | Cl | H | |
| $C_2H_5$ | $CH_3$ | $CH_3$ | O | $NO_2$ | H | |
| $C_2H_5$ | $CH_3$ | $CH_3$ | O | Cl | H | |
| $CH_3$ | $CH_3$ | H | O | F | H | |
| $C_2H_5$ | $C_2H_5$ | H | O | H | H | |
| $C_2H_5$ | $CH_3$ | H | O | $SO_2N(CH_3)_2$ | H | |
| $CH_3$ | $C_2H_5$ | H | O | $SO_2CH_3$ | H | |
| $CH_3$ | $C_2H_5$ | $CH_3$ | O | $SO_2(CH_2)CH_3$ | H | |
| $CH_3$ | $C_2H_5$ | H | O | $SO_2N(OCH_3)CH_3$ | H | |

Table 10 (continued)

| $R_1$ | $R_2$ | $R_3$ | W | $R_{14}$ | $R_{15}$ | m.p.(°C) |
|---|---|---|---|---|---|---|
| $C_2H_5$ | $C_2H_5$ | $CH_3$ | O | $SO_2C_2H_5$ | H | |
| $C_2H_5$ | $C_2H_5$ | H | O | $OSO_2CH_3$ | H | |
| $C_2H_5$ | $C_2H_5$ | H | O | $NO_2$ | H | |
| $C_2H_5$ | $C_2H_5$ | H | O | Cl | H | |
| $CH_3$ | $C_2H_5$ | H | O | $SO_2N(CH_3)C_2H_5$ | H | |
| $CH_3$ | $C_2H_5$ | H | O | H | 7-F | |
| $CH_3$ | $C_2H_5$ | H | O | H | 5-$OCH_3$ | |
| $CH_3$ | $C_2H_5$ | H | O | $NO_2$ | 8-Cl | |
| $CH_3$ | $CH_3$ | H | S | $CH_3$ | H | |
| $CH_2CH{=}CH_2$ | $CH_3$ | H | O | Cl | H | |
| $CH_2C(CH_3){=}CH_2$ | $CH_3$ | H | O | Cl | H | |
| $CH_2C{\equiv}CH$ | $CH_3$ | H | O | $NO_2$ | H | |
| $CH_2C{\equiv}CCH_3$ | $CH_3$ | H | O | Br | H | |
| $CH_3$ | $CH_2CH{=}CH_2$ | H | O | $OCH_3$ | H | |
| $CH_3$ | $CH_2C(CH_3){=}CH_2$ | H | O | $NO_2$ | H | |
| $CH_3$ | $CH_2C{\equiv}CCH_3$ | H | O | $SO_2CH_3$ | H | |
| $SCH_3$ | $CH_3$ | $CH_3$ | O | H | H | |
| $SCH_3$ | $CH_3$ | $CH_3$ | O | $NO_2$ | H | |
| $SCH_3$ | $CH_3$ | $CH_3$ | O | Cl | H | |
| $SCH_3$ | $CH_3$ | H | O | H | H | |
| $SCH_3$ | $CH_3$ | H | O | $NO_2$ | H | |
| $SCH_3$ | $CH_3$ | H | O | Cl | H | |
| $SCH_3$ | $CH_3$ | H | O | $OCH_3$ | H | |
| $SCH_3$ | $CH_3$ | H | O | $CH_3$ | H | |
| $SCH_3$ | $CH_3$ | H | O | F | H | |
| $SCH_3$ | $CH_3$ | H | S | H | H | |
| $SCH_3$ | $CH_3$ | H | S | Cl | H | |
| $SCH_3$ | $CH_3$ | H | S | $NO_2$ | H | |
| $SCH_3$ | $C_2H_5$ | H | O | $CH_3$ | H | |
| $SCH_3$ | $C_2H_5$ | $CH_3$ | O | $NO_2$ | H | |
| $SCH_3$ | $C_2H_5$ | $CH_3$ | O | H | H | |

Table 10 (continued)

| $R_1$ | $R_2$ | $R_3$ | W | $R_{14}$ | $R_{15}$ | m.p.(°C) |
|---|---|---|---|---|---|---|
| $SCH_3$ | $C_2H_5$ | H | O | $OSO_2CHCl_2$ | H | |
| $SCH_3$ | $CH_3$ | H | O | $OSO_2CH_2CHFCl$ | H | |
| $SCH_3$ | $CH_3$ | H | O | $OSO_2CH(CH_3)CH_2F$ | H | |
| $SCH_3$ | $CH_3$ | H | O | $OSO_2(CH_2)_3Br$ | H | |
| $SCH_3$ | $CH_3$ | H | O | $OSO_2(CH_2)_2CHCl_2$ | H | |
| $CH_3$ | $C_2H_5$ | H | O | $OSO_2CF_3$ | H | |
| $CH_3$ | $C_2H_5$ | H | O | $OSO_2CF_2CH_2F$ | H | |
| $C_2H_5$ | $C_2H_5$ | H | O | $OSO_2CH_2CF_3$ | H | |
| $SCH_3$ | $C_2H_5$ | H | O | $OSO_2CHF_2$ | H | |
| $CH_3$ | $C_2H_5$ | H | O | $OSO_2CCl_3$ | H | |
| $SC_2H_5$ | $CH_3$ | H | O | $OSO_2CH_3$ | H | |
| $SC_2H_5$ | $C_2H_5$ | H | O | $NO_2$ | 4-Br | |
| $SC_2H_5$ | $CH_3$ | H | S | H | 5-$OCH_3$ | |
| $SCH_3$ | $CH_3$ | $CH_3$ | O | F | 7-F | |
| $S(CH_2)_2CH_3$ | $CH_3$ | H | O | $CH_3$ | H | |
| $SCH(CH_3)_2$ | $CH_3$ | H | O | $SO_2N(CH_3)_2$ | H | |
| $SCH(CH_3)C_2H_5$ | $CH_3$ | H | O | Br | H | |
| $SCH_2CH{=}CH_2$ | $CH_3$ | H | O | Cl | H | |
| $SCH_2C(CH_3){=}CH_2$ | $CH_3$ | H | O | Cl | H | |
| $SCH_2C{\equiv}CCH_3$ | $CH_3$ | H | O | $NO_2$ | H | |
| $SCH_2CO_2CH_3$ | $CH_3$ | H | O | $SO_2C_2H_5$ | H | |
| $SCH_2CO_2CH_2CH_3$ | $CH_3$ | H | O | Cl | H | |
| $SCH(CH_3)CO_2CH_3$ | $CH_3$ | H | O | Br | H | |
| $SCH(CH_3)CO_2C_2H_5$ | $CH_3$ | H | O | H | H | |
| $CH_2OCH_3$ | $CH_3$ | $CH_3$ | O | H | H | |
| $CH_2OCH_3$ | $CH_3$ | H | S | H | H | |
| $CH_2OCH_3$ | $CH_3$ | H | O | $NO_2$ | H | |
| $CH_2OCH_3$ | $CH_3$ | H | O | Cl | H | |
| $CH_2OCH_3$ | $CH_3$ | H | O | Br | H | |
| $CH_2OCH_3$ | $CH_3$ | H | O | $OCH_3$ | H | |
| $CH_2OCH_3$ | $CH_3$ | H | S | H | 6-Br | |
| $CH_2OCH_3$ | $CH_3$ | H | S | H | 3-Cl | |

Table 10 (continued)

| $R_1$ | $R_2$ | $R_3$ | W | $R_{14}$ | $R_{15}$ | m.p.(°C) |
|---|---|---|---|---|---|---|
| $CH_2OCH_3$ | $C_2H_5$ | H | O | F | H | |
| $CH_2OCH_3$ | $CH_3$ | H | O | $SO_2C_2H_5$ | H | |
| $CH_2OCH_3$ | $CH_3$ | H | O | $SO_2N(CH_3)_2$ | H | |
| $CH_2OCH_3$ | $CH_3$ | H | O | $OSO_2C_2H_5$ | H | |
| $CH_2OC_2H_5$ | $CH_3$ | H | O | $CH_3$ | H | |
| $CH_2OC_2H_5$ | $CH_3$ | H | S | H | H | |
| $CH_2O(CH_2)_3CH_3$ | $CH_3$ | H | O | $NO_2$ | H | |
| $CH_2OCH(CH_3)_2$ | $CH_3$ | H | O | Cl | H | |
| $CH_2CH_2OCH_3$ | $CH_3$ | H | O | H | H | |
| $CH_2CH_2OCH(CH_3)_2$ | $CH_3$ | H | O | $OCH_3$ | H | |
| $OCH_3$ | $C_2H_5$ | H | O | H | H | |
| $OCH_3$ | $C_2H_5$ | H | S | H | H | |
| $OCH_3$ | $CH_3$ | $CH_3$ | O | H | H | |
| $OCH_3$ | $CH_3$ | $CH_3$ | O | Cl | H | |
| $OCH_3$ | $C_2H_5$ | H | O | $SO_2N(CH_3)]CH(CH_3)_2]$ | H | |
| $OCH_3$ | $C_2H_5$ | H | O | $SO_2N(CH_3)[(CH_2)_2CH_3]$ | H | |
| $OCH_3$ | $C_2H_5$ | H | O | $SO_2N(CH_3)_2$ | H | |
| $OCH_3$ | $CH_3$ | H | O | $OSO_2(CH_2)_2CHCl_2$ | H | |
| $OCH_3$ | $CH_3$ | H | O | $OSO_2CH(CH_3)CHF_2$ | H | |
| $OCH_3$ | $CH_3$ | H | O | $OSO_2CHFCF_3$ | H | |
| $OC_2H_5$ | $CH_3$ | H | O | Br | H | |
| $OC_2H_5$ | $CH_3$ | H | O | Cl | 4-Cl | |
| $OC_2H_5$ | $CH_3$ | H | O | H | 8-$NO_2$ | |
| $OC_2H_5$ | $CH_3$ | H | S | H | H | |
| $OC_2H_5$ | $CH_3$ | H | S | $NO_2$ | H | |
| $OC_2H_5$ | $CH_3$ | H | O | $SCH_3$ | H | |
| $OC_2H_5$ | $CH_3$ | H | O | $SCH_2CH(CH_3)_2$ | H | |
| $OC_2H_5$ | $CH_3$ | H | O | $S(CH_2)_3CH_3$ | H | |
| $OCH_3$ | $C_2H_5$ | H | O | $S(CH_2)_2CH_3$ | H | |
| $OCH_3$ | $C_2H_5$ | H | O | $SC_2H_5$ | H | |
| $OCH_3$ | $C_2H_5$ | H | O | $SO_2CH_2CH(CH_3)_2$ | H | |

Table 10 (continued)

| $R_1$ | $R_2$ | $R_3$ | W | $R_{14}$ | $R_{15}$ | m.p.(°C) |
|---|---|---|---|---|---|---|
| $OCH_3$ | $C_2H_5$ | H | O | $SO_2CH(CH_3)_2$ | H | |
| $OCH_3$ | $C_2H_5$ | H | O | $SO_2CH_3$ | H | |
| $OCH_3$ | $C_2H_5$ | H | O | $SO_2(CH_2)_2CH_3$ | H | |
| $O(CH_2)_2CH_3$ | $CH_3$ | H | O | $SO_2N(CH_3)_2$ | H | |
| $OCH(CH_3)_2$ | $CH_3$ | H | O | $CH_3$ | H | |
| $OCH(CH_3)C_2H_5$ | $CH_3$ | H | O | $OCH_3$ | H | |
| $OCH_2CH{=}CH_2$ | $CH_3$ | H | O | H | H | |
| $OCH_2C(CH_3){=}CH_2$ | $CH_3$ | H | O | Cl | H | |
| $OCH_2C{\equiv}CCH_3$ | $CH_3$ | H | O | $NO_2$ | H | |
| $N(CH_3)_2$ | $CH_3$ | H | O | H | H | |
| $N(CH_3)_2$ | $CH_3$ | H | O | Cl | H | |
| $CF_3$ | $CH_3$ | H | O | Cl | H | |
| $CF_3$ | $CH_3$ | H | O | $NO_2$ | H | |
| $CF_3$ | $CH_3$ | H | S | Cl | H | |
| $CF_3$ | $C_2H_5$ | H | O | H | H | |
| $CF_3$ | $C_2H_5$ | H | O | F | H | |
| $CF_3$ | $C_2H_5$ | H | O | $SO_2CH(CH_3)_2$ | H | |
| $CF_3$ | $C_2H_5$ | H | O | $OSO_2C_2H_5$ | H | |
| $CF_3$ | $C_2H_5$ | H | O | $OSO_2CH(CH_3)_2$ | H | |
| $CF_2CF_3$ | $CH_3$ | H | O | $OSO_2CH_3$ | H | |
| $CF_2CF_3$ | $CH_3$ | H | O | $OSO_2CH_2CH_2Cl$ | H | |
| $CF_2CF_3$ | $CH_3$ | H | O | $NO_2$ | H | |
| $CF_2CF_3$ | $C_2H_5$ | H | O | Cl | H | |
| $CH_3$ | $CH_2OCH_3$ | H | O | Br | H | |
| $CH_3$ | $CH_2OCH_3$ | H | O | $NO_2$ | H | |
| $CH_3$ | $CH_2OCH(CH_3)_2$ | H | O | Br | H | |
| $CH_3$ | $CH_2CH_2OCH_3$ | H | O | $NO_2$ | H | |
| $CH_3$ | $CH_2CH_2S(CH_2)_2CH_3$ | H | O | Cl | H | |
| $CH_3$ | $CH_2CH_2SCH_3$ | H | O | H | H | |
| $CH_3$ | $CH_2SCH_3$ | H | O | $CH_3$ | H | |
| $CH_3$ | $CH_2CH_2SCH_3$ | H | O | $OCH_3$ | H | |
| $CH_3$ | $CH_2OC_2H_5$ | H | O | F | H | |

Table 10 (continued)

| $R_1$ | $R_2$ | $R_3$ | W | $R_{14}$ | $R_{15}$ | m.p.(°C) |
|---|---|---|---|---|---|---|
| $OCH_3$ | $C_2H_5$ | H | O | $OSO_2CH_2CH_2OCH_3$ | H | |
| $OC_2H_5$ | $CH_3$ | H | O | $OSO_2(CH_2)_3OCH_3$ | H | |
| $SCH_3$ | $CH_3$ | H | O | $OSO_2(CH_2)_3CH_3$ | H | |
| $SCH_3$ | $CH_3$ | H | S | Br | H | |
| $SCH_3$ | $CH_3$ | H | O | Br | 8-Cl | |
| $SCH_3$ | $CH_3$ | H | O | H | 5-$OCH_3$ | |
| $SCH_3$ | $CH_3$ | H | O | H | 6-Br | |
| $SCH_3$ | $CH_3$ | $CH_3$ | O | $SO_2CH_3$ | H | |
| $SCH_3$ | $CH_3$ | $CH_3$ | O | $SO_2C_2H_5$ | H | |
| $OC_2H_5$ | $CH_3$ | $CH_3$ | O | Cl | H | |
| $OC_2H_5$ | $CH_3$ | $CH_3$ | O | $NO_2$ | H | |
| $SC_2H_5$ | $CH_3$ | H | O | F | H | |
| $C_2H_5$ | $C_2H_5$ | $CH_3$ | O | H | H | |
| $SCH_3$ | $CH_3$ | H | S | Cl | H | |
| $OC_2H_5$ | $CH_3$ | H | O | $OSO_2CH_2Cl$ | H | |
| $OC_2H_5$ | $CH_3$ | H | O | $SO_2CH_3$ | H | |
| $OC_2H_5$ | $CH_3$ | H | O | $SO_2(CH_2)_2CH_3$ | H | |
| $OC_2H_5$ | $CH_3$ | H | O | $SCH_2CH_3$ | H | |
| $OC_2H_5$ | $CH_3$ | H | O | $OSO_2CF_3$ | H | |
| $OC_2H_5$ | $CH_3$ | H | O | $SO_2N(CH_3)[(CH_2)_2CH_3]$ | H | |
| $OC_2H_5$ | $CH_3$ | H | O | $CH_3$ | H | |
| $OC_2H_5$ | $CH_3$ | H | O | $OCH_3$ | H | |
| $OC_2H_5$ | $CH_3$ | H | O | F | H | |
| $OC_2H_5$ | $CH_3$ | H | O | Cl | H | |
| $OC_2H_5$ | $CH_3$ | H | O | $NO_2$ | H | |
| $OC_2H_5$ | $CH_3$ | H | O | $SO_2N(CH_3)_2$ | H | |
| $OC_2H_5$ | $CH_3$ | H | O | $SO_2N(C_2H_5)_2$ | H | |
| $OC_2H_5$ | $CH_3$ | H | O | $SO_2N(OCH_3)CH_3$ | H | |
| $OC_2H_5$ | $CH_3$ | H | O | $OSO_2CH_3$ | H | |
| $OC_2H_5$ | $CH_3$ | H | O | $OSO_2(CH_2)_3CH_3$ | H | |
| $OC_2H_5$ | $CH_3$ | H | O | $SO_2CH_2CH{=}CH_2$ | H | |

Table 10 (continued)

| $R_1$ | $R_2$ | $R_3$ | W | $R_{14}$ | $R_{15}$ | m.p.(°C) |
|---|---|---|---|---|---|---|
| $OC_2H_5$ | $CH_3$ | H | O | $SO_2CF_3$ | H | |
| $OC_2H_5$ | $CH_3$ | H | O | $SO_2CF_2CF_3$ | H | |
| $OCH_3$ | $CH_3$ | H | O | H | H | |
| $OCH_3$ | $CH_3$ | H | O | $CH_3$ | H | |
| $OCH_3$ | $CH_3$ | H | O | $OCH_3$ | H | |
| $OCH_3$ | $CH_3$ | H | O | F | H | |
| $OCH_3$ | $CH_3$ | H | O | Cl | H | |
| $OCH_3$ | $CH_3$ | H | O | Br | H | |
| $OCH_3$ | $CH_3$ | H | O | $NO_2$ | H | |
| $OCH_3$ | $CH_3$ | H | O | $SO_2N(CH_3)_2$ | H | |
| $OCH_3$ | $CH_3$ | H | O | $SO_2N(C_2H_5)_2$ | H | |
| $OCH_3$ | $CH_3$ | H | O | $SO_2N(CH_3)C_2H_5$ | H | |
| $OCH_3$ | $CH_3$ | H | O | $SO_2N(OCH_3)CH_3$ | H | |
| $OCH_3$ | $CH_3$ | H | O | $OSO_2CH_3$ | H | |
| $OCH_3$ | $CH_3$ | H | O | $OSO_2C_2H_5$ | H | |
| $OCH_3$ | $CH_3$ | H | O | $OSO_2CF_3$ | H | |
| $OCH_3$ | $CH_3$ | H | O | $SCH_3$ | H | |
| $OCH_3$ | $CH_3$ | H | O | $SOCH_3$ | H | |
| $OCH_3$ | $CH_3$ | H | O | $SO_2CH_3$ | H | |
| $OCH_3$ | $CH_3$ | H | O | $SC_2H_5$ | H | |
| $OCH_3$ | $CH_3$ | H | O | $SO_2C_2H_5$ | H | |
| $OCH_3$ | $CH_3$ | H | O | $SO_2CH_2CH_2CH_3$ | H | |
| $OCH_3$ | $CH_3$ | H | O | $SO_2CH_2CH{=}CH_2$ | H | |
| $OCH_3$ | $CH_3$ | H | O | $SO_2CF_3$ | H | |
| $OCH_3$ | $CH_3$ | H | O | $SO_2CH_2CF_3$ | H | |
| $OCH_3$ | $CH_3$ | H | O | $SO_2CH_2CHClCF_3$ | H | |
| $OCH_3$ | $CH_2CF_3$ | H | O | $CH_3$ | H | |
| $OCH_3$ | $CH_2CF_3$ | H | O | $OCH_3$ | H | |
| $OCH_3$ | $CH_2CF_3$ | H | O | Cl | H | |
| $OCH_3$ | $CH_2CF_3$ | H | O | $NO_2$ | H | |
| $OCH_3$ | $CH_2CF_3$ | H | O | $SO_2N(CH_3)_2$ | H | |

Table 10 (continued)

| $R_1$ | $R_2$ | $R_3$ | W | $R_{14}$ | $R_{15}$ | m.p.(°C) |
|---|---|---|---|---|---|---|
| $OCH_3$ | $CH_2CF_3$ | H | O | $SO_2N(OCH_3)CH_3$ | H | |
| $OCH_3$ | $CH_2CF_3$ | H | O | $OSO_2CH_3$ | H | |
| $OCH_3$ | $CH_2CF_3$ | H | O | $OSO_2CF_3$ | H | |
| $OCH_3$ | $CH_2CF_3$ | H | O | $SO_2CH_3$ | H | |
| $OCH_3$ | $CH_2CF_3$ | H | O | $SO_2CH_2CH_2CH_3$ | H | |
| $OCH_3$ | $CH_2CF_3$ | H | O | $SO_2CH_2CH{=}CH_2$ | H | |
| Cl | $CH_3$ | H | O | $CH_3$ | H | |
| Cl | $CH_3$ | H | O | Cl | H | |
| Cl | $CH_3$ | H | O | $NO_2$ | H | |
| Cl | $CH_3$ | H | O | $SO_2N(CH_3)_2$ | H | |
| Cl | $CH_3$ | H | O | $OSO_2CH_3$ | H | |
| Cl | $CH_3$ | H | O | $SO_2CH_3$ | H | |
| $NHCH_3$ | $CH_3$ | H | O | $OCH_3$ | H | |
| $NHCH_3$ | $CH_3$ | H | O | Br | H | |
| $NHCH_3$ | $CH_3$ | H | O | $SO_2N(OCH_3)CH_3$ | H | |
| $NHCH_3$ | $CH_3$ | H | O | $OSO_2CF_3$ | H | |
| $NHCH_3$ | $CH_3$ | H | O | $SO_2CH_2CH_3$ | H | |
| $NHCH_3$ | $CH_3$ | H | O | $SO_2CH_2CH{=}CH_2$ | H | |
| $CH(OCH_3)_2$ | $CH_3$ | H | O | $CH_3$ | H | |
| $CH(OCH_3)_2$ | $CH_3$ | H | O | F | H | |
| $CH(OCH_3)_2$ | $CH_3$ | H | O | $NO_2$ | H | |
| $CH(OCH_3)_2$ | $CH_3$ | H | O | $SO_2N(C_2H_5)_2$ | H | |
| $CH(OCH_3)_2$ | $CH_3$ | H | O | $SO_2CH_3$ | H | |
| 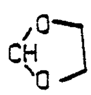 | $CH_3$ | H | O | H | H | |
| 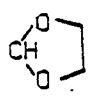 | $CH_3$ | H | O | $CH_3$ | H | |
| 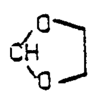 | $CH_3$ | H | O | $OCH_3$ | H | |
| 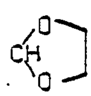 | $CH_3$ | H | O | F | H | |

Table 10 (continued)

| $R_1$ | $R_2$ | $R_3$ | W | $R_{14}$ | $R_{15}$ | m.p.(°C) |
|---|---|---|---|---|---|---|
| 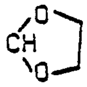 | $CH_3$ | H | O | Cl | H | |
| 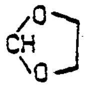 | $CH_3$ | H | O | Br | H | |
| 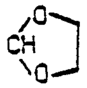 | $CH_3$ | H | O | $NO_2$ | H | |
| 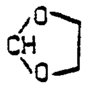 | $CH_3$ | H | O | $SO_2N(CH_3)_2$ | H | |
| 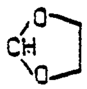 | $CH_3$ | H | O | $SO_2N(OCH_3)CH_3$ | H | |
| 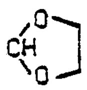 | $CH_3$ | H | O | $OSO_2CH_3$ | H | |
| 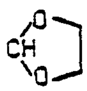 | $CH_3$ | H | O | $OSO_2CF_3$ | H | |
| 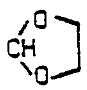 | $CH_3$ | H | O | $SCH_3$ | H | |
| 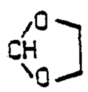 | $CH_3$ | H | O | $SO_2C_2H_5$ | H | |
| 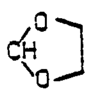 | $CH_3$ | H | O | $SO_2CH_2CH{=}CH_2$ | H | |
| 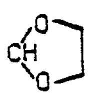 | $CH_3$ | H | O | $SO_2CH_2CH_2CH_3$ | H | |
| 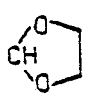 | $CH_3$ | H | O | $SO_2CH_3$ | H | |

Table 11

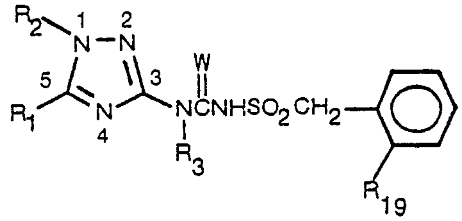

| $R_1$ | $R_2$ | $R_3$ | W | $R_{19}$ | m.p.(°C) |
|---|---|---|---|---|---|
| $OCH_3$ | $CH_3$ | H | O | Cl | |
| $OCH_3$ | $CH_3$ | H | O | $NO_2$ | |
| $OCH_3$ | $CH_3$ | H | O | $CF_3$ | |
| $OCH_3$ | $CH_3$ | H | O | $CO_2CH_3$ | 164-166° |
| $OCH_3$ | $CH_3$ | H | O | $CO_2C_2H_5$ | |
| $OCH_3$ | $CH_3$ | H | O | $CO_2(CH_2)_2CH_3$ | |
| $OCH_3$ | $CH_3$ | H | O | $CO_2(CH_2)_3CH_3$ | |
| $OCH_3$ | $CH_3$ | H | O | $CO_2(CH_2)_4CH_3$ | |
| $OCH_3$ | $CH_3$ | H | O | $CO_2(CH_2)_5CH_3$ | |
| $OCH_3$ | $CH_3$ | H | O | $CO_2CH(CH_3)_2$ | |
| $OCH_3$ | $CH_3$ | H | O | $CO_2CH_2CH_2OCH_3$ | |
| $OCH_3$ | $CH_3$ | H | O | $CO_2CH_2CH_2OC_2H_5$ | |
| $OCH_3$ | $CH_3$ | H | O | $CO_2(CH_2)_3OC_2H_5$ | |
| $OCH_3$ | $CH_3$ | H | O | $CO_2CH_2CH{=}CH_2$ | |
| $OCH_3$ | $CH_3$ | H | O | $CO_2CH_2CH{=}CHCH_3$ | |
| $OCH_3$ | $CH_3$ | H | O | $CO_2(CH_2)_4CH{=}CH_2$ | |
| $OCH_3$ | $CH_3$ | H | O | $CO_2CH_2C{\equiv}CH$ | |
| $OCH_3$ | $CH_3$ | H | O | $CO_2CH_2C{\equiv}CCH_3$ | |
| $OCH_3$ | $CH_3$ | H | O | $CO_2(CH_2)_4C{\equiv}CH$ | |
| $OCH_3$ | $CH_3$ | H | O | $CO_2CH(CH_3)C_2H_5$ | |
| $OCH_3$ | $CH_3$ | H | O | $CO_2CH_2CH(CH_3)_2$ | |
| $OCH_3$ | $CH_3$ | H | O | $CO_2CF_3$ | |
| $OCH_3$ | $CH_3$ | H | O | $CO_2CH_2CH_2Cl$ | |
| $OCH_3$ | $CH_3$ | H | O | $CO_2CH_2CF_3$ | |

Table 11 (continued)

| $R_1$ | $R_2$ | $R_3$ | W | $R_{19}$ | m.p.(°C) |
|---|---|---|---|---|---|
| $OCH_3$ | $CH_3$ | H | O | $CO_2CH_2CCl_3$ | |
| $OCH_3$ | $CH_3$ | H | O | $CO_2CH_2CH_2CF_3$ | |
| $OCH_3$ | $CH_3$ | H | O | $CO_2CH_2CH_2CH_2Cl$ | |
| $OCH_3$ | $CH_3$ | H | O | $CO_2CClF_2$ | |
| $OCH_3$ | $CH_3$ | H | O | $CO_2CF_2H$ | |
| $OCH_3$ | $CH_3$ | H | O | $CO_2CHFCF_2H$ | |
| $OCH_3$ | $CH_3$ | H | O | $CO_2CHClCF_2H$ | |
| $OCH_3$ | $CH_3$ | H | O | $CO_2CH_2CHFCF_2H$ | |
| $OCH_3$ | $CH_3$ | H | O | $SO_2N(OCH_3)CH_3$ | |
| $OCH_3$ | $CH_3$ | H | O | $SO_2N(CH_3)_2$ | |
| $OCH_3$ | $CH_3$ | H | O | $SO_2N(C_2H_5)_2$ | |
| $OCH_3$ | $CH_3$ | H | O | $SO_2N(CH_3)C_2H_5$ | |
| $OCH_3$ | $CH_3$ | H | O | $SO_2N(CH_3)CH_2CH_2CH_3$ | |
| $OCH_3$ | $CH_3$ | H | O | $OSO_2CH_3$ | |
| $OCH_3$ | $CH_3$ | H | O | $OSO_2C_2H_5$ | |
| $OCH_3$ | $CH_3$ | H | O | $OSO_2(CH_2)_2CH_3$ | |
| $OCH_3$ | $CH_3$ | H | O | $OSO_2(CH_2)_3CH_3$ | |
| $OCH_3$ | $CH_3$ | H | O | $OSO_2CH(CH_3)_2$ | |
| $OCH_3$ | $CH_3$ | H | O | $OSO_2CH(CH_3)C_2H_5$ | |
| $OCH_3$ | $CH_3$ | H | O | $OSO_2CH_2CH_2OCH_3$ | |
| $OCH_3$ | $CH_3$ | H | O | $OSO_2(CH_2)_3OCH_3$ | |
| $OCH_3$ | $CH_3$ | H | O | $OSO_2CF_3$ | |
| $OCH_3$ | $CH_3$ | H | O | $OSO_2CH_2CF_3$ | |
| $OCH_3$ | $CH_3$ | H | O | $OSO_2CHFCF_2H$ | |
| $OCH_3$ | $CH_3$ | H | O | $OSO_2CF_2CH_2CH_2CH_3$ | |
| $OCH_3$ | $CH_3$ | H | O | $OSO_2CClF_2$ | |
| $OCH_3$ | $CH_3$ | H | O | $OSO_2CBr_2CH_3$ | |
| $OCH_3$ | $CH_3$ | H | O | $N(CH_3)SO_2CH_3$ | |
| $OCH_3$ | $CH_3$ | H | O | $N(CH_3)SO_2C_2H_5$ | |
| $OCH_3$ | $CH_3$ | H | O | $N(CH_3)SO_2C_3H_5$ | |
| $OCH_3$ | $CH_3$ | H | O | $N(CH_3)SO_2CH(CH_3)C_2H_5$ | |

Table 11 (continued)

| $R_1$ | $R_2$ | $R_3$ | W | $R_{19}$ | m.p.(°C) |
|---|---|---|---|---|---|
| $OCH_3$ | $CH_3$ | H | O | $N(CH_3)SO_2CH_2CH_2OCH_3$ | |
| $OCH_3$ | $CH_3$ | H | O | $N(CH_3)SO_2CF_3$ | |
| $OCH_3$ | $CH_3$ | H | O | $SCH_3$ | |
| $OCH_3$ | $CH_3$ | H | O | $SOCH_3$ | |
| $OCH_3$ | $CH_3$ | H | O | $SO_2CH_3$ | |
| $OCH_3$ | $CH_3$ | H | O | $SO_2C_2H_5$ | |
| $OCH_3$ | $CH_3$ | H | O | $SO_2CH_2CH_2CH_3$ | |
| $OCH_3$ | $CH_3$ | H | O | $SO_2CH(CH_3)_2$ | |
| $OCH_3$ | $CH_3$ | H | O | $SO_2CH_2CH=CH_2$ | |
| $OCH_3$ | $CH_3$ | H | O | $SO_2CH(CH_3)C_2H_5$ | |
| $OCH_3$ | $CH_3$ | H | O | $SCH_2CH_2CH_3$ | |
| $OCH_3$ | $CH_3$ | H | O | $SCF_3$ | |
| $OCH_3$ | $CH_3$ | H | O | $SOCF_3$ | |
| $OCH_3$ | $CH_3$ | H | O | $SO_2CF_3$ | |
| $OCH_3$ | $CH_3$ | H | O | $OCCl_3$ | |
| $OCH_3$ | $CH_3$ | H | O | $OCF_3$ | |
| $OCH_3$ | $CH_3$ | H | O | $OCF_2CF_3$ | |
| $OCH_3$ | $CH_3$ | H | O | $OCF_2CF_2CH_3$ | |
| $OCH_3$ | $CH_3$ | H | O | $OCClF_2$ | |
| $OCH_3$ | $CH_3$ | H | O | $OCCl_2CF_3$ | |
| $OC_2H_5$ | $CH_3$ | H | O | Cl | |
| $OC_2H_5$ | $CH_3$ | H | O | $NO_2$ | |
| $OC_2H_5$ | $CH_3$ | H | O | $CF_3$ | |
| $OC_2H_5$ | $CH_3$ | H | O | $CO_2CH_3$ | |
| $OC_2H_5$ | $CH_3$ | H | O | $CO_2C_2H_5$ | |
| $OC_2H_5$ | $CH_3$ | H | O | $CO_2CH_2CH_2CH_3$ | |
| $OC_2H_5$ | $CH_3$ | H | O | $CO_2CH(CH_3)_2$ | |
| $OC_2H_5$ | $CH_3$ | H | O | $CO_2CH_2CH=CH_2$ | |
| $OC_2H_5$ | $CH_3$ | H | O | $CO_2CH_2C\equiv CH$ | |
| $OC_2H_5$ | $CH_3$ | H | O | $CO_2CH_2CH_2OCH_3$ | |
| $OC_2H_5$ | $CH_3$ | H | O | $CO_2CH_2CH_2OC_2H_5$ | |

Table 11 (continued)

| $R_1$ | $R_2$ | $R_3$ | W | $R_{19}$ | m.p.(°C) |
|---|---|---|---|---|---|
| $OC_2H_5$ | $CH_3$ | H | O | $CO_2CF_3$ | |
| $OC_2H_5$ | $CH_3$ | H | O | $SO_2N(OCH_3)CH_3$ | |
| $OC_2H_5$ | $CH_3$ | H | O | $SO_2N(CH_3)_2$ | |
| $OC_2H_5$ | $CH_3$ | H | O | $SO_2N(CH_3)C_2H_5$ | |
| $OC_2H_5$ | $CH_3$ | H | O | $SO_2N(C_2H_5)_2$ | |
| $OC_2H_5$ | $CH_3$ | H | O | $OSO_2CH_3$ | |
| $OC_2H_5$ | $CH_3$ | H | O | $OSO_2C_2H_5$ | |
| $OC_2H_5$ | $CH_3$ | H | O | $N(CH_3)SO_2CH_3$ | |
| $OC_2H_5$ | $CH_3$ | H | O | $SO_2CH_3$ | |
| $OC_2H_5$ | $CH_3$ | H | O | $SO_2C_2H_5$ | |
| $OC_2H_5$ | $CH_3$ | H | O | $SO_2CH_2CH_2CH_3$ | |
| $OC_2H_5$ | $CH_3$ | H | O | $SO_2CHCH{=}CH_2$ | |
| $OC_2H_5$ | $CH_3$ | H | O | $SO_2CF_3$ | |
| $OC_2H_5$ | $CH_3$ | H | O | $OCF_3$ | |
| $OC_2H_5$ | $CH_3$ | H | O | $OCF_2CF_3$ | |
| $OC_2H_5$ | $C_2H_5$ | H | O | Cl | |
| $OC_2H_5$ | $C_2H_5$ | H | O | $NO_2$ | |
| $OC_2H_5$ | $C_2H_5$ | H | O | $CF_3$ | |
| $OC_2H_5$ | $C_2H_5$ | H | O | $CO_2CH_3$ | |
| $OC_2H_5$ | $C_2H_5$ | H | O | $CO_2C_2H_5$ | |
| $OC_2H_5$ | $C_2H_5$ | H | O | $CO_2CH_2CH_2CH_3$ | |
| $OC_2H_5$ | $C_2H_5$ | H | O | $CO_2CH(CH_3)_2$ | |
| $OC_2H_5$ | $C_2H_5$ | H | O | $CO_2CHCH{=}CH_2$ | |
| $OC_2H_5$ | $C_2H_5$ | H | O | $CO_2CH_2C{\equiv}CH$ | |
| $OC_2H_5$ | $C_2H_5$ | H | O | $CO_2CH_2CH_2OCH_3$ | |
| $OC_2H_5$ | $C_2H_5$ | H | O | $SO_2N(OCH_3)CH_3$ | |
| $OC_2H_5$ | $C_2H_5$ | H | O | $SO_2N(CH_3)_2$ | |
| $OC_2H_5$ | $C_2H_5$ | H | O | $OSO_2CH_3$ | |
| $OC_2H_5$ | $C_2H_5$ | H | O | $N(CH_3)SO_2CH_3$ | |
| $OC_2H_5$ | $C_2H_5$ | H | O | $SO_2CH_3$ | |
| $OC_2H_5$ | $C_2H_5$ | H | O | $SO_2CH_2CH_3$ | |

Table 11 (continued)

| $R_1$ | $R_2$ | $R_3$ | W | $R_{19}$ | m.p.(°C) |
|---|---|---|---|---|---|
| $OC_2H_5$ | $C_2H_5$ | H | O | $SO_2CH_2CH_2CH_3$ | |
| $OC_2H_5$ | $C_2H_5$ | H | O | $OCF_3$ | |
| $OCH_2CH_2CH_3$ | $CH_3$ | H | O | Cl | |
| $OCH_2CH_2CH_3$ | $CH_3$ | H | O | $NO_2$ | |
| $OCH_2CH_2CH_3$ | $CH_3$ | H | O | $CF_3$ | |
| $OCH_2CH_2CH_3$ | $CH_3$ | H | O | $CO_2CH_3$ | |
| $OCH_2CH_2CH_3$ | $CH_3$ | H | O | $CO_2CH_2CH=CH_2$ | |
| $OCH_2CH_2CH_3$ | $CH_3$ | H | O | $SO_2N(OCH_3)CH_3$ | |
| $OCH_2CH_2CH_3$ | $CH_3$ | H | O | $SO_2N(CH_3)_2$ | |
| $OCH_2CH_2CH_3$ | $CH_3$ | H | O | $OSO_2CH_3$ | |
| $OCH_2CH_2CH_3$ | $CH_3$ | H | O | $N(CH_3)SO_2CH_3$ | |
| $OCH_2CH_2CH_3$ | $CH_3$ | H | O | $SO_2CH_3$ | |
| $OCH_2CH_2CH_3$ | $CH_3$ | H | O | $SO_2CH_2CH_2CH_3$ | |
| $OCH_2CH_2CH_3$ | $CH_3$ | H | O | $OCF_3$ | |
| $O(CH_2)_3CH_3$ | $CH_3$ | H | O | Cl | |
| $O(CH_2)_3CH_3$ | $CH_3$ | H | O | $NO_2$ | |
| $O(CH_2)_3CH_3$ | $CH_3$ | H | O | $CF_3$ | |
| $O(CH_2)_3CH_3$ | $CH_3$ | H | O | $CO_2CH_3$ | |
| $O(CH_2)_3CH_3$ | $CH_3$ | H | O | $CO_2C_2H_5$ | |
| $O(CH_2)_3CH_3$ | $CH_3$ | H | O | $CO_2CH(CH_3)_2$ | |
| $O(CH_2)_3CH_3$ | $CH_3$ | H | O | $SO_2N(CH_3)_2$ | |
| $O(CH_2)_3CH_3$ | $CH_3$ | H | O | $SO_2CH_3$ | |
| $O(CH_2)_3CH_3$ | $CH_3$ | H | O | $SO_2CH_2CH_2CH_3$ | |
| $O(CH_2)_3CH_3$ | $CH_3$ | H | O | $OCF_3$ | |
| $OCH_3$ | $C_2H_5$ | H | O | Cl | |
| $OCH_3$ | $C_2H_5$ | H | O | $NO_2$ | |
| $OCH_3$ | $C_2H_5$ | H | O | $CF_3$ | |
| $OCH_3$ | $C_2H_5$ | H | O | $CO_2CH_3$ | |
| $OCH_3$ | $C_2H_5$ | H | O | $CO_2C_2H_5$ | |
| $OCH_3$ | $C_2H_5$ | H | O | $CO_2CH(CH_3)_2$ | |
| $OCH_3$ | $C_2H_5$ | H | O | $CO_2CH_2CH=CH_2$ | |

Table 11 (continued)

| $R_1$ | $R_2$ | $R_3$ | W | $R_{19}$ | m.p.(°C) |
|---|---|---|---|---|---|
| $OCH_3$ | $C_2H_5$ | H | O | $CO_2CH_2CH_2OCH_3$ | |
| $OCH_3$ | $C_2H_5$ | H | O | $CO_2CH_2CH_2Cl$ | |
| $OCH_3$ | $C_2H_5$ | H | O | $SO_2N(OCH_3)CH_3$ | |
| $OCH_3$ | $C_2H_5$ | H | O | $SO_2N(CH_3)_2$ | |
| $OCH_3$ | $C_2H_5$ | H | O | $OSO_2CH_3$ | |
| $OCH_3$ | $C_2H_5$ | H | O | $N(CH_3)SO_2CH_3$ | |
| $OCH_3$ | $C_2H_5$ | H | O | $SO_2CH_3$ | |
| $OCH_3$ | $C_2H_5$ | H | O | $SO_2CH_2CH_2CH_3$ | |
| $OCH_3$ | $C_2H_5$ | H | O | $OCF_3$ | |
| $OCH_3$ | $CH_2CH=CH_2$ | H | O | Cl | |
| $OCH_3$ | $CH_2CH=CH_2$ | H | O | $NO_2$ | |
| $OCH_3$ | $CH_2CH=CH_2$ | H | O | $CF_3$ | |
| $OCH_3$ | $CH_2CH=CH_2$ | H | O | $CO_2CH_3$ | |
| $OCH_3$ | $CH_2CH=CH_2$ | H | O | $CO_2C_2H_5$ | |
| $OCH_3$ | $CH_2CH=CH_2$ | H | O | $CO_2CH(CH_3)_2$ | |
| $OCH_3$ | $CH_2CH=CH_2$ | H | O | $CO_2CH_2CH=CH_2$ | |
| $OCH_3$ | $CH_2CH=CH_2$ | H | O | $CO_2CH_2CH_2OCH_3$ | |
| $OCH_3$ | $CH_2CH=CH_2$ | H | O | $SO_2N(Me)_2$ | |
| $OCH_3$ | $CH_2CH=CH_2$ | H | O | $OSO_2CH_3$ | |
| $OCH_3$ | $CH_2CH=CH_2$ | H | O | $SO_2CH_3$ | |
| $OCH_3$ | $CH_2CH=CH_2$ | H | O | $SO_2CH_2CH_2CH_3$ | |
| $OCH_3$ | $CH_2CH=CH_2$ | H | O | $OCF_3$ | |
| $OCH_3$ | $CH_2OCH_3$ | H | O | Cl | |
| $OCH_3$ | $CH_2OCH_3$ | H | O | $NO_2$ | |
| $OCH_3$ | $CH_2OCH_3$ | H | O | $CO_2CH_3$ | |
| $OCH_3$ | $CH_2OCH_3$ | H | O | $CO_2CH_2CH_2CH_3$ | |
| $OCH_3$ | $CH_2OCH_3$ | H | O | $CO_2CH_2CH=CH_2$ | |
| $OCH_3$ | $CH_2OCH_3$ | H | O | $SO_2N(Me)_2$ | |
| $OCH_3$ | $CH_2OCH_3$ | H | O | $SO_2CH_3$ | |
| $OCH_3$ | $CH_2OCH_3$ | H | O | $SO_2CH_2CH_2CH_3$ | |
| $OCH_3$ | $CH_2OCH_3$ | H | O | $OSO_2CH_3$ | |

Table 11 (continued)

| $R_1$ | $R_2$ | $R_3$ | W | $R_{19}$ | m.p.(°C) |
|---|---|---|---|---|---|
| $OCH_3$ | $CH_2OCH_3$ | H | O | $OCF_3$ | |
| $OCH_3$ | $CH_2SCH_3$ | H | O | Cl | |
| $OCH_3$ | $CH_2SCH_3$ | H | O | $NO_2$ | |
| $OCH_3$ | $CH_2SCH_3$ | H | O | $CO_2CH_3$ | |
| $OCH_3$ | $CH_2SCH_3$ | H | O | $CO_2CH_2CH_3$ | |
| $OCH_3$ | $CH_2SCH_3$ | H | O | $CO_2CH(CH_3)_2$ | |
| $OCH_3$ | $CH_2SCH_3$ | H | O | $CO_2CH_2CH{=}CH_2$ | |
| $OCH_3$ | $CH_2SCH_3$ | H | O | $SO_2N(CH_3)_2$ | |
| $OCH_3$ | $CH_2SCH_3$ | H | O | $OSO_2CH_3$ | |
| $OCH_3$ | $CH_2SCH_3$ | H | O | $SO_2CH_3$ | |
| $OCH_3$ | $CH_2SCH_3$ | H | O | $SO_2CH_2CH_2CH_3$ | |
| $OCH_3$ | $CH_2SCH_3$ | H | O | $OCF_3$ | |
| $OCH_3$ | $CH_2CF_3$ | H | O | Cl | |
| $OCH_3$ | $CH_2CF_3$ | H | O | $NO_2$ | |
| $OCH_3$ | $CH_2CF_3$ | H | O | $CF_3$ | |
| $OCH_3$ | $CH_2CF_3$ | H | O | $CO_2CH_3$ | |
| $OCH_3$ | $CH_2CF_3$ | H | O | $CO_2CH_2CH{=}CH_2$ | |
| $OCH_3$ | $CH_2CF_3$ | H | O | $OSO_2CH_3$ | |
| $OCH_3$ | $CH_2CF_3$ | H | O | $SO_2CH_3$ | |
| $OCH_3$ | $CH_2CF_3$ | H | O | $SO_2CH_2CH_2CH_3$ | |
| $OCH_3$ | $CH_2CF_3$ | H | O | $SO_2N(CH_3)_2$ | |
| $OCH_3$ | $CH_2C{\equiv}CH$ | H | O | $CO_2CH_3$ | |
| $OCH_3$ | $CH_2CH_2OCH_3$ | H | O | $CO_2CH_3$ | |
| $OCH_3$ | $CH_2CH_2SCH_3$ | H | O | $CO_2CH_3$ | |
| $OCH_3$ | $CH_2CH_2CH_3$ | H | O | $CO_2CH_3$ | |
| $CH_3$ | $CH_3$ | H | O | Cl | |
| $CH_3$ | $CH_3$ | H | O | $NO_2$ | |
| $CH_3$ | $CH_3$ | H | O | $CF_3$ | |
| $CH_3$ | $CH_3$ | H | O | $CO_2CH_3$ | |
| $CH_3$ | $CH_3$ | H | O | $CO_2CH_2CH{=}CH_2$ | |
| $CH_3$ | $CH_3$ | H | O | $SO_2N(CH_3)_2$ | |

Table 11 (continued)

| $R_1$ | $R_2$ | $R_3$ | W | $R_{19}$ | m.p.(°C) |
|---|---|---|---|---|---|
| $CH_3$ | $CH_3$ | H | O | $SO_2CH_3$ | |
| $C_2H_5$ | $CH_3$ | H | O | $CO_2CH_3$ | |
| $(CH_2)_3CH_3$ | $CH_3$ | H | O | $CO_2CH_3$ | |
| $CH_2CH{=}CH_2$ | $CH_3$ | H | O | $CO_2CH_3$ | |
| $CH_2C{\equiv}CH$ | $CH_3$ | H | O | $CO_2CH_3$ | |
| $SCH_3$ | $CH_3$ | H | O | $CO_2CH_3$ | |
| $SCH_2CH_3$ | $CH_3$ | H | O | $CO_2CH_3$ | |
| $SCH_2CH{=}CH_2$ | $CH_3$ | H | O | $CO_2CH_3$ | |
| $SCH_2CO_2CH_3$ | $CH_3$ | H | O | $CO_2CH_3$ | |
| $CH_2OCH_3$ | $CH_3$ | H | O | $CO_2CH_3$ | |
| $CH_2OCH_2CH_3$ | $CH_3$ | H | O | $CO_2CH_3$ | |
| $CH_2CH_2OCH_3$ | $CH_3$ | H | O | $CO_2CH_3$ | |
| $N(CH_3)_2$ | $CH_3$ | H | O | $CO_2CH_3$ | |
| $CF_3$ | $CH_3$ | H | O | $CO_2CH_3$ | |
| $CF_2CF_3$ | $CH_3$ | H | O | $CO_2CH_3$ | |
| Cl | $CH_3$ | H | O | $CO_2CH_3$ | |
| $NH(CH_3)$ | $CH_3$ | H | O | $CO_2CH_3$ | |
| $CH(OCH_3)_2$ | $CH_3$ | H | O | $CO_2CH_3$ | |
| 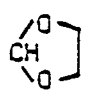 | $CH_3$ | H | O | $CO_2CH_3$ | |
| $OCH_3$ | $CH_3$ | $CH_3$ | O | $CO_2CH_3$ | |
| $OCH_3$ | $CH_3$ | H | S | $CO_2CH_3$ | |
| $OCH_3$ | $CH_3$ | H | S | $SO_2N(CH_3)_2$ | |
| $OCH_3$ | $CH_3$ | $CH_3$ | O | $SO_2N(CH_3)_2$ | |

Table 12

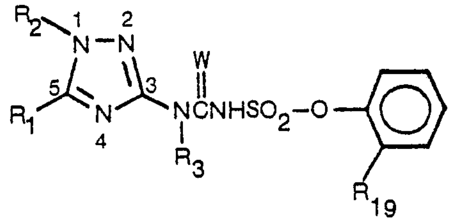

| $R_1$ | $R_2$ | $R_3$ | W | $R_{19}$ | m.p.(°C) |
|---|---|---|---|---|---|
| $OCH_3$ | $CH_3$ | H | O | Cl | |
| $OCH_3$ | $CH_3$ | H | O | $NO_2$ | |
| $OCH_3$ | $CH_3$ | H | O | $CF_3$ | |
| $OCH_3$ | $CH_3$ | H | O | $CO_2CH_3$ | |
| $OCH_3$ | $CH_3$ | H | O | $CO_2C_2H_5$ | |
| $OCH_3$ | $CH_3$ | H | O | $CO_2(CH_2)_2CH_3$ | |
| $OCH_3$ | $CH_3$ | H | O | $CO_2(CH_2)_3CH_3$ | |
| $OCH_3$ | $CH_3$ | H | O | $CO_2(CH_2)_4CH_3$ | |
| $OCH_3$ | $CH_3$ | H | O | $CO_2(CH_2)_5CH_3$ | |
| $OCH_3$ | $CH_3$ | H | O | $CO_2CH(CH_3)_2$ | |
| $OCH_3$ | $CH_3$ | H | O | $CO_2CH_2CH_2OCH_3$ | |
| $OCH_3$ | $CH_3$ | H | O | $CO_2CH_2CH_2OC_2H_5$ | |
| $OCH_3$ | $CH_3$ | H | O | $CO_2(CH_2)_3OC_2H_5$ | |
| $OCH_3$ | $CH_3$ | H | O | $CO_2CH_2CH{=}CH_2$ | |
| $OCH_3$ | $CH_3$ | H | O | $CO_2CH_2CH{=}CHCH_3$ | |
| $OCH_3$ | $CH_3$ | H | O | $CO_2(CH_2)_4CH{=}CH_2$ | |
| $OCH_3$ | $CH_3$ | H | O | $CO_2CH_2C{\equiv}CH$ | |
| $OCH_3$ | $CH_3$ | H | O | $CO_2CH_2C{\equiv}CCH_3$ | |
| $OCH_3$ | $CH_3$ | H | O | $CO_2(CH_2)_4C{\equiv}CH$ | |
| $OCH_3$ | $CH_3$ | H | O | $CO_2CH(CH_3)C_2H_5$ | |
| $OCH_3$ | $CH_3$ | H | O | $CO_2CH_2CH(CH_3)_2$ | |
| $OCH_3$ | $CH_3$ | H | O | $CO_2CF_3$ | |
| $OCH_3$ | $CH_3$ | H | O | $CO_2CH_2CH_2Cl$ | |
| $OCH_3$ | $CH_3$ | H | O | $CO_2CH_2CF_3$ | |

Table 12 (continued)

| $R_1$ | $R_2$ | $R_3$ | W | $R_{19}$ | m.p.(°C) |
|---|---|---|---|---|---|
| $OCH_3$ | $CH_3$ | H | O | $CO_2CH_2CCl_3$ | |
| $OCH_3$ | $CH_3$ | H | O | $CO_2CH_2CH_2CF_3$ | |
| $OCH_3$ | $CH_3$ | H | O | $CO_2CH_2CH_2CH_2Cl$ | |
| $OCH_3$ | $CH_3$ | H | O | $CO_2CClF_2$ | |
| $OCH_3$ | $CH_3$ | H | O | $CO_2CF_2H$ | |
| $OCH_3$ | $CH_3$ | H | O | $CO_2CHFCF_2H$ | |
| $OCH_3$ | $CH_3$ | H | O | $CO_2CHClCF_2H$ | |
| $OCH_3$ | $CH_3$ | H | O | $CO_2CH_2CHFCF_2H$ | |
| $OCH_3$ | $CH_3$ | H | O | $SO_2N(OCH_3)CH_3$ | |
| $OCH_3$ | $CH_3$ | H | O | $SO_2N(CH_3)_2$ | |
| $OCH_3$ | $CH_3$ | H | O | $SO_2N(C_2H_5)_2$ | |
| $OCH_3$ | $CH_3$ | H | O | $SO_2N(CH_3)C_2H_5$ | |
| $OCH_3$ | $CH_3$ | H | O | $SO_2N(CH_3)CH_2CH_2CH_3$ | |
| $OCH_3$ | $CH_3$ | H | O | $OSO_2CH_3$ | |
| $OCH_3$ | $CH_3$ | H | O | $OSO_2C_2H_5$ | |
| $OCH_3$ | $CH_3$ | H | O | $OSO_2(CH_2)_2CH_3$ | |
| $OCH_3$ | $CH_3$ | H | O | $OSO_2(CH_2)_3CH_3$ | |
| $OCH_3$ | $CH_3$ | H | O | $OSO_2CH(CH_3)_2$ | |
| $OCH_3$ | $CH_3$ | H | O | $OSO_2CH(CH_3)C_2H_5$ | |
| $OCH_3$ | $CH_3$ | H | O | $OSO_2CH_2CH_2OCH_3$ | |
| $OCH_3$ | $CH_3$ | H | O | $OSO_2(CH_2)_3OCH_3$ | |
| $OCH_3$ | $CH_3$ | H | O | $OSO_2CF_3$ | |
| $OCH_3$ | $CH_3$ | H | O | $OSO_2CH_2CF_3$ | |
| $OCH_3$ | $CH_3$ | H | O | $OSO_2CHFCF_2H$ | |
| $OCH_3$ | $CH_3$ | H | O | $OSO_2CF_2CH_2CH_2CH_3$ | |
| $OCH_3$ | $CH_3$ | H | O | $OSO_2CClF_2$ | |
| $OCH_3$ | $CH_3$ | H | O | $OSO_2CBr_2CH_3$ | |
| $OCH_3$ | $CH_3$ | H | O | $N(CH_3)SO_2CH_3$ | |
| $OCH_3$ | $CH_3$ | H | O | $N(CH_3)SO_2C_2H_5$ | |
| $OCH_3$ | $CH_3$ | H | O | $N(CH_3)SO_2C_3H_5$ | |
| $OCH_3$ | $CH_3$ | H | O | $N(CH_3)SO_2CH(CH_3)C_2H_5$ | |

Table 12 (continued)

| $R_1$ | $R_2$ | $R_3$ | W | $R_{19}$ | m.p.(°C) |
|---|---|---|---|---|---|
| $OCH_3$ | $CH_3$ | H | O | $N(CH_3)SO_2CH_2CH_2OCH_3$ | |
| $OCH_3$ | $CH_3$ | H | O | $N(CH_3)SO_2CF_3$ | |
| $OCH_3$ | $CH_3$ | H | O | $SCH_3$ | |
| $OCH_3$ | $CH_3$ | H | O | $SOCH_3$ | |
| $OCH_3$ | $CH_3$ | H | O | $SO_2CH_3$ | |
| $OCH_3$ | $CH_3$ | H | O | $SO_2C_2H_5$ | |
| $OCH_3$ | $CH_3$ | H | O | $SO_2CH_2CH_2CH_3$ | |
| $OCH_3$ | $CH_3$ | H | O | $SO_2CH(CH_3)_2$ | |
| $OCH_3$ | $CH_3$ | H | O | $SO_2CH_2CH{=}CH_2$ | |
| $OCH_3$ | $CH_3$ | H | O | $SO_2CH(CH_3)C_2H_5$ | |
| $OCH_3$ | $CH_3$ | H | O | $SCH_2CH_2CH_3$ | |
| $OCH_3$ | $CH_3$ | H | O | $SCF_3$ | |
| $OCH_3$ | $CH_3$ | H | O | $SOCF_3$ | |
| $OCH_3$ | $CH_3$ | H | O | $SO_2CF_3$ | |
| $OCH_3$ | $CH_3$ | H | O | $OCCl_3$ | |
| $OCH_3$ | $CH_3$ | H | O | $OCF_3$ | |
| $OCH_3$ | $CH_3$ | H | O | $OCF_2CF_3$ | |
| $OCH_3$ | $CH_3$ | H | O | $OCF_2CF_2CH_3$ | |
| $OCH_3$ | $CH_3$ | H | O | $OCClF_2$ | |
| $OCH_3$ | $CH_3$ | H | O | $OCCl_2CF_3$ | |
| $OC_2H_5$ | $CH_3$ | H | O | Cl | |
| $OC_2H_5$ | $CH_3$ | H | O | $NO_2$ | |
| $OC_2H_5$ | $CH_3$ | H | O | $CF_3$ | |
| $OC_2H_5$ | $CH_3$ | H | O | $CO_2CH_3$ | |
| $OC_2H_5$ | $CH_3$ | H | O | $CO_2C_2H_5$ | |
| $OC_2H_5$ | $CH_3$ | H | O | $CO_2CH_2CH_2CH_3$ | |
| $OC_2H_5$ | $CH_3$ | H | O | $CO_2CH(CH_3)_2$ | |
| $OC_2H_5$ | $CH_3$ | H | O | $CO_2CH_2CH{=}CH_2$ | |
| $OC_2H_5$ | $CH_3$ | H | O | $CO_2CH_2C{\equiv}CH$ | |
| $OC_2H_5$ | $CH_3$ | H | O | $CO_2CH_2CH_2OCH_3$ | |
| $OC_2H_5$ | $CH_3$ | H | O | $CO_2CH_2CH_2OC_2H_5$ | |

Table 12 (continued)

| $R_1$ | $R_2$ | $R_3$ | W | $R_{19}$ | m.p.(°C) |
|---|---|---|---|---|---|
| $OC_2H_5$ | $CH_3$ | H | O | $CO_2CF_3$ | |
| $OC_2H_5$ | $CH_3$ | H | O | $SO_2N(OCH_3)CH_3$ | |
| $OC_2H_5$ | $CH_3$ | H | O | $SO_2N(CH_3)_2$ | |
| $OC_2H_5$ | $CH_3$ | H | O | $SO_2N(CH_3)C_2H_5$ | |
| $OC_2H_5$ | $CH_3$ | H | O | $SO_2N(C_2H_5)_2$ | |
| $OC_2H_5$ | $CH_3$ | H | O | $OSO_2CH_3$ | |
| $OC_2H_5$ | $CH_3$ | H | O | $OSO_2C_2H_5$ | |
| $OC_2H_5$ | $CH_3$ | H | O | $N(CH_3)SO_2CH_3$ | |
| $OC_2H_5$ | $CH_3$ | H | O | $SO_2CH_3$ | |
| $OC_2H_5$ | $CH_3$ | H | O | $SO_2C_2H_5$ | |
| $OC_2H_5$ | $CH_3$ | H | O | $SO_2CH_2CH_2CH_3$ | |
| $OC_2H_5$ | $CH_3$ | H | O | $SO_2CHCH{=}CH_2$ | |
| $OC_2H_5$ | $CH_3$ | H | O | $SO_2CF_3$ | |
| $OC_2H_5$ | $CH_3$ | H | O | $OCF_3$ | |
| $OC_2H_5$ | $CH_3$ | H | O | $OCF_2CF_3$ | |
| $OC_2H_5$ | $C_2H_5$ | H | O | Cl | |
| $OC_2H_5$ | $C_2H_5$ | H | O | $NO_2$ | |
| $OC_2H_5$ | $C_2H_5$ | H | O | $CF_3$ | |
| $OC_2H_5$ | $C_2H_5$ | H | O | $CO_2CH_3$ | |
| $OC_2H_5$ | $C_2H_5$ | H | O | $CO_2C_2H_5$ | |
| $OC_2H_5$ | $C_2H_5$ | H | O | $CO_2CH_2CH_2CH_3$ | |
| $OC_2H_5$ | $C_2H_5$ | H | O | $CO_2CH(CH_3)_2$ | |
| $OC_2H_5$ | $C_2H_5$ | H | O | $CO_2CHCH{=}CH_2$ | |
| $OC_2H_5$ | $C_2H_5$ | H | O | $CO_2CH_2C{\equiv}CH$ | |
| $OC_2H_5$ | $C_2H_5$ | H | O | $CO_2CH_2CH_2OCH_3$ | |
| $OC_2H_5$ | $C_2H_5$ | H | O | $SO_2N(OCH_3)CH_3$ | |
| $OC_2H_5$ | $C_2H_5$ | H | O | $SO_2N(CH_3)_2$ | |
| $OC_2H_5$ | $C_2H_5$ | H | O | $OSO_2CH_3$ | |
| $OC_2H_5$ | $C_2H_5$ | H | O | $N(CH_3)SO_2CH_3$ | |
| $OC_2H_5$ | $C_2H_5$ | H | O | $SO_2CH_3$ | |
| $OC_2H_5$ | $C_2H_5$ | H | O | $SO_2CH_2CH_3$ | |

Table 12 (continued)

| $R_1$ | $R_2$ | $R_3$ | W | $R_{19}$ | m.p.(°C) |
|---|---|---|---|---|---|
| $OC_2H_5$ | $C_2H_5$ | H | O | $SO_2CH_2CH_2CH_3$ | |
| $OC_2H_5$ | $C_2H_5$ | H | O | $OCF_3$ | |
| $OCH_2CH_2CH_3$ | $CH_3$ | H | O | Cl | |
| $OCH_2CH_2CH_3$ | $CH_3$ | H | O | $NO_2$ | |
| $OCH_2CH_2CH_3$ | $CH_3$ | H | O | $CF_3$ | |
| $OCH_2CH_2CH_3$ | $CH_3$ | H | O | $CO_2CH_3$ | |
| $OCH_2CH_2CH_3$ | $CH_3$ | H | O | $CO_2CH_2CH{=}CH_2$ | |
| $OCH_2CH_2CH_3$ | $CH_3$ | H | O | $SO_2N(OCH_3)CH_3$ | |
| $OCH_2CH_2CH_3$ | $CH_3$ | H | O | $SO_2N(CH_3)_2$ | |
| $OCH_2CH_2CH_3$ | $CH_3$ | H | O | $OSO_2CH_3$ | |
| $OCH_2CH_2CH_3$ | $CH_3$ | H | O | $N(CH_3)SO_2CH_3$ | |
| $OCH_2CH_2CH_3$ | $CH_3$ | H | O | $SO_2CH_3$ | |
| $OCH_2CH_2CH_3$ | $CH_3$ | H | O | $SO_2CH_2CH_2CH_3$ | |
| $OCH_2CH_2CH_3$ | $CH_3$ | H | O | $OCF_3$ | |
| $O(CH_2)_3CH_3$ | $CH_3$ | H | O | Cl | |
| $O(CH_2)_3CH_3$ | $CH_3$ | H | O | $NO_2$ | |
| $O(CH_2)_3CH_3$ | $CH_3$ | H | O | $CF_3$ | |
| $O(CH_2)_3CH_3$ | $CH_3$ | H | O | $CO_2CH_3$ | |
| $O(CH_2)_3CH_3$ | $CH_3$ | H | O | $CO_2C_2H_5$ | |
| $O(CH_2)_3CH_3$ | $CH_3$ | H | O | $CO_2CH(CH_3)_2$ | |
| $O(CH_2)_3CH_3$ | $CH_3$ | H | O | $SO_2N(CH_3)_2$ | |
| $O(CH_2)_3CH_3$ | $CH_3$ | H | O | $SO_2CH_3$ | |
| $O(CH_2)_3CH_3$ | $CH_3$ | H | O | $SO_2CH_2CH_2CH_3$ | |
| $O(CH_2)_3CH_3$ | $CH_3$ | H | O | $OCF_3$ | |
| $OCH_3$ | $C_2H_5$ | H | O | Cl | |
| $OCH_3$ | $C_2H_5$ | H | O | $NO_2$ | |
| $OCH_3$ | $C_2H_5$ | H | O | $CF_3$ | |
| $OCH_3$ | $C_2H_5$ | H | O | $CO_2CH_3$ | |
| $OCH_3$ | $C_2H_5$ | H | O | $CO_2C_2H_5$ | |
| $OCH_3$ | $C_2H_5$ | H | O | $CO_2CH(CH_3)_2$ | |
| $OCH_3$ | $C_2H_5$ | H | O | $CO_2CH_2CH{=}CH_2$ | |

Table 12 (continued)

| $R_1$ | $R_2$ | $R_3$ | W | $R_{19}$ | m.p.(°C) |
|---|---|---|---|---|---|
| $OCH_3$ | $C_2H_5$ | H | O | $CO_2CH_2CH_2OCH_3$ | |
| $OCH_3$ | $C_2H_5$ | H | O | $CO_2CH_2CH_2Cl$ | |
| $OCH_3$ | $C_2H_5$ | H | O | $SO_2N(OCH_3)CH_3$ | |
| $OCH_3$ | $C_2H_5$ | H | O | $SO_2N(CH_3)_2$ | |
| $OCH_3$ | $C_2H_5$ | H | O | $OSO_2CH_3$ | |
| $OCH_3$ | $C_2H_5$ | H | O | $N(CH_3)SO_2CH_3$ | |
| $OCH_3$ | $C_2H_5$ | H | O | $SO_2CH_3$ | |
| $OCH_3$ | $C_2H_5$ | H | O | $SO_2CH_2CH_2CH_3$ | |
| $OCH_3$ | $C_2H_5$ | H | O | $OCF_3$ | |
| $OCH_3$ | $CH_2CH=CH_2$ | H | O | Cl | |
| $OCH_3$ | $CH_2CH=CH_2$ | H | O | $NO_2$ | |
| $OCH_3$ | $CH_2CH=CH_2$ | H | O | $CF_3$ | |
| $OCH_3$ | $CH_2CH=CH_2$ | H | O | $CO_2CH_3$ | |
| $OCH_3$ | $CH_2CH=CH_2$ | H | O | $CO_2C_2H_5$ | |
| $OCH_3$ | $CH_2CH=CH_2$ | H | O | $CO_2CH(CH_3)_2$ | |
| $OCH_3$ | $CH_2CH=CH_2$ | H | O | $CO_2CH_2CH=CH_2$ | |
| $OCH_3$ | $CH_2CH=CH_2$ | H | O | $CO_2CH_2CH_2OCH_3$ | |
| $OCH_3$ | $CH_2CH=CH_2$ | H | O | $SO_2N(Me)_2$ | |
| $OCH_3$ | $CH_2CH=CH_2$ | H | O | $OSO_2CH_3$ | |
| $OCH_3$ | $CH_2CH=CH_2$ | H | O | $SO_2CH_3$ | |
| $OCH_3$ | $CH_2CH=CH_2$ | H | O | $SO_2CH_2CH_2CH_3$ | |
| $OCH_3$ | $CH_2CH=CH_2$ | H | O | $OCF_3$ | |
| $OCH_3$ | $CH_2OCH_3$ | H | O | Cl | |
| $OCH_3$ | $CH_2OCH_3$ | H | O | $NO_2$ | |
| $OCH_3$ | $CH_2OCH_3$ | H | O | $CO_2CH_3$ | |
| $OCH_3$ | $CH_2OCH_3$ | H | O | $CO_2CH_2CH_2CH_3$ | |
| $OCH_3$ | $CH_2OCH_3$ | H | O | $CO_2CH_2CH=CH_2$ | |
| $OCH_3$ | $CH_2OCH_3$ | H | O | $SO_2N(Me)_2$ | |
| $OCH_3$ | $CH_2OCH_3$ | H | O | $SO_2CH_3$ | |
| $OCH_3$ | $CH_2OCH_3$ | H | O | $SO_2CH_2CH_2CH_3$ | |
| $OCH_3$ | $CH_2OCH_3$ | H | O | $OSO_2CH_3$ | |

Table 12 (continued)

| $R_1$ | $R_2$ | $R_3$ | W | $R_{19}$ | m.p.(°C) |
|---|---|---|---|---|---|
| $OCH_3$ | $CH_2OCH_3$ | H | O | $OCF_3$ | |
| $OCH_3$ | $CH_2SCH_3$ | H | O | Cl | |
| $OCH_3$ | $CH_2SCH_3$ | H | O | $NO_2$ | |
| $OCH_3$ | $CH_2SCH_3$ | H | O | $CO_2CH_3$ | |
| $OCH_3$ | $CH_2SCH_3$ | H | O | $CO_2CH_2CH_3$ | |
| $OCH_3$ | $CH_2SCH_3$ | H | O | $CO_2CH(CH_3)_2$ | |
| $OCH_3$ | $CH_2SCH_3$ | H | O | $CO_2CH_2CH{=}CH_2$ | |
| $OCH_3$ | $CH_2SCH_3$ | H | O | $SO_2N(CH_3)_2$ | |
| $OCH_3$ | $CH_2SCH_3$ | H | O | $OSO_2CH_3$ | |
| $OCH_3$ | $CH_2SCH_3$ | H | O | $SO_2CH_3$ | |
| $OCH_3$ | $CH_2SCH_3$ | H | O | $SO_2CH_2CH_2CH_3$ | |
| $OCH_3$ | $CH_2SCH_3$ | H | O | $OCF_3$ | |
| $OCH_3$ | $CH_2CF_3$ | H | O | Cl | |
| $OCH_3$ | $CH_2CF_3$ | H | O | $NO_2$ | |
| $OCH_3$ | $CH_2CF_3$ | H | O | $CF_3$ | |
| $OCH_3$ | $CH_2CF_3$ | H | O | $CO_2CH_3$ | |
| $OCH_3$ | $CH_2CF_3$ | H | O | $CO_2CH_2CH{=}CH_2$ | |
| $OCH_3$ | $CH_2CF_3$ | H | O | $OSO_2CH_3$ | |
| $OCH_3$ | $CH_2CF_3$ | H | O | $SO_2CH_3$ | |
| $OCH_3$ | $CH_2CF_3$ | H | O | $SO_2CH_2CH_2CH_3$ | |
| $OCH_3$ | $CH_2CF_3$ | H | O | $SO_2N(CH_3)_2$ | |
| $OCH_3$ | $CH_2C{=}CH$ | H | O | $CO_2CH_3$ | |
| $OCH_3$ | $CH_2CH_2OCH_3$ | H | O | $CO_2CH_3$ | |
| $OCH_3$ | $CH_2CH_2SCH_3$ | H | O | $CO_2CH_3$ | |
| $OCH_3$ | $CH_2CH_2CH_3$ | H | O | $CO_2CH_3$ | |
| $CH_3$ | $CH_3$ | H | O | Cl | |
| $CH_3$ | $CH_3$ | H | O | $NO_2$ | |
| $CH_3$ | $CH_3$ | H | O | $CF_3$ | |
| $CH_3$ | $CH_3$ | H | O | $CO_2CH_3$ | |
| $CH_3$ | $CH_3$ | H | O | $CO_2CH_2CH{=}CH_2$ | |
| $CH_3$ | $CH_3$ | H | O | $SO_2N(CH_3)_2$ | |

Table 12 (continued)

| $R_1$ | $R_2$ | $R_3$ | W | $R_{19}$ | m.p.(°C) |
|---|---|---|---|---|---|
| $CH_3$ | $CH_3$ | H | O | $SO_2CH_3$ | |
| $C_2H_5$ | $CH_3$ | H | O | $CO_2CH_3$ | |
| $(CH_2)_3CH_3$ | $CH_3$ | H | O | $CO_2CH_3$ | |
| $CH_2CH{=}CH_2$ | $CH_3$ | H | O | $CO_2CH_3$ | |
| $CH_2C{\equiv}CH$ | $CH_3$ | H | O | $CO_2CH_3$ | |
| $SCH_3$ | $CH_3$ | H | O | $CO_2CH_3$ | |
| $SCH_2CH_3$ | $CH_3$ | H | O | $CO_2CH_3$ | |
| $SCH_2CH{=}CH_2$ | $CH_3$ | H | O | $CO_2CH_3$ | |
| $SCH_2CO_2CH_3$ | $CH_3$ | H | O | $CO_2CH_3$ | |
| $CH_2OCH_3$ | $CH_3$ | H | O | $CO_2CH_3$ | |
| $CH_2OCH_2CH_3$ | $CH_3$ | H | O | $CO_2CH_3$ | |
| $CH_2CH_2OCH_3$ | $CH_3$ | H | O | $CO_2CH_3$ | |
| $N(CH_3)_2$ | $CH_3$ | H | O | $CO_2CH_3$ | |
| $CF_3$ | $CH_3$ | H | O | $CO_2CH_3$ | |
| $CF_2CF_3$ | $CH_3$ | H | O | $CO_2CH_3$ | |
| Cl | $CH_3$ | H | O | $CO_2CH_3$ | |
| $NH(CH_3)$ | $CH_3$ | H | O | $CO_2CH_3$ | |
| $CH(OCH_3)_2$ | $CH_3$ | H | O | $CO_2CH_3$ | |
| 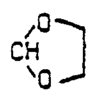 | $CH_3$ | H | O | $CO_2CH_3$ | |
| $OCH_3$ | $CH_3$ | $CH_3$ | O | $CO_2CH_3$ | |
| $OCH_3$ | $CH_3$ | H | S | $CO_2CH_3$ | |
| $OCH_3$ | $CH_3$ | H | S | $SO_2N(CH_3)_2$ | |
| $OCH_3$ | $CH_3$ | $CH_3$ | O | $SO_2N(CH_3)_2$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TABLE 13

| | Active Ingredient | Weight Percent* Diluent(s) | Surfactant(s) |
|---|---|---|---|
| Wettable Powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example X

Wettable Powder

| | |
|---|---|
| 2-[[(1-methyl-5-methylthio-1H-1,2,4-triazol-3-yl)-aminocarbonyl]aminosulfonyl]benzoic acid, methyl ester | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 30 microns, reblended, and packaged.

Example XI

Wettable Powder

| | |
|---|---|
| 2-[[(5-ethylthio-1-methyl-1H-1,2,4-triazol-3-yl)-aminocarbonyl]aminosulfonyl]benzoic acid, methyl ester | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example XII

Granule

| | |
|---|---|
| Wettable Powder of Example XI | 5% |
| attapulgite granules (U.S.S. 20—40 mesh; 0.84—0.42 mm) | 95% |

A slurry of wettable powder containing ≈25% solids is sprayed on the surface of attapulgite granules in a double-cone blender. The granules are dried and packaged.

Example XIII

Extruded Pellet

| | |
|---|---|
| 2-[[(5-ethyl-1-methyl-1H-1,2,4-triazol-3-yl)aminocarbonyl]aminosulfonyl]benzoic acid, methyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture

is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example XIV

Oil Suspension

| | |
|---|---|
| 2-[[(5-methoxy-1-methyl-1H-1,2,4-triazol-3-yl)aminocarbonyl]aminosulfonyl]benzoic acid, methyl ester | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example XV

Wettable Powder

| | |
|---|---|
| N-[(5-methoxy-1-methyl-1H-1,2,4-triazol-3-yl)aminocarbonyl]-2-chlorobenzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example XVI

Low Strength Granule

| | |
|---|---|
| N-[(5-methoxy-1-methyl-1H-1,2,4-triazol-3-yl)aminocarbonyl]-N',N'-dimethyl-1,2-benzenedisulfonamide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example XVII

Aqueous Suspension

| | |
|---|---|
| 2-[[(5-methylthio-1-methyl-1H-1,2,4-triazol-3-yl)-aminocarbonyl]aminosulfonyl]benzoic acid, methyl ester | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example XVIII

Solution

| | |
|---|---|
| 2-[[(5-ethyl-1-methyl-1H-1,2,4-triazol-3-yl)aminocarbonyl]aminosulfonyl]benzoic acid, methyl ester, sodium salt | 5% |
| water | 95% |

The salt is added directly to the water with stirring to produce the solution, which may then be packaged for use.

Example XIX

Low Strength Granule

| | |
|---|---|
| 2-[[(5-ethylthio-1-methyl-1H-1,2,4-triazol-3-yl)-aminocarbonyl]aminosulfonyl]benzoic acid, methyl ester | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example XX

Granule

| | |
|---|---|
| 2-[[(5-methoxy-1-methyl-1H-1,2,4-triazol-3-yl)aminocarbonyl]aminosulfonyl]benzoic acid, methyl ester | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example XXI

High Strength Concentrate

| | |
|---|---|
| N-[(5-methoxy-1-methyl-1H-1,2,4-triazol-3-yl)amino-carbonyl]-2-chlorobenzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example XXII

Wettable Powder

| | |
|---|---|
| N-[(5-methoxy-1-methyl-1H-1,2,4-triazol-3-yl)aminocarbonyl]-N',N'-dimethyl-1,2-benzenedisulfon-amide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example XXIII

Wettable Powder

| | |
|---|---|
| 2-[[(5-methylthio-1-methyl-1H-1,2,4-triazol-3-yl)aminocarbonyl]aminosulfonyl]benzoic acid, methyl ester | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example XXIV

Oil Suspension

| | |
|---|---|
| 2-[[(5-ethylthio-1-methyl-1H-1,2,4-triazol-3-yl)aminocarbonyl]aminosulfonyl]benzoic acid, methyl ester | 35% |
| blend of polyalcohol carboxylic esters and oil soluble petroleum sulfonates | 6% |
| xylene | 59% |

The ingredients are combined and ground together in a sand mill to produce particles essentially all

below 5 microns. The product can be used directly, extended with oils, or emulsified in water.

Example XXV

Dust

| | |
|---|---|
| 2-[[(5-ethyl-1-methyl-1H-1,2,4-triazol-3-yl)aminocarbonyl]aminosulfonyl]benzoic acid, methyl ester | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

The compounds of the present invention have herbicidal activity. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. In addition, some of the compounds are useful for the selective control of weeds in certain crops, including wheat and soybeans.

The rates of application for the compounds of the invention are determined by a number of factors, including their use as selective or general herbicides, the crop species involved, the types of weeds to be controlled, weather conditions, formulations selected, mode of application, amount of foliage present, etc. In general terms, the subject compounds should be applied at levels of around 0.125 to 10 kg/ha, the lower rates being suggested for use on lighter soils and/or those having a low organic matter content, or for situations where only short-term persistence is required.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

Test A

Seeds of crabgrass (*Digitaria* sp.), barnyardgrass (*Echinochloa crusgalli*), wild oats (*Avena fatua*), cassia (*Cassia tora*), morningglory (*Ipomoea* sp.), cocklebur (*Xanthium* sp.), sorghum, corn, soybean, rice, wheat and nutsedge tubers (*Cyperus rotundus*) were planted in a growth medium and treated pre-emergence with the chemicals dissolved in a non-phytotoxic solvent. At the same time, cotton having five leaves (including cotyledonary ones), bush beans with the third trifoliate leaf expanding, crabgrass, barnyardgrass and wild oats with two leaves, cassia with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three to five leaves were sprayed. Treated plants and controls were maintained in a greenhouse for sixteen days, whereupon all species were compared to controls and visually rated for response to treatment.

The ratings are based on a numerical scale extending from 0 = no injury, to 10 = complete kill. The accompanying descriptive symbols have the following meanings:

A = growth acceleration;
C = chlorosis or necrosis;
E = emergence inhibition;
G = growth retardation;
H = formative effects;
P = terminal bud injury;
U = unusual pigmentation;
X = axillary stimulation; and
6Y = abscised buds or flowers.

The ratings for the compounds tested by this procedure are presented in Table A. The compounds tested demonstrate good control of nutsedge, especially in pre-emergence application. The results show one compound to be relatively tolerant to soybeans, and a few to be tolerant to wheat.

Table A Structures

Compound 1

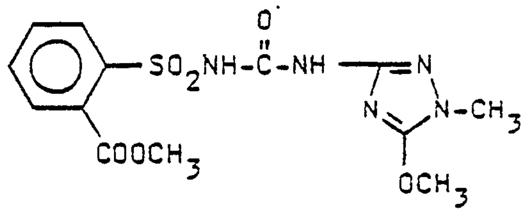

Compound 2

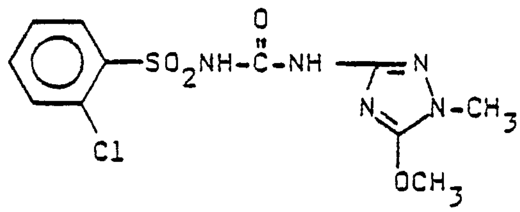

Compound 3

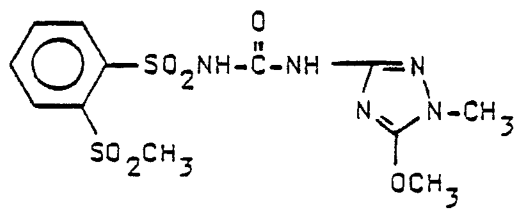

Compound 4

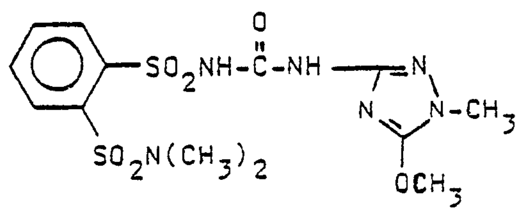

Compound 5

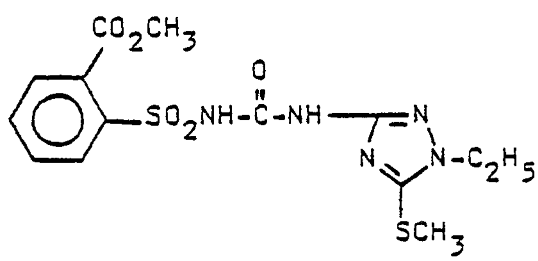

Compound 6

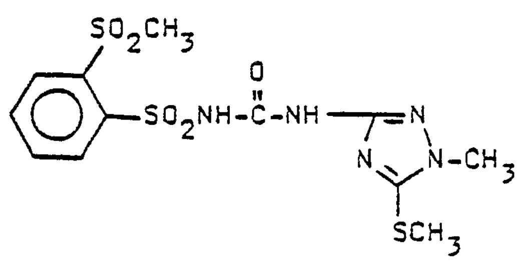

Table A Structures (continued)

Compound 7

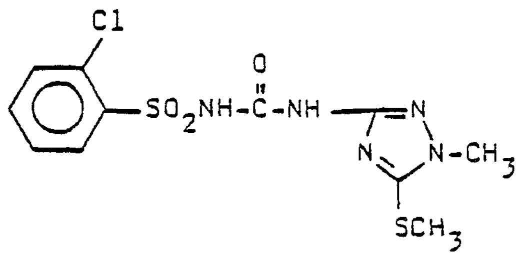

Compound 8

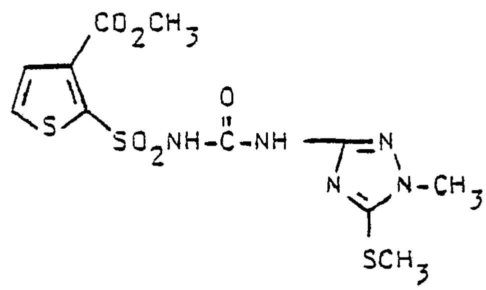

Compound 9

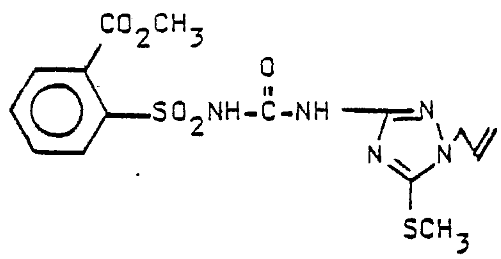

Compound 10

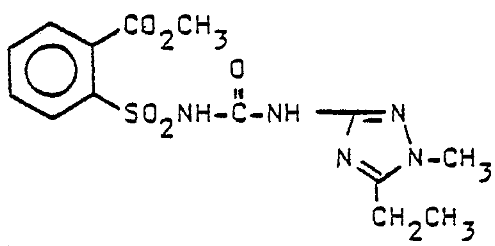

Compound 11

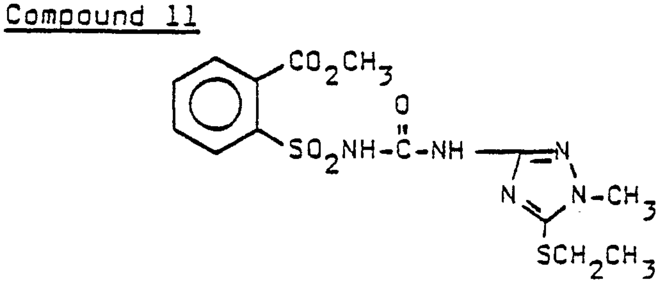

Compound 12

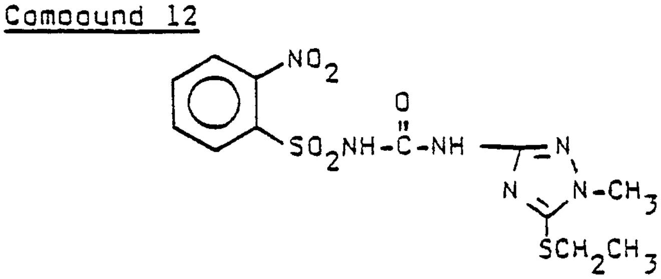

Table A Structures (continued)

Compound 13

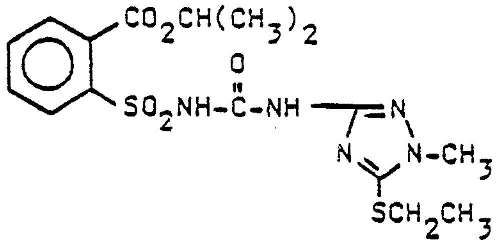

Compound 14

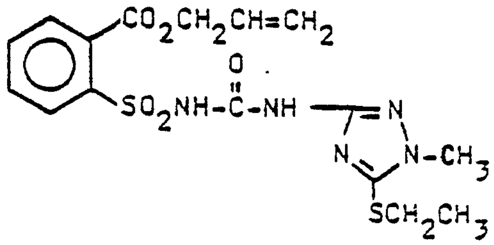

Compound 15

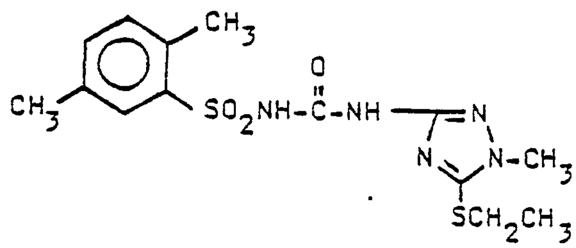

Compound 16

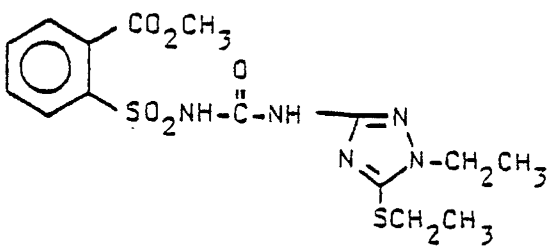

Compound 17

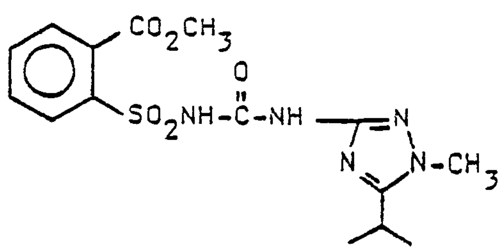

Compound 18

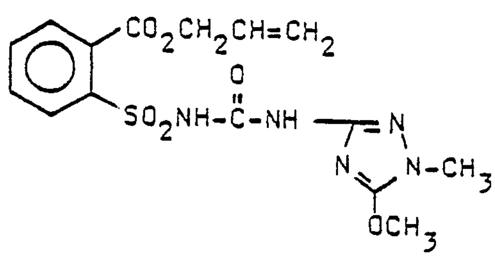

Table A Structures (continued)

Compound 19

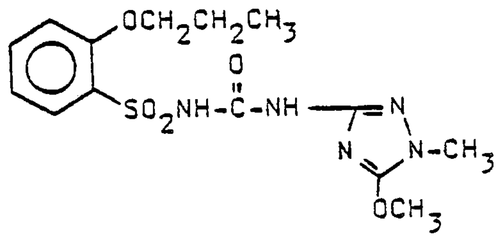

Compound 20

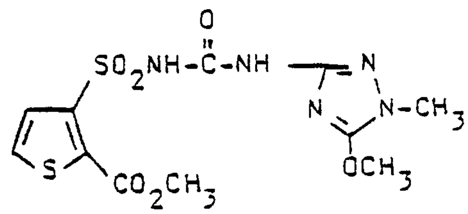

Compound 21

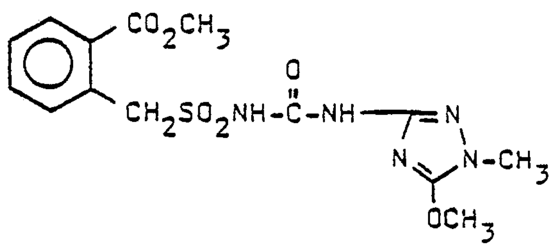

Compound 22

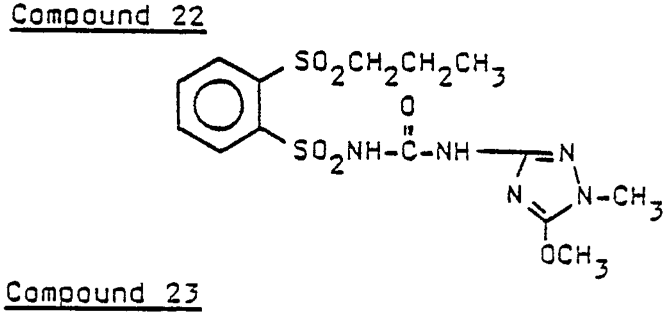

Compound 23

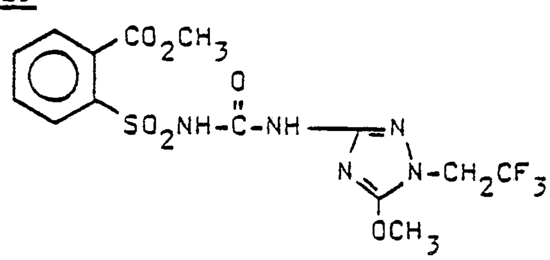

Compound 24

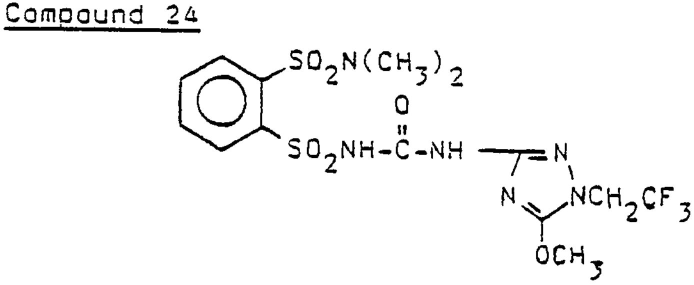

Table A Structures (continued)

Compound 25

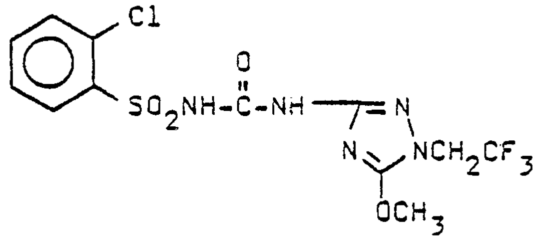

Compound 26

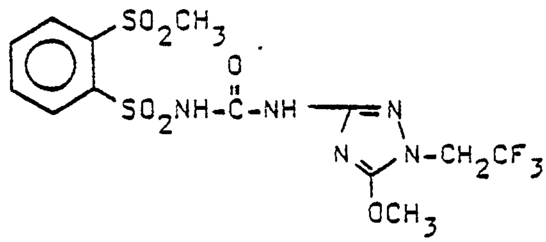

Compound 27

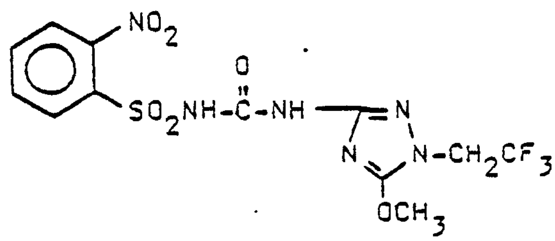

Compound 28

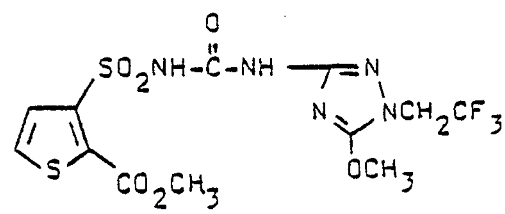

Compound 29

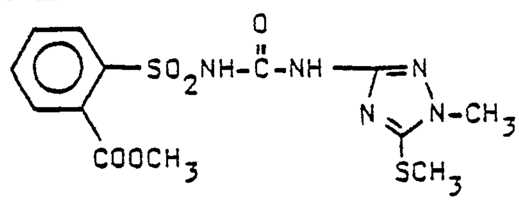

Compound 30

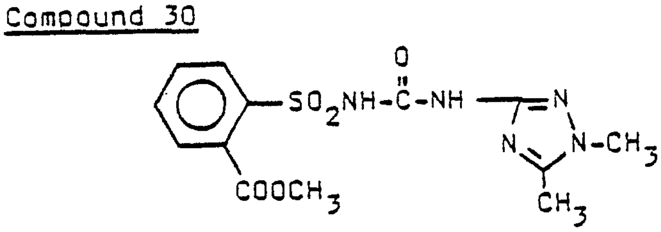

Table A Structures (continued)

Compound 31

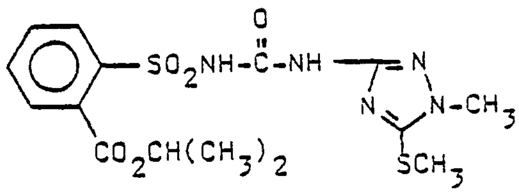

Compound 32

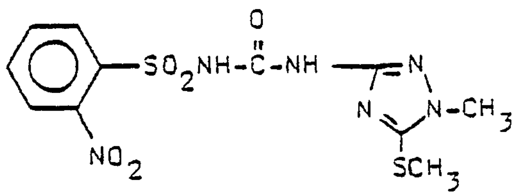

Compound 33

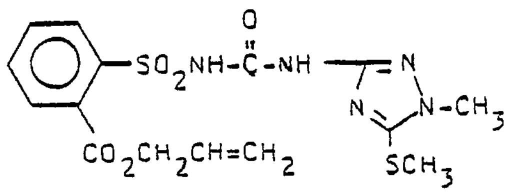

Table A

| | Compound 1 | | Compound 2 | |
|---|---|---|---|---|
| Rate kg/ha | 0.4 | .05 | 0.4 | .05 |
| POST-EMERGENCE | | | | |
| Bush bean | 9C | 5C,9G,6Y | 5C,9G,6Y | 5C,9G,6Y |
| Cotton | 5C,9G | 2C,9G | 4C,9G | 4C,8G |
| Morningglory | 5C,9G | 9C | 5C,9G | 4C,8H |
| Cocklebur | 10C | 9C | 9C | 3G |
| Cassia | 9C | 9C | 5C,9G | 3C,8G |
| Nutsedge | 9C | 10C | 4C,9G | 2C,8G |
| Crabgrass | 9C | 9C | 5C,9G | 2C,8G |
| Barnyardgrass | 9C | 9C | 9C | 9C |
| Wild Oats | 9C | 9C | 2C,9G | 2C,8G |
| Wheat | 9C | 9C | 2C,9G | 5G |
| Corn | 10C | 9C | 5U,9C | 9C |
| Soybean | 9C | 9C | 5C,9G | 4C,9G |
| Rice | 9C | 9C | 5C,9G | 5C,9G |
| Sorghum | 9C | 9C | 4C,9G | 4C,9G |
| Sugar beet | 9C | 9C | 9C | 5C,9G |
| PRE-EMERGENCE | | | | |
| Morningglory | 9C | 9G | 9G | 2C,8H |
| Cocklebur | 9H | 9H | 9H | 8H |
| Cassia | 9C | 3C,9G | 2C,9G | 2C,7G |
| Nutsedge | 10E | 10E | 5C,9G | 2C,7G |
| Crabgrass | 6C,9G | 5C,9G | 3C,7G | 2C,3G |
| Barnyardgrass | 10E | 6C,9H | 5C,9H | 3C,8H |
| Wild Oats | 6C,9H | 6C,9H | 4C,9G | 2C,7G |
| Wheat | 6C,9H | 6C,9H | 9H | 2C,8G |
| Corn | 10E | 10H | 10H | 3C,9H |
| Soybean | 9H | 3C,8H | 9H | 3C,4H |
| Rice | 10E | 10E | 10E | 10E |
| Sorghum | 6C,9H | 5C,9H | 6C,9H | 5C,9H |
| Sugar beet | 10E | 10E | 6C,9G | 5C,9G |

Table A (continued)

| | Compound 3 | | Compound 4 | |
|---|---|---|---|---|
| Rate kg/ha | 0.4 | .05 | 0.4 | .05 |
| POST-EMERGENCE | | | | |
| Bush bean | 5C,9G,6Y | 4C,9G,6Y | 9C | 5C,9G,6Y |
| Cotton | 5C,5H | 3C,3G | 6C,9G | 4C,8G |
| Morningglory | 4C,9G | 4C,8H | 9C | 5C,9G |
| Cocklebur | 2C,8G | 1C,4G | 9C | 9C |
| Cassia | 4C,8H | 2C,5G | 5C,9G | 4C,8H |
| Nutsedge | 2C,9G | 2C,5G | 9C | 2C,9G |
| Crabgrass | 3C,9G | 2C,3G | 9C | 9C |
| Barnyardgrass | 9C | 5C,8H | 9C | 9C |
| Wild Oats | 4C,9G | 2C,3G | 9C | 9C |
| Wheat | 5C,9G | 2C,8G | 9C | 9C |
| Corn | 5U,9C | 2C,9H | 10C | 9C |
| Soybean | 5C,9G | 3C,8G | 9C | 9C |
| Rice | 6C,9G | 5C,9G | 9C | 9C |
| Sorghum | 3C,9G | 4C,8H | 9C | 5C,9G |
| Sugar beet | 5C,9G | 2C,5G | 9C | 5C,9G |
| PRE-EMERGENCE | | | | |
| Morningglory | 4C,8H | 1C | 9C | 2C,9G |
| Cocklebur | 4G | 0 | 9H | 9H |
| Cassia | 1C | 1C | 2C,8G | 3C,8G |
| Nutsedge | 2C,6G | 0 | 10E | 5C,9G |
| Crabgrass | 2G | 0 | 6C,9G | 1C,3G |
| Barnyardgrass | 3C,6G | 1C | 10H | 5C,9H |
| Wild Oats | 3C,6G | 1C | 6C,9H | 6C,9H |
| Wheat | 2C,9G | 2G | 7C,9H | 10H |
| Corn | 3C,9H | 2C,3G | 10H | 5C,9H |
| Soybean | 2C,3G | 1C | 9H | 4C,6H |
| Rice | 5C,8H | 3C | 10E | 10E |
| Sorghum | 4C,9H | 3G | 6C,9H | 6C,9H |
| Sugar beet | 1C | 0 | 6C,9G | 5C,9G |

Table A (continued)

| | Cmpd. 5 | Cmpd. 6 | Cmpd. 7 | Cmpd. 8 |
|---|---|---|---|---|
| Rate kg/ha | 0.1 | 0.1 | 0.1 | 0.1 |
| POST-EMERGENCE | | | | |
| Bush bean | 9C | 2H | 2C,9G,6Y | 4C,9G,6Y |
| Cotton | 5C,9G | 0 | 1C | 1C,1H |
| Morningglory | 2C,8G | 0 | 1C,4H | 4H |
| Cocklebur | 8G | 0 | 2G | 3C,9G |
| Cassia | 3C,5G | 0 | 2C | 0 |
| Nutsedge | 9C,9G | 0 | 1C,4G | 0 |
| Crabgrass | 3C,8G | 0 | 1C,3G | 2C |
| Barnyardgrass | 5C,9H | 2H | 5C,9H | 2C,8H |
| Wild Oats | 1C,9G | 0 | 0 | 0 |
| Wheat | 9G | 0 | 0 | 0 |
| Corn | 3U,9H | 4G | 1C,5G | 2C,8H |
| Soybean | 6C,9G | 2H | 2C,9G,5X | 1C,3G |
| Rice | 6C,9G | 5G | 2C,9G | 6G |
| Sorghum | 7U,9G | 5G | 2C,8H | 2C,8H |
| Sugar beet | - | - | - | - |
| PRE-EMERGENCE | | | | |
| Morningglory | 9G | 0 | 2C | 0 |
| Cocklebur | 9H | 0 | 1C | 4G |
| Cassia | 2C,8G | 0 | 2C | 3H |
| Nutsedge | 10E | 0 | 3G | 0 |
| Crabgrass | 2C,4G | 0 | 1C | 0 |
| Barnyardgrass | 3C,9G | 0 | 1C | 0 |
| Wild Oats | 2C,9G | 0 | 0 | 0 |
| Wheat | 2C,9G | 0 | 0 | 0 |
| Corn | 1C,9G | 0 | 2C,6G | 1C,7H |
| Soybean | 3C,8G | 0 | 2C,3H | 2A |
| Rice | 10E | 0 | 6G | 2C,7H |
| Sorghum | 3C,9H | 0 | 2C,6G | 2C,6G |
| Sugar beet | - | - | - | - |

Table A (continued)

| | Cmpd. 9 | Cmpd. 10 | Cmpd. 11 | Cmpd. 12 |
|---|---|---|---|---|
| Rate kg/ha | 0.1 | 0.1 | .05 | .05 |
| POST-EMERGENCE | | | | |
| Bush bean | 9C | 9C | 6C,9G,6Y | 0 |
| Cotton | 5C,8H | 5C,9G | 4C,8G | 0 |
| Morningglory | 5C,9H | 3C,9G | 2C | 0 |
| Cocklebur | 2C,8G | 8H | 1C | 0 |
| Cassia | 5C,8G | 5C,8H | 1C | 0 |
| Nutsedge | 2C,7G | 7C,9G | 3C,6G | 0 |
| Crabgrass | 9C | 9C | 2C | 0 |
| Barnyardgrass | 9C | 10C | 3C,7H | 0 |
| Wild Oats | 1C,9G | 9C | 1C | 0 |
| Wheat | 9C | 9C | 0 | 0 |
| Corn | 9C | 10C | 2C,4H | 0 |
| Soybean | 5C,9G | 6C,9G | 4C,9G | 4H |
| Rice | 5C,9G | 10C | 4C,9G | 0 |
| Sorghum | 5C,9G | 10C | 2C,9G | 0 |
| Sugar beet | - | - | - | - |
| PRE-EMERGENCE | | | | |
| Morningglory | 9G | 8H | 3C | 0 |
| Cocklebur | 9H | 9H | 9H | - |
| Cassia | 8G | 3C,7G | 2C | 2C |
| Nutsedge | 9G | 10E | 1C,4G | 0 |
| Crabgrass | 2C,3G | 3C,5G | 1C,2H | 0 |
| Barnyardgrass | 5C,9H | 3C,9H | 2C | 0 |
| Wild Oats | 2C,8G | 3C,9G | 2C | 0 |
| Wheat | 9H | 2C,9G | 2G | 0 |
| Corn | 2C,9G | 3C,9G | 3C,7H | 0 |
| Soybean | 9H | 3C,7H | 2C,5H | 0 |
| Rice | 10E | 10E | 5C,7G | 5G |
| Sorghum | 2C,9H | 4C,9H | 3C,9H | 0 |
| Sugar beet | - | - | - | - |

Table A (continued)

| | Cmpd. 13 | Cmpd. 14 | Cmpd. 15 | Cmpd. 16 |
|---|---|---|---|---|
| Rate kg/ha | .05 | .05 | .05 | .05 |
| POST-EMERGENCE | | | | |
| Bush bean | 4C,9G,6Y | 3C,8G,6Y | 0 | 4C,8G,6Y |
| Cotton | 2C,6G | 2C | 0 | 4C,7H |
| Morningglory | 2C,5G | 1C | 0 | 2C,6G |
| Cocklebur | 2C,8H | 5G | 5H | 2G |
| Cassia | 1C | 3G | 0 | 1C |
| Nutsedge | 0 | 0 | 0 | 0 |
| Crabgrass | 0 | 0 | 0 | 0 |
| Barnyardgrass | 0 | 0 | 0 | 5H |
| Wild Oats | 0 | 0 | 0 | 0 |
| Wheat | 0 | 0 | 0 | 0 |
| Corn | 0 | 0 | 0 | 2C |
| Soybean | 2C,6H | 2C,6G | 2G | 2C,9G,5X |
| Rice | 6G | 2G | 0 | 4C,8G |
| Sorghum | 2C | 0 | 0 | 3C,8H |
| Sugar beet | - | - | - | - |
| PRE-EMERGENCE | | | | |
| Morningglory | 3C,5G | 1C | 0 | 0 |
| Cocklebur | 7G | - | 0 | - |
| Cassia | 0 | 0 | 0 | 0 |
| Nutsedge | 8G | 0 | 0 | 0 |
| Crabgrass | 0 | 0 | 0 | 0 |
| Barnyardgrass | 1C | 1C | 0 | 3C |
| Wild Oats | 0 | 0 | 0 | 0 |
| Wheat | 0 | 0 | 0 | 0 |
| Corn | 2C,5G | 2G | 0 | 2G |
| Soybean | 1C | 0 | 2H | 1C |
| Rice | 2C | 0 | 2G | 1C |
| Sorghum | 1C | 0 | 0 | 1C |
| Sugar beet | - | - | - | - |

Table A (continued)

| | Cmpd. 17 | Cmpd. 18 | Cmpd. 19 | Cmpd. 20 |
|---|---|---|---|---|
| Rate kg/ha | .05 | 1/20 | 1/20 | 1/20 |
| POST-EMERGENCE | | | | |
| Bush bean | 1C | 9C | 5C,9G,6Y | 4C,9G,6Y |
| Cotton | 1C | 4C,8H | 5C,8G | 4C,5H |
| Morningglory | 1C | 4C,8H | 4C,7G | 5C,8H |
| Cocklebur | 0 | 5C,9G | 9C | 9C |
| Cassia | 1C | 4C,7H | 3C,6H | 3C,3G |
| Nutsedge | 0 | 2C,8G | 3C,9G | 2C,7G |
| Crabgrass | 0 | 2C,5G | 3C,7G | 3C,8G |
| Barnyardgrass | 1C | 9C | 9C | 9C |
| Wild Oats | 0 | 3C,9G | 0 | 1C |
| Wheat | 0 | 3C,9G | 0 | 7G |
| Corn | 1C | 3C,9H | 5C,9G | 2C,9G |
| Soybean | 1C | 5C,9G | 6C,9G | 1C,2H |
| Rice | 0 | 5C,9G | 5C,9G | 5C,9G |
| Sorghum | 2C | 3U,9G | 2C,9G | 4C,9G |
| Sugar beet | - | 9C | 5C,9G | 9C |
| PRE-EMERGENCE | | | | |
| Morningglory | 0 | 2C,8H | 2C,8H | 4C,8H |
| Cocklebur | 0 | 8H | 10E | 9H |
| Cassia | 0 | 3C,6G | 9G | 2C,3G |
| Nutsedge | 0 | 10E | 4C,9G | 2C,9G |
| Crabgrass | 0 | 2C,3G | 2C | 3C,6G |
| Barnyardgrass | 0 | 3C,8H | 3C,8H | 5C,9H |
| Wild Oats | 0 | 2C,6G | 5G | 2C,4G |
| Wheat | 0 | 2C,8H | 6G | 3C,7G |
| Corn | 1C | 5C,9G | 5C,9G | 5C,9H |
| Soybean | 0 | 3C,5H | 3C,7G | 0 |
| Rice | 0 | 10E | 10E | 10E |
| Sorghum | 0 | 5C,8H | 2C,9G | 5C,9H |
| Sugar beet | - | 5C,9G | 9C | 5C,9G |

Table A (continued)

| | Cmpd. 21 | Cmpd. 22 | Cmpd. 23 | Cmpd. 23 |
|---|---|---|---|---|
| Rate kg/ha | 1/20 | 1/20 | 1/20 | 2/5 |
| POST-EMERGENCE | | | | |
| Bush bean | 0 | 3C,8G,6Y | 9C | 9C |
| Cotton | 2C | 3C,4H | 4C,9G | 5C,9G |
| Morningglory | 2C,5G | 3C,8G | 4C,9G | 5C,9G |
| Cocklebur | 1C | 2C,6G | 6G | 5C,9G |
| Cassia | 1C | 3C | 3C,8G | 3C,9G |
| Nutsedge | 0 | 3G | 3C,8G | 9C |
| Crabgrass | 0 | 3G | 5C,9G | 9C |
| Barnyardgrass | 2H | 9C | 9C | 9C |
| Wild Oats | 0 | 3C,9G | 9C | 9C |
| Wheat | 0 | 2C,6G | 6U,9G | 9C |
| Corn | 1C,3G | 2C,5H | 6U,9G | 10C |
| Soybean | 1C | 1C,3H | 4C,9G | 5C,9G |
| Rice | 0 | 5C,9G | 3C,9G | 5C,9G |
| Sorghum | 2C,5G | 4C,9G | 3U,9G | 9C |
| Sugar beet | 2C,5G | 4C,9H | 9C | 9C |
| PRE-EMERGENCE | | | | |
| Morningglory | 3C,3H | 2C,6H | 9C | 9C |
| Cocklebur | - | 1C | - | 9H |
| Cassia | 2C | 0 | 5C,8G | 5C,9G |
| Nutsedge | 0 | 0 | 9G | 10E |
| Crabgrass | 3G | 1C,4G | 5G | 5C,9G |
| Barnyardgrass | 0 | 2C,6H | 2C,4G | 5C,9H |
| Wild Oats | 0 | 4G | 4C,9G | 5C,9H |
| Wheat | 0 | 0 | 4C,9H | 10H |
| Corn | 0 | 3C,8H | 5C,9H | 10H |
| Soybean | 1H | 0 | 8H | 9H |
| Rice | 2C | 4C,9H | 10E | 10E |
| Sorghum | 3C | 4C,9H | 5C,9H | 6C,9H |
| Sugar beet | 9G | 2C,4H | 5C,9G | 10E |

Table A (continued)

| | Cmpd. 24 | Cmpd. 24 | Cmpd. 25 | Cmpd. 25 |
|---|---|---|---|---|
| Rate kg/ha | 1/20 | 2/5 | 2/5 | 1/20 |
| POST-EMERGENCE | | | | |
| Bush bean | 2C,7G,6Y | 5C,9G | 9C | 4C,9G,6Y |
| Cotton | 3C,7G | 5C,8G | 4C,8G | 4C,4H |
| Morningglory | 3C,7G | 4C,9G | 5C,9G | 3C,8G |
| Cocklebur | 8G | 2C,9G | 7G | 0 |
| Cassia | 3C,5H | 3C,5H | 5C,9G | 3C,8G |
| Nutsedge | 4G | 2C,7G | 9C | 3C,8G |
| Crabgrass | 3C,7G | 5C,9G | 9C | 4C,9G |
| Barnyardgrass | 9C | 9C | 9C | 9C |
| Wild Oats | 3C,9G | 9C | 2C,8G | 4G |
| Wheat | 5C,9G | 5U,9G | 5U,9G | 3U,9G |
| Corn | 2C,8H | 3U,9G | 9C | 3U,9G |
| Soybean | 5C,9G | 5C,9G | 5C,9G | 3C,9G |
| Rice | 5C,9G | 5C,9G | 5C,9G | 5C,9G |
| Sorghum | 3C,9H | 5C,9G | 3U,9G | 5C,9G |
| Sugar beet | 5C,8H | 5C,9H | 9C | 9C |
| PRE-EMERGENCE | | | | |
| Morningglory | 2C,3H | 5C,9G | 5C,9H | 4C,9G |
| Cocklebur | 5H | 9H | 9H | 9H |
| Cassia | 2C | 5C,8G | 5C,9G | 4C,9G |
| Nutsedge | 0 | 2C,8G | 10E | 10E |
| Crabgrass | 0 | 3C,5G | 6C,9G | 4G |
| Barnyardgrass | 1C | 3C,8H | 5C,9H | 5C,9H |
| Wild Oats | 0 | 4C,9H | 3C,9G | 4G |
| Wheat | 0 | 4C,9H | 10H | 3C,9H |
| Corn | 3C,8H | 5C,9G | 9H | 4C,9H |
| Soybean | 1C,1H | 3C,5H | 8H | 3C,3H |
| Rice | 2C,8G | 10E | 10E | 10E |
| Sorghum | 3C,8H | 5C,9H | 5C,9H | 5C,9H |
| Sugar beet | 2C,5G | 5C,9H | 10E | 10E |

Table A (continued)

| | Cmpd. 26 | Cmpd. 27 | Cmpd. 27 |
|---|---|---|---|
| Rate kg/ha | 2/5 | 2/5 | 1/20 |
| POST-EMERGENCE | | | |
| Bush bean | 2C | 5C,9G,6Y | 4C,9G,6Y |
| Cotton | 1C | 4C,8G | 2C,3H |
| Morningglory | 2C,5G | 4C,8G | 2C,9G |
| Cocklebur | 1H | 6G | 5H |
| Cassia | 3G | 4C,8G | 3C,6H |
| Nutsedge | 0 | 4C,9G | 6G |
| Crabgrass | 0 | 9C | 2C,8G |
| Barnyardgrass | 0 | 9C | 9C |
| Wild Oats | 0 | 9C | 2C,5G |
| Wheat | 0 | 3U,9G | 2G |
| Corn | 2C,3H | 9C | 4C,9G |
| Soybean | 2C,2H | 5C,9G | 4C,9H |
| Rice | 0 | 4C,9G | 5C,9G |
| Sorghum | 2G | 4U,9G | 2C,9G |
| Sugar beet | 0 | 4C,9H | 4C,8H |
| PRE-EMERGENCE | | | |
| Morningglory | 2C | 5C,9G | 2C,9H |
| Cocklebur | 0 | 9H | 5H |
| Cassia | 0 | 5C,9G | 3H |
| Nutsedge | 0 | 10E | 2C,9G |
| Crabgrass | 0 | 5C,9G | 1C |
| Barnyardgrass | 0 | 2C,8H | 0 |
| Wild Oats | 0 | 4C,9G | 0 |
| Wheat | 0 | 2C,9G | 0 |
| Corn | 0 | 10E | 3C,8G |
| Soybean | 0 | 2C,8H | 1H,1A |
| Rice | 0 | 10E | 10E |
| Sorghum | 0 | 5C,9H | 2C,9G |
| Sugar beet | 0 | 5C,9G | 2C,7G |

Table A (continued)

| | Cmpd. 28 | Cmpd. 28 | Cmpd. 29 | Cmpd. 30 |
|---|---|---|---|---|
| Rate kg/ha | 2/5 | 1/20 | 0.4 | 2 |
| POST-EMERGENCE | | | | |
| Bush bean | 3C,8G,6Y | 4C,8G,6Y | 9C | 2S,9G,6Y |
| Cotton | 4C,7G | 2C,2H | 6C,9G | 3C,8G |
| Morningglory | 4C,8G | 4C,8H | 2C,9G | 3C,8G |
| Cocklebur | 3G | 2G | 9C | 2C |
| Cassia | 2C,4H | 1C,3G | 4C,9G | 2C |
| Nutsedge | 2C,9G | 3G | 9C | 1C,7G |
| Crabgrass | 4C,9G | 2C,7G | 9C | 1C,6G |
| Barnyardgrass | 9C | 3C,8H | 9C | 2C,6H |
| Wild Oats | 1C,2G | 0 | 9C | 2C,5G |
| Wheat | 3U,9G | 3C,9G | 5C,9G | 2C,5G |
| Corn | 5C,9G | 2C,9G | 10C | 3C |
| Soybean | 1C,2H | 1C | 4C,9G | 3C,7G |
| Rice | 5C,9G | 1C,4G | 4C,9G | 2C,9G |
| Sorghum | 5C,9G | 3C,9G | 10C | 2C,6G |
| Sugar beet | 9C | 3C,9H | - | - |
| PRE-EMERGENCE | | | | |
| Morningglory | 9G | 2C,2H | 9H | 8G |
| Cocklebur | 9H | 7H | - | 6G |
| Cassia | 2C,7G | 1C | 2C,9G | 10E |
| Nutsedge | 5G | 0 | 10E | 10E |
| Crabgrass | 3G | 1C | 4C,8G | 5G |
| Barnyardgrass | 2C,6G | 1C | 5C,9H | 2C,8H |
| Wild Oats | 1C | 0 | 5C,9G | 2C |
| Wheat | 5C,9H | 0 | 9H | 5G |
| Corn | 5C,9H | 2C,8G | 10H | 2C,5G |
| Soybean | 0 | 0 | 9H | 1C |
| Rice | 5C,9H | 2C,5G | 10E | 9H |
| Sorghum | 5C,9H | 3C,9H | 10E | 3C,8G |
| Sugar beet | 5C,9G | 2C,7G | - | - |

Table A (continued)

| | Cmpd. 31 | Cmpd. 32 | Cmpd. 33 |
|---|---|---|---|
| Rate kg/ha | 2/5 | 2/5 | 2/5 |
| POST-EMERGENCE | | | |
| Bush bean | 6C,9G,6Y | 9C | 9C |
| Cotton | 5C,9G | 4C,4H,8G | 6C,9G |
| Morningglory | 10C | 4C,9H | 1C,3G |
| Cocklebur | 9C | 5G | 9C |
| Cassia | 4C,9G | 2C,4G | 3C |
| Nutsedge | 3C,9G | 2C,9G | 1C,9G |
| Crabgrass | 2C,5G | 4C,8G | 1H |
| Barnyardgrass | 9C | 4C,7H | 2C,9H |
| Wild Oats | 3C,9G | 3C | 2C |
| Wheat | 1C,9G | 1C | 0 |
| Corn | 5C,9G | 8H | 2C,6H |
| Soybean | 4C,9G | 4C,9G | 5C,9G |
| Rice | 5C,9G | 6C,9G | 6C,9G |
| Sorghum | 4U,9G | 2C,9H | 2C,9G |
| Sugar beet | - | - | - |
| PRE-EMERGENCE | | | |
| Morningglory | 9H | 9G | 8H |
| Cocklebur | 9H | 9H | 9H |
| Cassia | 3C,9G | 2C,8G | 8G |
| Nutsedge | 10E | 10E | 10E |
| Crabgrass | 3G | 2C,6G | 1C |
| Barnyardgrass | 9H | 3C,9H | 2C,9H |
| Wild Oats | 2C,9H | 2C,8G | 1C,9G |
| Wheat | 2C,9H | 0 | 1C,8G |
| Corn | 2C,9G | 9H | 2C,9H |
| Soybean | 9H | 9H | 8H |
| Rice | 10E | 9H | 9H |
| Sorghum | 9H | 1C,5G | 2C,8H |
| Sugar beet | - | - | - |

Test B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederacea*), cassia (*Cassia tora*), teaweed (*Sida spinosa*), velvetleaf (*Abutilon theophrasti*), and jimsonweed (*Datura stramonium*). A 12.5 cm diameter plastic pot was also filled with prepared soil and planted with rice and wheat. Another 12.5 cm pot was planted with sugarbeets. The above four containers were treated pre-emergence with several test compounds within the scope of the invention.

Twenty-eight days after treatment, the plants were evaluated and visually rated for response to the chemical treatments utilizing the rating system described previously for Test A. The data are summarized in Table B.

Table B

PRE-EMERGENCE ON

WOODSTOWN SANDY LOAM

| | Compound 1 | | Compound 2 | |
|---|---|---|---|---|
| Rate kg/ha | 0.03 | 0.12 | 0.03 | 0.12 |
| Crabgrass | 9G,9C | 9G,9C | 7G | 8G |
| Barnyardgrass | 9G,9C | 9G,9C | 6G | 9G |
| Sorghum | 10C | 10C | 9G | 10C |
| Wild Oats | 9G | 9G | 3G | 7G |
| Johnsongrass | 9G | 9G | 7G | 8G |
| Dallisgrass | 9G,9C | 9G,9C | 0 | 7G |
| Giant foxtail | 9G,9C | 9G,9C | 4G | 9G |
| Ky. bluegrass | 9G,9C | 9G,9C | 7G | 8G |
| Cheatgrass | 10C | 10C | 8G | 10C |
| Sugar beets | 10C | 10C | 9G | 10C |
| Corn | 9G | 10C | 3G | 9G |
| Mustard | 10C | 10C | 9G | 10C |
| Cocklebur | 8G | 9G | 0 | 7G |
| Pigweed | - | - | - | - |
| Nutsedge | 10C | 10C | 9G | 10C |
| Cotton | 8G | 9G | 3G | 7G |
| Morningglory | 8G | 9G | 5G | 7G |
| Cassia | 8G | 9G | 4G | 8G |
| Teaweed | 8G | 9G | 3G | 7G |
| Velvetleaf | 9G | 9G | 8G | 8G |
| Jimsonweed | 9G,9C | 9G,9C | 6G | 8G |
| Soybean | 9G | 9G | 5G,3C | 7G,5C |
| Rice | 10C | 10C | 10C | 10C |
| Wheat | 9G,9C | 9G,9C | 2G | 5G |

Table 8 (continued)

PRE-EMERGENCE ON

WOODSTOWN SANDY LOAM

| | Compound 3 | | Compound 4 | |
|---|---|---|---|---|
| Rate kg/ha | 0.03 | 0.12 | 0.03 | 0.12 |
| Crabgrass | 0 | 0 | 6G | 9G |
| Barnyardgrass | 0 | 0 | 6G | 9G |
| Sorghum | 0 | 3G | 10C | 10C |
| Wild Oats | 0 | 2G | 6G | 8G |
| Johnsongrass | 2G | 5G | 7G | 8G |
| Dallisgrass | 0 | 0 | 7G | 8G |
| Giant foxtail | 0 | 0 | 9G | 9G,9C |
| Ky. bluegrass | 0 | 0 | 8G | 9G |
| Cheatgrass | 0 | 5G | 9G | 10C |
| Sugar beets | 5G | 5G | 8G | 9G |
| Corn | 0 | 2G | 2G | 9G,9C |
| Mustard | 8G | 8G | 10C | 10C |
| Cocklebur | 0 | 0 | 8G | 9G |
| Pigweed | - | - | - | - |
| Nutsedge | 0 | 0 | 9G | 9G |
| Cotton | 0 | 0 | 2G | 7G |
| Morningglory | 0 | 2G | 6G | 8G |
| Cassia | 0 | 0 | 0 | 3G |
| Teaweed | 0 | 0 | 0 | 4G |
| Velvetleaf | 0 | 0 | 5G | 8G |
| Jimsonweed | 0 | 0 | 7G | 8G |
| Soybean | 0 | 0 | 2G,2C | 7G,5C |
| Rice | 7G | 9G | 10C | 10C |
| Wheat | 0 | 0 | 8G | 9G |

Table 8 (continued)

PRE-EMERGENCE ON

WOODSTOWN SANDY LOAM

| | Compound 5 | | Compound 9 | |
|---|---|---|---|---|
| Rate kg/ha | 0.060 | 0.250 | 0.030 | 0.120 |
| Crabgrass | 0 | 7G | 4G | 5G |
| Barnyardgrass | 4G | 10C | 3G | 7G,3C |
| Sorghum | 10C | 10E | - | - |
| Wild Oats | 0 | 6G,3H | 3G | 6G |
| Johnsongrass | 8G,5H | 10C | 6G,3H | 7G,3H |
| Dallisgrass | 7G | 9G,9C | 7G | 8G |
| Giant foxtail | 2G | 8G,8C | 0 | 4G |
| Ky. bluegrass | 8G | 10E | 7G,5C | 10C |
| Cheatgrass | 7G | 10C | 7G,3C | 7G,7C |
| Sugar beets | 8G,9C | 10C | 8G,7C | 9G,9C |
| Corn | 4G | 10C | 4G | 8G,5H |
| Mustard | 8G,8C | 10C | 7G | 8G |
| Cocklebur | 3G | 7G | 5G | 6G |
| Pigweed | 9G,9C | 10C | 10C | 10C |
| Nutsedge | 8G | 10E | 6G | 8G |
| Cotton | 3G,2C | 8G,5H | 3G,3H | 9G,5H |
| Morningglory | 6G | 9G,3H | 5G | 9G |
| Cassia | 3G | 7G,3H | 6G | 7G,3C |
| Teaweed | 4G,3C | 8G,5H | 3G | 7G,3H |
| Velvetleaf | 6G,5H | 9G,9C | 4G | 7G,5H |
| Jimsonweed | 6G | 9G,9C | 2G | 5G |
| Soybean | 5G,5H | 9G,9C | 5G,3H | 9G,5H |
| Rice | 10C | 10C | 10E | 10E |
| Wheat | 3G | 6G | 2G | 6G |

Table 8 (continued)

PRE-EMERGENCE ON

WOODSTOWN SANDY LOAM

| | Compound 10 | | Compound 30 |
|---|---|---|---|
| Rate kg/ha | 0.030 | 0.120 | 0.50 |
| Crabgrass | 0 | 5G | 0 |
| Barnyardgrass | 0 | 6G | 0 |
| Sorghum | - | - | 0 |
| Wild Oats | 0 | 6G | 0 |
| Johnsongrass | 5G,3H | 8G,3H | 0 |
| Dallisgrass | 6G,3H | 9G,9C | 0 |
| Giant foxtail | 0 | 0 | 0 |
| Ky. bluegrass | 6G,3H | 7G,5H | 0 |
| Cheatgrass | 7G,5C | 8G,5C | 0 |
| Sugar beets | 3G | 8G,8C | 0 |
| Corn | 2G | 9G,9C | 0 |
| Mustard | 9G,7C | 9G,9C | 6G |
| Cocklebur | 5G | - | 6G |
| Pigweed | - | - | 0 |
| Nutsedge | 8G | 10E | 4G |
| Cotton | 0 | 4G | 0 |
| Morningglory | 5G | 7G,3H | 0 |
| Cassia | 4G | 6G | 0 |
| Teaweed | 0 | 6G,3H | 9G,9C |
| Velvetleaf | 0 | 5G,5H | 0 |
| Jimsonweed | 0 | 6G,3H | 0 |
| Soybean | 0 | 7G,5H | 0 |
| Rice | 8G,5H | 10E | 0 |
| Wheat | 0 | 7G,5H | 0 |

Test C

The test chemicals, dissolved in a non-phytotoxic solvent, were applied in an overall spray to the foliage and surrounding soil of selected plant species. One day after treatment, plants were observed for rapid burn injury. Approximately fourteen days after treatment, all species were visually compared to untreated controls and rated for response to treatment. The rating system was as described previously for Test A. The data are presented in Table C.

All plant species were seeded in Woodstown sandy loam soil and grown in a greenhouse. The following species were grown in soil contained in plastic pots (25 cm diameter by 13 cm deep): soybeans, cotton, alfalfa, corn, rice, wheat, sorghum, velvetleaf (*Abutilon theophrasti*), sesbania (*Sesbania exaltata*), Cassia (*Cassia tora*), morningglory (*Ipomoea hederacea*), jimsonweed (*Datura stramonium*), cocklebur (*Xanthium pensylvanicum*), crabgrass (*Digitaria* sp.), nutsedge (*Cyperus rotundus*), barnyardgrass (*Echinochloa crusgalli*), giant foxtail (*Setaria faberii*) and wild oats (*Avena fatua*). The following species were grown in soil in a paper cup (12 cm diameter by 13 cm deep): sunflower, sugarbeets, and mustard. All plants were sprayed approximately 14 days after planting.

The compounds tested by this procedure are highly active post-emergence herbicides.

Table C

Over-the-Top Soil/Foliage Treatment

Compound 1

| Rate kg/ha | 0.004 | 0.016 | 0.064 | 0.25 |
|---|---|---|---|---|
| Soybeans | 10C | 10C | 10C | 10C |
| Velvetleaf | 8G | 9G,5C | 9G | 10C |
| Sesbania | 6G | 9G | 10G | 10C |
| Cassia | 5G | 9G | 10G | 10C |
| Cotton | 6G | 9G | 9G | 10C |
| Morningglory | 8G | 9G | 10C | 10C |
| Alfalfa | 7G | 8G | 9G | 10C |
| Jimsonweed | 8G | 9G | 9G | 9G |
| Cocklebur | 8G | 8G | 8G | 10P |
| Corn | 9G | 9G | 10C | 10C |
| Crabgrass | 3G | 9G | 9C | 9C |
| Rice | 8G | 9G | 10C | 10C |
| Nutsedge | 9C | 9C | 10C | 10C |
| Barnyardgrass | 9C | 10C | 10C | 10C |
| Wheat | 4G | 8G | 9G | 9G |
| Giant foxtail | 9G | 10C | 10C | 10C |
| Wild Oats | 8G | 9G | 9G | 9G |
| Sorghum | 9U | 9U | 9G | 10C |
| Sunflower | 9G,3H | 10C | 10C | 10C |
| Rape | 8G | 9G | 9G | 9G |
| Johnsongrass | 10U | 10U | 10C | 10C |
| Sugar beets | 8G | 10C | 10C | 10C |
| Bindweed | 8G | 9G | 9G | 9G |

Table C (continued)

Over-the-Top Soil/Foliage Treatment

| | Compound 2 | | |
|---|---|---|---|
| Rate kg/ha | 0.016 | 0.064 | 0.25 |
| Soybeans | 8G | 9G,4C | 10C |
| Velvetleaf | 8G | 9G | 9G |
| Sesbania | 6G | 9G | 10C |
| Cassia | 4G | 7G | 8G |
| Cotton | 6G | 8G | 8G |
| Morningglory | 6G | 7G | 9G |
| Alfalfa | 3G | 6G | 8G |
| Jimsonweed | 3G | 6G | 8G |
| Cocklebur | 4G | 5G | 8G |
| Corn | 9G | 9G | 9G |
| Crabgrass | 0 | 6G | 8G |
| Rice | 9G | 9C | 9C |
| Nutsedge | 4G | 8G | 10C |
| Barnyardgrass | 9G | 10C | 10C |
| Wheat | 0 | 4G | 9G |
| Giant foxtail | 7G | 9G | 9G |
| Wild Oats | 0 | 7G | 9G |
| Sorghum | 7G | 9G | 9U |
| Sunflower | 5G,2H | 10P | 10P |
| Rape | 7G | 8G | 9G |
| Johnsongrass | 8G | 9U | 10U |
| Sugar beets | 9G | 10G | 10C |
| Bindweed | 3G | 7G | 8G |

Table C (continued)

Over-the-Top Soil/Foliage Treatment

| | Compound 3 | | |
|---|---|---|---|
| Rate kg/ha | 0.016 | 0.064 | 0.25 |
| Soybeans | 0 | 1G | 7G |
| Velvetleaf | 0 | 2G | 4G |
| Sesbania | 0 | 0 | 5G |
| Cassia | 0 | 0 | 0 |
| Cotton | 0 | 0 | 2G |
| Morningglory | 0 | 0 | 3C |
| Alfalfa | 0 | 0 | 3G |
| Jimsonweed | 0 | 2G | 7G |
| Cocklebur | 0 | 3G | 4G |
| Corn | 2G | 2G | 9G |
| Crabgrass | 0 | 0 | 4G |
| Rice | 5G | 8G | 9G |
| Nutsedge | 0 | 0 | 2G |
| Barnyardgrass | 2G | 3G | 6G |
| Wheat | 0 | 3G | 6G |
| Giant foxtail | 0 | 5G | - |
| Wild Oats | 2G | 4G | 9G |
| Sorghum | 6G | 9G | 9G |
| Sunflower | 0 | 2G | 4G |
| Rape | 0 | 1G | 5G |
| Johnsongrass | 0 | 6G | 9U |
| Sugar beets | 0 | 2G | 3G |
| Bindweed | 0 | 0 | 2G |

Table C (continued)

Over-the-Top Soil/Foliage Treatment

| | Compound 4 | | | |
|---|---|---|---|---|
| Rate kg/ha | 0.004 | 0.016 | 0.064 | 0.25 |
| Soybeans | 8G | 9G | 9G | 10C |
| Velvetleaf | 4C | 4G | 8G | 9G |
| Sesbania | 3G | 5G | 9G | 9G |
| Cassia | 0 | 0 | 4G | 9G |
| Cotton | 4G | 4G | 6G | 9G |
| Morningglory | 7G | 9G | 9G | 9G |
| Alfalfa | 6G | 6G | 7G | 9C |
| Jimsonweed | 6G | 6G | 9G | 9G |
| Cocklebur | 5G | 5G | 9G | 10P |
| Corn | 0 | 4G,3H | 9G | 9G |
| Crabgrass | 0 | 8G | 9G | 10C |
| Rice | 6G | 8C | 9C | 9C |
| Nutsedge | 3G | 5G | 7G | 10C |
| Barnyardgrass | 8G | 10C | 10C | 10C |
| Wheat | 7G | 8G | 9G | 10C |
| Giant foxtail | 8G | 9G | 9G | 9G |
| Wild Oats | 8G | 9G | 9G | 9G |
| Sorghum | 8G | 8U | 9U | 9U |
| Sunflower | 2G | 4G,3H | 9G | 10C |
| Rape | 6G | 8G | 9G | 9G |
| Johnsongrass | 8G | 9U | 10U | 10U |
| Sugar beets | 6G | 9G | 9G | 9G |
| Bindweed | 4G | 6G | 8G | 8G |

**Claims**

1. A compound of the formula:

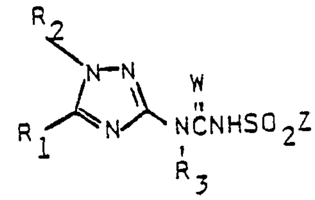

wherein

$R_1$ is H, $C_1$—$C_4$ alkyl, $C_3$—$C_4$ alkenyl, $C_3$—$C_4$ alkynyl, $SR_4$, $OR_5$, $CH_2OR_6$, $CH_2CH_2OR_6$, $N(CH_3)_2$, $CF_3$, $CF_2CF_3$, Cl, $NHCH_3$, $N(CH_3)_2$, $CH(OCH_3)_2$ or

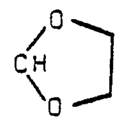 ;

$R_2$ is $C_1$—$C_4$ alkyl, $C_3$—$C_4$ alkenyl, $C_3$—$C_4$ alkynyl, $CH_2OCH_3$, $CH_2CH_2OCH_3$, $CH_2SCH_3$, $CH_2CH_2SCH_3$ or $C_1$—$C_4$ alkyl substituted with 1—3 F atoms;

$R_3$ is H or $CH_3$;

$R_4$ is $C_1$—$C_4$ alkyl, $C_3$—$C_4$ alkenyl, $C_3$—$C_4$ alkynyl, $CH_2CO_2R_6$ or $CH(CH_3)CO_2R_6$;

$R_5$ is $C_1$—$C_4$ alkyl, $C_3$—$C_4$ alkenyl, $C_3$—$C_4$ alkynyl, $CH_2CO_2R_6$, $CH(C_3)CO_2R_6$ or $CH_2CF_3$;

$R_6$ is $C_1$—$C_4$ alkyl;

W is O or S;

Z is

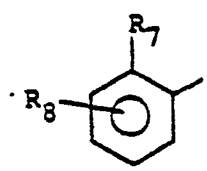 , 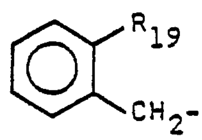 , 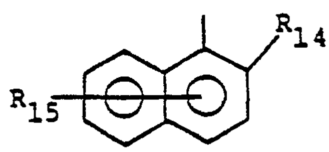

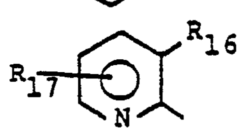 , 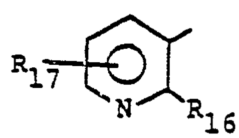 , 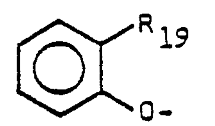 ,

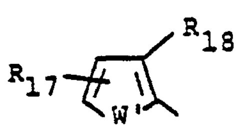 , 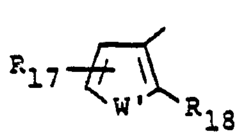 or 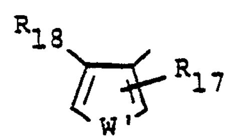

$R_7$ is H, $C_1$—$C_4$ alkyl, $C_1$—$C_4$ alkoxy, F, $C(O)NR_{21}R_{22}$, Cl, Br, $NO_2$, $CF_3$, $CO_2R_9$, $SO_2NR_{10}R_{11}$, $C(O)SR_{10}$, $SO_2N(OCH_3)CH_3$, $QSO_2R_{12}$, $S(O)_nR_{13}$, $CH_2CO_2R_{20}$, $CH(CH_3)CO_2R_{20}$, $CH_2S(O)_nR_{13}$, $CH(CH_3)S(O)_nR_{13}$, $C_3$—$C_4$ alkenyloxy, $C_3$—$C_4$ alkynyloxy, $C_1$—$C_2$ alkyl substituted with either $OCH_3$ or $OC_2H_5$, or $C_1$—$C_3$ alkoxy substituted with either a) 1—5 atoms of Cl, Br or F or b) $OCH_3$ or $OC_2H_5$;

$R_8$ is H, F, Cl, Br, $CF_3$, $NO_2$, $C_1$—$C_3$ alkyl or $C_1$—$C_3$ alkoxy;

$R_9$ is $C_1$—$C_6$ alkyl, $CH_2CH_2OCH_3$, $CH_2CH_2OCH_2CH_3$, $CH_2CH_2CH_2OCH_2CH_3$, $C_3$—$C_6$ alkenyl, $C_3$—$C_6$ alkynyl or $C_1$—$C_3$ alkyl substituted with 1—3 atoms of Cl or F;

$R_{10}$ and $R_{11}$ are independently $C_1$—$C_3$ alkyl;

$R_{12}$ is $C_1$—$C_4$ alkyl, $CH_2CH_2OCH_3$, $CH_2CH_2CH_2OCH_3$ or $C_1$—$C_4$ alkyl substituted with 1—3 atoms of F, Cl or Br;

$R_{13}$ is $C_1$—$C_4$ alkyl, allyl, $C_1$—$C_3$ alkyl substituted with 1—5 atoms of F, Cl or Br;

n is 0, 1 or 2;

Q is O or $NCH_3$;

$R_{14}$ is H, $CH_3$, $OCH_3$, F, Cl, Br, $NO_2$, $SO_2NR_{10}R_{11}$, $SO_2N(OCH_3)CH_3$, $OSO_2R_{12}$ or $S(O)_nR_{13}$;

$R_{15}$ is H, Cl, Br, $CH_3$, $OCH_3$ or $NO_2$;

$R_{16}$ is H, $C_1$—$C_4$ alkyl, $C_1$—$C_4$ alkoxy, F, Cl, Br, $CF_3$, $CO_2R_{20}$, $SO_2NR_{10}R_{11}$, $SO_2N(OCH_3)CH_3$ or $S(O)_nR_{13}$;

$R_{17}$ is H, F, Cl, Br, $CH_3$ or $OCH_3$;

W′ is O or S;

$R_{18}$ is H, $C_1$—$C_4$ alkyl, $C_1$—$C_4$ alkoxy, F, Cl, Br, $NO_2$, $CO_2R_{20}$, $SO_2NR_{10}R_{11}$, $SO_2N(OCH_3)CH_3$ or $S(O)_nR_{13}$;

$R_{19}$ is Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ or $C_1$—$C_3$ alkoxy substituted with 1—5 atoms of Cl or F;

$R_{20}$ is $C_1$—$C_4$ alkyl, $CH_2CH_2OCH_2$, $CH_2CH_2Cl$ or $CH_2CH{=}CH_2$;

$R_{21}$ is $C_1$—$C_3$ alkyl or $C_6H_5$;

$R_{22}$ is $C_1$—$C_3$ alkyl; and

$R_{21}$ and $R_{22}$ may be taken together to be

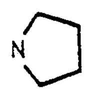 ;

provided that

(1) the total number of carbon atoms of $R_{10}$ and $R_{11}$ is less than or equal to 4;

(2) the total number of carbon atoms of $R_1$ and $R_2$ is less than or equal to 6;

(3) when W′ is O, then $R_{18}$ is H, Cl, Br, $CH_3$ or $CO_2R_{20}$;

(4) when W′ is O and $R_{18}$ is H, Cl, Br or $CH_3$, then Z is

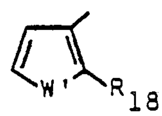 ;

(5) when W is S, then $R_3$ is H;

(6) when $R_7$ is H, then $R_8$ is H; and

(7) $R_{14}$ and $R_{15}$ may not both be $NO_2$.

2. Compounds of Claim 1 wherein W is O and $R_3$ is H.

3. Compounds of Claim 2 wherein Z is

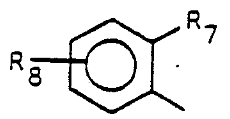 , 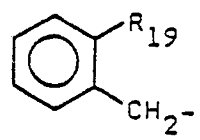 , 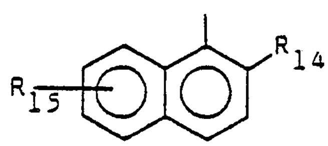 ,

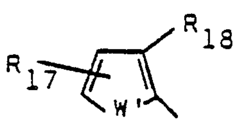 , 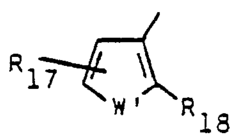 , 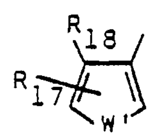 or 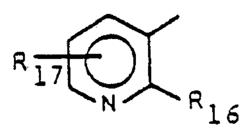 ;

W′ is S;

$R_{19}$ is $CO_2CH_3$, $SO_2N(CH_3)_2$ or $SO_2CH_3$; and

$R_{15}$ and $R_{17}$ are H.

4. Compounds of Claim 3 wherein Z is

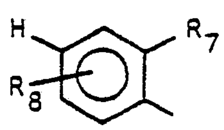 , 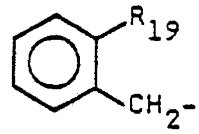 , 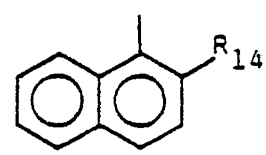 ,

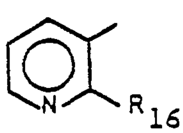 , 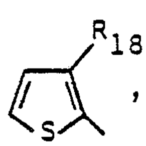 , 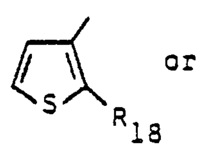 or 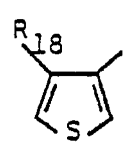 .

5. Compounds of Claim 4, where

$R_1$ is $C_1$—$C_2$ alkyl, $SR_4$, $OR_5$, $CH_2OCH_3$, $N(CH_3)_2$ or Cl;

$R_2$ is $C_1$—$C_2$ alkyl, $CH_2CF_3$, $CH_2CH{=}CH_2$ or $CH_2C{\equiv}CH$; and

$R_4$ and $R_5$ are independently $CH_3$ or $C_2H_5$.

6. Compounds of Claim 5 where Z is

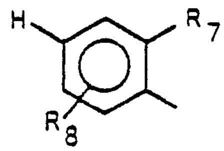

;

R is other than H; and
$R_8$ is H, F, Cl, Br, $CF_3$, $CH_3$ or $OCH_3$.

7. Compounds of Claim 6 where
$R_7$ is Cl, $NO_2$ $CO_2R_9$, $SO_2NR_{10}R_{11}$ or $OSO_2R_{12}$;
$R_8$ is H;
$R_9$ is $C_1$—$C_3$ alkyl, $CH_2CH{=}CH_2$, $CH_2CH_2OCH_3$ or $CH_2CH_2Cl$;
$R_{10}$ and $R_{11}$ are independently $C_1$—$C_2$ alkyl; and
$R_{12}$ and $R_{13}$ are independently $C_1$—$C_3$ alkyl.

8. Compounds of Claim 7 wherein $R_9$, $R_{10}$, $R_{11}$, $R_{12}$ and $R_{13}$ are $CH_3$.

9. The compound of Claim 1 which is 2-[[(5-methylthio-1-methyl-1H-1,2,4-triazol-3-yl)aminocarbonyl]aminosulfonyl]benzoic acid, methyl ester.

10. The compound of Claim 1 which is 2-[[(5-ethylthio-1-methyl-1H-1,2,4-triazol-3-yl)aminocarbonyl]aminosulfonyl]benzoic acid, methyl ester.

11. The compound of Claim 1 which is 2-[[(5-ethyl-1-methyl-1H-1,2,4-triazol-3-yl)aminocarbonyl]aminosulfonyl]benzoic acid, methyl ester.

12. The compound of Claim 1 which is 2-[[(5-methoxy-1-methyl-1H-1,2,4-triazol-3-yl)aminocarbonyl]aminosulfonyl]benzoic acid, methyl ester.

13. The compound of Claim 1 which is N-[(5-methoxy-1-methyl-1H-1,2,4-triazol-3-yl)aminocarbonyl]-2-chlorobenzenesulfonamide.

14. The compound of Claim 1 which is N-[(5-methyl-1H-1,2,4-triazol-3-yl)aminocarbonyl]-N',N'-dimethyl-1,2-benzenedisulfonamide.

15. A compound of Claim 1 wherein
$R_1$ is H, $C_1$—$C_4$ alkyl, $C_3$—$C_4$ alkenyl, $C_3$—$C_4$ alkynyl, $SR_4$, $OR_5$, $CH_2OR_6$, $CH_2CH_2OR_6$, $N(CH_3)_2$, $CF_3$ or $CF_2CF_3$;
$R_2$ is $C_1$—$C_4$ alkyl, $C_3$—$C_4$ alkenyl, $C_3$—$C_4$ alkynyl, $CH_2OCH_3$, $CH_2CH_2OCH_3$, $CH_2SCH_3$ or $CH_2CH_2SCH_3$;
$R_5$ is $C_1$—$C_4$ alkyl, $C_3$—$C_4$ alkenyl or $C_3$—$C_4$ alkynyl;
W is O or S;
Z is

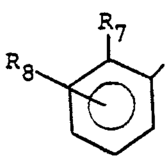 , 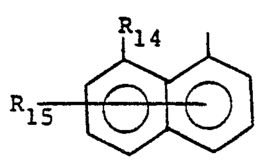 , 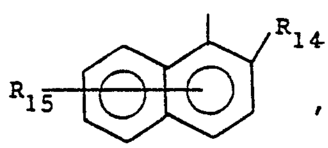 ,

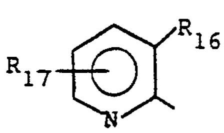 , 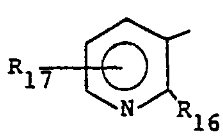 , 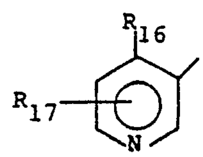 ,

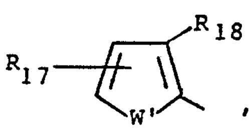 , 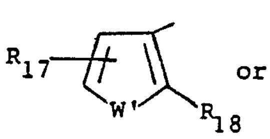 or 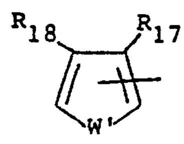

$R_7$ is H, $C_1$—$C_4$ alkyl, $C_1$—$C_4$ alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $OCF_3$, $OCHF_2$ $OCH_2CF_2H$ or $CH_2OR_{13}$;
$R_9$ is $C_1$—$C_4$ alkyl, $CH_2CH_2OCH_3$, $CH_2CH_2Cl$ or $CH_2CH{=}CH_2$;
$R_{13}$ is $C_1$—$C_4$ alkyl;
n is 0 or 2; and
$R_3$, $R_4$, $R_6$, $R_8$, $R_{10}$, $R_{11}$, $R_{12}$, $R_{14}$, $R_{15}$, $R_{16}$, $R_{17}$, $R_{18}$, W and W' are as defined in claim 1.

16. A composition suitable for controlling the growth of undesired vegetation comprising an effective amount of a herbicidal compound and at least one of the following: surfactant, solid or liquid diluent,

characterised in that said herbicidal compound comprises a compound of any of claims 1 to 15.

17. A method for controlling the growth of undesired vegetation by applying to the locus to be protected an effective amount of a herbicidal compound, characterised in that said herbicidal compound comprises a compound of any of claims 1 to 15.

18. A process for making a compound of claim 1 which comprises

(a) reacting together compounds of formulae

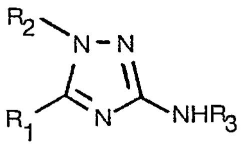

and $ZSO_2NCW$

wherein

$R_1$, $R_2$ $R_3$, W and Z are as defined in claim 1; or

(b) alkylating a compound of formula

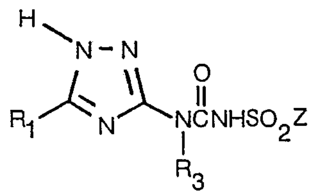

wherein

$R_1$, $R_3$ and Z are as defined in claim 1, with a reagent of formula

$$R_2X$$

wherein

$R_2$ is as defined in claim 1 and X is a leaving group.

**Patentansprüche**

1. Verbindung der Formel

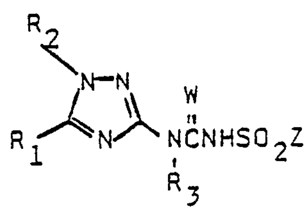

worin

$R_1$ H, $C_1$—$C_4$-Alkyl, $C_3$—$C_4$-Alkenyl, $C_3$—$C_4$-Alkinyl, $SR_4$, $OR_5$, $CH_2OR_6$, $CH_2CH_2OR_6$, $N(CH_3)_2$, $CF_3$, $CF_2CF_3$, Cl, $NHCH_3$, $N(CH_3)_2$, $CH(OCH_3)_2$ oder

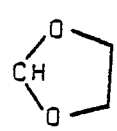

ist;

$R_2$ $C_1$—$C_4$-Alkyl, $C_3$—$C_4$-Alkenyl, $C_3$—$C_4$-Alkinyl, $CH_2OCH_3$, $CH_2CH_2OCH_3$, $CH_2SCH_3$, $CH_2CH_2SCH_3$ oder mit 1-2-F-Atopen substituiertes $C_1$—$C_4$-Alkyl ist;

$R_3$ H oder $CH_3$ ist;

$R_4$ $C_1$—$C_4$-Alkyl, $C_3$—$C_4$-Alkenyl, $C_3$—$C_4$-Alkinyl, $CH_2CO_2R_6$ oder $CH(CH_3)CO_2R_6$ ist;

$R_5$ is $C_1$—$C_4$-Alkyl, $C_3$—$C_4$-Alkenyl, $C_3$—$C_4$-Alkinyl, $CH_2CO_2R_6$, $CH(CH_3)CO_2R_6$ oder $CH_2CF_3$ ist;

$R_6$ $C_1$—$C_4$-Alkyl ist;

W O oder S ist;

Z

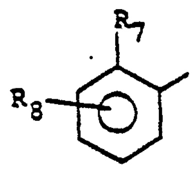 , 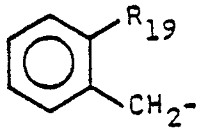 , 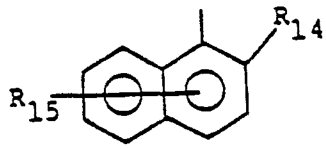

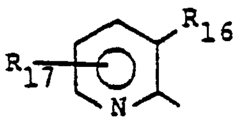 , 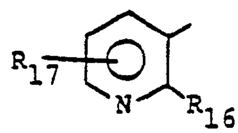 , 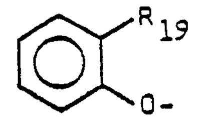 ,

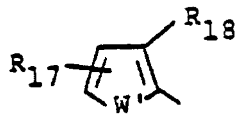 , 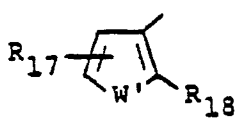 oder 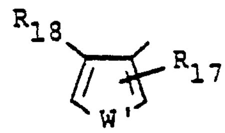

ist;

$R_7$ H, $C_1$—$C_4$-Alkyl, $C_1$—$C_4$-Alkoxy, F, $C(O)NR_{21}R_{22}$, Cl, Br, $NO_2$, $CF_3$, $CO_2R_9$, $SO_2NR_{10}R_{11}$, $C(O)SR_{10}$, $SO_2N(OCH_3)CH_3$, $QSO_2R_{12}$, $S(O)_nR_{13}$, $CH_2CO_2R_{20}$, $CH(CH_3)CO_2R_{20}$, $CH_2S(O)_nR_{13}$, $CH(CH_3)S(O)_nR_{13}$, $C_3$—$C_4$-Alkenyloxy, $C_3$—$C_4$-Akinyloxy, mit entweder $OCH_3$ oder $OC_2H_5$, substituiertes $C_1$—$C_2$-Alkyl, mit entweder a) 1 bis 5 Cl- Br- oder F-Atomen oder b) $OCH_3$ oder $OC_2H_5$ substituiertes $C_1$—$C_3$-Alkoxy ist;

$R_8$ H, F, Cl, Br, $CF_3$, $NO_2$, $C_1$—$C_3$-Alkyl oder $C_1$—$C_3$-Alkoxy ist;

$R_9$ $C_1$—$C_6$-Alkyl, $CH_2CH_2OCH_3$, $CH_2CH_2OCH_2CH_3$, $CH_2CH_2CH_2OCH_2CH_3$, $C_3$—$C_6$-Alkenyl, $C_3$—$C_6$-Alkinyl oder mit 1 bis 3 Cl- oder F-Atomen substituiertes $C_1$—$C_3$-Alkyl ist;

$R_{10}$ und $R_{11}$ unabhängig $C_1$—$C_3$-Alkyl sind;

$R_{12}$ $C_1$—$C_4$-Alkyl, $CH_2CH_2OCH_3$, $CH_2CH_2CH_2OCH_3$ oder mit 1 bis 3 F-, Cl- oder Br-Atomen substituiertes $C_1$—$C_4$-Alkyl ist;

$R_{13}$ $C_1$—$C_4$-Alkyl, Allyl, mit 1 bis 5 F-, Cl- oder Br-Atomen substituiertes $C_1$—$C_3$-Alkyl ist;

n 0, 1 oder 2 ist;

Q O oder $NCH_3$ ist;

$R_{14}$ H, $CH_3$, $OCH_3$, F, Cl, Br, $NO_2$, $SO_2NR_{10}R_{11}$, $SO_2N(OCH_3)CH_3$, $OSO_2R_{12}$ oder $S(O)_nR_{13}$ ist;

$R_{15}$ H, Cl, Br, $CH_3$, $OCH_3$ oder $NO_2$ ist;

$R_{16}$ H, $C_1$—$C_4$-Alkyl, $C_1$—$C_4$-Alkoxy, F, Cl, Br, $CF_3$, $CO_2R_{20}$, $SO_2NR_{10}R_{11}$, $SO_2N(OCH_3)CH_3$ oder $S(O)_nR_{13}$ ist;

$R_{17}$ H, F, Cl, Br, $CH_3$ oder $OCH_3$ ist;

W' O oder S ist;

$R_{18}$ H, $C_1$—$C_4$-Alkyl, $C_1$—$C_4$-Alkoxy, F, Cl, Br, $NO_2$, $CO_2R_{20}$, $SO_2NR_{10}R_{11}$, $SO_2N(OCH_3)CH_3$ oder $S(O)_nR_{13}$ ist;

$R_{19}$ Cl, $NO_2$, $CF_3$, $CO_2R_9$, $SO_2N(OCH_3)CH_3$, $SO_2NR_{10}R_{11}$, $QSO_2R_{12}$, $S(O)_nR_{13}$ oder mit 1 bis 5 Cl- oder F-Atomen substituiertes $C_1$—$C_3$-Alkoxy ist;

$R_{20}$ $C_1$—$C_4$-Alkyl, $CH_2CH_2OCH_2$, $CH_2CH_2Cl$ oder $CH_2CH=CH_2$ ist;

$R_{21}$ $C_1$—$C_3$-Alkyl oder $C_6H_5$ ist;

$R_{22}$ $C_1$—$C_3$-Alkyl ist; und

$R_{21}$ und $R_{22}$ zusammengenommen werden können zu

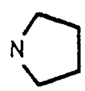 ;

mit der Massgabe, dass

(1) die Gesamtzahl der Kohlenstoffatome von $R_{10}$ und $R_{11}$ weniger als oder gleich 4 ist;

(2) die Gesamtzahl oder Kohlenstoffatome von $R_1$ und $R_2$ weniger als oder gleich 6 ist;

(3) wenn W' O, ist dann $R_{18}$ H, Cl, Br, $CH_3$ oder $CO_2R_{20}$ ist;

(4) wenn W' O ist und $R_{18}$ H, Cl, Br oder $CH_3$ ist, dann Z

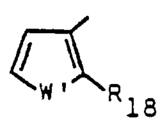

ist;

(5) wenn W S ist, dann $R_3$ H ist;

(6) wenn $R_7$ H ist, dann $R_8$ H ist und
(7) $R_{14}$ und $R_{15}$ nicht beide $NO_2$ sein können.
2. Verbindung nach Anspuch 1, worin W O ist und und $R_3$ H ist.
3. Verbindungen nach Anspruch 2, worin Z

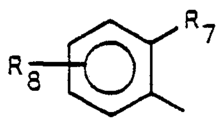 , 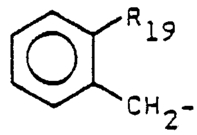 , 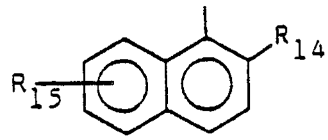 ,

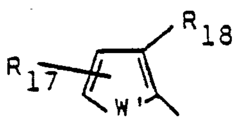 , 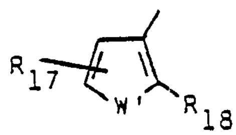 , 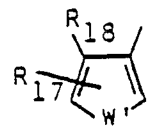 oder 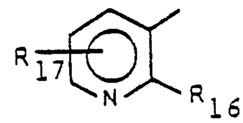 ;

ist;
W' S ist;
$R_{19}$ $CO_2CH_3$, $SO_2N(CH_3)_2$ oder $SO_2CH_3$ ist; und
$R_{15}$ und $R_{17}$ H sind.
4. Verbindungen nach Anspruch 3, worin Z

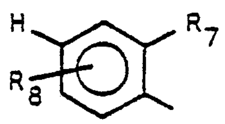 , 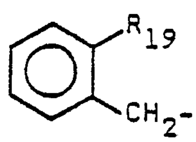 , 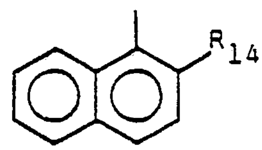 ,

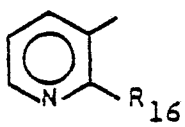 , 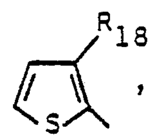 , 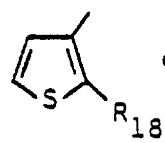 oder 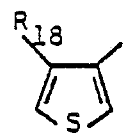 .

ist;
5. Verbindungen nach Anspruch 4 worin
$R_1$ is $C_1$—$C_2$-Alkyl, $SR_4$, $OR_5$, $CH_2OCH_3$, $N(CH_3)_2$ oder Cl ist;
$R_2$ $C_1$—$C_2$-Alkyl, $CH_2CF_3$, $CH_2CH{=}CH_2$ oder $CH_2C{\equiv}CH$ ist; und
$R_4$ und $R_5$ unabhängig $CH_3$ oder $C_2H_5$ sind.
6. Verbindung nach Anspruch 5,
worin Z

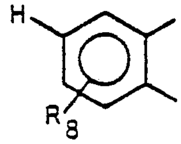

ist;
$R_7$ von H verschieden ist; und
$R_8$ H, F, Cl, Br, $CF_3$, $CH_3$ oder $OCH_3$ ist.
7. Verbindungen nach Anspruch 6, worin
$R_7$ Cl, $NO_2$ $CO_2R_9$, $SO_2NR_{10}R_{11}$ oder $OSO_2R_{12}$ ist;
$R_8$ H ist;
$R_9$ $C_1$—$C_3$ alkyl, $CH_2CH{=}CH_2$, $CH_2CH_2OCH_3$ oder $CH_2CH_2Cl$ ist;
$R_{10}$ und $R_{11}$ unabhängig $C_1$—$C_2$-Alkyl sind; und
$R_{12}$ und $R_{13}$ unabhängig $C_1$—$C_3$-Alkyl sind.
8. Verbindungen nach Anspruch 7, worin $R_9$, $R_{10}$, $R_{11}$, $R_{12}$ und $R_{13}$ $CH_3$ sind.
9. Verbindung nach Anspruch 1, welche 2-[[(5-Methylthio-1-methyl-1H-1,2,4-triazol-3-yl)aminocarbonyl]aminosulfonyl]benzosäure-methylester ist.
10. Verbindung nach Anspruch 1, welche 2-[[(5-Ethylthio-1-methyl-1H-1,2,4-triazol-3-yl)aminocarbonyl]aminosulfonyl]benzoesäure-methylester ist.
11. Verbindung nach Anspruch 1, welche 2-[[(5-Ethyl-1-methyl-1H-1,2,4-triazol-3-yl)aminocarbonyl]aminosulfonyl]benzosäure-methylester ist.

12. Verbindung nach Anspruch 1, welche 2-[[(5-Methoxy-1-methyl-1H-1,2,4-triazol-3-yl)aminocarbonyl]aminosulfonyl]benzoesäure-methylester ist.

13. Verbindung nach Anspruch 1, welche N-[(5-methoxy-1-methyl-1H-1,2,4-triazol-3-yl)aminocarbonyl]-2-chlorobenzolsulfonamid ist.

14. Verbindung nach Anspruch 1, welche N-[(5-Methoxy-1-methyl-1H-1,2,,4-triazol-3-yl)aminocarbonyl]-N',N'-dimethyl-1,2-benzodisulfonamid ist.

15. Verbindung nach Anspruch 1, worin

$R_1$ H, $C_1$—$C_4$-Alkyl, $C_3$—$C_4$-Alkenyl, $C_3$—$C_4$-Alkinyl, $SR_4$, $OR_5$, $CH_2OR_6$, $CH_2CH_2OR_6$, $N(CH_3)_2$, $CF_3$ oder $CF_2CF_3$ ist;

$R_2$ $C_1$—$C_4$-Alkyl, $C_3$—$C_4$-Alkenyl, $C_3$—$C_4$-Alkinyl, $CH_2OCH_3$, $CH_2CH_2OCH_3$, $CH_2SCH_3$ oder $CH_2CH_2SCH_3$ ist;

$R_5$ $C_1$—$C_4$-Alkyl, $C_3$—$C_4$-Alkenyl oder $C_3$—$C_4$-Alkinyl ist;

W is O ist S ist;

Z

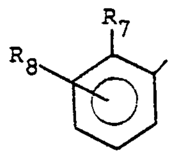,
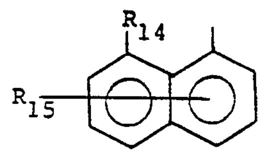,
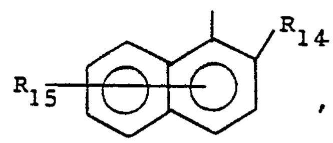,

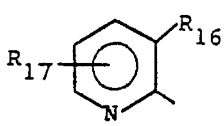,
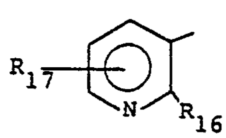,
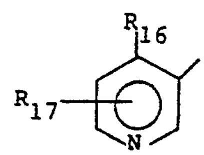,

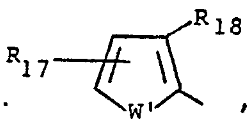,
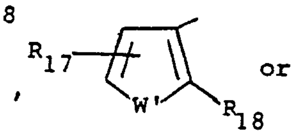
or
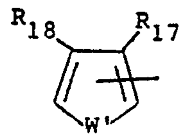

ist;

$R_7$ H, $C_1$—$C_4$-Alkyl, $C_1$—$C_4$-Alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $OCF_3$, $OCHF_2$ $OCH_2CF_2H$ oder $CH_2OR_{13}$ ist;

$R_9$ $C_1$—$C_4$-Alkyl, $CH_2CH_2OCH_3$, $CH_2CH_2Cl$ oder $CH_2CH{=}CH_2$ ist;

$R_{12}$ $C_1$—$C_4$-Alkyl ist;

n 0 oder 2 ist; und

$R_3$, $R_4$, $R_6$, $R_8$, $R_{10}$, $R_{11}$, $R_{12}$, $R_{14}$, $R_{15}$, $R_{16}$, $R_{17}$, $R_{18}$, W und W' wie in Anspruch 1 definiert sind.

16. Zusammensetzung, geeignet zur Bekämpfung des Wachstums unerwünschter Vegetation, welche eine wirksame Menge einer herbiziden Verbindung und wenigstens eines der folgenden umfaßt: oberflächenaktives Mittel, festes oder flüssigres Verdünnungsmittel, dadurch gekennzeichnet, daß die herbizide Verbindung eine Verbindung nach einem der Ansprüche 1 bis 15 umfaßt.

17. Verfahren zur Bekämpfung des Wachstums unerwünschter Vegetation durch Anwendung einer wirksamen Menge einer herbiziden Verbindung auf den zu schützenden Ort, dadurch gekennzeichnet, daß die herbizide Verbindung eine Verbindung nach einem der Ansprüche 1 bis 15 umfaßt.

18. Verfahren zur Herstellung einer Verbindung nach Anspruch 1 durch

(a) miteinander Reagieren lassen der Verbindungen der Formeln

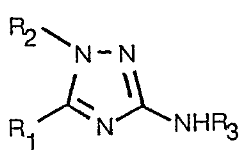

und $ZSO_2NCW$

worin

$R_1$, $R_2$ $R_3$, W und Z wie in Anspruch 1 definiert sind; oder

(b) Alkylieren einer Verbindung der Formel

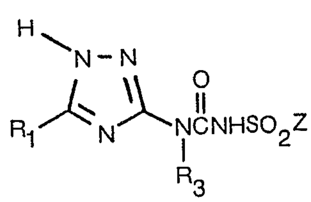

worin

$R_1$, $R_3$ und Z wie in Anspruch 1 definiert sind, mit einem Reagens der Formel

$$R_2X$$

worin

$R_2$ wie in Anspruch 1 definiert ist und X eine austretende Gruppe ist.

**Revendications**

1. Un composé de la formule:

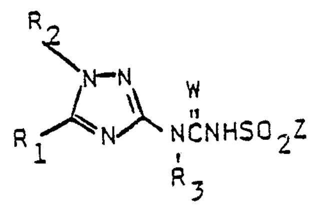

dans laquelle

$R_1$ est H, un groupe alkyle en $C_1$—$C_4$, alcényle en $C_3$—$C_4$ ou alcynyle en $C_3$—$C_4$, $SR_4$, $OR_5$, $CH_2OR_6$, $CH_2CH_2OR_6$, $N(CH_3)_2$, $CF_3$ $CF_2CF_3$, Cl, $NHCH_3$, $N(CH_3)_2$, $CH(OCH_3)_2$ ou

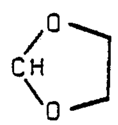 ;

$R_2$ est un groupe alkyle en $C_1$—$C_4$, alcényle en $C_3$—$C_4$, alcynyle en $C_3$—$C_4$, $CH_2OCH_3$, $CH_2CH_2OCH_3$, $CH_2SCH_3$, $CH_2CH_2SCH_3$, ou un groupe alkyle en $C_1$—$C_4$ substitué par 1—3 atomes de F;

$R_3$ est H ou $CH_3$;

$R_4$ est un group alkyle en $C_1$—$C_4$, alcényle en $C_3$—$C_4$, alcynyle en $C_3$—$C_4$, $CH_2CO_2R_6$ ou $CH(CH_3)CO_2R_6$;

$R_5$ est un group alkyle en $C_1$—$C_4$, alcényle en $C_3$—$C_4$, alcynyle en $C_3$—$C_4$, $CH_2CO_2R_6$, $CH(CH_3)CO_2R_6$ ou $CH_2CF_3$;

$R_6$ est un groupe alkyle en $C_1$—$C_4$;

W est O ou S;

Z est

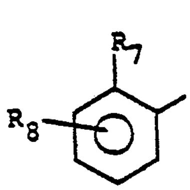 , 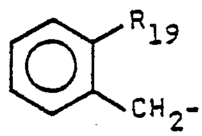 , 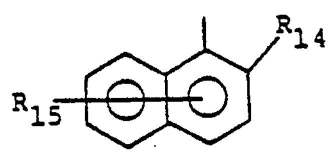

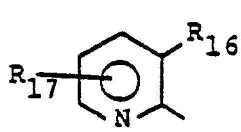 , 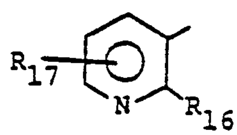 , 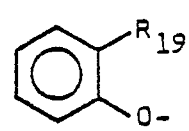 ,

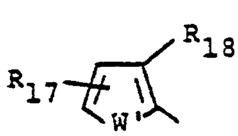 , 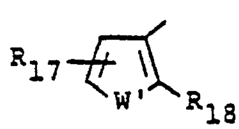 ou 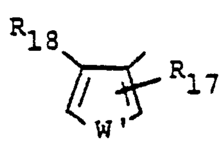

$R_7$ est H, un groupe alkyle en $C_1$—$C_4$, alcoxy en $C_1$—$C_4$, F, $C(O)NR_{21}R_{22}$, Cl, Br, $NO_2$, $CF_3$, $CO_2R_9$, $SO_2NR_{10}R_{11}$, $C(O)SR_{10}$, $SO_2N(OCH_3)CH_3$, $OSO_2R_{12}$, $S(O)_nR_{13}$, $CH_2CO_2R_{20}$, $CH(CH_3)CO_2R_{20}$,$CH_2S(O)_nR_{13}$, $CH(CH_3)S(O)_nR_{13}$, un groupe alcényloxy en $C_3$—$C_4$, alcynyloxy en $C_3$—$C_4$, alkyle en $C_1$—$C_2$ substitué par $OCH_3$ ou par $OC_2H_5$, ou alcoxy en $C_1$—$C_3$ substitué par a) 1—5 atomes de Cl, Br ou F, ou par b) $OCH_3$ ou $OC_2H_5$;

$R_8$ est H, F, Cl, Br, $CF_3$, $NO_2$, un groupe alkyle en $C_1$—$C_3$ ou alcoxy en $C_1$—$C_3$;

$R_9$ est un groupe alkyle en $C_1$—$C_6$, $CH_2CH_2OCH_3$, $CH_2CH_2OCH_2CH_3$, $CH_2CH_2CH_2OCH_2CH_3$, un groupe alcényle en $C_3$—$C_6$, alcynyle en $C_3$—$C_6$ ou alkyle en $C_1$—$C_3$ substitué par 1—3 atomes de Cl ou F;

$R_{10}$ et $R_{11}$ sont chacun indépendament un groupe alkyle en $C_1$—$C_3$;

$R_{12}$ est un groupe alkyle en $C_1$—$C_4$, $CH_2CH_2OCH_3$, $CH_2CH_2CH_2OCH_3$, ou un groupe alkyle en $C_1$—$C_4$ substitué par 1—3 atomes de F, Cl ou Br;

$R_{13}$ est un groupe alkyle en $C_1$—$C_4$, allyle, alkyle en $C_1$—$C_3$ substitué par 1—5 atomes de F, Cl ou Br;

n a la valeur 0, 1 ou 2;

Q est O ou $NCH_3$;

$R_{14}$ est H, $CH_3$, $OCH_3$, F, Cl, Br, $NO_2$, $SO_2NR_{10}R_{11}$, $SO_2N(OCH_3)CH_3$, $OSO_2R_{12}$ ou $S(O)_nR_{13}$;

$R_{15}$ est H, Cl, Br, $CH_3$, $OCH_3$ ou $NO_2$;

$R_{16}$ est H, un groupe alkyle en $C_1$—$C_4$, alcoxy en $C_1$—$C_4$, F, Cl, Br, $CF_3$, $CO_2R_{20}$, $SO_2NR_{10}R_{11}$, $SO_2N(OCH_3)CH_3$ ou $S(O)_nR_{13}$;

$R_{17}$ est H, F, Cl, Br, $CH_3$ ou $OCH_3$;

W' est O ou S;

$R_{18}$ est H, un groupe alkyle en $C_1$—$C_4$, alcoxy en $C_1$—$C_4$, F, Cl, Br, $NO_2$, $CO_2R_{20}$, $SO_2NR_{10}R_{11}$, $SO_2N(OCH_3)CH_3$ ou $S(O)_nR_{13}$;

$R_{19}$ est Cl, $NO_2$, $CF_3$, $CO_2R_9$, $SO_2N(OCH_3)CH_3$, $SO_2NR_{10}R_{11}$, $QSO_2R_{12}$, $S(O)_nR_{13}$ ou un groupe alcoxy en $C_1$—$C_3$ substitué par 1—5 atomes de Cl ou F;

$R_{20}$ est un groupe alkyle en $C_1$—$C_4$, $CH_2CH_2OCH_3$, $CH_2CH_2Cl$ ou $CH_2CH{=}CH_2$;

$R_{21}$ est un groupe alkyle en $C_1$—$C_3$ ou $C_6H_5$;

$R_{22}$ est un groupe alkyle en $C_1$—$C_3$; et

$R_{21}$ et $R_{22}$ peuvent être pris ensemble pour représenter

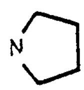 ;

avec la condition que

(1) le nombre total d'atomes de carbone de $R_{10}$ et $R_{11}$ est inférieur ou égal à 4;

(2) le nombre total d'atomes de carbone de $R_1$ et $R_2$ est inférieur ou égal à 6;

(3) si W' est O, $R_{18}$ est alors H, Cl, Br, $CH_3$ ou $CO_2R_{20}$;

(4) si W' est O et $R_{18}$ est H, Cl, Br ou $CH_3$, Z est alors

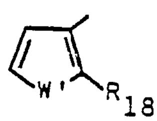 ;

(5) si W est S, $R_3$ est alors H;

(6) si $R_7$ est H, $R_8$ est alors H; et

(7) $R_{14}$ et $R_{15}$ ne peuvent pas tous les deux être $NO_2$.

2. Composés de la revendication 1, dans lesquels W est O et $R_3$ est H.

3. Composés de la revendication 2, dans lesquels Z est

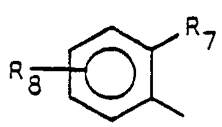 , 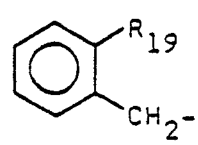 , 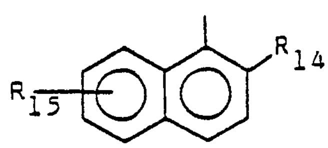 ,

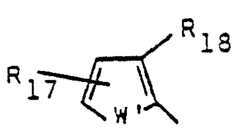 , 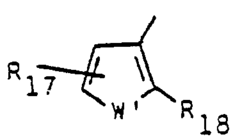 , 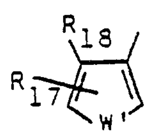 ou 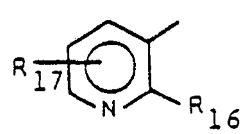 ;

W' est S;

$R_{19}$ est $CO_2CH_3$, $SO_2N(CH_3)_2$ ou $SO_2CH_3$; et

$R_{15}$ et $R_{17}$ sont H.

4. Composés de la revendication 3, dans lesquels Z est

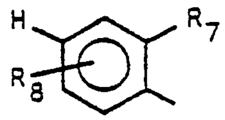 , 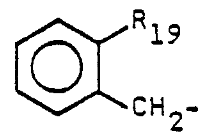 , 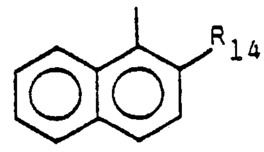 ,

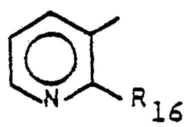 , 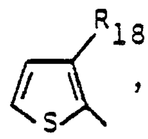 , 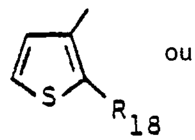 ou 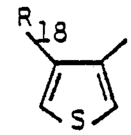 .

5. Composés de la revendication 4, dans lesquels
$R_1$ est un groupe alkyle en $C_1$—$C_2$, $SR_4$, $OR_5$, $CH_2OCH_3$, $N(CH_3)_3$ ou Cl;
$R_2$ est un groupe alkyle en $C_1$—$C_2$, $CH_2CF_3$, $CH_2CH{=}CH_2$ ou $CH_2C{\equiv}CH$; et
$R_4$ et $R_5$ sont indépendamment $CH_3$ ou $C_2H_5$.

6. Composés de la revendication 5, dans lesquels Z est

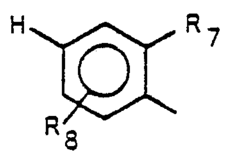 ;

$R_7$ est autre chose que H; et
$R_8$ est H, F, Cl, Br, $CF_3$, $CH_3$ ou $OCH_3$.

7. Composés de la revendication 6, dans lesquels
$R_7$ est Cl, $NO_2$, $CO_2R_9$, $SO_2NR_{10}R_{11}$ ou $OSO_2R_{12}$;
$R_8$ est H;
$R_9$ est un groupe alkyle en $C_1$—$C_3$, $CH_2CH{=}CH_2$, $CH_2CH_2OCH_3$ ou $CH_2CH_2Cl$;
$R_{10}$ et $R_{11}$ sont indépendamment un groupe alkyle en $C_1$—$C_2$; et
$R_{12}$ et $R_{13}$ sont indépendamment un groupe alkyle en $C_1$—$C_3$.

8. Composés de la revendication 7, dans lesquels $R_9$, $R_{10}$, $R_{11}$, $R_{12}$ et $R_{13}$ sont $CH_3$.

9. Le composé de la revendication 1 qui est l'ester méthylique de l'acide 2-[[(5-méthylthio-1-méthyl-1H-1,2,4-triazol-3-yl)aminocarbonyl]aminosulfonyl]benzoïque.

10. Le composé de la revendication 1 qui est l'ester méthylique de l'acide 2-[[(5-éthylthio-1-méthyl-1H-1,2,4-triazol-3-yl)aminocarbonyl]aminosulfonyl]benzoïque.

11. Le composé de la revendication 1 qui est l'ester méthylique de l'acide 2-[[(5-éthyl-1-méthyl-1H-1,2,4-triazol-3-yl)aminocarbonyl]aminosulfonyl]benzoïque.

12. Le composé de la revendication 1 qui est l'ester méthylique de l'acide 2-[[(5-méthoxy-1-méthyl-1H-1,2,4-triazol-3-yl)aminocarbonyl]aminosulfonyl]benzoïque.

13. Le composé de la revendication 1 qui est le N-[(5-méthoxy-1-méthyl-1H-1,2,4-triazol-3-yl)aminocarbonyl]-2-chlorobenzènesulfonamide.

14. Le composé de la revendication 1 qui est le N-[(5-éthylique de l'acide méthoxy-1-méthyl-1H-1,2,4-triazol-3-yl)aminocarbonyl]-N',N'-diméthyl-1,2-benzènedisulfonamide.

15. Un composé de la revendiction 1 dans lequel
$R_1$ est H, un group alkyle en $C_1$—$C_4$, alcényle en $C_3$—$C_4$, alcynyle en $C_3$—$C_4$, $SR_4$, $OR_5$, $CH_2OR_6$, $CH_2CH_2OR_6$, $N(CH_3)_2$, $CF_3$ ou $CF_2CF_2$;
$R_2$ est un groupe alkyle en $C_1$—$C_4$, alcényle en $C_3$—$C_4$, alcynyle en $C_3$—$C_4$, $CH_2OCH_3$, $CH_2CH_2OCH_3$, $CH_2SCH_3$ ou $CH_2CH_2SCH_3$;
$R_5$ est un groupe alkyle en $C_1$—$C_4$, alcényle en $C_3$—$C_4$ ou alcynyle en $C_3$—$C_4$;
W est O ou S;
Z est

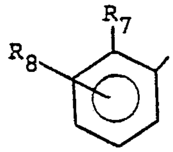 , 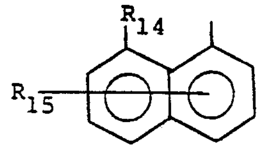 , 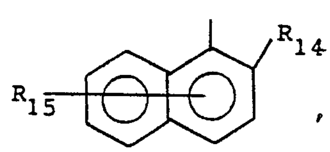 ,

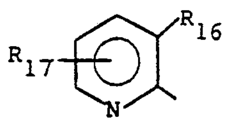, 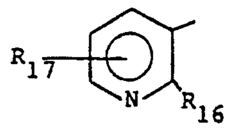, 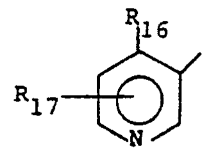,

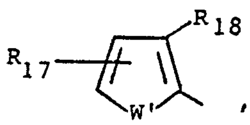, 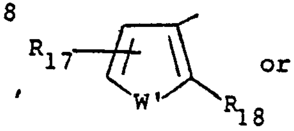 or 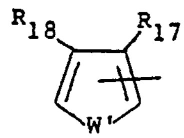

$R_7$ est H, un groupe alkyle en $C_1$—$C_4$, alcoxy en $C_1$—$C_4$, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $OCF_3$, $OCHF_2$, $OCF_2CF_2H$ ou $CH_2OR_{13}$;

$R_9$ est un groupe alkyle en $C_1$—$C_4$, $CH_2CH_2OCH_3$, $CH_2CH_2Cl$ ou $CH_2CH{=}CH_2$;

$R_{13}$ est un groupe alkyle en $C_1$—$C_4$;

n a la valeur O ou 2; et

$R_3$, $R_4$, $R_6$, $R_8$, $R_{10}$, $R_{11}$, $R_{12}$, $R_{14}$, $R_{15}$, $R_{16}$, $R_{17}$, $R_{18}$, W et W' sont tels que définis dans la revendication 1.

16. Une composition convenant pour combattre la croissance d'une végétation indésirable, comprenant une quantité efficace d'un composé herbicide et d'au moins l'un des ingrédients suivants: agent tensioactif, diluant solide ou liquide, caractérisé en ce que ledit composé herbicide comprend un composé selon l'une quelconque des revendication 1 à 15.

17. Un procédé pour combattre la croissance d'un végétation indésirable en appliquent sur la site à protéger une quantité efficace d'un composé herbicide, caractérisé en ce que ledit composé herbicide comprend un composé selon l'une quelconque des revendications 1 à 15.

18. Un procédé pour préparer un composé de la revendication 1 qui consiste

(a) à fair réagir ensemble des composés de formules

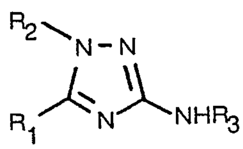 et $ZSO_2NCW$

dans lesquelles $R_1$, $R_2$, $R_3$, W et Z sont tels que définis dans la revendication 1; ou bien

(b) à alkyler un composé de formule

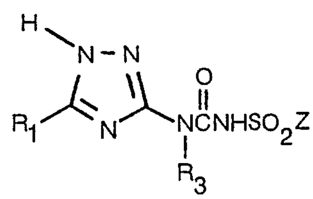

dans laquelle $R_1$, $R_3$ et Z sont tels que définis dans la revendication 1, avec un réactif de formule

$R_2X$

dans laquelle $R_2$ est tel que défini dans la revendication 1 et X est un groupe partant.